# EUROPEAN PATENT APPLICATION

(11) **EP 2 002 714 A1**
(43) Date of publication of application: **17.12.2008**
(21) Application number: 08012287.2
(22) Date of filing: 16.11.2006
(51) Int. Cl.: A01K 67/027, A61K 49/00

(54) **Novel gene disruptions, compositions and methods relating thereto**

(30) Priority: 21.11.2005 US 739105 P
(62) Divisional of application: 06849173.7
(71) Applicant: Genentech, Inc., South San Francisco CA 94080-4990 (US); Lexicon Pharmaceuticals, Inc., The Woodlands, TX 77381 (US)
(72) Inventor: Byers-Horner, Allison Anne, Dickinson, TX 77539 (US); Combs, Katherin, Spring, TX 77380 (US); Desauvage, Frederic, Forster City California 94404 (US); Fan, Liangfen, Spring Texas 77388 (US); Filvaroff, Ellen, San Francisco California 94121 (US); Irving, Bryan, San Francisco California 94107 (US); Junutula, Jagath Reddy, Fremont California 94555 (US); Massey, Erin Marie, Conroe Texas 77384 (US); McLain, Dina Rebecca, Houston Texas 77019 (US); Minze, Laurie Jeannette, Katy Texas 77493 (US); Montgomery, Charles A., Jay Oklahoma 74346 (US); Payne, Bobby Joe, The Woodlands Texas 77381 (US); Phillips, Heidi, Palo Alto California 94303 (US); Rangel, Carolina, Houston Texas 77009 (US); Shi, Zheng-zheng, The Woodlands Texas 77382 (US); Sparks, Mary Jean, Magnolia Texas 77354 (US); Stala, Joy, The Woodlands Texas 77382 (US); Townsend, Teresa Gail, Cypress Texas 77429 (US); Vogel, Peter, The Woodlands Texas 77381 (US); Willis-Sevaux, Tracy Ellen, Conroe Texas 77304 (US)
(74) Representative: Denison, Christopher Marcus

(57) **Abstract**

The present invention relates to transgenic animals, as well as compositions and methods relating to the characterization of gene function. Specifically, the present invention provides transgenic mice comprising disruptions in PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069 PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 genes. Such in vivo studies and characterizations may provide valuable identification and discovery of therapeutics and/or treatments useful in the prevention, amelioration or correction of diseases or dysfunctions associated with gene disruptions such as neurological disorders; cardiovascular, endothelial or angiogenic disorders; eye abnormalities; immunological disorders; oncological disorders; bone metabolic abnormalities or disorders; lipid metabolic disorders; or developmental abnormalities.

## Description

### FIELD OF THE INVENTION

The present invention relates to compositions, including transgenic and knockout animals and methods of using such compositions for the diagnosis and treatment of diseases or disorders.

### BACKGROUND OF THE INVENTION

Extracellular proteins play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. These secreted polypeptides or signaling molecules normally pass through the cellular secretory pathway to reach their site of action in the extracellular environment.

Secreted proteins have various industrial applications, including as pharmaceuticals, diagnostics, biosensors and bioreactors. Most protein drugs available at present, such as thrombolytic agents, interferons, interleukins, erythropoietins, colony stimulating factors, and various other cytokines, are secretory proteins. Their receptors, which are membrane proteins, also have potential as therapeutic or diagnostic agents. Efforts are being undertaken by both industry and academia to identify new, native secreted proteins. Many efforts are focused on the screening of mammalian recombinant DNA libraries to identify the coding sequences for novel secreted proteins. Examples of screening methods and techniques are described in the literature [see, for example, Klein et al., Proc. Natl. Acad. Sci. 93:7108-7113 (1996); U.S. Patent No. 5,536,637)].

Membrane-bound proteins and receptors can play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. Such membrane-bound proteins and cell receptors include, but are not limited to, cytokine receptors, receptor kinases, receptor phosphatases, receptors involved in cell-cell interactions, and cellular adhesion molecules like selectins and integrins. For instance, transduction of signals that regulate cell growth and differentiation is regulated in part by phosphorylation of various cellular proteins. Protein tyrosine kinases, enzymes that catalyze that process, can also act as growth factor receptors. Examples include fibroblast growth factor receptor and nerve growth factor receptor.

Membrane-bound proteins and receptor molecules have various industrial applications, including as pharmaceutical and diagnostic agents. Receptor immuno-adhesions, for instance, can be employed as therapeutic agents to block receptor-ligand interactions. The membrane-bound proteins can also be employed for screening of potential peptide or small molecule inhibitors of the relevant receptor/ligand interaction.

Efforts are being undertaken by both industry and academia to identify new, native receptor or membrane-bound proteins. Many efforts are focused on the screening of mammalian recombinant DNA libraries to identify the coding sequences for novel receptor or membrane-bound proteins.

Given the importance of secreted and membrane-bound proteins in biological and disease processes, in *vivo* studies and characterizations may provide valuable identification and discovery of therapeutics and/or treatments useful in the prevention, amelioration or correction of diseases or dysfunctions. In this regard, genetically engineered mice have proven to be invaluable tools for the functional dissection of biological processes relevant to human disease, including immunology, cancer, neuro-biology, cardiovascular biology, obesity and many others. Gene knockouts can be viewed as modeling the biological mechanism of drug action by presaging the activity of highly specific antagonists in *vivo.* Knockout mice have been shown to model drug activity; phenotypes of mice deficient for specific pharmaceutical target proteins can resemble the human clinical phenotype caused by the corresponding antagonist drug. Gene knockouts enable the discovery of the mechanism of action of the target, the predominant physiological role of the target, and mechanism-based side-effects that might result from inhibition of the target in mammals. Examples of this type include mice deficient in the angiotensin converting enzyme (ACE) [Esther, C.R. et al., Lab. Invest., 74:953-965 (1996)] and cyclooxygenase-1 (COX1) genes [Langenbach, R. et al., Cell, 83:483-492 (1995)]. Conversely, knocking the gene out in the mouse can have an opposite phenotypic effect to that observed in humans after administration of an agonist drug to the corresponding target. Examples include the erythropoietin knockout [Wu, C.S. et al., Cell, 83:59-67 (1996)], in which a consequence of the mutation is deficient red blood cell production, and the GABA(A)-R-β3 knockout [DeLorey, T.M., J. Neurosci., 18:8505-8514 (1998)], in which the mutant mice show hyperactivity and hyper-responsiveness. Both these phenotypes are opposite to the effects of erythropoietin and benzodiazepine administration in humans. A striking example of a target validated using mouse genetics is the ACC2 gene. Although the human ACC2 gene had been identified several years ago, interest in ACC2 as a target for drug development was stimulated only recently after analysis of ACC2 function using a knockout mouse. ACC2 mutant mice eat more than their wild-type littermates, yet burn more fat and store less fat in their adipocytes, making this enzyme a probable target for chemical antagonism in the treatment of obesity [Abu-Elheiga, L. et al., Science, 291:2613-2616 (2001)].

In the instant application, mutated gene disruptions have resulted in phenotypic observations related to various disease conditions or dysfunctions including: CNS/neurological disturbances or disorders such as anxiety; eye abnormalities and associated diseases; cardiovascular, endothelial or angiogenic disorders including atherosclerosis; abnormal metabolic disorders including diabetes and dyslipidemias associated with elevated serum triglycerides and cholesterol levels; immunological and inflammatory disorders; oncological disorders; bone metabolic abnormalities or disorders such as arthritis, osteoporosis and osteopetrosis; or a developmental disease such as embryonic lethality.

### SUMMARY OF THE INVENTION

### A. Embodiments

The invention provides an isolated nucleic acid molecule comprising a nucleotide sequence that encodes a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.

In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 polypeptide cDNA as disclosed herein, the coding sequence of a PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 polypeptide lacking the signal peptide as disclosed herein, the coding sequence of an extracellular domain of a transmembrane PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect of the invention provides an isolated nucleic acid molecule comprising a nucleotide sequence encoding a PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PR04329, PR04352, PRO5733, PR09859, PR09864, PR09904, PR09907, PRO10013, PR090948, PR028694, PRO16089, PRO19563, PRO19675, PR020084, PR021434, PR050332, PR038465 or PR0346 polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PR0218, PR0228, PR0271, PR0273, PRO295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PRO20084, PR021434, PR050332, PR038465 or PR0346 polypeptides are contemplated.

The invention also provides fragments of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide coding sequence, or the complement thereof, that may find use as, for example, hybridization probes, for encoding fragments of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide that may optionally encode a polypeptide comprising a binding site for an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR03 8465 or anti-PR0346 antibody or as antisense oligonucleotide probes. Such nucleic acid fragments usually are or are at least about 10 nucleotides in length, alternatively are or are at least about 15 nucleotides in length, alternatively are or are at least about 20 nucleotides in length, alternatively are or are at least about 30 nucleotides in length, alternatively are or are at least about 40 nucleotides in length, alternatively are or are at least about 50 nucleotides in length, alternatively are or are at least about 60 nucleotides in length, alternatively are or are at least about 70 nucleotides in length, alternatively are or are at least about 80 nucleotides in length, alternatively are or are at least about 90 nucleotides in length, alternatively are or are at least about 100 nucleotides in length, alternatively are or are at least about 110 nucleotides in length, alternatively are or are at least about 120 nucleotides in length, alternatively are or are at least about 130 nucleotides in length, alternatively are or are at least about 140 nucleotides in length, alternatively are or are at least about 150 nucleotides in length, alternatively are or are at least about 160 nucleotides in length, alternatively are or are at least about 170 nucleotides in length, alternatively are or are at least about 180 nucleotides in length, alternatively are or are at least about 190 nucleotides in length, alternatively are or are at least about 200 nucleotides in length, alternatively are or are at least about 250 nucleotides in length, alternatively are or are at least about 300 nucleotides in length, alternatively are or are at least about 350 nucleotides in length, alternatively are or are at least about 400 nucleotides in length, alternatively are or are at least about 450 nucleotides in length, alternatively are or are at least about 500 nucleotides in length, alternatively are or are at least about 600 nucleotides in length, alternatively are or are at least about 700 nucleotides in length, alternatively are or are at least about 800 nucleotides in length, alternatively are or are at least about 900 nucleotides in length and alternatively are or are at least about 1000 nucleotides in length, wherein in this context the term "about" means the referenced nucleotide sequence length plus or minus 10% of that referenced length. It is noted that novel fragments of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide-encoding nucleotide sequence may be determined in a routine manner by aligning the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide-encoding nucleotide sequence with other known nucleotide sequences using any of a number of well known sequence alignment programs and determining which PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide-encoding nucleotide sequence fragment(s) are novel. All of such PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide-encoding nucleotide sequences are contemplated herein. Also contemplated are the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide fragments encoded by these nucleotide molecule fragments, preferably those PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide fragments that comprise a binding site for an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PR01016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PRO19563, anti-PRO19675, anti-PR020084, anti-PR021434, anti-PR050332, anti-PR038465 or anti-PRO346antibody.

The invention provides isolated PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides encoded by any of the isolated nucleic acid sequences hereinabove identified.

In a certain aspect, the invention concerns an isolated PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PR0218, PR0228, PR0271, PR0273, PR0295, PR0302, PRO305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PRO21434, PR050332, PR038465 or PR0346 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PR0218, PR0228, PR0271, PR0273, PR0295, PRO302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In one aspect, the invention concerns PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant polypeptides which are or are at least about 10 amino acids in length, alternatively are or are at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 amino acids in length, or more. Optionally, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR0523 8, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PR019563, PRO19675, PR020084, PR021434, PR050332, PR038465 or PR0346 variant polypeptides will have or have no more than one conservative amino acid substitution as compared to the native PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence, alternatively will have or will have no more than 2, 3, 4, 5, 6, 7, 8, 9, or 10 conservative amino acid substitution as compared to the native PR0218, PR0228, PR0271, PR0273, PR0295, PR0302, PR0305, PR0326, PR0386, PR0655, PRO162, PR0788, PR0792, PRO940, PRO941, PRO1004, PRO1012, PRO1016, PR0474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence.

In a specific aspect, the invention provides an isolated PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1R65, PRO1879, PR03446, PR03543, PR04329, PR04352, PR05733, PR09859, PR09864, PR09904, PR09907, PR010013, PR090948, PR028694, PRO16089, PRO19563, PRO19675, PR020084, PR021434, PR050332, PR038465 or PR0346 polypeptide and recovering the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide from the cell culture.

Another aspect the invention provides an isolated PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide and recovering the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PR01865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide from the cell culture.

The invention provides agonists and antagonists of a native PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide as defined herein. In particular, the agonist or antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody or a small molecule.

The invention provides a method of identifying agonists or antagonists to a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide which comprise contacting the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide with a candidate molecule and monitoring a biological activity mediated by said PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PRO346 polypeptide. Preferably, the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide is a native PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.

The invention provides a composition of matter comprising a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, or an agonist or antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PRO28694, PRO16089, PRO19563, PRO19675, PR020084, PR021434, PR050332, PR038465 or PR0346 polypeptide as herein described, or an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PRO19563, anti-PRO19675, anti-PR020084, anti-PR021434, anti-PR050332, anti-PR038465 or anti-PR0346 antibody, in combination with a carrier. Optionally, the carrier is a pharmaceutically acceptable carrier.

The invention provides the use of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, or an agonist or antagonist thereof as hereinbefore described, or an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PR019563, anti-PR019675, anti-PR020084, anti-PR021434, anti-PR050332, anti-PR038465 or anti-PR0346 antibody, for the preparation of a medicament useful in the treatment of a condition which is responsive to the anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PRO941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PR0346 antibody.

The invention provides vectors comprising DNA encoding any of the herein described polypeptides. Host cell comprising any such vector are also provided. By way of example, the host cells may be CHO cells, E. *coli,* or yeast. A process for producing any of the herein described polypeptides is further provided and comprises culturing host cells under conditions suitable for expression of the desired polypeptide and recovering the desired polypeptide from the cell culture.

The invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

The invention provides an antibody which binds, preferably specifically, to any of the above or below described polypeptides. Optionally, the antibody is a monoclonal antibody, humanized antibody, antibody fragment or single-chain antibody.

The invention provides oligonucleotide probes which may be useful for isolating genomic and cDNA nucleotide sequences, measuring or detecting expression of an associated gene or as antisense probes, wherein those probes may be derived from any of the above or below described nucleotide sequences. Preferred probe lengths are described above.

The invention also provides a method of identifying a phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PR01016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
(a) providing a non-human transgenic animal whose genome comprises a disruption of the gene which encodes for a PR0218, PR0228, PR0271, PR0273, PR0295, PR0302, PR0305, PR0326, PR0386, PR0655, PR0162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PR010013, PR090948, PRO28694, PRO16089, PRO19563, PRO19675, PR020084, PR021434, PR050332, PR038465 or PR0346 polypeptide;
(b) measuring a physiological characteristic of the non-human transgenic animal; and
(c) comparing the measured physiological characteristic with that of a gender matched wild-type animal, wherein the physiological characteristic of the non-human transgenic animal that differs from the physiological characteristic of the wild-type animal is identified as a phenotype resulting from the gene disruption in the non-human transgenic animal. In one aspect, the non-human transgenic animal is a mammal. In another aspect, the mammal is a rodent. In still another aspect, the mammal is a rat or a mouse. In one aspect, the non-human transgenic animal is heterozygous for the disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In another aspect, the phenotype exhibited by the non-human transgenic animal as compared with gender matched wild-type littermates is at least one of the following: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.

In still another aspect, the developmental abnormality comprises embryonic lethality or reduced viability.

In still yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.

In still another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barr6 syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In still another aspect, the bone metabolic abnormality or disorder is arthritis, osteoporosis, osteopenia or osteopetrosis.

In another aspect, the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits of optic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages ofCD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages of CD4 cells and increased percentages of B cells in tissues; increase in percentages of B cells in Peyer's patches;; decreased germinal center, isotype-switched B cells in Peyer's patches (CD38low;IgM negative); decreased CD23 intensity in spleen; increased mean percentages of B220 Med/CD23-cells and B220+/CD 11b-Low/CD23- cells in peritoneal lavage; increased mean percentages ofB cells in peripheral blood; decreased CD4 and CD8 T cells and increased B cells; increase in peritoneal B cells; reduction in CD 11b-Hi cells in peritoneal cavity; decreased mean CD4 to CD8 ratio in spleen; decreased CD8 cells; decreased mean percentages of B220+/CD23+ cells and B220+/CD11bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG 1; increased mean serum IgG2a; increased mean serum IgG2b; decreased mean serum IgG1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.

The invention also provides an isolated cell derived from a non-human transgenic animal whose genome comprises a disruption of the gene which encodes for a PR0218, PRO228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide. In one aspect, the isolated cell is a murine cell. In yet another aspect, the murine cell is an embryonic stem cell. In still another aspect, the isolated cell is derived from a non-human transgenic animal which exhibits at least one ofthe following phenotypes compared with gender matched wild-type littermates: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality. The invention also provides a method of identifying an agent that modulates a phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide, the method comprising:
(a) providing a non-human transgenic animal whose genome comprises a disruption of the gene which encodes for the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PRO4352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a phenotype resulting from the gene disruption in the non-human transgenic animal;
(d) administering a test agent to the non-human transgenic animal of (a); and
(e) determining whether the test agent modulates the identified phenotype associated with gene disruption in the non-human transgenic animal.

In one aspect, the phenotype associated with the gene disruption comprises a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In yet another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism, or Conradi syndrome.

In still another aspect, the developmental abnormality comprises embryonic lethality or reduced viability.

In still another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

In another aspect, the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits of optic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages of CD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages of CD4 cells and increased percentages of B cells in tissues; increase in percentages of B cells in Peyer's patches;; decreased germinal center, isotype-switched B cells in Peyer's patches (CD38low;IgM negative); decreased CD23 intensity in spleen; increased mean percentages of B220 Med/CD23- cells and B220+/CD11b-Low/CD23- cells in peritoneal lavage; increased mean percentages ofB cells in peripheral blood; decreased CD4 and CD8 T cells and increased B cells; increase in peritoneal B cells; reduction in CD11b-Hi cells in peritoneal cavity; decreased mean CD4 to CD8 ratio in spleen; decreased CD8 cells; decreased mean percentages of B220+/CD23+cells and B220+/CD11bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG 1; increased mean serum IgG2a; increased mean serum IgG2b; decreased mean serum IgGI response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.

The invention also provides an agent which modulates the phenotype associated with gene disruption. In one aspect, the agent is an agonist or antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PRO3446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PR01012, anti-PR01016, anti-PR0474, anti-PR05238, anti-PRO1069, anti-PRO1111, anti-PRO1113, anti-PRO1130, anti-PR01195, anti-PR01271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PRO28694, anti-PRO16089, anti-PRO19563, anti-PRO19675, anti-PRO20084, anti-PR021434, anti-PR050332, anti-PR038465 or anti-PR0346 antibody. In still another aspect, the antagonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PR01130, anti-PRO1195, anti-PR01271, anti-PR01865, anti-PR01879, anti-PR03446, anti-PR03543, anti-PR04329, anti-PR04352, anti-PR05733, anti-PRO9859, anti-PR09864, anti-PR09904, anti-PR09907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PR0346 antibody.

The invention also provides a method of identifying an agent that modulates a physiological characteristic associated with a disruption of the gene which encodes for a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
(a) providing a non-human transgenic animal whose genome comprises a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
(b) measuring a physiological characteristic exhibited by the non-human transgenic animal of (a);
(c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
(d) administering a test agent to the non-human transgenic animal of (a); and
(e) determining whether the physiological characteristic associated with gene disruption is modulated.

In one aspect, the non-human transgenic animal exhibits at least one of the following physiological characteristics compared with gender matched wild-type littermates:

In another aspect, the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits of optic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage ofCD4 spleen thymocytes; decreased percentages of CD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages of CD4 cells and increased percentages of B cells in tissues; increase in percentages of B cells in Peyer's patches;; decreased germinal center, isotype-switched B cells in Peyer's patches (CD381ow;IgM negative); decreased CD23 intensity in spleen; increased mean percentages of B220 Med/CD23- cells and B220+/CD 11b-Low/CD23- cells in peritoneal lavage; increased mean percentages of B cells in peripheral blood; decreased CD4 and CD8 T cells and increased B cells; increase in peritoneal B cells; reduction in CD 11 b-Hi cells in peritoneal cavity; decreased mean CD4 to CD8 ratio in spleen; decreased CD8 cells; decreased mean percentages of B220+/CD23+ cells and B220+/CD 11bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG1; increased mean serum IgG2a; increased mean serum IgG2b; decreased mean serum IgG1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.

The invention also provides an agent that modulates a physiological characteristic which is associated with gene disruption. In one aspect, the agent is an agonist or antagonist of the phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PRO941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agent is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PR01016, anti-PR0474, anti-PR05238, anti-PRO1069, anti-PRO1111, anti-PRO1113, anti-PRO1130, anti-PRO1195, anti-PR01271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody. In still another aspect, the antagonist agent is an anti-PR0218, anti-PR0228, anti-PRO271, anti-PR0273, anti-PR0295, anti-PR0302, anti-PR0305, anti-PR0326, anti-PR0386, anti-PRO655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PR01113, anti-PRO1130, anti-PRO1195, anti-PR01271, anti-PR01865, anti-PR01879, anti-PR03446, anti-PR03543, anti-PR04329, anti-PR04352, anti-PR05733, anti-PR09859, anti-PR09864, anti-PR09904, anti-PRO9907, anti-PRO10013, anti-PR090948, anti-PR028694, anti-PRO16089, anti-PR019563, anti-PR019675, anti-PR020084, anti-PR021434, anti-PR050332, anti-PR038465 or anti-PR0346 antibody.

The invention also provides a method of identifying an agent which modulates a behavior associated with a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
(a) providing a non-human transgenic animal whose genome comprises a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
(b) observing the behavior exhibited by the non-human transgenic animal of (a);
(c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
(d) administering a test agent to the non-human transgenic animal of (a); and
(e) determining whether the agent modulates the behavior associated with gene disruption.

In one aspect, the observed behavior is an increased anxiety-like response during open field activity testing. In yet another aspect, the observed behavior is a decreased anxiety-like response during open field activity testing. In yet another aspect, the observed behavior is an abnormal circadian rhythm during home-cage activity testing. In yet another aspect, the observed behavior is an enhanced motor coordination during inverted screen testing. In yet another aspect, the observed behavior is impaired motor coordination during inverted screen testing. In yet another aspect, the observed behavior includes depression, generalized anxiety disorders, attention deficit disorder, sleep disorder, hyperactivity disorder, obsessive compulsive disorder, schizophrenia, cognitive disorders, hyperalgesia and sensory disorders. Such disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

The invention also provides an agent that modulates a behavior which is associated with gene disruption. In one aspect, the agent is an agonist or antagonist of the phenotype associated with a disruption of a gene which encodes for a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide. In yet another aspect, the agent is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PRO941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PRO19563, anti-PRO19675, anti-PR020084, anti-PR021434, anti-PR050332, anti-PR038465 or anti-PR0346 antibody. In still another aspect, the antagonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PR01130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PR010013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.

The invention also provides a method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide, the method comprising:
(a) providing a non-human transgenic animal whose genome comprises a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
(b) administering a test agent to said non-human transgenic animal; and
(c) determining whether the test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality associated with the gene disruption in the non-human transgenic animal.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities the retinal abnormalities are consistent with retinal dysplasia, various retinopathies, including retinopathy ofprematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy o the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism, or Conradi syndrome.

In still another aspect, the developmental abnormality comprises embryonic lethality or reduced viability.

In yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still yet another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

In another aspect, the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits of optic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages ofCD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages of CD4 cells and increased percentages of B cells in tissues; increase in percentages of B cells in Peyer's patches;; decreased germinal center, isotype-switched B cells in Peyer's patches (CD381ow;IgM negative); decreased CD23 intensity in spleen; increased mean percentages of B220 Med/CD23- cells and B220+/CD11b-Low/CD23- cells in peritoneal lavage; increased mean percentages of B cells in peripheral blood; decreased CD4 and CD8 T cells and increased B cells; increase in peritoneal B cells; reduction in CD 11 b-Hi cells in peritoneal cavity; decreased mean CD4 to CD8 ratio in spleen; decreased CD8 cells; decreased mean percentages of B220+/CD23+ cells and B220+/CD1 1bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG1; increased mean serum IgG2a; increased mean serum IgG2b; decreased mean serum IgG 1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasiaof sebaceous glands and multi focal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.

The invention also provides an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality which is associated with gene disruption. In one aspect, the agent is an agonist or antagonist of the phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agent is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agonist agent is an anti-PR0218, anti-PR0228, anti-PR0271, anti-PR0273, anti-PR0295, anti-PRO302, anti-PR0305, anti-PR0326, anti-PR0386, anti-PR0655, anti-PRO162, anti-PR0788, anti-PR0792, anti-PRO940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PR0346 antibody. In still another aspect, the antagonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibody.

The invention also provides a therapeutic agent for the treatment of a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

The invention also provides a method of identifying an agent that modulates the expression of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
(a) contacting a test agent with a host cell expressing a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide; and
(b) determining whether the test agent modulates the expression of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide by the host cell.

The invention also provides an agent that modulates the expression of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In one aspect, the agent is an agonist or antagonist of the phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agent is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PRO38465 or PR0346 polypeptide. In yet another aspect, the agonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PRO20084, anti-PR021434, anti-PR050332, anti-PR038465 or anti-PR0346 antibody. In still another aspect, the antagonist agent is an anti-PR0218, anti-PR0228, anti-PRO271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PR01016, anti-PR0474, anti-PR05238, anti-PRO1069, anti-PRO1111, anti-PRO1113, anti-PRO1130, anti-PR01195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PRO28694, anti-PRO16089, anti-PRO19563, anti-PRO19675, anti-PRO20084, anti-PRO21434, anti-PR050332, anti-PR038465 or anti-PR0346 antibody.

The invention also provides a method of evaluating a therapeutic agent capable of affecting a condition associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
(a) providing a non-human transgenic animal whose genome comprises a disruption of the gene which encodes for the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a condition resulting from the gene disruption in the non-human transgenic animal;
(d) administering a test agent to the non-human transgenic animal of (a); and
(e) evaluating the effects of the test agent on the identified condition associated with gene disruption in the non-human transgenic animal.

In one aspect, the condition is a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

The invention also provides a therapeutic agent which is capable of affecting a condition associated with gene disruption. In one aspect, the agent is an agonist or antagonist of the phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PRO20084, PR021434, PR050332, PR038465 or PR0346 polypeptide. In yet another aspect, the agent is an agonist or antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody. In still another aspect, the antagonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PRO9907, anti-PRO10013, anti-PRO90948, anti-PR028694, anti-PRO16089, anti-PRO19563, anti-PRO19675, anti-PR020084, anti-PR021434, anti-PR050332, anti-PR038465 or anti-PR0346 antibody.

The invention also provides a pharmaceutical composition comprising a therapeutic agent capable of affecting the condition associated with gene disruption.

The invention also provides a method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PR0218, PR0228, PR0271, PR0273, PR0295, PR0302, PR0305, PR0326, PR0386, PR0655, PRO162, PR0788, PR0792, PR0940, PRO941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject in need of such treatment whom may already have the disorder, or may be prone to have the disorder or may be in whom the disorder is to be prevented, a therapeutically effective amount of a therapeutic agent, or agonists or antagonists thereof, , thereby effectively treating or preventing or ameliorating said disorder or disease.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.

In still another aspect, the developmental abnormality comprises embryonic lethality or reduced viability.

In yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.,* hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still yet another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

In another aspect the therapeutic agent is an agonist or antagonist of the phenotype associated with a disruption of a gene which encodes for a PR0218, PR0228, PR0271, PR0273, PR0295, PR0302, PR0305, PR0326, PR0386, PRO655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agent is an agonist or antagonist of a PRO218, PR0228, PRO271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PRO9907, anti-PRO10013, anti-PR090948, anti-PR028694, anti-PRO16089, anti-PR019563, anti-PR019675, anti-PR020084, anti-PR021434, anti-PR050332, anti-PR038465 or anti-PR0346 antibody. In still another aspect, the antagonist agent is an anti-PR0218, anti-PR0228, anti-PR0271, anti-PR0273, anti-PR0295, anti-PRO302, anti-PR0305, anti-PR0326, anti-PR0386, anti-PR0655, anti-PRO162, anti-PR0788, anti-PR0792, anti-PRO940, anti-PR0941, anti-PRO1004, anti-PRO1012, anti-PRO1016, anti-PR0474, anti-PRO5238, anti-PRO1069, anti-PRO1111, anti-PRO1113, anti-PRO1130, anti-PRO1195, anti-PR01271, anti-PRO1865, anti-PR01879, anti-PR03446, anti-PR03543, anti-PR04329, anti-PR04352, anti-PR05733, anti-PR09859, anti-PR09864, anti-PRO9904, anti-PR09907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PR0346 antibody.

The invention also provides a method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PRO9864, PR09904, PR09907, PRO10013, PR090948, PR028694, PRO16089, PRO19563, PRO19675, PR020084, PR021434, PR050332, PR038465 or PR0346 polypeptide, the method comprising:
(a) providing a non-human transgenic animal cell culture, each cell of said culture comprising a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
(b) administering a test agent to said cell culture; and
(c) determining whether the test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in said culture. In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing.

In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.

In still another aspect, the developmental abnormality comprises embryonic lethality or reduced viability.

In yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still yet another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft-versus-host disease.

In yet another aspect, the bone metabolic abnormality or disorder is arthritis, osteoporosis, osteopenia or osteopetrosis.

The invention also provides an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality which is associated with gene disruption in said culture. In one aspect, the agent is an agonist or antagonist of the phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agent is an agonist or antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In yet another aspect, the agonist agent is an anti-PR0218, anti-PR0228, anti-PR0271, anti-PR0273, anti-PR0295, anti-PRO302, anti-PR0305, anti-PR0326, anti-PRO386, anti-PR0655, anti-PRO162, anti-PR0788, anti-PR0792, anti-PRO940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PR01879, anti-PRO3446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PR019563, anti-PRO19675, anti-PR020084, anti-PR021434, anti-PR050332, anti-PR038465 or anti-PR0346 antibody. In still another aspect, the antagonist agent is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PR019675, anti-PR020084, anti-PR021434, anti-PR050332, anti-PR038465 or anti-PR0346 antibody.

The invention also provides a method of modulating a phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PRO5733, PR09859, PR09864, PR09904, PR09907, PRO10013, PR090948, PR028694, PR016089, PRO19563, PRO19675, PR020084, PR021434, PR050332, PR038465 or PR0346 polypeptide, the method comprising administering to a subject whom may already have the phenotype, or may be prone to have the phenotype or may be in whom the phenotype is to be prevented, an effective amount of an agent identified as modulating said phenotype, or agonists or antagonists thereof, thereby effectively modulating the phenotype.

The invention also provides a method of modulating a physiological characteristic associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346polypeptide, the method comprising administering to a subject whom may already exhibit the physiological characteristic, or may be prone to exhibit the physiological characteristic or may be in whom the physiological characteristic is to be prevented, an effective amount of an agent identified as modulating said physiological characteristic, or agonists or antagonists thereof, thereby effectively modulating the physiological characteristic.

The invention also provides a method of modulating a behavior associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PRO346 polypeptide, the method comprising administering to a subject whom may already exhibit the behavior, or may be prone to exhibit the behavior or may be in whom the exhibited behavior is to be prevented, an effective amount of an agent identified as modulating said behavior, or agonists or antagonists thereof, thereby effectively modulating the behavior.

The invention also provides a method of modulating the expression of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a host cell expressing said PR0218, PR0228, PR0271, PR0273, PR0295, PR0302, PR0305, PR0326, PR03 86, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, an effective amount of an agent identified as modulating said expression, or agonists or antagonists thereof, thereby effectively modulating the expression of said polypeptide.

The invention also provides a method of modulating a condition associated with a disruption of a gene which encodes for a PR0218, PR0228, PR0271, PR0273, PR0295, PR0302, PR0305, PR0326, PR0386, PRO655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject whom may have the condition, or may be prone to have the condition or may be in whom the condition is to be prevented, a therapeutically effective amount of a therapeutic agent identified as modulating said condition, or agonists or antagonists thereof, thereby effectively modulating the condition.

The invention also provides a method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a non-human transgenic animal cell culture, each cell of said culture comprising a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, an effective amount of an agent identified as treating or preventing or ameliorating said disorder, or agonists or antagonists thereof, thereby effectively treating or preventing or ameliorating said disorder.

### B. Further Embodiments

In yet further embodiments, the invention is directed to the following set of potential claims for this application:
1. A method of identifying a phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) measuring a physiological characteristic of the non-human transgenic animal; and
   (c) comparing the measured physiological characteristic with that of a gender matched wild-type animal, wherein the physiological characteristic of the non-human transgenic animal that differs from the physiological characteristic of the wild-type animal is identified as a phenotype resulting from the gene disruption in the non-human transgenic animal.
2. The method of Claim 1, wherein the non-human transgenic animal is heterozygous for the disruption of a gene which encodes for a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
3. The method of Claim 1, wherein the phenotype exhibited by the non-human transgenic animal as compared with gender matched wild-type littermates is at least one of the following: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
4. The method of Claim 3, wherein the neurological disorder is an increased anxiety-like response during open field activity testing.
5. The method of Claim 3, wherein the neurological disorder is a decreased anxiety-like response during open field activity testing.
6. The method of Claim 3, wherein the neurological disorder is an abnormal circadian rhythm during home-cage activity testing.
7. The method of Claim 3, wherein the neurological disorder is an enhanced motor coordination during inverted screen testing.
8. The method of Claim 3, wherein the neurological disorder is an impaired motor coordination during inverted screen testing.
9. The method of Claim 3, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
10. The method of Claim 3, wherein the eye abnormality is a retinal abnormality.
11. The method of Claim 3, wherein the eye abnormality is consistent with vision problems or blindness.
12. The method of Claim 10, wherein the retinal abnormality is consistent with retinitis pigmentosa.
13. The method of Claim 10, wherein the retinal abnormality is characterized by retinal degeneration or retinal dysplasia.
14. The method of Claim 10, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
15. The method of Claim 3, wherein the eye abnormality is a cataract.
16. The method of Claim 15, wherein the cataract is consistent with systemic diseases such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
17. The method of Claim 3, wherein the developmental abnormality comprises embryonic lethality or reduced viability.
18. The method of Claim 3, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
19. The method of Claim 3, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
20. The method of Claim 3, wherein the bone metabolic abnormality or disorder is arthritis, osteoporosis or osteopetrosis.
21. The method of Claim 1, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits ofoptic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages of CD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages of CD4 cells and increased percentages ofB cells in tissues; increase in percentages of B cells in Peyer's patches;; decreased germinal center, isotype-switched B cells in Peyer's patches (CD38low;IgM negative); decreased CD23 intensity in spleen; increased mean percentages of B220 Med/CD23- cells and B220+/CD11b-Low/CD23- cells in peritoneal lavage; increased mean percentages ofB cells in peripheral blood; decreased CD4 and CD8 T cells and increased B cells; increase in peritoneal B cells; reduction in CD11b-Hi cells in peritoneal cavity; decreased mean CD4 to CD8 ratio in spleen; decreased CD8 cells; decreased mean percentages of B220+/CD23+ cells and B220+/CD 11bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG1; increased mean serum IgG2a; increased mean serum IgG2b; decreased mean serum IgG1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.
22. An isolated cell derived from a non-human transgenic animal whose genome comprises a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
23. The isolated cell of Claim 22 which is a murine cell.
24. The isolated cell of Claim 23, wherein the murine cell is an embryonic stem cell.
25. The isolated cell of Claim 22, wherein the non-human transgenic animal exhibits at least one of the following phenotypes compared with gender matched wild-type littermates: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
26. A method of identifying an agent that modulates a phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of the gene which encodes for the PR0218, PR0228, PR0271, PR0273, PR0295, PR0302, PR0305, PR0326, PR0386, PR0655, PRO162, PR0788, PR0792, PRO940, PRO941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) measuring a physiological characteristic of the non-human transgenic animal of (a);
   (c) comparing the measured physiological characteristic of (b) with that of a gender matched wild-type animal, wherein the physiological characteristic of the non-human transgenic animal that differs from the physiological characteristic of the wild-type animal is identified as a phenotype resulting from the gene disruption in the non-human transgenic animal;
   (d) administering a test agent to the non-human transgenic animal of (a); and
   (e) determining whether the test agent modulates the identified phenotype associated with gene disruption in the non-human transgenic animal.
27. The method of Claim 26, wherein the phenotype associated with the gene disruption comprises a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
28. The method of Claim 27, wherein the neurological disorder is an increased anxiety-like response during open field activity testing.
29. The method of Claim 27, wherein the neurological disorder is a decreased anxiety-like response during open field activity testing.
30. The method of Claim 27, wherein the neurological disorder is an abnormal circadian rhythm during home-cage activity testing.
31. The method of Claim 27, wherein the neurological disorder is an enhanced motor coordination during inverted screen testing.
32. The method of Claim 27, wherein the neurological disorder is an impaired motor coordination during inverted screen testing.
33. The method of Claim 27, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
34. The method of Claim 27, wherein the eye abnormality is a retinal abnormality.
35. The method of Claim 27, wherein the eye abnormality is consistent with vision problems or blindness.
36. The method of Claim 34, wherein the retinal abnormality is consistent with retinitis pigmentosa.
37. The method of Claim 34, wherein the retinal abnormality is characterized by retinal degeneration or retinal dysplasia.
38. The method of Claim 34, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
39. The method of Claim 27, wherein the eye abnormality is a cataract.
40. The method of Claim 39, wherein the cataract is consistent with systemic diseases such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
41. The method of Claim 27, wherein the developmental abnormality comprises embryonic lethality or reduced viability.
42. The method of Claim 27, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
43. The method of Claim 27, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sj6gren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation-associated diseases including graft rejection and graft -versus-host disease.
44. The method of Claim 27, wherein said bone metabolic abnormality or disorder is arthritis, osteoporosis or osteopetrosis.
45. The method of Claim 26, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits ofoptic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages of CD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages ofB cells in blood; decreased percentages of CD4 cells and increased percentages of B cells in tissues; increase in percentages of B cells in Peyer's patches;; decreased germinal center, isotype-switched B cells in Peyer's patches (CD38low;IgM negative); decreased CD23 intensity in spleen; increased mean percentages of B220 Med/CD23- cells and B220+/CD11b-Low/CD23- cells in peritoneal lavage; increased mean percentages of B cells in peripheral blood; decreased CD4 and CD8 T cells and increased B cells; increase in peritoneal B cells; reduction in CD11b-Hi cells in peritoneal cavity; decreased mean CD4 to CD8 ratio in spleen; decreased CD8 cells; decreased mean percentages of B220+/CD23+ cells and B220+/CD11bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG1; increased mean serum IgG2a; increased mean serum IgG2b; decreased mean serum IgG1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.
46. An agent identified by the method of Claim 26.
47. The agent of Claim 46 which is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide.
48. The agent of Claim 47, wherein the agonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibody.
49. The agent of Claim 47, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
50. A method of identifying an agent that modulates a physiological characteristic associated with a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of the gene which encodes for a PR0218, PR0228, PR0271, PR0273, PR0295, PR0302, PR0305, PR0326, PR0386, PR0655, PRO162, PR0788, PR0792, PR0940, PRO941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide;
   (b) measuring a physiological characteristic exhibited by the non-human transgenic animal of (a);
   (c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
   (d) administering a test agent to the non-human transgenic animal of (a); and
   (e) determining whether the physiological characteristic associated with gene disruption is modulated.
51. The method of Claim 50, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits of optic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages of CD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages of CD4 cells and increased percentages ofB cells in tissues; increase in percentages of B cells in Peyer's patches;; decreased germinal center, isotype-switched B cells in Peyer's patches (CD38low;IgM negative); decreased CD23 intensity in spleen; increased mean percentages of B220 Med/CD23- cells and B220+/CD11b-Low/CD23- cells in peritoneal lavage; increased mean percentages of B cells in peripheral blood; decreased CD4 and CD8 T cells and increased B cells; increase in peritoneal B cells; reduction in CD11b-Hi cells in peritoneal cavity; decreased mean CD4 to CD8 ratio in spleen; decreased CD8 cells; decreased mean percentages ofB220+/CD23+ cells and B220+/CD 11 bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG 1; increased mean serum IgG2a; increased mean serum IgG2b; decreased mean serum IgG1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.
52. An agent identified by the method of Claim 50.
53. The agent of Claim 52 which is an agonist or antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
54. The agent of Claim 53, wherein the agonist is an anti-PR0218, anti-PR0228, anti-PR0271, anti-PR0273,
   anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PR016089, anti-PRO19563, anti-PRO19675, anti-PR020084, anti-PR021434, anti-PR050332, anti-PR038465 or anti-PR0346 antibody.
55. The agent of Claim 53, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
56. A method of identifying an agent which modulates a behavior associated with a disruption of the gene which encodes for a PR0218, PR0228, PR0271, PR0273, PR0295, PR0302, PR0305, PR0326, PR0386, PRO655, PRO162, PR0788, PRO792, PRO940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR0503 3 2, PR03 8465 or PR0346 polypeptide, the method comprising:
   (a) providing a non-human transgenic animal whose genome comprises a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) observing the behavior exhibited by the non-human transgenic animal of (a);
   (c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
   (d) administering a test agent to the non-human transgenic animal of (a); and
   (e) determining whether the agent modulates the behavior associated with gene disruption.
57. The method of Claim 56, wherein the behavior is an increased anxiety-like response during open field activity testing.
58. The method of Claim 56, wherein the behavior is a decreased anxiety-like response during open field activity testing.
59. The method of Claim 56, wherein the behavior is an abnormal circadian rhythm during home-cage activity testing.
60. The method of Claim 56, wherein the behavior is an enhanced motor coordination during inverted screen testing.
61. The method of Claim 56, wherein the behavior is an impaired motor coordination during inverted screen testing.
62. The method of Claim 56, wherein the behavior is depression, generalized anxiety disorders, attention deficit disorder, sleep disorder, hyperactivity disorder, obsessive compulsive disorder, schizophrenia, cognitive disorders, hyperalgesia or sensory disorders.
63. An agent identified by the method of Claim 56.
64. The agent of Claim 63 which is an agonist or antagonist of a PR0218, PR0228, PR0271, PR0273, PR0295, PR0302, PR0305, PR0326, PR0386, PR0655, PRO162, PR0788, PRO792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
65. The agent of Claim 64, wherein the agonist is an anti-PR0218, anti-PR0228, anti-PR0271, anti-PR0273,
   anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
66. The agent of Claim 64, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PR019675, anti-PR020084, anti-PR021434, anti-PR050332, anti-PR038465 or anti-PR0346 antibody.
67. A method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of the gene which encodes for a PRO218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PRO1069, PRO1111, PRO1113, PRO1130, PRO1195, PR01271, PR01865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide;
   (b) administering a test agent to said non-human transgenic animal; and
   (c) determining whether said test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in the non-human transgenic animal.
68. The method of Claim 67, wherein the neurological disorder is an increased anxiety-like response during open field activity testing.
69. The method of Claim 67, wherein the neurological disorder is a decreased anxiety-like response during open field activity testing.
70. The method of Claim 67, wherein the neurological disorder is an abnormal circadian rhythm during home-cage activity testing.
71. The method of Claim 67, wherein the neurological disorder is an enhanced motor coordination during inverted screen testing.
72. The method of Claim 67, wherein the neurological disorder is an impaired motor coordination during inverted screen testing.
73. The method of Claim 73, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
74. The method of Claim 67, wherein the eye abnormality is a retinal abnormality.
75. The method of Claim 67, wherein the eye abnormality is consistent with vision problems or blindness.
76. The method of Claim 74, wherein the retinal abnormality is consistent with retinitis pigmentosa.
77. The method of Claim 74, wherein the retinal abnormality is characterized by retinal degeneration or retinal dysplasia.
78. The method of Claim 74, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
79. The method of Claim 67, wherein the eye abnormality is a cataract.
80. The method of Claim 79, wherein the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
81. The method of Claim 67, wherein the developmental abnormality comprises embryonic lethality or reduced viability.
82. The method of Claim 67, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.,* hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
83. The method of Claim 67, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
84. The method of Claim 67, wherein said bone metabolic abnormality or disorder is arthritis, osteoporosis or osteopetrosis.
85. The method of Claim 67, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits ofoptic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages ofCD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages ofB cells in blood; decreased percentages ofCD4 cells and increased percentages of B cells in tissues; increase in percentages of B cells in Peyer's patches;; decreased germinal center, isotype-switched B cells in Peyer's patches (CD3 8low;IgM negative); decreased CD23 intensity in spleen; increased mean percentages of B220 Med/CD23- cells and B220+/CD 11b-Low/CD23- cells in peritoneal lavage; increased mean percentages of B cells in peripheral blood; decreased CD4 and CD8 T cells and increased B cells; increase in peritoneal B cells; reduction in CD11b-Hi cells in peritoneal cavity; decreased mean CD4 to CD8 ratio in spleen; decreased CD8 cells; decreased mean percentages of B220+/CD23+ cells and B220+/CD11bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG1; increased mean serum IgG2a; increased mean serum IgG2b; decreased mean serum IgG 1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.
86. An agent identified by the method of Claim 67.
87. The agent of Claim 86 which is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
88. The agent of Claim 87, wherein the agonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibody.
89. The agent of Claim 87, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PRO19563, anti-PRO19675, anti-PRO20084, anti-PRO21434, anti-PR050332, anti-PR038465 or anti-PR0346 antibody.
90. A therapeutic agent identified by the method of Claim 67.
91. A method of identifying an agent that modulates the expression of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) contacting a test agent with a host cell expressing a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide; and
   (b) determining whether the test agent modulates the expression of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide by the host cell.
92. An agent identified by the method of Claim 91.
93. The agent of Claim 92 which is an agonist or antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
94. The agent of Claim 93, wherein the agonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibody.
95. The agent of Claim 93, wherein the antagonist is an anti-PR0218, anti-PR0228, anti-PR0271, anti-PR0273, anti-PR0295, anti-PR0302, anti-PR0305, anti-PR0326, anti-PRO386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
96. A method of evaluating a therapeutic agent capable of affecting a condition associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of the gene which encodes for the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide;
   (b) measuring a physiological characteristic of the non-human transgenic animal of (a);
   (c) comparing the measured physiological characteristic of (b) with that of a gender matched wild-type animal, wherein the physiological characteristic of the non-human transgenic animal that differs from the physiological characteristic of the wild-type animal is identified as a condition resulting from the gene disruption in the non-human transgenic animal;
   (d) administering a test agent to the non-human transgenic animal of (a); and
   (e) evaluating the effects of the test agent on the identified condition associated with gene disruption in the non-human transgenic animal.
97. The method of Claim 96, wherein the condition is a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
98. A therapeutic agent identified by the method of Claim 96.
99. The therapeutic agent of Claim 98 which is an agonist or antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
100. The therapeutic agent of Claim 99, wherein the agonist is an anti-PR0218, anti-PR0228, anti-PR0271, anti-PR0273, anti-PR0295, anti-PR0302, anti-PRO305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PR01069, anti-PRO1111, anti-PRO1113, anti-PRO1130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PR010013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
101. The therapeutic agent of Claim 99, wherein the antagonist is an anti-PR0218, anti-PR0228, anti-PR0271,
   anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
102. A pharmaceutical composition comprising the therapeutic agent of Claim 98.
103. A method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PRO20084, PRO21434, PR050332, PR038465 or PR0346 polypeptide, the method comprising administering to a subject in need of such treatment whom may already have the disorder, or may be prone to have the disorder or may be in whom the disorder is to be prevented, a therapeutically effective amount of the therapeutic agent of Claim 94, or agonists or antagonists thereof, thereby effectively treating or preventing or ameliorating said disorder.
104. The method of Claim 103, wherein the neurological disorder is an increased anxiety-like response during open field activity testing.
105. The method of Claim 103, wherein the neurological disorder is a decreased anxiety-like response during open field activity testing.
106. The method of Claim 103, wherein the neurological disorder is an abnormal circadian rhythm during home-cage activity testing.
107. The method of Claim 103, wherein the neurological disorder is an enhanced motor coordination during inverted screen testing.
108. The method of Claim 103, wherein the neurological disorder is an impaired motor coordination during inverted screen testing.
109. The method of Claim 103, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
110. The method of Claim 103, wherein the eye abnormality is a retinal abnormality.
111. The method of Claim 103, wherein the eye abnormality is consistent with vision problems or blindness.
112. The method of Claim 110, wherein the retinal abnormality is consistent with retinitis pigmentosa.
113. The method of Claim 110, wherein the retinal abnormality is characterized by retinal degeneration or retinal dysplasia.
114. The method of Claim 110, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
115. The method of Claim 103, wherein the eye abnormality is a cataract.
116. The method of Claim 115, wherein the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
117. The method of Claim 103, wherein the developmental abnormality comprises embryonic lethality or reduced viability.
118. The method of Claim 103, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.,* hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
119. The method of Claim 103, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
120. The method of Claim 103, wherein said bone metabolic abnormality or disorder is arthritis, osteoporosis or osteopetrosis.
121. A method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal cell culture, each cell of said culture comprising a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) administering a test agent to said cell culture; and
   (c) determining whether said test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in said cell culture.
122. The method of Claim 121, wherein the neurological disorder is an increased anxiety-like response during open field activity testing.
123. The method of Claim 121, wherein the neurological disorder is a decreased anxiety-like response during open field activity testing.
124. The method of Claim 121, wherein the neurological disorder is an abnormal circadian rhythm during home-cage activity testing.
125. The method of Claim 121, wherein the neurological disorder is an enhanced motor coordination during inverted screen testing.
126. The method of Claim 121, wherein the neurological disorder is an impaired motor coordination during inverted screen testing.
127. The method of Claim 121, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
128. The method of Claim 121, wherein the eye abnormality is a retinal abnormality.
129. The method of Claim 121, wherein the eye abnormality is consistent with vision problems or blindness.
130. The method of Claim 128, wherein the retinal abnormality is consistent with retinitis pigmentosa.
131. The method of Claim 128, wherein the retinal abnormality is characterized by retinal degeneration or retinal dysplasia.
132. The method of Claim 128, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
133. The method of Claim 121, wherein the eye abnormality is a cataract.
134. The method of Claim 133, wherein the cataract is a systemic disease such as human Down's syndrome, Hallerman-Streiffsyndrome, Lowe syndrome, galactosemia, Marfan syndrome, Trismoy 13-15, Alport syndrome, myotonic dystrophy, Fabry disease, hypoparathroidism or Conradi syndrome.
135. The method of Claim 121, wherein the developmental abnormality comprises embryonic lethality or reduced viability.
136. The method of Claim 121, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
137. The method of Claim 121, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
138. The method of Claim 121, wherein said bone metabolic abnormality or disorder is arthritis, osteoporosis or osteopetrosis.
139. An agent identified by the method of Claim 121.
140. The agent of Claim 139 which is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
141. The agent of Claim 140, wherein the agonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PR019563, anti-PRO19675, anti-PR020084, anti-PR021434, anti-PR050332, anti-PR038465 or anti-PR0346 antibody.
142. The agent of Claim 140, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibody.
143. A therapeutic agent identified by the method of Claim 121.
144. A method of modulating a phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject whom may already have the phenotype, or may be prone to have the phenotype or may be in whom the phenotype is to be prevented, an effective amount of the agent of Claim 46, or agonists or antagonists thereof, thereby effectively modulating the phenotype.
145. A method of modulating a physiological characteristic associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject whom may already exhibit the physiological characteristic, or may be prone to exhibit the physiological characteristic or may be in whom the physiological characteristic is to be prevented, an effective amount of the agent of Claim 52, or agonists or antagonists thereof, thereby effectively modulating the physiological characteristic.
146. A method of modulating a behavior associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject whom may already exhibit the behavior, or may be prone to exhibit the behavior or may be in whom the exhibited behavior is to be prevented, an effective amount of the agent of Claim 63, or agonists or antagonists thereof, thereby effectively modulating the behavior.
147. A method of modulating the expression of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide, the method comprising administering to a host cell expressing said PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, an effective amount of the agent of Claim 92, or agonists or antagonists thereof, thereby effectively modulating the expression of said polypeptide.
148.A method of modulating a condition associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject whom may have the condition, or may be prone to have the condition or may be in whom the condition is to be prevented, a therapeutically effective amount of the therapeutic agent of Claim 98, or agonists or antagonists thereof, thereby effectively modulating the condition.
149. A method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a non-human transgenic animal cell culture, each cell of said culture comprising a disruption of the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PRO9859, PR09864, PR09904, PR09907, PRO10013, PRO90948, PR028694, PRO16089, PRO19563, PRO19675, PR020084, PRO21434, PRO50332, PR038465 or PR0346 polypeptide, a therapeutically effective amount of the agent of Claim 139, or agonists or antagonists thereof, thereby effectively treating or preventing or ameliorating said disorder.

### BRIEF DESCRIPTION OF THE DRAWINGS

Figure 1 shows a nucleotide sequence (SEQ ID NO:1) of a native sequence PR0218 cDNA, wherein SEQ ID NO:1 is a clone designated herein as "DNA30867-1335" (UNQ192).
Figure 2 shows the amino acid sequence (SEQ ID NO:2) derived from the coding sequence of SEQ ID NO:1 shown in Figure 1.
Figure 3 shows a nucleotide sequence (SEQ ID NO:3) of a native sequence PR0228 cDNA, wherein SEQ ID NO:3 is a clone designated herein as "DNA33092-1202" (UNQ202).
Figure 4 shows the amino acid sequence (SEQ ID NO:4) derived from the coding sequence of SEQ ID NO:3 shown in Figure 3.
Figure 5 shows a nucleotide sequence (SEQ ID NO:5) of a native sequence PR0271 cDNA, wherein SEQ ID NO:5 is a clone designated herein as "DNA39423-1182" (UNQ238).
Figure 6 shows the amino acid sequence (SEQ ID NO:6) derived from the coding sequence of SEQ ID NO:5 shown in Figure 5.
Figure 7 shows a nucleotide sequence (SEQ ID NO:7) of a native sequence PR0273 cDNA, wherein SEQ ID NO:7 is a clone designated herein as "DNA39523-1192" (UNQ240).
Figure 8 shows the amino acid sequence (SEQ ID NO:8) derived from the coding sequence of SEQ ID NO:7 shown in Figure 7.
Figure 9 shows a nucleotide sequence (SEQ ID NO:9) of a native sequence PR0295 cDNA, wherein SEQ ID NO:9 is a clone designated herein as "DNA38268-1188" (UNQ258).
Figure 10 shows the amino acid sequence (SEQ ID NO:10) derived from the coding sequence of SEQ ID NO:9 shown in Figure 9.
Figure 11 shows a nucleotide sequence (SEQ ID NO:11) of a native sequence PR0302 cDNA, wherein SEQ ID NO:11 is a clone designated herein as "DNA40370-1217" (UNQ265).
Figure 12 shows the amino acid sequence (SEQ ID NO:12) derived from the coding sequence of SEQ ID NO:11 shown in Figure 11.
Figure 13 shows a nucleotide sequence (SEQ ID NO:13) of a native sequence PR0305 cDNA, wherein SEQ ID NO: 13 is a clone designated herein as "DNA40619-1220" (UNQ268).
Figure 14 shows the amino acid sequence (SEQ ID NO:14) derived from the coding sequence of SEQ ID NO:13 shown in Figure 13.
Figure 15 shows a nucleotide sequence (SEQ ID NO:15) of a native sequence PR0326 cDNA, wherein SEQ ID NO:15 is a clone designated herein as "DNA37140-1234" (UNQ287).
Figure 16 shows the amino acid sequence (SEQ ID NO:16) derived from the coding sequence of SEQ ID NO:15 shown in Figure 15.
Figure 17 shows a nucleotide sequence (SEQ ID NO:17) of a native sequence PR0386 cDNA, wherein SEQ ID NO:17 is a clone designated herein as "DNA45415-1318" (UNQ326).
Figure 18 shows the amino acid sequence (SEQ ID NO:18) derived from the coding sequence of SEQ ID NO:17 shown in Figure 17.
Figure 19 shows a nucleotide sequence (SEQ ID NO: 19) of a native sequence PR0655 cDNA, wherein SEQ ID NO:19 is a clone designated herein as "DNA50960-1224" (UNQ360).
Figure 20 shows the amino acid sequence (SEQ ID NO:20) derived from the coding sequence of SEQ ID NO: 19 shown in Figure 19.
Figure 21 shows a nucleotide sequence (SEQ ID NO:21) of a native sequence PRO162 cDNA, wherein SEQ ID NO:21 is a clone designated herein as "DNA56965-1356" (UNQ429).
Figure 22 shows the amino acid sequence (SEQ ID NO:22) derived from the coding sequence of SEQ ID NO:21 shown in Figure 21.
Figure 23 shows a nucleotide sequence (SEQ ID NO:23) of a native sequence PR0788 cDNA, wherein SEQ ID NO:23 is a clone designated herein as "DNA56405-1357" (UNQ430).
Figure 24 shows the amino acid sequence (SEQ ID NO:24) derived from the coding sequence of SEQ ID NO:23 shown in Figure 23.
Figure 25 shows a nucleotide sequence (SEQ ID NO:25) of a native sequence PR0792 cDNA, wherein SEQ ID NO:25 is a clone designated herein as "DNA56352-1358" (UNQ431).
Figure 26 shows the amino acid sequence (SEQ ID NO:26) derived from the coding sequence of SEQ ID NO:25 shown in Figure 25.
Figure 27 shows a nucleotide sequence (SEQ ID NO:27) of a native sequence PR0940 cDNA, wherein SEQ ID NO:27 is a clone designated herein as "DNA54002-1367" (UNQ477).
Figure 28 shows the amino acid sequence (SEQ ID NO:28) derived from the coding sequence of SEQ ID NO:27 shown in Figure 27.
Figure 29 shows a nucleotide sequence (SEQ ID NO:29) of a native sequence PR0941 cDNA, wherein SEQ ID NO:29 is a clone designated herein as "DNA53906-1368" (UNQ478).
Figure 30 shows the amino acid sequence (SEQ ID NO:30) derived from the coding sequence of SEQ ID NO:29 shown in Figure 29.
Figure 31 shows a nucleotide sequence (SEQ ID NO:31) of a native sequence PRO1004 cDNA, wherein SEQ ID NO:31 is a clone designated herein as "DNA57844-1410" (UNQ488).
Figure 32 shows the amino acid sequence (SEQ ID NO:32) derived from the coding sequence of SEQ ID NO:31 shown in Figure 31.
Figure 33 shows a nucleotide sequence (SEQ ID NO:33) of a native sequence PRO1012 cDNA, wherein SEQ ID NO:33 is a clone designated herein as "DNA56439-1376" (UNQ495).
Figure 34 shows the amino acid sequence (SEQ ID NO:34) derived from the coding sequence of SEQ ID NO:33 shown in Figure 33.
Figure 35 shows a nucleotide sequence (SEQ ID NO:35) of a native sequence PRO1016 cDNA, wherein SEQ ID NO:35 is a clone designated herein as "DNA56113-1378" (UNQ499).
Figure 36 shows the amino acid sequence (SEQ ID NO:36) derived from the coding sequence of SEQ ID NO:35 shown in Figure 35.
Figure 37 shows a nucleotide sequence (SEQ ID NO:37) of a native sequence PR0474 cDNA, wherein SEQ ID NO:37 is a clone designated herein as "DNA56045-1380" (UNQ502).
Figure 38 shows the amino acid sequence (SEQ ID NO:38) derived from the coding sequence of SEQ ID NO:37 shown in Figure 37.

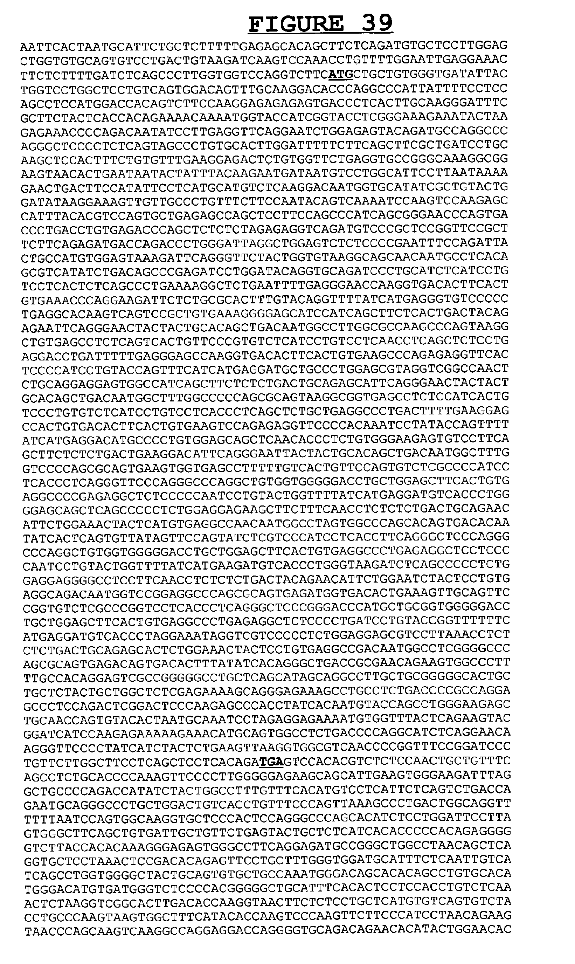

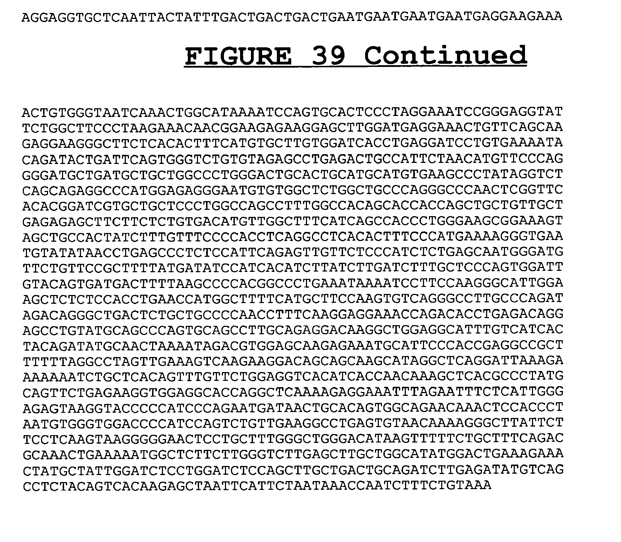

Figure 39 shows a nucleotide sequence (SEQ ID NO:39) of a native sequence PR05238 cDNA, wherein SEQ ID NO:39 is a clone designated herein as "DNA257845" (UNQ503).

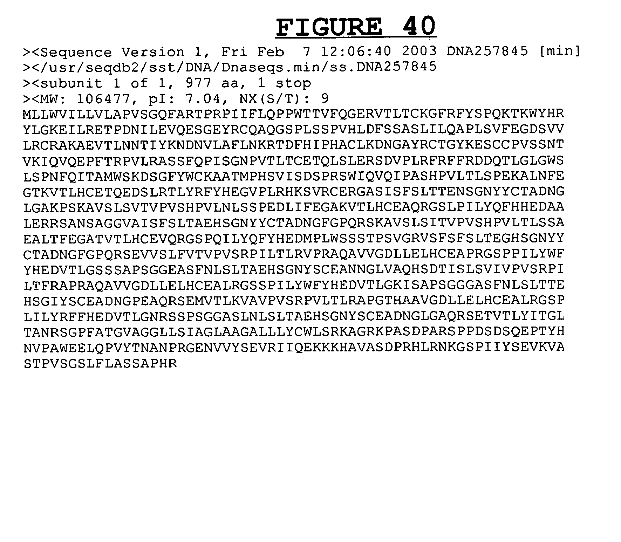

Figure 40 shows the amino acid sequence (SEQ ID NO:40) derived from the coding sequence of SEQ ID NO:39 shown in Figure 39.
Figure 41 shows a nucleotide sequence (SEQ ID NO:41) of a native sequence PRO1069 cDNA, wherein SEQ ID NO:41 is a clone designated herein as "DNA59211-1450" (UNQ526).
Figure 42 shows the amino acid sequence (SEQ ID NO:42) derived from the coding sequence of SEQ ID NO:41 shown in Figure 41.
Figure 43 shows a nucleotide sequence (SEQ ID NO:43) of a native sequence PRO1111 cDNA, wherein SEQ ID NO:43 is a clone designated herein as "DNA58721-1475" (UNQ554).
Figure 44 shows the amino acid sequence (SEQ ID NO:44) derived from the coding sequence of SEQ ID NO:43 shown in Figure 43.
Figure 45 shows a nucleotide sequence (SEQ ID NO:45) of a native sequence PRO1113 cDNA, wherein SEQ ID NO:45 is a clone designated herein as "DNA57254-1477" (UNQ556).
Figure 46 shows the amino acid sequence (SEQ ID NO:46) derived from the coding sequence of SEQ ID NO:45 shown in Figure 45.
Figure 47 shows a nucleotide sequence (SEQ ID NO:47) of a native sequence PRO1130 cDNA, wherein SEQ ID NO:47 is a clone designated herein as "DNA59814-1486" (UNQ567).
Figure 48 shows the amino acid sequence (SEQ ID NO:48) derived from the coding sequence of SEQ ID NO:47 shown in Figure 47.
Figure 49 shows a nucleotide sequence (SEQ ID NO:49) of a native sequence PRO1195 cDNA, wherein SEQ ID NO:49 is a clone designated herein as "DNA65412-1523" (UNQ608).
Figure 50 shows the amino acid sequence (SEQ ID NO:50) derived from the coding sequence of SEQ ID NO:49 shown in Figure 49.
Figure 51 shows a nucleotide sequence (SEQ ID NO:51) of a native sequence PRO1271 cDNA, wherein SEQ ID NO:51 is a clone designated herein as "DNA66309-1538" (UNQ641).
Figure 52 shows the amino acid sequence (SEQ ID NO:52) derived from the coding sequence of SEQ ID NO:51 shown in Figure 51.
Figure 53 shows a nucleotide sequence (SEQ ID NO:53) of a native sequence PRO1865 cDNA, wherein SEQ ID NO:53 is a clone designated herein as "DNA81757-2512" (UNQ856).
Figure 54 shows the amino acid sequence (SEQ ID NO:54) derived from the coding sequence of SEQ ID NO:53 shown in Figure 53.
Figure 55 shows a nucleotide sequence (SEQ ID NO:55) of a native sequence PRO1879 cDNA, wherein SEQ ID NO:55 is a clone designated herein as "DNA54009-2517" (UNQ863).
Figure 56 shows the amino acid sequence (SEQ ID NO:56) derived from the coding sequence of SEQ ID NO:55 shown in Figure 55.
Figure 57 shows a nucleotide sequence (SEQ ID NO:57) of a native sequence PR03446 cDNA, wherein SEQ ID NO:57 is a clone designated herein as "DNA92219-2541" (UNQ1833).
Figure 58 shows the amino acid sequence (SEQ ID NO:58) derived from the coding sequence of SEQ ID NO:57 shown in Figure 57.
Figure 59 shows a nucleotide sequence (SEQ ID NO:59) of a native sequence PR03543 cDNA, wherein SEQ ID NO:51 is a clone designated herein as "DNA86571-2551" (UNQ1835).
Figure 60 shows the amino acid sequence (SEQ ID NO:60) derived from the coding sequence of SEQ ID NO:59 shown in Figure 59.
Figure 61 shows a nucleotide sequence (SEQ ID NO:61) of a native sequence PR04329 cDNA, wherein SEQ ID NO:61 is a clone designated herein as "DNA77629-2573" (UNQ1885).
Figure 62 shows the amino acid sequence (SEQ ID NO:62) derived from the coding sequence of SEQ ID NO:61 shown in Figure 61.
Figure 63 shows a nucleotide sequence (SEQ ID NO:63) of a native sequence PR04352 cDNA, wherein SEQ ID NO:63 is a clone designated herein as "DNA87976-2593" (UNQ1906).
Figure 64 shows the amino acid sequence (SEQ ID NO:64) derived from the coding sequence of SEQ ID NO:63 shown in Figure 63.

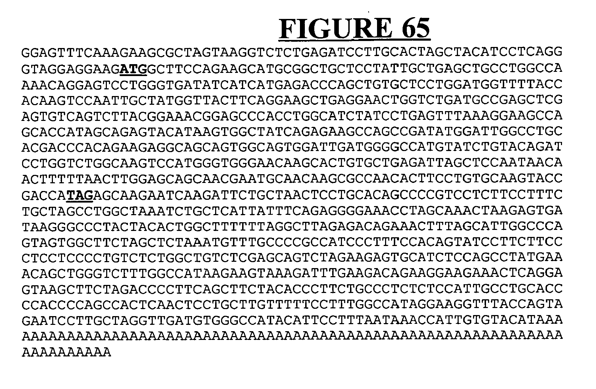

Figure 65 shows a nucleotide sequence (SEQ ID NO:65) of a native sequence PR05733 cDNA, wherein SEQ ID NO:65 is a clone designated herein as "DNA82343" (UNQ2453).

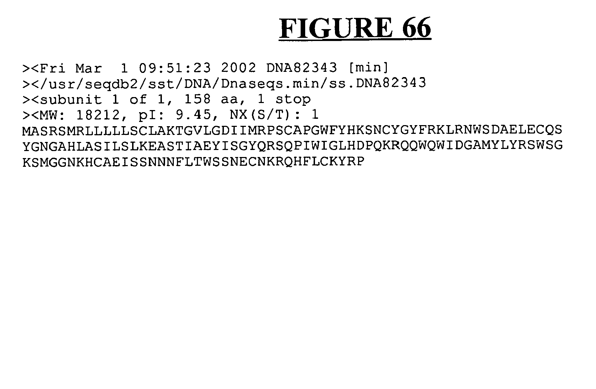

Figure 66 shows the amino acid sequence (SEQ ID NO:66) derived from the coding sequence of SEQ ID NO:65 shown in Figure 65.
Figure 67 shows a nucleotide sequence (SEQ ID NO:67) of a native sequence PR09859 cDNA, wherein SEQ ID NO:67 is a clone designated herein as "DNA125170-2780" (UNQ3043).
Figure 68 shows the amino acid sequence (SEQ ID NO:68) derived from the coding sequence of SEQ ID NO:67 shown in Figure 67.
Figure 69 shows a nucleotide sequence (SEQ ID NO:69) of a native sequence PR09864 cDNA, wherein SEQ ID NO:69 is a clone designated herein as "DNA125151-2784" (UNQ3048).
Figure 70 shows the amino acid sequence (SEQ ID NO:70) derived from the coding sequence of SEQ ID NO:69 shown in Figure 69.
Figure 71 shows a nucleotide sequence (SEQ ID NO:71) of a native sequence PR09904 cDNA, wherein SEQ ID NO:71 is a clone designated herein as "DNA129549-2798" (UNQ3072).
Figure 72 shows the amino acid sequence (SEQ ID NO:72) derived from the coding sequence of SEQ ID NO:71 shown in Figure 71.
Figure 73 shows a nucleotide sequence (SEQ ID NO:73) of a native sequence PR09907 cDNA, wherein SEQ ID NO:73 is a clone designated herein as "DNA142392-2800" (UNQ3075).
Figure 74 shows the amino acid sequence (SEQ ID NO:74) derived from the coding sequence of SEQ ID NO:73 shown in Figure 73.
Figure 75 shows a nucleotide sequence (SEQ ID NO:75) of a native sequence PRO10013 cDNA, wherein SEQ ID NO:75 is a clone designated herein as "DNA125181-2804" (UNQ3082).
Figure 76 shows the amino acid sequence (SEQ ID NO:76) derived from the coding sequence of SEQ ID NO:75 shown in Figure 75.

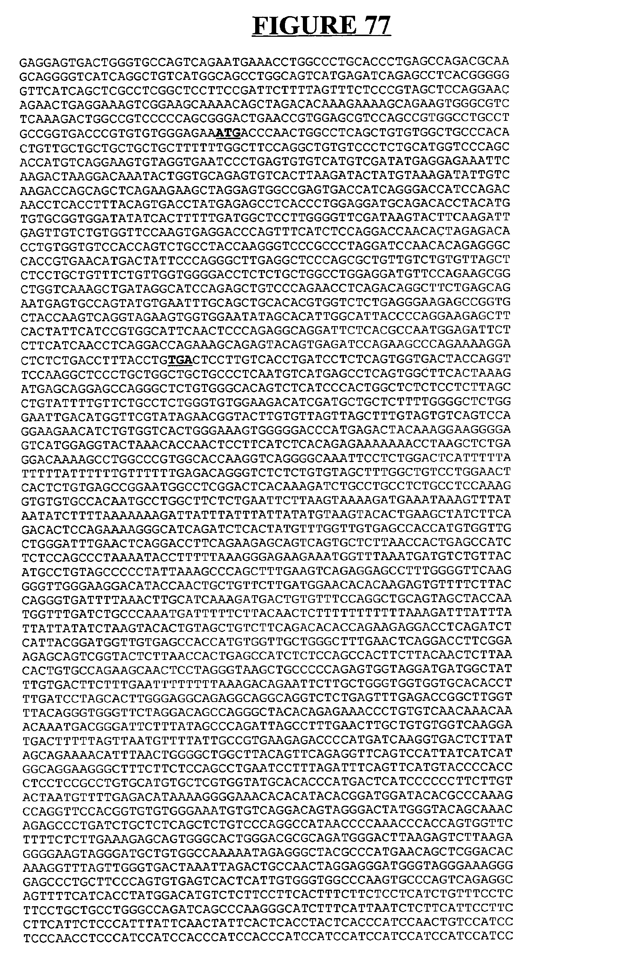

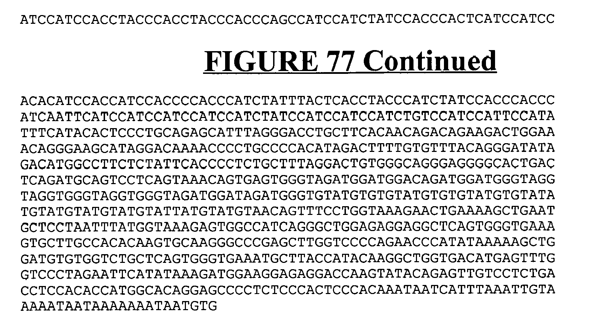

Figure 77 shows a nucleotide sequence (SEQ ID NO:77) of a native sequence PR090948 cDNA, wherein SEQ ID NO:77 is a clone designated herein as "DNA336882" (UNQ5043).

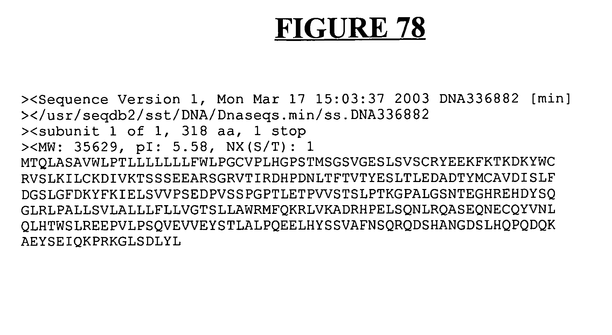

Figure 78 shows the amino acid sequence (SEQ ID NO:78) derived from the coding sequence of SEQ ID NO:77 shown in Figure 77.

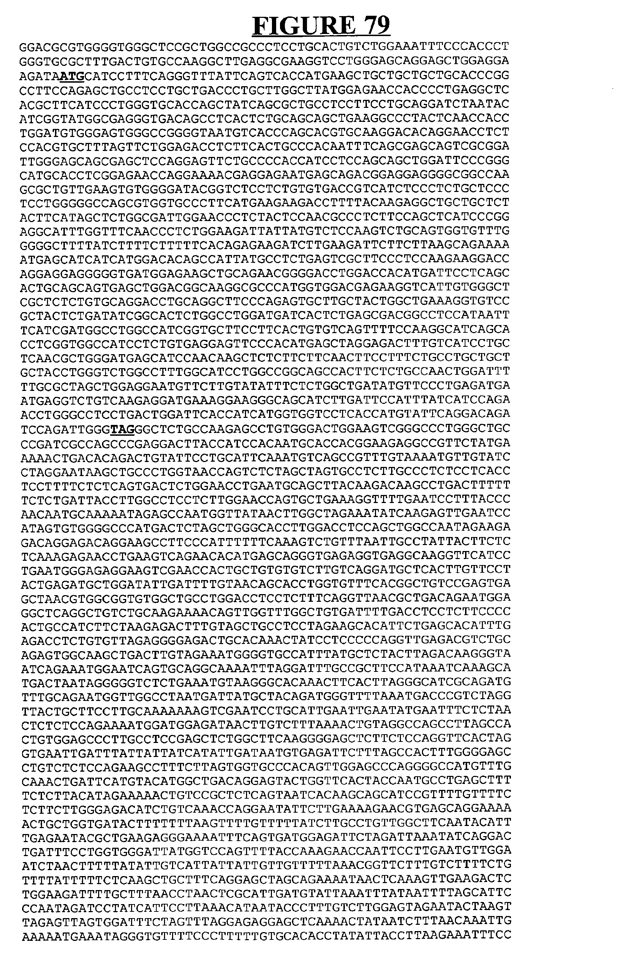

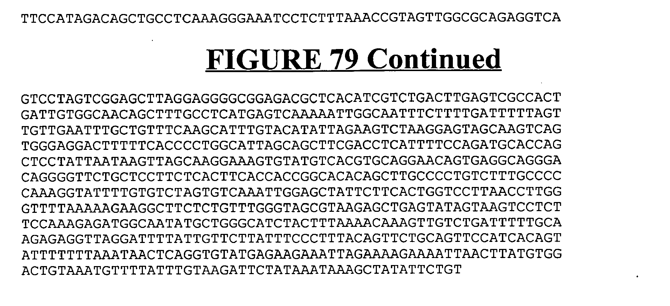

Figure 79 shows a nucleotide sequence (SEQ ID NO:79) of a native sequence PR028694 cDNA, wherein SEQ ID NO:79 is a clone designated herein as "DNA184073" (UNQ5384).

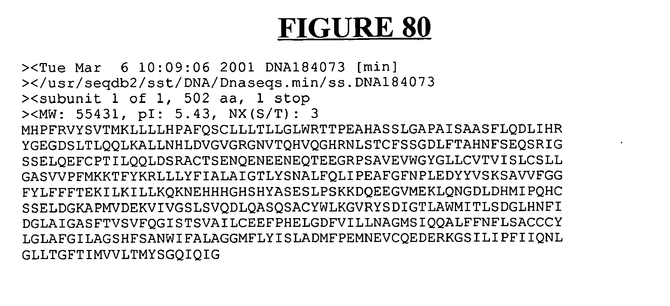

Figure 80 shows the amino acid sequence (SEQ ID NO:80) derived from the coding sequence of SEQ ID NO:79 shown in Figure 79.
Figure 81 shows a nucleotide sequence (SEQ ID NO:81) of a native sequence PRO16089 cDNA, wherein SEQ ID NO:81 is a clone designated herein as "DNA150163-2842" (UNQ5782).
Figure 82 shows the amino acid sequence (SEQ ID NO:82) derived from the coding sequence of SEQ ID NO:81 shown in Figure 81.
Figure 83 shows a nucleotide sequence (SEQ ID NO:83) of a native sequence PRO19563 cDNA, wherein SEQ ID NO:83 is a clone designated herein as "DNA96861-2844" (UNQ5785).
Figure 84 shows the amino acid sequence (SEQ ID NO:84) derived from the coding sequence of SEQ ID NO:83 shown in Figure 83.
Figure 85 shows a nucleotide sequence (SEQ ID NO:85) of a native sequence PRO19675 cDNA, wherein SEQ ID NO:85 is a clone designated herein as "DNA131658-2875" (LINQ5835).
Figure 86 shows the amino acid sequence (SEQ ID NO:86) derived from the coding sequence of SEQ ID NO:85 shown in Figure 85.
Figure 87 shows a nucleotide sequence (SEQ ID NO:87) of a native sequence PR020084 Cdna, wherein SEQ ID NO:87 is a clone designated herein as "DNA168061-2897" (UNQ6124).
Figure 88 shows the amino acid sequence (SEQ ID NO:88) derived from the coding sequence of SEQ ID NO:87 shown in Figure 87.
Figure 89 shows a nucleotide sequence (SEQ ID NO:89) of a native sequence PR021434 cDNA, wherein SEQ ID NO:89 is a clone designated herein as "DNA147253-2983" (UNQ6509).
Figure 90 shows the amino acid sequence (SEQ ID NO:90) derived from the coding sequence of SEQ ID NO:89 shown in Figure 89.

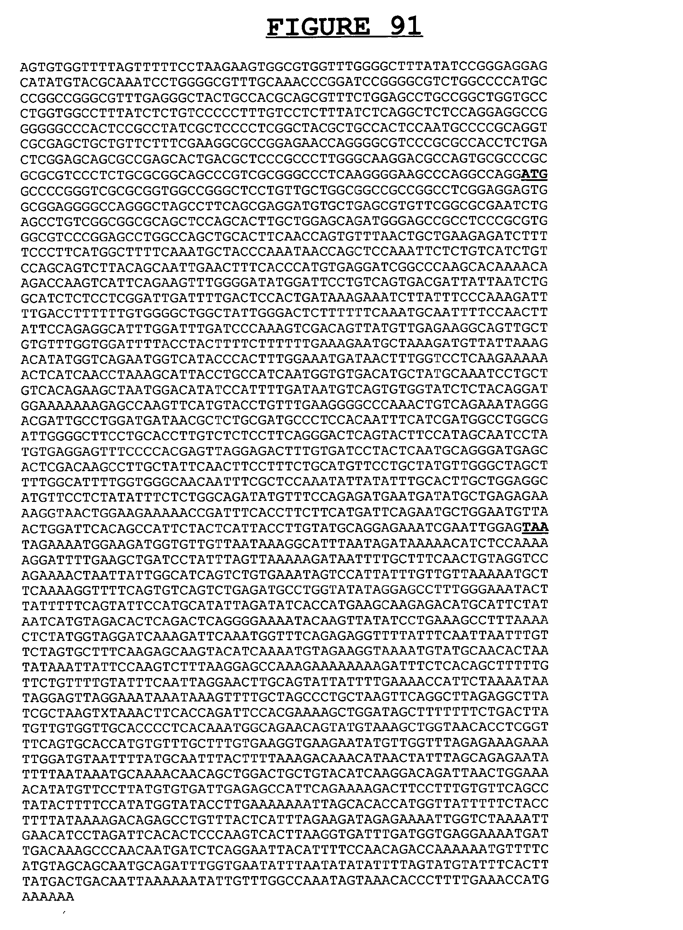

Figure 91 shows a nucleotide sequence (SEQ ID NO:91) of a native sequence PR050332 cDNA, wherein SEQ ID NO:91 is a clone designated herein as "DNA255255" (UNQ11645).

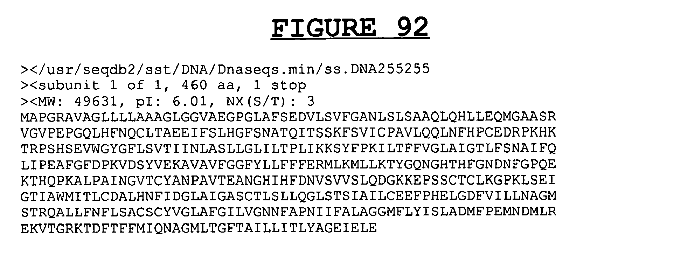

Figure 92 shows the amino acid sequence (SEQ ID NO:92) derived from the coding sequence of SEQ ID NO:91 shown in Figure 91.

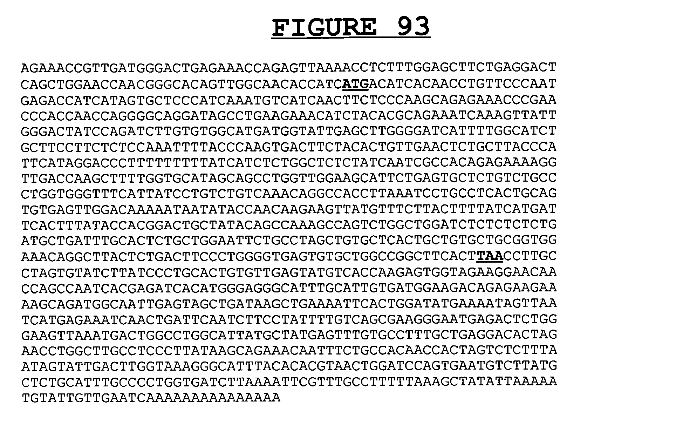

Figure 93 shows a nucleotide sequence (SEQ ID NO:93) of a native sequence PR038465 cDNA, wherein SEQ ID NO:93 is a clone designated herein as "DNA228002" (UNQ15965).

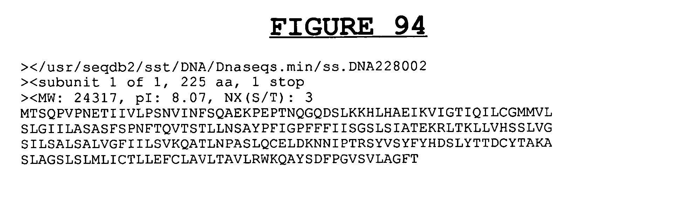

Figure 94 shows the amino acid sequence (SEQ ID NO:94) derived from the coding sequence of SEQ ID NO:93 shown in Figure 93.
Figure 95 shows a nucleotide sequence (SEQ ID NO:95) of a native sequence PR0346 cDNA, wherein SEQ ID NO:95 is a clone designated herein as "DNA44167-1243" (UNQ305).
Figure 96 shows the amino acid sequence (SEQ ID NO:96) derived from the coding sequence of SEQ ID NO:95 shown in Figure 95.

### DETAILED DESCRIPTION OF THE PREFERRED EMBODIMENTS

### I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PR0218, PR0228, PR0271, PR0273, PR0295, PR0302, PR0305, PR0326, PR0386, PR0655, PR0162, PR0788, PR0792, PR0940, PR0941, PR01004, PR01012, PR01016, PR0474, PR05238, PRO1069, PRO1111, PRO1113, PRO1130, PRO1195, PR01271, PRO1865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide" comprises a polypeptide having the same amino acid sequence as the corresponding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide derived from nature. Such native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PR010013, PR090948, PR028694, PRO16089, PRO19563, PRO19675, PR020084, PR021434, PR050332, PR038465 or PR0346 polypeptides can be isolated from nature or can be produced by recombinant or synthetic means. The term "native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide" specifically encompasses naturally-occurring truncated or secreted forms of the specific PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide *(e.g.,* an extracellular domain sequence), naturally-occurring variant forms (*e.g.,* alternatively spliced forms) and naturally-occurring allelic variants of the polypeptide. The invention provides native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR03 8465 or PR0346 polypeptides disclosed herein which are mature or full-length native sequence polypeptides comprising the full-length amino acids sequences shown in the accompanying figures. Start and stop codons are shown in bold font and underlined in the figures. However, while the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides.

The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide "extracellular domain" or "ECD" refers to a form of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide which is essentially free of the transmembrane and cytoplasmic domains. Ordinarily, a PR0218, PR0228, PR0271, PR0273, PR0295, PR0302, PR0305, PR0326, PR0386, PR0655, PRO162, PRO788, PRO792, PR0940, PR0941, PRO1004, PRO1012, PRO1016, PR0474, PR05238, PRO1069, PRO1111, PRO1113, PRO1130, PRO1195, PRO1271, PRO1865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide ECD will have less than 1% of such transmembrane and/or cytoplasmic domains and preferably, will have less than 0.5% of such domains. It will be understood that any transmembrane domains identified for the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide variant" means a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PRO3543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, preferably an active PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PRO5238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, as defined herein having at least about 80% amino acid sequence identity with a full-length native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence as disclosed herein, a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence lacking the signal peptide as disclosed herein, an extracellular domain of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 polypeptide, with or without the signal peptide, as disclosed herein or any other fragment of a full-length PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence as disclosed herein (such as those encoded by a nucleic acid that represents only a portion of the complete coding sequence for a full-length PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide). Such PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide variants include, for instance, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides wherein one or more amino acid residues are added, or deleted, at the N- or C-terminus of the full-length native amino acid sequence. Ordinarily, a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide variant will have or will have at least about 80% amino acid sequence identity, alternatively will have or will have at least about 81 %, 82%, 83%, 84%, 85%, 86%, 87%, 88%, 89%, 90%, 91%, 92%, 93%, 94%, 95%, 96%, 97%, 98%, or 99% amino acid sequence identity, to a full-length native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence as disclosed herein, a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence lacking the signal peptide as disclosed herein, an extracellular domain of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PRO9859, PR09864, PR09904, PR09907, PRO10013, PR090948, PR028694, PRO16089, PRO19563, PRO19675, PR020084, PR021434, PR050332, PR038465 or PR0346 polypeptide, with or without the signal peptide, as disclosed herein or any other specifically defined fragment of a full-length PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence as disclosed herein. Ordinarily, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant polypeptides are or are at least about 10 amino acids in length, alternatively are or are at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 amino acids in length, or more. Optionally, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant polypeptides will have no more than one conservative amino acid substitution as compared to the native PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence, alternatively will have or will have no more than 2, 3, 4, 5, 6, 7, 8, 9, or 10 conservative amino acid substitution as compared to the native PR0218, PR0228, PR0271, PR0273, PR0295, PR0302, PR0305, PR0326, PR03 86, PR0655, PRO162, PR0788, PR0792, PR0940, PR0941, PRO1004, PRO1012, PRO1016, PR0474, PR05238, PRO1069, PRO1111, PR01113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence.

"Percent (%) amino acid sequence identity" with respect to the PR0218, PRO228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequences identified herein is defined as the percentage of amino acid residues in a candidate sequence that are identical with the amino acid residues in the specific PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues. Unless specifically stated otherwise, all % amino acid sequence identity values used herein are obtained as described in the immediately preceding paragraph using the ALIGN-2 computer program.

"PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant polynucleotide" or "PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant nucleic acid sequence" means a nucleic acid molecule which encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, preferably an active PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, as defined herein and which has at least about 80% nucleic acid sequence identity with a nucleotide acid sequence encoding a full-length native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence as disclosed herein, a full-length native sequence PR0218, PRO228, PRO271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence lacking the signal peptide as disclosed herein, an extracellular domain of a PR0218, PR0228, PRO271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 polypeptide, with or without the signal peptide, as disclosed herein or any other fragment of a full-length PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence as disclosed herein (such as those encoded by a nucleic acid that represents only a portion of the complete coding sequence for a full-length PR0218, PR0228, PR0271, PR0273, PR0295, PR0302, PR0305, PR0326, PR0386, PR0655, PRO162, PR0788, PR0792, PRO940, PRO941, PRO1004, PRO1012, PRO1016, PR0474, PRO5238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide). Ordinarily, a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant polynucleotide will have or will have at least about 80% nucleic acid sequence identity, alternatively will have or will have at least about 81%, 82%, 83%, 84%, 85%, 86%, 87%, 88%, 89%, 90%, 91%, 92%, 93%, 94%, 95%, 96%, 97%, 98%, or 99% nucleic acid sequence identity with a nucleic acid sequence encoding a full-length native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence as disclosed herein, a full-length native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence lacking the signal peptide as disclosed herein, an extracellular domain of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PRO3543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, with or without the signal sequence, as disclosed herein or any other fragment of a full-length PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide sequence as disclosed herein. Variants do not encompass the native nucleotide sequence.

Ordinarily, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant polynucleotides are or are at least about 5 nucleotides in length, alternatively are or are at least about 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length.

"Percent (%) nucleic acid sequence identity" with respect to PR0218-, PR0228-, PR0271-, PRO273-, PR0295-, PR0302-, PR0305-, PR0326-, PR0386-, PR0655-, PRO162-, PR0788-, PR0792-, PR0940-, PRO941-, PRO1004-, PRO1012-, PR01016-, PR0474-, PR05238-, PRO1069-, PRO1111-, PRO1113-, PRO1130- , PRO1195-, PRO1271-, PRO1865-, PRO1879-, PR03446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PRO346-encoding nucleic acid sequences identified herein is defined as the percentage of nucleotides in a candidate sequence that are identical with the nucleotides in the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PRO9904, PRO9907, PRO10013, PR090948, PR028694, PRO16089, PRO19563, PRO19675, PR020084, PR021434, PR050332, PR038465 or PR0346 nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides. Unless specifically stated otherwise, all % nucleic acid sequence identity values used herein are obtained as described in the immediately preceding paragraph using the ALIGN-2 computer program.

The invention also provides PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant polynucleotides which are nucleic acid molecules that encode a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide and which are capable of hybridizing, preferably under stringent hybridization and wash conditions, to nucleotide sequences encoding a full-length PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide as disclosed herein. PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 variant polypeptides may be those that are encoded by a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant polynucleotide.

The term "full-length coding region" when used in reference to a nucleic acid encoding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide refers to the sequence ofnucleotides which encode the full-length PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PRO20084, PR021434, PR050332, PR038465 or PR0346 polypeptide ofthe invention (which is often shown between start and stop codons, inclusive thereof, in the accompanying figures). The term "full-length coding region" when used in reference to an ATCC deposited nucleic acid refers to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide-encoding portion ofthe cDNA that is inserted into the vector deposited with the ATCC (which is often shown between start and stop codons, inclusive thereof, in the accompanying figures).

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. The invention provides that the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide natural environment will not be present. Ordinarily, however, isolated polypeptide will be prepared by at least one purification step.

An "isolated" PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PR0218, PR0228, PR0271, PR0273, PR0295, PR0302, PR0305, PR0326, PR0386, PR0655, PR0162, PR0788, PR0792, PR0940, PR0941, PRO1004, PRO1012, PRO1016, PR0474, PR05238, PRO1069, PRO1111, PR01113, PR01130, PR01195, PR01271, PR01865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

"Active" or "activity" for the purposes herein refers to form(s) of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide which retain a biological and/or an immunological activity of native or naturally-occurring PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, wherein "biological" activity refers to a biological function (either inhibitory or stimulatory) caused by a native or naturally-occurring PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide other than the ability to induce the production of an antibody against an antigenic epitope possessed by a native or naturally-occurring PR0218, PR0228, PR0271, PR0273, PR0295, PR0302, PR0305, PR0326, PR0386, PR0655, PRO162, PR0788, PRO792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide and an "immunological" activity refers to the ability to induce the production of an antibody against an antigenic epitope possessed by a native or naturally-occurring PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.

The term "antagonist" is used in the broadest sense [unless otherwise qualified], and includes any molecule that partially or fully blocks, inhibits, or neutralizes a biological activity of a native PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide disclosed herein. In a similar manner, the term "agonist" is used in the broadest sense [unless otherwise qualified] and includes any molecule that mimics a biological activity of a native PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PRO4352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide disclosed herein. Suitable agonist or antagonist molecules specifically include agonist or antagonist antibodies or antibody fragments, fragments or amino acid sequence variants of native PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides, peptides, antisense oligonucleotides, small organic molecules, etc. Methods for identifying agonists or antagonists of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PR01016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide may comprise contacting a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PRO9904, PRO9907, PRO10013, PRO90948, PR028694, PRO16089, PRO19563, PRO19675, PR020084, PR021434, PR050332, PR038465 or PRO346 polypeptide with a candidate agonist or antagonist molecule and measuring a detectable change in one or more biological activities normally associated with the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.

"Treating" or "treatment" or "alleviation" refers to both therapeutic treatment and prophylactic or preventative measures, wherein the object is to prevent or slow down (lessen) the targeted pathologic condition or disorder. A subject in need of treatment may already have the disorder, or may be prone to have the disorder or may be in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, rodents such as rats or mice, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. Depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PR0218, PR0228, PR0271, PR0273, PR0295, PR0302, PR0305, PR0326, PR0386, PR0655, PRO162, PR0788, PR0792, PR0940, PRO941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

An "effective amount" of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, an anti-PR0218, anti-PR0228, anti-PR0271, anti-PR0273, anti-PR0295, anti-PR0302, anti-PR0305, anti-PR0326, anti-PRO386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PR019675, anti-PR020084, anti-PR021434, anti-PR050332, anti-PR038465 or anti-PR0346 antibody, a PR0218, PR0228, PR0271, PR0273, PR0295, PR0302, PR0305, PR0326, PR0386, PR0655, PRO162, PR0788, PR0792, PRO940, PRO941, PRO1004, PRO1012, PRO1016, PRO474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding oligopeptide, a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding organic molecule or an agonist or antagonist thereof as disclosed herein is an amount sufficient to carry out a specifically stated purpose. An "effective amount" may be determined empirically and in a routine manner, in relation to the stated purpose.

The term "therapeutically effective amount" refers to an amount of an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody, a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding oligopeptide, a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding organic molecule or other drug effective to "treat" a disease or disorder in a subject or mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. See the definition herein of "treating". To the extent the drug may prevent growth and/or kill existing cancer cells, it may be cytostatic and/or cytotoxic.

The phrases "cardiovascular, endothelial and angiogenic disorder", "cardiovascular, endothelial and angiogenic dysfunction", "cardiovascular, endothelial or angiogenic disorder" and "cardiovascular, endothelial or angiogenic dysfunction" are used interchangeably and refer in part to systemic disorders that affect vessels, such as diabetes mellitus, as well as diseases of the vessels themselves, such as of the arteries, capillaries, veins, and/or lymphatics. This would include indications that stimulate angiogenesis and/or cardiovascularization, and those that inhibit angiogenesis and/or cardiovascularization. Such disorders include, for example, arterial disease, such as atherosclerosis, hypertension, inflammatory vasculitides, Reynaud's disease and Reynaud's phenomenon, aneurysms, and arterial restenosis; venous and lymphatic disorders such as thrombophlebitis, lymphangitis, and lymphedema; and other vascular disorders such as peripheral vascular disease, cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma, tumor angiogenesis, trauma such as wounds, burns, and other injured tissue, implant fixation, scarring, ischemia reperfusion injury, rheumatoid arthritis, cerebrovascular disease, renal diseases such as acute renal failure, or osteoporosis. This would also include angina, myocardial infarctions such as acute myocardial infarctions, cardiac hypertrophy, and heart failure such as CHF.

"Hypertrophy", as used herein, is defined as an increase in mass of an organ or structure independent of natural growth that does not involve tumor formation. Hypertrophy of an organ or tissue is due either to an increase in the mass of the individual cells (true hypertrophy), or to an increase in the number of cells making up the tissue (hyperplasia), or both. Certain organs, such as the heart, lose the ability to divide shortly after birth. Accordingly, "cardiac hypertrophy" is defined as an increase in mass of the heart, which, in adults, is characterized by an increase in myocyte cell size and contractile protein content without concomitant cell division. The character of the stress responsible for inciting the hypertrophy, (*e.g.,* increased preload, increased afterload, loss of myocytes, as in myocardial infarction, or primary depression of contractility), appears to play a critical role in determining the nature of the response. The early stage of cardiac hypertrophy is usually characterized morphologically by increases in the size of myofibrils and mitochondria, as well as by enlargement of mitochondria and nuclei. At this stage, while muscle cells are larger than normal, cellular organization is largely preserved. At a more advanced stage of cardiac hypertrophy, there are preferential increases in the size or number of specific organelles, such as mitochondria, and new contractile elements are added in localized areas of the cells, in an irregular manner. Cells subjected to long-standing hypertrophy show more obvious disruptions in cellular organization, including markedly enlarged nuclei with highly lobulated membranes, which displace adjacent myofibrils and cause breakdown of normal Z-band registration. The phrase "cardiac hypertrophy" is used to include all stages of the progression of this condition, characterized by various degrees of structural damage of the heart muscle, regardless of the underlying cardiac disorder. Hence, the term also includes physiological conditions instrumental in the development of cardiac hypertrophy, such as elevated blood pressure, aortic stenosis, or myocardial infarction.

"Heart failure" refers to an abnormality of cardiac function where the heart does not pump blood at the rate needed for the requirements of metabolizing tissues. The heart failure can be caused by a number of factors, including ischemic, congenital, rheumatic, or idiopathic forms.

"Congestive heart failure" (CHF) is a progressive pathologic state where the heart is increasingly unable to supply adequate cardiac output (the volume of blood pumped by the heart over time) to deliver the oxygenated blood to peripheral tissues. As CHF progresses, structural and hemodynamic damages occur. While these damages have a variety of manifestations, one characteristic symptom is ventricular hypertrophy. CHF is a common end result of a number of various cardiac disorders.

"Myocardial infarction" generally results from atherosclerosis of the coronary arteries, often with superimposed coronary thrombosis. It may be divided into two major types: transmural infarcts, in which myocardial necrosis involves the full thickness of the ventricular wall, and subendocardial (nontransmural) infarcts, in which the necrosis involves the subendocardium, the intramural myocardium, or both, without extending all the way through the ventricular wall to the epicardium. Myocardial infarction is known to cause both a change in hemodynamic effects and an alteration in structure in the damaged and healthy zones of the heart. Thus, for example, myocardial infarction reduces the maximum cardiac output and the stroke volume of the heart. Also associated with myocardial infarction is a stimulation of the DNA synthesis occurring in the interstice as well as an increase in the formation of collagen in the areas of the heart not affected.

As a result of the increased stress or strain placed on the heart in prolonged hypertension due, for example, to the increased total peripheral resistance, cardiac hypertrophy has long been associated with "hypertension". A characteristic of the ventricle that becomes hypertrophic as a result of chronic pressure overload is an impaired diastolic performance. Fouad et al., J. Am. Coll. Cardiol., 4: 1500-1506 (1984); Smith et al., J. Am. Coll. Cardiol., 5: 869-874 (1985). A prolonged left ventricular relaxation has been detected in early essential hypertension, in spite of normal or supranormal systolic function. Hartford et al., Hypertension, 6: 329-338 (1984). However, there is no close parallelism between blood pressure levels and cardiac hypertrophy. Although improvement in left ventricular function in response to antihypertensive therapy has been reported in humans, patients variously treated with a diuretic (hydrochlorothiazide), a β-blocker (propranolol), or a calcium channel blocker (diltiazem), have shown reversal of left ventricular hypertrophy, without improvement in diastolic function. Inouye et al., Am. J. Cardiol., 53: 1583-7 (1984).

Another complex cardiac disease associated with cardiac hypertrophy is "hypertrophic cardiomyopathy". This condition is characterized by a great diversity of morphologic, functional, and clinical features (Maron et al., N. Engl. J. Med., 316: 780-789 (1987); Spirito et al., N. Engl. J. Med., 320: 749-755 (1989); Louie and Edwards, Prog. Cardiovasc. Dis., 36: 275-308 (1994); Wigle et al., Circulation, 92: 1680-1692 (1995)), the heterogeneity of which is accentuated by the fact that it afflicts patients of all ages. Spirito et al., N. Engl. J. Med., 336: 775-785 (1997). The causative factors of hypertrophic cardiomyopathy are also diverse and little understood. In general, mutations in genes encoding sarcomeric proteins are associated with hypertrophic cardiomyopathy. Recent data suggest that β-myosin heavy chain mutations may account for approximately 30 to 40 percent of cases of familial hypertrophic cardiomyopathy. Watkins et al., N. Engl. J. Med., 326: 1108-1114 (1992); Schwartz et al, Circulation, 91: 532-540 (1995); Marian and Roberts, Circulation, 92: 1336-1347 (1995); Thierfelder et al., Cell, 77:701-712 (1994); Watkins et al., Nat. Gen., 11:434-437 (1995). Besides β-myosin heavy chain, other locations of genetic mutations include cardiac troponin T, alpha topomyosin, cardiac myosin binding protein C, essential myosin light chain, and regulatory myosin light chain. *See,* Malik and Watkins, Curr. Opin. Cardiol., 12: 295-302 (1997).

Supravalvular "aortic stenosis" is an inherited vascular disorder characterized by narrowing of the ascending aorta, but other arteries, including the pulmonary arteries, may also be affected. Untreated aortic stenosis may lead to increased intracardiac pressure resulting in myocardial hypertrophy and eventually heart failure and death. The pathogenesis of this disorder is not fully understood, but hypertrophy and possibly hyperplasia of medial smooth muscle are prominent features of this disorder. It has been reported that molecular variants of the elastin gene are involved in the development and pathogenesis of aortic stenosis. U.S. Patent No. 5,650,282 issued July 22, 1997.

"Valvular regurgitation" occurs as a result of heart diseases resulting in disorders of the cardiac valves. Various diseases, like rheumatic fever, can cause the shrinking or pulling apart of the valve orifice, while other diseases may result in endocarditis, an inflammation of the endocardium or lining membrane ofthe atrioventricular orifices and operation of the heart. Defects such as the narrowing of the valve stenosis or the defective closing of the valve result in an accumulation of blood in the heart cavity or regurgitation of blood past the valve. If uncorrected, prolonged valvular stenosis or insufficiency may result in cardiac hypertrophy and associated damage to the heart muscle, which may eventually necessitate valve replacement.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, etc.

The term "T cell mediated disease" means a disease in which T cells directly or indirectly mediate or otherwise contribute to a morbidity in a mammal. The T cell mediated disease may be associated with cell mediated effects, lymphokine mediated effects, etc., and even effects associated with B cells if the B cells are stimulated, for example, by the lymphokines secreted by T cells.

Examples of immune-related and inflammatory diseases, some of which are immune or T cell mediated, include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonia, idiopathic pulmonary fibrosis and hypersensitivity pneumonitis, or transplantation associated diseases including graft rejection and graft -versus-host-disease. Infectious diseases including viral diseases such as AIDS (HIV infection), hepatitis A, B, C, D, and E, herpes, etc., bacterial infections, fungal infections, protozoal infections and parasitic infections.

An "autoimmune disease" herein is a disease or disorder arising from and directed against an individual's own tissues or organs or a co-segregate or manifestation thereof or resulting condition therefrom. In many of these autoimmune and inflammatory disorders, a number of clinical and laboratory markers may exist, including, but not limited to, hypergammaglobulinemia, high levels of autoantibodies, antigen-antibody complex deposits in tissues, benefit from corticosteroid or immunosuppressive treatments, and lymphoid cell aggregates in affected tissues. Without being limited to any one theory regarding B-cell mediated autoimmune disease, it is believed that B cells demonstrate a pathogenic effect in human autoimmune diseases through a multitude of mechanistic pathways, including autoantibody production, immune complex formation, dendritic and T-cell activation, cytokine synthesis, direct chemokine release, and providing a nidus for ectopic neo-lymphogenesis. Each of these pathways may participate to different degrees in the pathology of autoimmune diseases.

"Autoimmune disease" can be an organ-specific disease (i.e., the immune response is specifically directed against an organ system such as the endocrine system, the hematopoietic system, the skin, the cardiopulmonary system, the gastrointestinal and liver systems, the renal system, the thyroid, the ears, the neuromuscular system, the central nervous system, etc.) or a systemic disease which can affect multiple organ systems (for example, systemic lupus erythematosus (SLE), rheumatoid arthritis, polymyositis, etc.). Preferred such diseases include autoimmune rheumatologic disorders (such as, for example, rheumatoid arthritis, Sj6gren's syndrome, scleroderma, lupus such as SLE and lupus nephritis, polymyositis/dermatomyositis, cryoglobulinemia, anti-phospholipid antibody syndrome, and psoriatic arthritis), autoimmune gastrointestinal and liver disorders (such as, for example, inflammatory bowel diseases (e.g., ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-associated vasculitis, including Churg-Strauss vasculitis, Wegener's granulomatosis, and polyarteriitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, and autoimmune endocrine disorders (such as, for example, diabetic-related autoimmune diseases such as insulin-dependent diabetes mellitus (IDDM), Addison's disease, and autoimmune thyroid disease (e.g., Graves' disease and thyroiditis)). More preferred such diseases include, for example, rheumatoid arthritis, ulcerative colitis, ANCA-associated vasculitis, lupus, multiple sclerosis, Sjögren's syndrome, Graves' disease, IDDM, pernicious anemia, thyroiditis, and glomerulonephritis.
Specific examples of other autoimmune diseases as defined herein, which in some cases encompass those listed above, include, but are not limited to, arthritis (acute and chronic, rheumatoid arthritis including juvenile-onset rheumatoid arthritis and stages such as rheumatoid synovitis, gout or gouty arthritis, acute immunological arthritis, chronic inflammatory arthritis, degenerative arthritis, type II collagen-induced arthritis, infectious arthritis, Lyme arthritis, proliferative arthritis, psoriatic arthritis, Still's disease, vertebral arthritis, osteoarthritis, arthritis chronica progrediente, arthritis deformans, polyarthritis chronica primaria, reactive arthritis, menopausal arthritis, estrogen-depletion arthritis, and ankylosing spondylitis/rheumatoid spondylitis), autoimmune lymphoproliferative disease, inflammatory hyperproliferative skin diseases, psoriasis such as plaque psoriasis, gutatte psoriasis, pustular psoriasis, and psoriasis of the nails, atopy including atopic diseases such as hay fever and Job's syndrome, dermatitis including contact dermatitis, chronic contact dermatitis, exfoliative dermatitis, allergic dermatitis, allergic contact dermatitis, hives, dermatitis herpetiformis, nummular dermatitis, seborrheic dermatitis, non-specific dermatitis, primary irritant contact dermatitis, and atopic dermatitis, x-linked hyper IgM syndrome, allergic intraocular inflammatory diseases, urticaria such as chronic allergic urticaria and chronic idiopathic urticaria, including chronic autoimmune urticaria, myositis, polymyositis/dermatomyositis, juvenile dermatomyositis, toxic epidermal necrolysis, scleroderma (including systemic scleroderma), sclerosis such as systemic sclerosis, multiple sclerosis (MS) such as spino-optical MS, primary progressive MS (PPMS), and relapsing remitting MS (RRMS), progressive systemic sclerosis, atherosclerosis, arteriosclerosis, sclerosis disseminata, ataxic sclerosis, neuromyelitis optica (NMO), inflammatory bowel disease (IBD) (for example, Crohn's disease, autoimmune-mediated gastrointestinal diseases, gastrointestinal inflammation, colitis such as ulcerative colitis, colitis ulcerosa, microscopic colitis, collagenous colitis, colitis polyposa, necrotizing enterocolitis, and transmural colitis, and autoimmune inflammatory bowel disease), bowel inflammation, pyoderma gangrenosum, erythema nodosum, primary sclerosing cholangitis, respiratory distress syndrome, including adult or acute respiratory distress syndrome (ARDS), meningitis, inflammation of all or part of the uvea, iritis, choroiditis, an autoimmune hematological disorder, graft-versus-host disease, angioedema such as hereditary angioedema, cranial nerve damage as in meningitis, herpes gestationis, pemphigoid gestationis, pruritis scroti, autoimmune premature ovarian failure, sudden hearing loss due to an autoimmune condition, IgE-mediated diseases such as anaphylaxis and allergic and atopic rhinitis, encephalitis such as Rasmussen's encephalitis and limbic and/or brainstem encephalitis, uveitis, such as anterior uveitis, acute anterior uveitis, granulomatous uveitis, nongranulomatous uveitis, phacoantigenic uveitis, posterior uveitis, or autoimmune uveitis, glomerulonephritis (GN) with and without nephrotic syndrome such as chronic or acute glomerulonephritis such as primary GN, immune-mediated GN, membranous GN (membranous nephropathy), idiopathic membranous GN or idiopathic membranous nephropathy, membrano- or membranous proliferative GN (MPGN), including Type I and Type II, and rapidly progressive GN (RPGN), proliferative nephritis, autoimmune polyglandular endocrine failure, balanitis including balanitis circumscripta plasmacellularis, balanoposthitis, erythema annulare centrifugum, erythema dyschromicum perstans, eythema multiform, granuloma annulare, lichen nitidus, lichen sclerosus et atrophicus, lichen simplex chronicus, lichen spinulosus, lichen planus, lamellar ichthyosis, epidermolytic hyperkeratosis, premalignant keratosis, pyoderma gangrenosum, allergic conditions and responses, food allergies, drug allergies, insect allergies, rare allergic disorders such as mastocytosis, allergic reaction, eczema including allergic or atopic eczema, asteatotic eczema, dyshidrotic eczema, and vesicular palmoplantar eczema, asthma such as asthma bronchiale, bronchial asthma, and auto-immune asthma, conditions involving infiltration ofT cells and chronic inflammatory responses, immune reactions against foreign antigens such as fetal A-B-O blood groups during pregnancy, chronic pulmonary inflammatory disease, autoimmune myocarditis, leukocyte adhesion deficiency, lupus, including lupus nephritis, lupus cerebritis, pediatric lupus, non-renal lupus, extra-renal lupus, discoid lupus and discoid lupus erythematosus, alopecia lupus, SLE, such as cutaneous SLE or subacute cutaneous SLE, neonatal lupus syndrome (NLE), and lupus erythematosus disseminatus, juvenile onset (Type I) diabetes mellitus, including pediatric IDDM, adult onset diabetes mellitus (Type II diabetes), autoimmune diabetes, idiopathic diabetes insipidus, diabetic retinopathy, diabetic nephropathy, diabetic colitis, diabetic large-artery disorder, immune responses associated with acute and delayed hypersensitivity mediated by cytokines and T-lymphocytes, tuberculosis, sarcoidosis, granulomatosis including lymphomatoid granulomatosis, Wegener's granulomatosis, agranulocytosis, vasculitides, including vasculitis, large-vessel vasculitis (including polymyalgia rheumatica and giant-cell (Takayasu's) arteritis), medium-vessel vasculitis (including Kawasaki's disease and polyarteritis nodosa/periarteritis nodosa), microscopic polyarteritis, immunovasculitis, CNS vasculitis, cutaneous vasculitis, hypersensitivity vasculitis, necrotizing vasculitis such as systemic necrotizing vasculitis, and ANCA-associated vasculitis, such as Churg-Strauss vasculitis or syndrome (CSS) and ANCA-associated small-vessel vasculitis, temporal arteritis, aplastic anemia, autoimmune aplastic anemia, Coombs positive anemia, Diamond Blackfan anemia, hemolytic anemia or immune hemolytic anemia including autoimmune hemolytic anemia (AIHA), pernicious anemia (anemia pemiciosa), Addison's disease, pure red cell anemia or aplasia (PRCA), Factor VIII deficiency, hemophilia A, autoimmune neutropenia(s), cytopenias such as pancytopenia, leukopenia, diseases involving leukocyte diapedesis, CNS inflammatory disorders, Alzheimer's disease, Parkinson's disease, multiple organ injury syndrome such as those secondary to septicemia, trauma or hemorrhage, antigen-antibody complex- mediated diseases, anti-glomerular basement membrane disease, anti-phospholipid antibody syndrome, motoneuritis, allergic neuritis, Behçet's disease/syndrome, Castleman's syndrome, Goodpasture's syndrome, Reynaud's syndrome, Sjögren's syndrome, Stevens-Johnson syndrome, pemphigoid such as pemphigoid bullous and skin pemphigoid, pemphigus (including pemphigus vulgaris, pemphigus foliaceus, pemphigus mucus-membrane pemphigoid, and pemphigus erythematosus), autoimmune polyendocrinopathies, Reiter's disease or syndrome, thermal injury due to an autoimmune condition, preeclampsia, an immune complex disorder such as immune complex nephritis, antibody-mediated nephritis, neuroinflammatory disorders, polyneuropathies, chronic neuropathy such as IgM polyneuropathies or IgM-mediated neuropathy, thrombocytopenia (as developed by myocardial infarction patients, for example), including thrombotic thrombocytopenic purpura (TTP), post-transfusion purpura (PTP), heparin-induced thrombocytopenia, and autoimmune or immune-mediated thrombocytopenia including, for example, idiopathic thrombocytopenic purpura (ITP) including chronic or acute ITP, scleritis such as idiopathic cerato-scleritis, episcleritis, autoimmune disease of the testis and ovary including autoimmune orchitis and oophoritis, primary hypothyroidism, hypoparathyroidism, autoimmune endocrine diseases including thyroiditis such as autoimmune thyroiditis, Hashimoto's disease, chronic thyroiditis (Hashimoto's thyroiditis), or subacute thyroiditis, autoimmune thyroid disease, idiopathic hypothyroidism, Grave's disease, polyglandular syndromes such as autoimmune polyglandular syndromes, for example, type I (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis or encephalomyelitis allergica and experimental allergic encephalomyelitis (EAE), myasthenia gravis such as thymoma-associated myasthenia gravis, cerebellar degeneration, neuromyotonia, opsoclonus or opsoclonus myoclonus syndrome (OMS), and sensory neuropathy, multifocal motor neuropathy, Sheehan's syndrome, autoimmune hepatitis, chronic hepatitis, lupoid hepatitis, giant-cell hepatitis, chronic active hepatitis or autoimmune chronic active hepatitis, pneumonitis such as lymphoid interstitial pneumonitis (LIP), bronchiolitis obliterans (non-transplant) vs NSIP, Guillain-Barré syndrome, Berger's disease (IgA nephropathy), idiopathic IgA nephropathy, linear IgA dermatosis, acute febrile neutrophilic dermatosis, subcorneal pustular dermatosis, transient acantholytic dermatosis, cirrhosis such as primary biliary cirrhosis and pneumonocirrhosis, autoimmune enteropathy syndrome, Celiac or Coeliac disease, celiac sprue (gluten enteropathy), refractory sprue, idiopathic sprue, cryoglobulinemia such as mixed cryoglobulinemia, amylotrophic lateral sclerosis (ALS; Lou Gehrig's disease), coronary artery disease, autoimmune ear disease such as autoimmune inner ear disease (AIED), autoimmune hearing loss, polychondritis such as refractory or relapsed or relapsing polychondritis, pulmonary alveolar proteinosis, Cogan's syndrome/nonsyphilitic interstitial keratitis, Bell's palsy, Sweet's disease/syndrome, rosacea autoimmune, zoster-associated pain, amyloidosis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal or segmental or focal segmental glomerulosclerosis (FSGS), endocrine ophthalmopathy, uveoretinitis, chorioretinitis, autoimmune hepatological disorder, fibromyalgia, multiple endocrine failure, Schmidt's syndrome, adrenalitis, gastric atrophy, presenile dementia, demyelinating diseases such as autoimmune demyelinating diseases and chronic inflammatory demyelinating polyneuropathy, Dressler's syndrome, alopecia areata, alopecia totalis, CREST syndrome (calcinosis, Raynaud's phenomenon, esophageal dysmotility, sclerodactyly, and telangiectasia), male and female autoimmune infertility, e.g., due to anti-spermatozoan antibodies, mixed connective tissue disease, Chagas' disease, rheumatic fever, recurrent abortion, farmer's lung, erythema multiforme, post-cardiotomy syndrome, Cushing's syndrome, bird-fancier's lung, allergic granulomatous angiitis, benign lymphocytic angiitis, Alport's syndrome, alveolitis such as allergic alveolitis and fibrosing alveolitis, interstitial lung disease, transfusion reaction, leprosy, malaria, parasitic diseases such as leishmaniasis, kypanosomiasis, schistosomiasis, ascariasis, aspergillosis, Sampter's syndrome, Caplan's syndrome, dengue, endocarditis, endomyocardial fibrosis, diffuse interstitial pulmonary fibrosis, interstitial lung fibrosis, fibrosing mediastinitis, pulmonary fibrosis, idiopathic pulmonary fibrosis, cystic fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, iridocyclitis (acute or chronic), or Fuch's cyclitis, Henoch-Schonlein purpura, human immunodeficiency virus (HIV) infection, SCID, acquired immune deficiency syndrome (AIDS), echovirus infection, sepsis (systemic inflammatory response syndrome (SIRS)), endotoxemia, pancreatitis, thyroxicosis, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, chorioiditis, giant-cell polymyalgia, chronic hypersensitivity pneumonitis, conjunctivitis, such as vernal catarrh, keratoconjunctivitis sicca, and epidemic keratoconjunctivitis, idiopathic nephritic syndrome, minimal change nephropathy, benign familial and ischemia-reperfusion injury, transplant organ reperfusion, retinal autoimmunity, joint inflammation, bronchitis, chronic obstructive airway/pulmonary disease, silicosis, aphthae, aphthous stomatitis, arteriosclerotic disorders (cerebral vascular insufficiency) such as arteriosclerotic encephalopathy and arteriosclerotic retinopathy, aspermiogenese, autoimmune hemolysis, Boeck's disease, cryoglobulinemia, Dupuytren's contracture, endophthalmia phacoanaphylactica, enteritis allergica, erythema nodosum leprosum, idiopathic facial paralysis, chronic fatigue syndrome, febris rheumatica, Hamman-Rich's disease, sensoneural hearing loss, haemoglobinuria paroxysmatica, hypogonadism, ileitis regionalis, leucopenia, mononucleosis infectiosa, traverse myelitis, primary idiopathic myxedema, nephrosis, ophthalmia symphatica, orchitis granulomatosa, pancreatitis, polyradiculitis acuta, pyoderma gangrenosum, Quervain's thyreoiditis, acquired spenic atrophy, non-malignant thymoma, lymphofollicular thymitis, vitiligo, toxic-shock syndrome, food poisoning, conditions involving infiltration of T cells, leukocyte-adhesion deficiency, immune responses associated with acute and delayed hypersensitivity mediated by cytokines and T-lymphocytes, diseases involving leukocyte diapedesis, multiple organ injury syndrome, antigen-antibody complex-mediated diseases, antiglomerular basement membrane disease, autoimmune polyendocrinopathies, oophoritis, primary myxedema, autoimmune atrophic gastritis, sympathetic ophthalmia, rheumatic diseases, mixed connective tissue disease, nephrotic syndrome, insulitis, polyendocrine failure, autoimmune polyglandular syndromes, including polyglandular syndrome type I, adult-onset idiopathic hypoparathyroidism (AOIH), cardiomyopathy such as dilated cardiomyopathy, epidermolisis bullosa acquisita (EBA), hemochromatosis, myocarditis, nephrotic syndrome, primary sclerosing cholangitis, purulent or nonpurulent sinusitis, acute or chronic sinusitis, ethmoid, frontal, maxillary, or sphenoid sinusitis, allergic sinusitis, an eosinophil-related disorder such as eosinophilia, pulmonary infiltration eosinophilia, eosinophilia-myalgia syndrome, Loffler's syndrome, chronic eosinophilic pneumonia, tropical pulmonary eosinophilia, bronchopneumonic aspergillosis, aspergilloma, or granulomas containing eosinophils, anaphylaxis, spondyloarthropathies, seronegative spondyloarthritides, polyendocrine autoimmune disease, sclerosing cholangitis, sclera, episclera, chronic mucocutaneous candidiasis, Bruton's syndrome, transient hypogammaglobulinemia of infancy, Wiskott-Aldrich syndrome, ataxia telangiectasia syndrome, angiectasis, autoimmune disorders associated with collagen disease, rheumatism such as chronic arthrorheumatism, lymphadenitis, reduction in blood pressure response, vascular dysfunction, tissue injury, cardiovascular ischemia, hyperalgesia, renal ischemia, cerebral ischemia, and disease accompanying vascularization, allergic hypersensitivity disorders, glomerulonephritides, reperfusion injury, ischemic re-perfusion disorder, reperfusion injury of myocardial or other tissues, lymphomatous tracheobronchitis, inflammatory dermatoses, dermatoses with acute inflammatory components, multiple organ failure, bullous diseases, renal cortical necrosis, acute purulent meningitis or other central nervous system inflammatory disorders, ocular and orbital inflammatory disorders, granulocyte transfusion-associated syndromes, cytokine-induced toxicity, narcolepsy, acute serious inflammation, chronic intractable inflammation, pyelitis, endarterial hyperplasia, peptic ulcer, valvulitis, and endometriosis.

The phrase "anxiety related disorders" refers to disorders of anxiety, mood, and substance abuse, including but not limited to: depression, generalized anxiety disorders, attention deficit disorder, sleep disorder, hyperactivity disorder, obsessive compulsive disorder, schizophrenia, cognitive disorders, hyperalgesia and sensory disorders. Such disorders include the mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

The term "lipid metabolic disorder" refers to abnormal clinical chemistry levels of cholesterol and triglycerides, wherein elevated levels of these lipids is an indication for atherosclerosis. Additionally, abnormal serum lipid levels may be an indication ofvarious cardiovascular diseases including hypertension, stroke, coronary artery diseases, diabetes and/or obesity.

The phrase "eye abnormality" refers to such potential disorders of the eye as they may be related to atherosclerosis or various ophthalmological abnormalities. Such disorders include but are not limited to the following: retinal dysplasia, various retinopathies, restenosis, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis. Cataracts are also considered an eye abnormality and are associated with such systemic diseases as: Human Down's syndrome, Hallerman-Streiff syndrome, Lowe syndrome, galactosemia, Marfan syndrome, Trismoy 13-15 condition, Alport syndrome, myotonic dystrophy, Fabry disease, hypothroidisms, or Conradi syndrome. Other ocular developmental anomalies include: Aniridia, anterior segment and dysgenesis syndrome. Cataracts may also occur as a result of an intraocular infection or inflammation (uveitis).

A "growth inhibitory amount" of an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195,anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PR0346 antibody, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PRO9859, PR09864, PR09904, PR09907, PRO10013, PRO90948, PR028694, PR016089, PRO19563, PRO19675, PR020084, PR021434, PR050332, PR038465 or PR0346 polypeptide, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding oligopeptide or PR0218, PR0228, PR0271, PR0273, PR0295, PR0302, PR0305, PR0326, PR0386, PR0655, PRO162, PRO788, PRO792, PRO940, PRO941, PRO1004, PRO1012, PRO1016, PRO474, PRO5238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding organic molecule is an amount capable of inhibiting the growth of a cell, especially tumor, e.g., cancer cell, either *in vitro* or *in vivo.* A "growth inhibitory amount" of an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PR0474, anti-PR05238, anti-PR01069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PR019563, anti-PR019675, anti-PR020084, anti-PR021434, anti-PR050332, anti-PR038465 or anti-PRO346 antibody, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR0523 8, PRO1069, PRO1111, PRO1113, PRO1130, PR01195, PRO1271, PRO1865, PRO1879, PR03446, PR03543, PRO4329, PR04352, PR05733, PR09859, PR09864, PR09904, PR09907, PRO10013, PRO90948, PR028694, PR016089, PRO19563, PRO19675, PR020084, PR021434, PR050332, PR038465 or PR0346 polypeptide, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding oligopeptide or PR0218, PR0228, PR0271, PR0273, PR0295, PR0302, PR0305, PR0326, PRO386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding organic molecule for purposes of inhibiting neoplastic cell growth may be determined empirically and in a routine manner.

A "cytotoxic amount" of an anti-PR0218, anti-PR0228, anti-PR0271, anti-PR0273, anti-PR0295, anti-PRO302, anti-PR0305, anti-PR0326, anti-PR0386, anti-PR0655, anti-PRO162, anti-PRO788, anti-PR0792, anti-PRO940, anti-PR0941, anti-PRO1004, anti-PRO1012, anti-PRO1016, anti-PR0474, anti-PR05238, anti-PR01069, anti-PRO1111, anti-PRO1113, anti-PRO1130, anti-PRO1195, anti-PR01271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PRO346 antibody, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PRO9864, PR09904, PR09907, PRO10013, PR090948, PR028694, PRO16089, PRO19563, PRO19675, PR020084, PR021434, PR050332, PR038465 or PR0346 polypeptide, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding oligopeptide or PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding organic molecule is an amount capable of causing the destruction of a cell, especially tumor, e.g., cancer cell, either *in vitro* or *in vivo.*

A "cytotoxic amount" of an anti-PR0218, anti-PR0228, anti-PR0271, anti-PR0273, anti-PR0295, anti-PRO302, anti-PR0305, anti-PR0326, anti-PR0386, anti-PR0655, anti-PRO162, anti-PRO788, anti-PR0792, anti-PRO940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PR0346 antibody, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PRO4352, PRO5733, PR09859, PR09864, PR09904, PR09907, PRO10013, PR090948, PR028694, PRO16089, PRO19563, PRO19675, PR020084, PR021434, PR050332, PR038465 or PR0346 polypeptide, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PR01271, PR01865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding oligopeptide or PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding organic molecule for purposes of inhibiting neoplastic cell growth may be determined empirically and in a routine manner.

The term "antibody" is used in the broadest sense and specifically covers, for example, single anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody monoclonal antibodies (including agonist, antagonist, and neutralizing antibodies), anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PR019563, anti-PRO19675, anti-PR020084, anti-PR021434, anti-PR050332, anti-PR038465 or anti-PR0346 antibody compositions with polyepitopic specificity, polyclonal antibodies, single chain anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PRO19563 , anti-PRO19675, anti-PR020084, anti-PR021434, anti-PR050332, anti-PR038465 or anti-PR0346 antibodies, and fragments of anti-PRO218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PR01069, anti-PRO1111, anti-PRO1113, anti-PRO1130, anti-PRO1195, anti-PR01271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PR0346 antibodies (see below) as long as they exhibit the desired biological or immunological activity. The term "immunoglobulin" (Ig) is used interchangeable with antibody herein.

An "isolated antibody" is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. The invention provides that the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The basic 4-chain antibody unit is a heterotetrameric glycoprotein composed of two identical light (L) chains and two identical heavy (H) chains (an IgM antibody consists of 5 of the basic heterotetramer unit along with an additional polypeptide called J chain, and therefore contain 10 antigen binding sites, while secreted IgA antibodies can polymerize to form polyvalent assemblages comprising 2-5 of the basic 4-chain units along with J chain). In the case of IgGs, the 4-chain unit is generally about 150,000 daltons. Each L chain is linked to a H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (V_{H}) followed by three constant domains (C_{H}) for each of the α and γ chains and four C_{H} domains for µ and ∈ isotypes. Each L chain has at the N-terminus, a variable domain (V_{L}) followed by a constant domain (C_{L}) at its other end. The V_{L} is aligned with the V_{H} and the C_{L} is aligned with the first constant domain of the heavy chain (C_{H} 1). Particular amino acid residues are believed to form an interface between the light chain and heavy chain variable domains. The pairing of a V_{H} and V_{L} together forms a single antigen-binding site. For the structure and properties of the different classes of antibodies, see, e.g., Basic and Clinical Immunology, 8th edition, Daniel P. Stites, Abba I. Terr and Tristram G. Parslow (eds.), Appleton & Lange, Norwalk, CT, 1994, page 71 and Chapter 6.

The L chain from any vertebrate species can be assigned to one of two clearly distinct types, called kappa and lambda, based on the amino acid sequences of their constant domains. Depending on the amino acid sequence of the constant domain of their heavy chains (C_{H}), immunoglobulins can be assigned to different classes or isotypes. There are five classes of immunoglobulins: IgA, IgD, IgE, IgG, and IgM, having heavy chains designated α, δ, ∈, γ, and µ, respectively. The γ and α classes are further divided into subclasses on the basis of relatively minor differences in C_{H} sequence and function, e.g., humans express the following subclasses: IgG1, IgG2, IgG3, IgG4, IgA1, and IgA2.

The term "variable" refers to the fact that certain segments of the variable domains differ extensively in sequence among antibodies. The V domain mediates antigen binding and define specificity of a particular antibody for its particular antigen. However, the variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains ofnative heavy and light chains each comprise four FRs, largely adopting a P -sheet configuration, connected by three hypervariable regions, which form loops connecting, and in some cases forming part of, the P -sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (e.g. around about residues 24-34 (L1), 50-56 (L2) and 89-97 (L3) in the V_{L}, and around about 1-35 (H1), 50-65 (H2) and 95-102 (H3) in the V_{H}; Kabat et al., Sequences of Proteins of Immunological Interest, 5th Ed. Public Health Service, National Institutes of Health, Bethesda, MD. (1991)) and/or those residues from a "hypervariable loop" (e.g. residues 26-32 (L1), 50-52 (L2) and 91-96 (L3) in the V_{L}, and 26-32 (H1), 53-55 (H2) and 96-101 (H3) in the V_{H}; Chothia and Lesk J. Mol. Biol. 196:901-917 (1987)).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, the monoclonal antibodies useful in the present invention may be prepared by the hybridoma methodology first described by Kohler et al., Nature, 256:495 (1975), or may be made using recombinant DNA methods in bacterial, eukaryotic animal or plant cells (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see U.S. Patent No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (e.g. Old World Monkey, Ape etc), and human constant region sequences.

An "intact" antibody is one which comprises an antigen-binding site as well as a C_{L} and at least heavy chain constant domains, C_{H} 1, C_{H} 2 and C_{H} 3. The constant domains may be native sequence constant domains (e.g. human native sequence constant domains) or amino acid sequence variant thereof. Preferably, the intact antibody has one or more effector functions.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (see U.S. Patent No. 5,641,870, Example 2; Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (C_{H} 1). Each Fab fragment is monovalent with respect to antigen binding, i.e., it has a single antigen-binding site. Pepsin treatment of an antibody yields a single large F(ab')₂ fragment which roughly corresponds to two disulfide linked Fab fragments having divalent antigen-binding activity and is still capable of cross-linking antigen. Fab' fragments differ from Fab fragments by having additional few residues at the carboxy terminus of the C_{H} 1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, which region is also the part recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to small antibody fragments prepared by constructing sFv fragments (see preceding paragraph) with short linkers (about 5-10 residues) between the V_{H} and V_{L} domains such that inter-chain but not intra-chain pairing of the V domains is achieved, resulting in a bivalent fragment, i.e., fragment having two antigen-binding sites. Bispecific diabodies are heterodimers of two "crossover" sFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms of non-human (e.g., rodent) antibodies are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "species-dependent antibody," e.g., a mammalian anti-human IgE antibody, is an antibody which has a stronger binding affinity for an antigen from a first mammalian species than it has for a homologue of that antigen from a second mammalian species. Normally, the species-dependent antibody "bind specifically" to a human antigen (i.e., has a binding affinity (Kd) value of no more than about 1 x 10⁻⁷ M, preferably no more than about 1 x 10⁻⁸ and most preferably no more than about 1 x 10⁻⁹ M) but has a binding affinity for a homologue of the antigen from a second non-human mammalian species which is at least about 50 fold, or at least about 500 fold, or at least about 1000 fold, weaker than its binding affinity for the human antigen. The species-dependent antibody can be of any of the various types of antibodies as defined above, but preferably is a humanized or human antibody.

A "PR0218, PR0228, PR0271, PR0273, PR0295, PR0302, PR0305, PR0326, PR0386, PR0655, PRO162, PR0788, PRO792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding oligopeptide" is an oligopeptide that binds, preferably specifically, to a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide as described herein. PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding oligopeptides may be chemically synthesized using known oligopeptide synthesis methodology or may be prepared and purified using recombinant technology. PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PRO50332, PR038465 or PR0346 binding oligopeptides usually are or are at least about 5 amino acids in length, alternatively are or are at least about 6, 7, 8, 9, 10, 11, 12, 13, 14, 15, 16, 17, 18, 19, 20, 21,22,23,24,25,26,27,28,29,30,31,32,33,34,35,36,37,38,39,40,41,42,43,44,45,46,47,48,49,50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such oligopeptides that are capable of binding, preferably specifically, to a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide as described herein. PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PR01865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871, 4,833,092, 5,223,409, 5,403,484,5,571,689,5,663,143; PCT PublicationNos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

A "PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PRO9859, PR09864, PR09904, PR09907, PRO10013, PR090948, PR028694, PRO16089, PRO19563, PRO19675, PR020084, PR021434, PR050332, PR038465 or PR0346 binding organic molecule" is an organic molecule other than an oligopeptide or antibody as defined herein that binds, preferably specifically, to a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide as described herein. PR0218, PR0228, PR0271, PR0273, PR0295, PR0302, PR0305, PRO326, PRO386, PRO655, PRO162, PRO788, PRO792, PR0940, PR0941, PRO1004, PRO1012, PRO1016, PR0474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PR019675, PR020084, PR021434, PR050332, PR038465 or PR0346 binding organic molecules may be identified and chemically synthesized using known methodology (see, e.g., PCT Publication Nos. WO00/00823 and WO00/39585). PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 binding organic molecules are usually less than about 2000 daltons in size, alternatively less than about 1500, 750, 500, 250 or 200 daltons in size, wherein such organic molecules that are capable of binding, preferably specifically, to a PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PR016089, PRO19563, PRO19675, PR020084, PR021434, PR050332, PR038465 or PR0346 polypeptide as described herein may be identified without undue experimentation using well known techniques. In this regard, it is noted that techniques for screening organic molecule libraries for molecules that are capable of binding to a polypeptide target are well known in the art (see, e.g., PCT Publication Nos. WO00/00823 and WO00/39585).

An antibody, oligopeptide or other organic molecule "which binds" an antigen of interest, e.g. a tumor-associated polypeptide antigen target, is one that binds the antigen with sufficient affinity such that the antibody, oligopeptide or other organic molecule is preferably useful as a diagnostic and/or therapeutic agent in targeting a cell or tissue expressing the antigen, and does not significantly cross-react with other proteins. The extent of binding of the antibody, oligopeptide or other organic molecule to a "non-target" protein will be less than about 10% of the binding of the antibody, oligopeptide or other organic molecule to its particular target protein as determined by fluorescence activated cell sorting (FACS) analysis or radioimmunoprecipitation (RIA). With regard to the binding of an antibody, oligopeptide or other organic molecule to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of at least about 10⁻⁴ M, alternatively at least about 10⁻⁵ M, alternatively at least about 10⁻⁶ M, alternatively at least about 10⁻⁷ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M, or greater. The term "specific binding" refers to binding where a molecule binds to a particular polypeptide or epitope on a particular polypeptide without substantially binding to any other polypeptide or polypeptide epitope.

An antibody, oligopeptide or other organic molecule that "inhibits the growth of tumor cells expressing a "PR0218, PR0228, PR0271, PR0273, PR0295, PR0302, PR0305, PR0326, PR0386, PR0655, PRO162, PR0788, PR0792, PR0940, PR0941, PRO1004, PR01012, PRO1016, PR0474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346" or a "growth inhibitory" antibody, oligopeptide or other organic molecule is one which results in measurable growth inhibition of cancer cells expressing or overexpressing the appropriate PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide may be a transmembrane polypeptide expressed on the surface of a cancer cell or may be a polypeptide that is produced and secreted by a cancer cell. Preferred growth inhibitory anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PRO788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PR01195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibodies, oligopeptides or organic molecules inhibit growth of PR0218-, PR0228-, PR0271-, PR0273-, PR0295-, PR0302-, PR0305-, PR0326-, PR0386-, PR0655-, PRO162-, PR0788-, PR0792-, PR0940-, PR0941-, PRO1004-, PRO1012-, PRO1016-, PR0474-, PR05238-, PRO1069-, PRO1111-, PRO1113-, PR01130-, PRO1195-, PRO1271-, PRO1865-, PRO1879-, PR03446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PR0346-expressing tumor cells by or by greater than 20%, preferably from about 20% to about 50%, and even more preferably, by or by greater than 50% (e.g., from about 50% to about 100%) as compared to the appropriate control, the control typically being tumor cells not treated with the antibody, oligopeptide or other organic molecule being tested. Growth inhibition can be measured at an antibody concentration of about 0.1 to 30 µg/ml or about 0.5 nM to 200 nM in cell culture, where the growth inhibition is determined 1-10 days after exposure of the tumor cells to the antibody. Growth inhibition of tumor cells *in vivo* can be determined in various ways. The antibody is growth inhibitory in *vivo* if administration of the anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PR01004, anti-PR01012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PR010013, anti-PR090948, anti-PR028694, anti-PRO16089, anti-PRO19563, anti-PRO19675, anti-PR020084, anti-PR021434, anti-PR050332, anti-PR038465 or anti-PR0346 antibody at about 1 µg/kg to about 100 mg/kg body weight results in reduction in tumor size or tumor cell proliferation within about 5 days to 3 months from the first administration of the antibody, preferably within about 5 to 30 days.

An antibody, oligopeptide or other organic molecule which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell is usually one which overexpresses a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. Preferably the cell is a tumor cell, e.g., a prostate, breast, ovarian, stomach, endometrial, lung, kidney, colon, bladder cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody, oligopeptide or other organic molecule which induces apoptosis is one which results in or in about 2 to 50 fold, preferably in or in about 5 to 50 fold, and most preferably in or in about 10 to 50 fold, induction of annexin binding relative to untreated cell in an annexin binding assay.

Antibody "effector functions" refer to those biological activities attributable to the Fc region (a native sequence Fc region or amino acid sequence variant Fc region) of an antibody, and vary with the antibody isotype. Examples of antibody effector functions include: C 1q binding and complement dependent cytotoxicity; Fc receptor binding; antibody-dependent cell-mediated cytotoxicity (ADCC); phagocytosis; down regulation of cell surface receptors (e.g., B cell receptor); and B cell activation.

"Antibody-dependent cell-mediated cytotoxicity" or "ADCC" refers to a form of cytotoxicity in which secreted Ig bound onto Fc receptors (FcRs) present on certain cytotoxic cells (e.g., Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are absolutely required for such killing. The primary cells for mediating ADCC, NK cells, express FcγRIII only, whereas monocytes express FcyRI, FcγRII and FcγRIII. FcR expression on hematopoietic cells is summarized in Table 3 on page 464 of Ravetch and Kinet, Annu. Rev. Immunol. 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo,* e.g., in a animal model such as that disclosed in Clynes et al.Proc. Natl. Acad. Sci. U.S.A. 95:652-656 (1998).

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see review M. in Daëron, Annu. Rev. Immunol. 15:203-234 (1997)). FcRs are reviewed in Ravetch and Kinet, Annu. Rev. Immunol. 9:457-492 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

"Human effector cells" are leukocytes which express one or more FcRs and perform effector functions. Preferably, the cells express at least FcγRIII and perform ADCC effector function. Examples ofhuman leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source, e.g., from blood.

"Complement dependent cytotoxicity" or "CDC" refers to the lysis of a target cell in the presence of complement. Activation of the classical complement pathway is initiated by the binding of the first component of the complement system (Clq) to antibodies (of the appropriate subclass) which are bound to their cognate antigen. To assess complement activation, a CDC assay, e.g., as described in Gazzano-Santoro et al., J. Immunol. Methods 202:163 (1996), may be performed.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma ofthe lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD). Preferably, the cancer comprises a tumor that expresses an IGF receptor, more preferably breast cancer, lung cancer, colorectal cancer, or prostate cancer, and most preferably breast or prostate cancer.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol- Myers Squibb Oncology, Princeton, N.J.), ABRAXANE^{™} Cremophor-free, albumin-engineered nanoparticle formulation of paclitaxel (American Pharmaceutical Partners, Schaumberg, Illinois), and TAXOTERE® doxetaxel (Rhône- Poulenc Rorer, Antony, France); chloranbucil; GEMZAR® gemcitabine; 6- thioguanine; mercaptopurine; methotrexate; platinum analogs such as cisplatin and carboplatin; vinblastine; platinum; etoposide (VP-16); ifosfamide; mitoxantrone; vincristine; NAVELBINE® vinorelbine; novantrone; teniposide; edatrexate; daunomycin; aminopterin; xeloda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.

Also included in this definition are anti-hormonal agents that act to regulate or inhibit hormone action on tumors such as anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Ralf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELIX® rmRH; and pharmaceutically acceptable salts, acids or derivatives of any of the above.

The terms "cell proliferative disorder" and "proliferative disorder" refer to disorders that are associated with some degree of abnormal cell proliferation. In one aspect of the invention, the cell proliferative disorder is cancer.

"Tumor", as used herein, refers to all neoplastic cell growth and proliferation, whether malignant or benign, and all pre-cancerous and cancerous cells and tissues.

An antibody, oligopeptide or other organic molecule which "induces cell death" is one which causes a viable cell to become nonviable. The cell is one which expresses a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, preferably a cell that overexpresses a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PR019563, PR019675, PR020084, PR021434, PR050332, PR038465 or PR0346 polypeptide as compared to a normal cell of the same tissue type. The PR0218, PR0228, PR0271, PR0273, PR0295, PR0302, PR0305, PR0326, PRO386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide may be a transmembrane polypeptide expressed on the surface of a cancer cell or may be a polypeptide that is produced and secreted by a cancer cell. Preferably, the cell is a cancer cell, e.g., a breast, ovarian, stomach, endometrial, salivary gland, lung, kidney, colon, thyroid, pancreatic or bladder cell. Cell death *in vitro* may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (i.e., in the absence of complement) and in the absence of immune effector cells. To determine whether the antibody, oligopeptide or other organic molecule is able to induce cell death, loss of membrane integrity as evaluated by uptake ofpropidium iodide (PI), trypan blue (see Moore et al. Cytotechnology 17:1-11 (1995)) or 7AAD can be assessed relative to untreated cells. Preferred cell death-inducing antibodies, oligopeptides or other organic molecules are those which induce PI uptake in the PI uptake assay in BT474 cells.

As used herein, the term "immunoadhesion" designates antibody-like molecules which combine the binding specificity of a heterologous protein (an "adhesion") with the effector functions of immunoglobulin constant domains. Structurally, the immunoadhesions comprise a fusion of an amino acid sequence with the desired binding specificity which is other than the antigen recognition and binding site of an antibody (i.e., is "heterologous"), and an immunoglobulin constant domain sequence. The adhesion part of an immunoadhesion molecule typically is a contiguous amino acid sequence comprising at least the binding site of a receptor or a ligand. The immunoglobulin constant domain sequence in the immunoadhesion may be obtained from any immunoglobulin, such as IgG-1, IgG-2, IgG-3, or IgG-4 subtypes, IgA (including IgA-1 and IgA-2), IgE, IgD or IgM.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

"Replication-preventing agent" is an agent wherein replication, function, and/or growth of the cells is inhibited or prevented, or cells are destroyed, no matter what the mechanism, such as by apoptosis, angiostasis, cytosis, tumoricide, mytosis inhibition, blocking cell cycle progression, arresting cell growth, binding to tumors, acting as cellular mediators, etc. Such agents include a chemotherapeutic agent, cytotoxic agent, cytokine, growth-inhibitory agent, or anti-hormonal agent, e.g., an anti-estrogen compound such as tamoxifen, an anti-progesterone such as onapristone (see, EP 616 812); or an anti-androgen such as flutamide, as well as aromidase inhibitors, or a hormonal agent such as an androgen.

The term "cytotoxic agent" as used herein refers to a substance that inhibits or prevents the function of cells and/or causes destruction of cells. The term is intended to include radioactive isotopes (e.g., At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

Preferred cytotoxic agents herein for the specific tumor types to use in combination with the antagonists herein are as follows:
1. Prostate cancer: androgens, docetaxel, paclitaxel, estramustine, doxorubicin, mitoxantrone, antibodies to ErbB2 domain(s) such as 2C4 (WO 01/00245; hybridoma ATCC HB-12697), which binds to a region in the extracellular domain of ErbB2 (e.g., any one or more residues in the region from about residue 22 to about residue 584 of ErbB2, inclusive), AVASTIN^{™} anti-vascular endothelial growth factor (VEGF), TARCEVA^{™} OSI-774 (erlotinib) (Genenetech and OSI Pharmaceuticals), or other epidermal growth factor receptor tyrosine kinase inhibitors (EGFR TKI's).
2. Stomach cancer: 5-fluorouracil (5FU), XELODA^{™} capecitabine, methotrexate, etoposide, cisplatin/carboplatin, pacliitaxel, docetaxel, gemcitabine, doxorubicin, and CPT-11 (camptothcin-11; irinotecan, USA Brand Name: CAMPTOSAR^{®}).
3. Pancreatic cancer: gemcitabine, 5FU, XELODA^{™} capecitabine, CPT-11, docetaxel, paclitaxel, cisplatin, carboplatin, TARCEVA^{™} erlotinib, and other EGFR TKI's.
4. Colorectal cancer: 5FU, XELODA^{™} capecitabine, CPT-11, oxaliplatin, AVASTIN^{™} anti-VEGF, TARCEVA^{™} erlotinib and other EGFR TKI's, and ERBITUX™ (formerly known as IMC-C225) human:murine-chimerized monoclonal antibody that binds to EGFR and blocks the ability of EGF to initiate receptor activation and signaling to the tumor.
5. Renal cancer: IL-2, interferon alpha, AVASTIN^{™} anti-VEGF, MEGACE^{™} (Megestrol acetate) progestin, vinblastine, TARCEVA^{™} erlotinib, and other EGFR TKI's.

A "growth inhibitory agent" when used herein refers to a compound or composition which inhibits growth of a cell, especially a PR0218-, PR0228-, PR0271-, PR0273-, PR0295-, PR0302-, PR0305-, PR0326-, PR0386-, PR0655-, PRO162-, PR0788-, PR0792-, PR0940-, PR0941-, PRO1004-, PRO1012-, PRO1016-, PR0474-, PR05238-, PRO1069-, PRO1111-, PRO1113-, PRO1130-, PRO1195-, PRO1271-, PRO1865-, PRO1879-, PR03446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PR0346-expressing cancer cell, either *in vitro* or *in vivo.* Thus, the growth inhibitory agent may be one which significantly reduces the percentage of PR0218-, PR0228-, PR0271-, PR0273-, PR0295-, PR0302-, PR0305-, PR0326-, PR0386-, PR0655-, PRO162-, PR0788-, PR0792-, PR0940-, PR0941-, PRO1004-, PRO1012-, PRO1016-, PRO474-, PRO5238-, PRO1069-, PRO1111-, PRO1113-, PRO1130-, PRO1195-, PRO1271-, PRO1865-, PRO1879-, PR03446-, PR03543-, PR04329-, PR04352-, PRO5733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PR0346-expressing cells in S phase. Examples of growth inhibitory agents include agents that block cell cycle progression (at a place other than S phase), such as agents that induce G 1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

"Doxorubicin" is an anthracycline antibiotic. The full chemical name ofdoxorubicin is (8S-cis)-10-[(3-amino-2,3,6-trideoxy-α-L-lyxo-hexapyranosyl)oxy]-7,8,9,10-tetrahydro-6,8,11-tribydroxy-8-(hydroxyacetyl)-1-methoxy-5,12-naphthacenedione.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon -α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL- 1a, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "package insert" is used to refer to instructions customarily included in commercial packages of therapeutic products, that contain information about the indications, usage, dosage, administration, contraindications and/or warnings concerning the use of such therapeutic products.

The term "gene" refers to (a) a gene containing at least one of the DNA sequences disclosed herein; (b) any DNA sequence that encodes the amino acid sequence encoded by the DNA sequences disclosed herein and/or; ©) any DNA sequence that hybridizes to the complement of the coding sequences disclosed herein. Preferably, the term includes coding as well as noncoding regions, and preferably includes all sequences necessary for normal gene expression.

The term "gene targeting" refers to a type of homologous recombination that occurs when a fragment of genomic DNA is introduced into a mammalian cell and that fragment locates and recombines with endogenous homologous sequences. Gene targeting by homologous recombination employs recombinant DNA technologies to replace specific genomic sequences with exogenous DNA of particular design.

The term "homologous recombination" refers to the exchange of DNA fragments between two DNA molecules or chromatids at the site of homologous nucleotide sequences.

The term "target gene" (alternatively referred to as "target gene sequence" or "target DNA sequence") refers to any nucleic acid molecule, polynucleotide, or gene to be modified by homologous recombination. The target sequence includes an intact gene, an exon or intron, a regulatory sequence or any region between genes. The target gene my comprise a portion of a particular gene or genetic locus in the individual's genomic DNA.

"Disruption" of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene occurs when a fragment of genomic DNA locates and recombines with an endogenous homologous sequence wherein the disruption is a deletion of the native gene or a portion thereof, or a mutation in the native gene or wherein the disruption is the functional inactivation of the native gene. Alternatively, sequence disruptions may be generated by nonspecific insertional inactivation using a gene trap vector (i.e. non-human transgenic animals containing and expressing a randomly inserted transgene; *see* for example U.S. Pat. No. 6,436,707 issued August 20, 2002). These sequence disruptions or modifications may include insertions, missense, frameshift, deletion, or substitutions, or replacements of DNA sequence, or any combination thereof. Insertions include the insertion of entire genes, which may be of animal, plant, fungal, insect, prokaryotic, or viral origin. Disruption, for example, can alter the normal gene product by inhibiting its production partially or completely or by enhancing the normal gene product's activity. Preferably, the disruption is a null disruption, wherein there is no significant expression of the PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene.

The term "native expression" refers to the expression of the full-length polypeptide encoded by the PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene, at expression levels present in the wild-type mouse. Thus, a disruption in which there is "no native expression" of the endogenous PR0218, PR0228, PR0271, PR0273, PR0295, PR0302, PR0305, PR0326, PR0386, PR0655, PRO162, PRO788, PRO792, PRO940, PRO941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene refers to a partial or complete reduction of the expression of at least a portion of a polypeptide encoded by an endogenous PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PR01113, PRO1130, PRO1195, PR01271, PRO1865, PRO1879, PR03446, PR03543, PR04329, PR04352, PR05733, PR09859, PR09864, PR09904, PR09907, PRO10013, PR090948, PR028694, PRO16089, PR019563, PRO19675, PR020084, PR021434, PR050332, PR038465 or PR0346 gene of a single cell, selected cells, or all of the cells of a mammal.

The term "knockout" refers to the disruption of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 gene wherein the disruption results in: the functional inactivation of the native gene; the deletion of the native gene or a portion thereof; or a mutation in the native gene.

The term "knock-in" refers to the replacement of the mouse ortholog (or other mouse gene) with a human cDNA encoding any of the specific human PR0218-, PR0228-, PR0271-, PR0273-, PR0295-, PR0302-, PR0305-, PR0326-, PR0386-, PR0655-, PRO162-, PR0788-, PR0792-, PR0940-, PR0941-, PRO1004-, PRO1012-, PRO1016-, PR0474-, PR05238-, PRO1069-, PRO1111-, PRO1113-, PRO1130-, PRO1195-, PRO1271-, PRO1865-, PRO1879-, PR03446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PRO346-encoding genes or variants thereof (ie. the disruption results in a replacement of a native mouse gene with a native human gene).

The term "construct" or "targeting construct" refers to an artificially assembled DNA segment to be transferred into a target tissue, cell line or animal. Typically, the targeting construct will include a gene or a nucleic acid sequence of particular interest, a marker gene and appropriate control sequences. As provided herein, the targeting construct comprises a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 targeting construct. A "PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 targeting construct" includes a DNA sequence homologous to at least one portion of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene and is capable of producing a disruption in a PR0218, PR0228, PR0271, PR0273, PR0295, PR0302, PR0305, PR0326, PR0386, PR0655, PRO162, PR0788, PR0792, PRO940, PRO941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene in a host cell.

The term "transgenic cell" refers to a cell containing within its genome a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene that has been disrupted, modified, altered, or replaced completely or partially by the method of gene targeting.

The term "transgenic animal" refers to an animal that contains within its genome a specific gene that has been disrupted or otherwise modified or mutated by the methods described herein or methods otherwise well known in the art. Preferably the non-human transgenic animal is a mammal. More preferably, the mammal is a rodent such as a rat or mouse. In addition, a "transgenic animal" may be a heterozygous animal (i.e., one defective allele and one wild-type allele) or a homozygous animal (i.e., two defective alleles). An embryo is considered to fall within the definition of an animal. The provision of an animal includes the provision of an embryo or foetus *in utero,* whether by mating or otherwise, and whether or not the embryo goes to term.

As used herein, the terms "selective marker" and position selection marker" refer to a gene encoding a product that enables only the cells that carry the gene to survive and/or grow under certain conditions. For example, plant and animal cells that express the introduced neomycin resistance (Neo^{r}) gene are resistant to the compound G418. Cells that do not carry the Neo^{r} gene marker are killed by G418. Other positive selection markers are known to, or are within the purview of, those of ordinary skill in the art.

The term "modulates" or "modulation" as used herein refers to the decrease, inhibition, reduction, amelioration, increase or enhancement of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene function, expression, activity, or alternatively a phenotype associated with PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene.

The term "ameliorates" or "amelioration" as used herein refers to a decrease, reduction or elimination of a condition, disease, disorder, or phenotype, including an abnormality or symptom.

The term "abnormality" refers to any disease, disorder, condition, or phenotype in which PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 is implicated, including pathological conditions and behavioral observations.

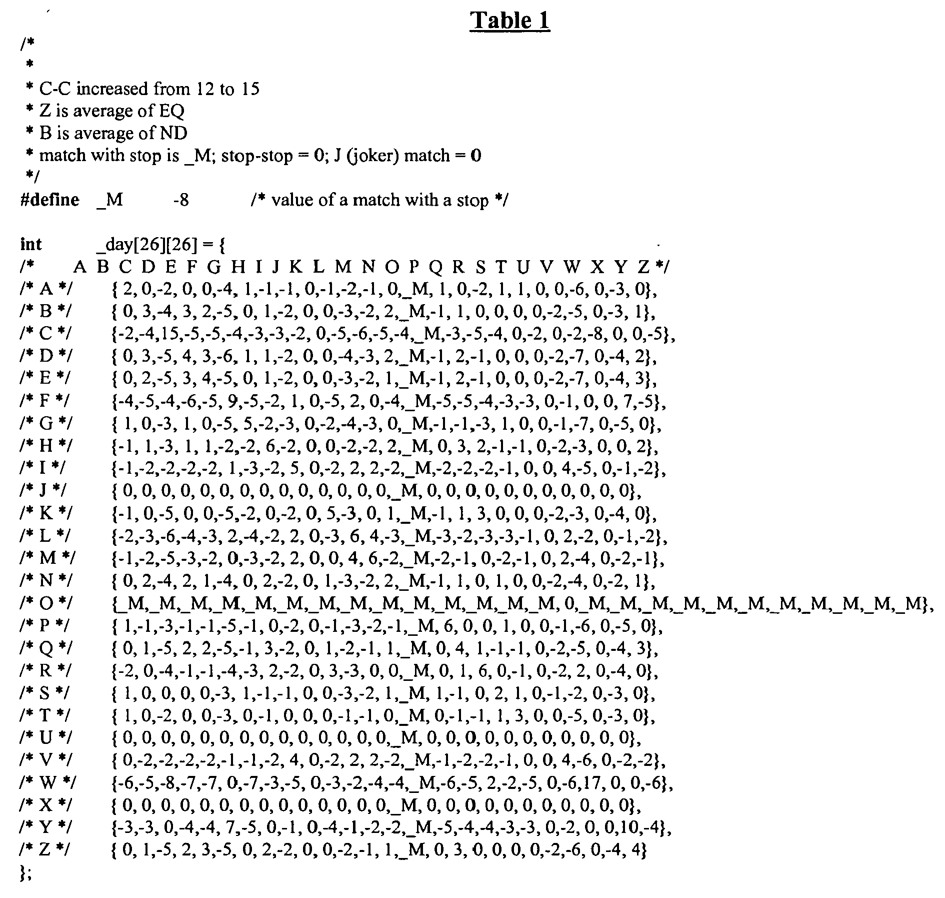

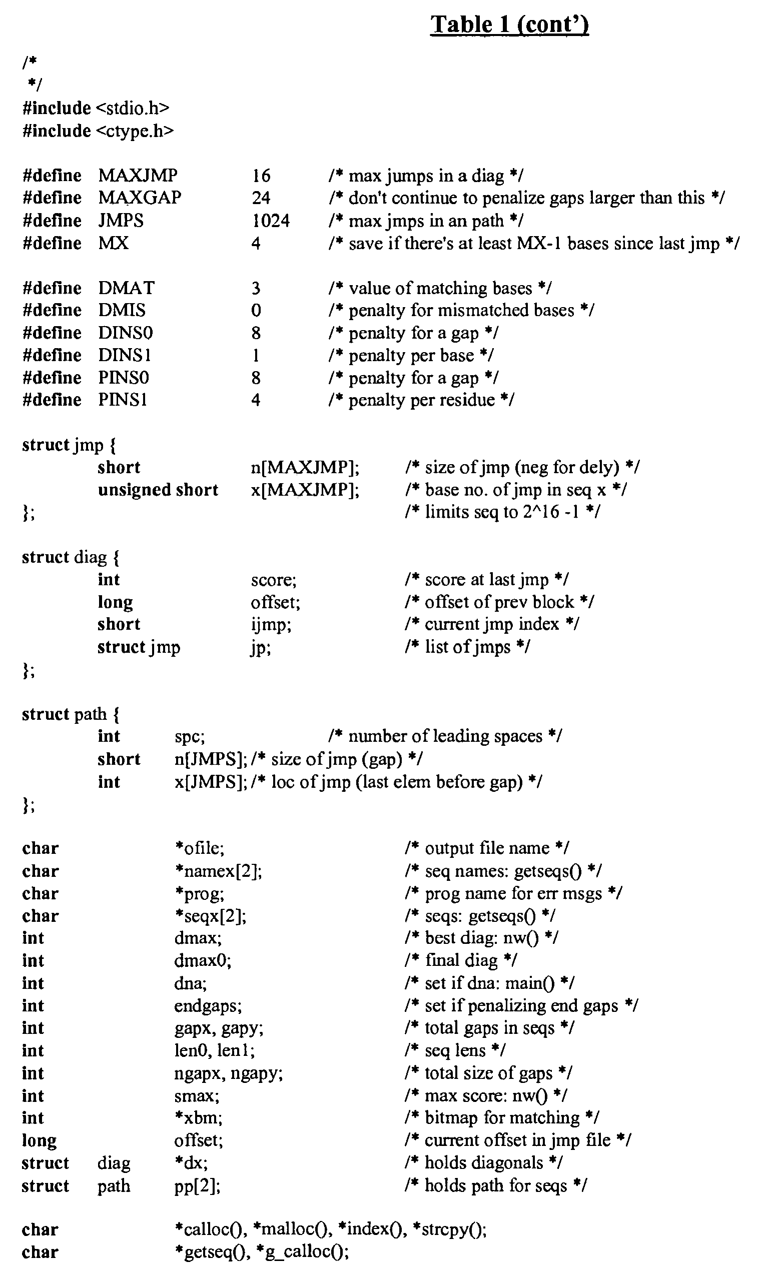

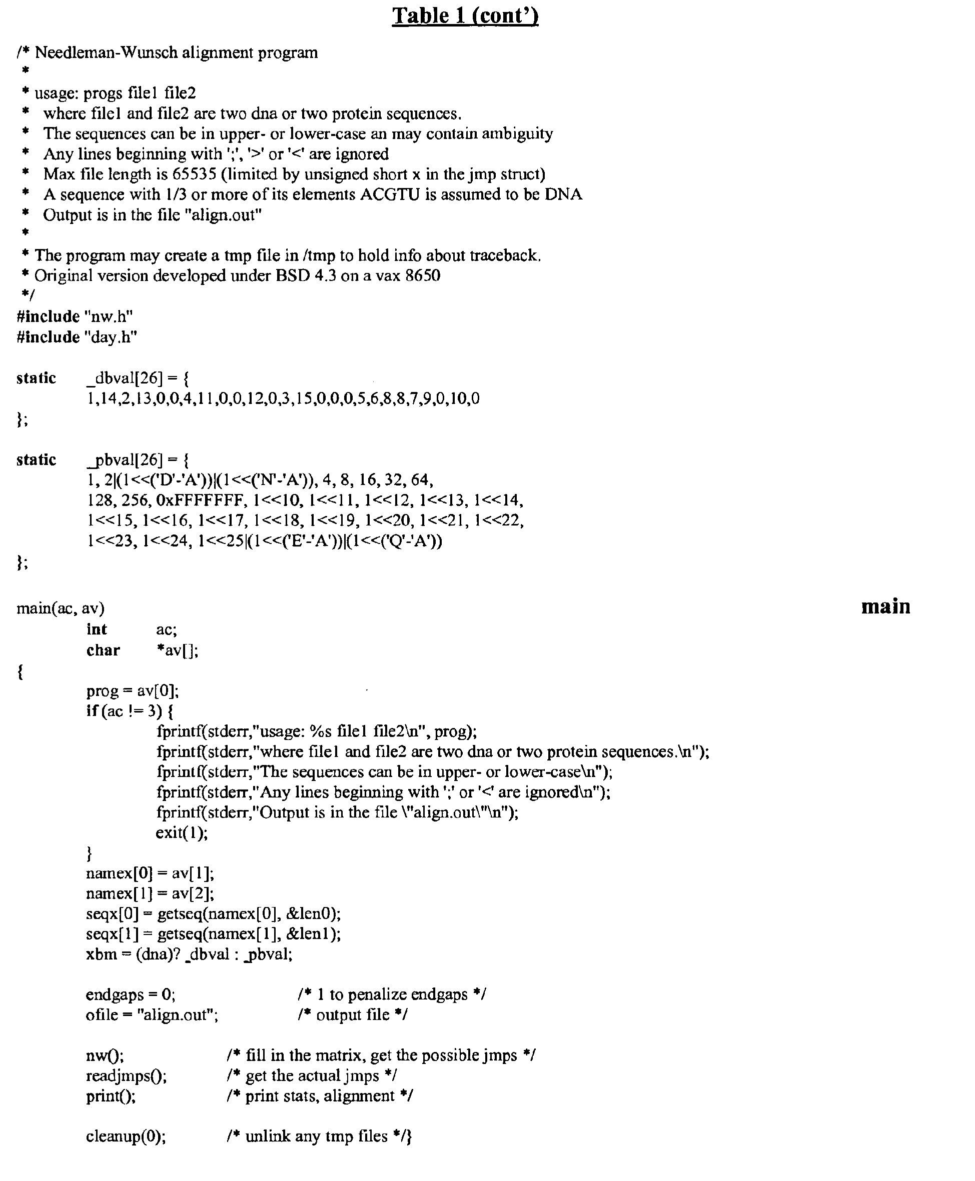

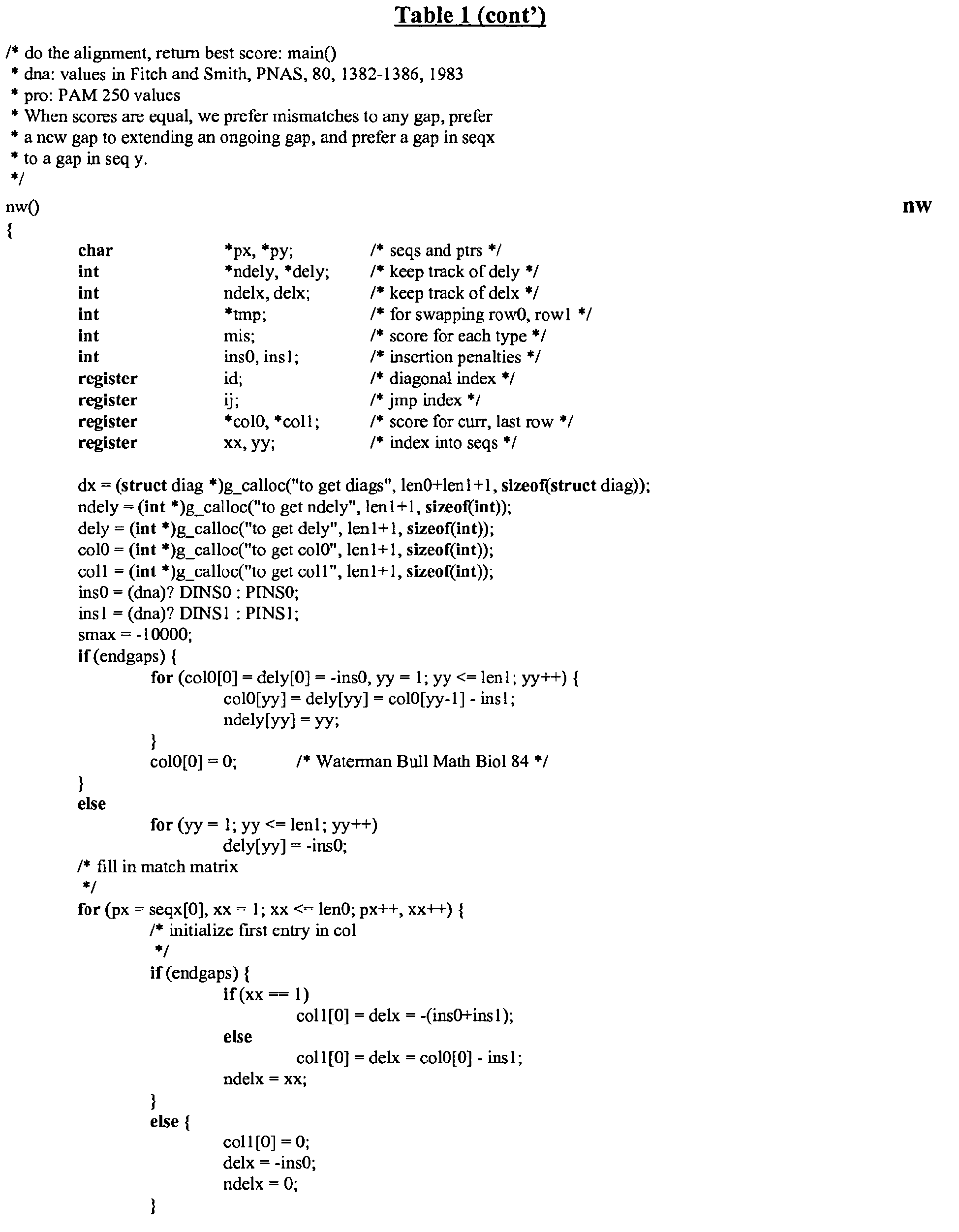

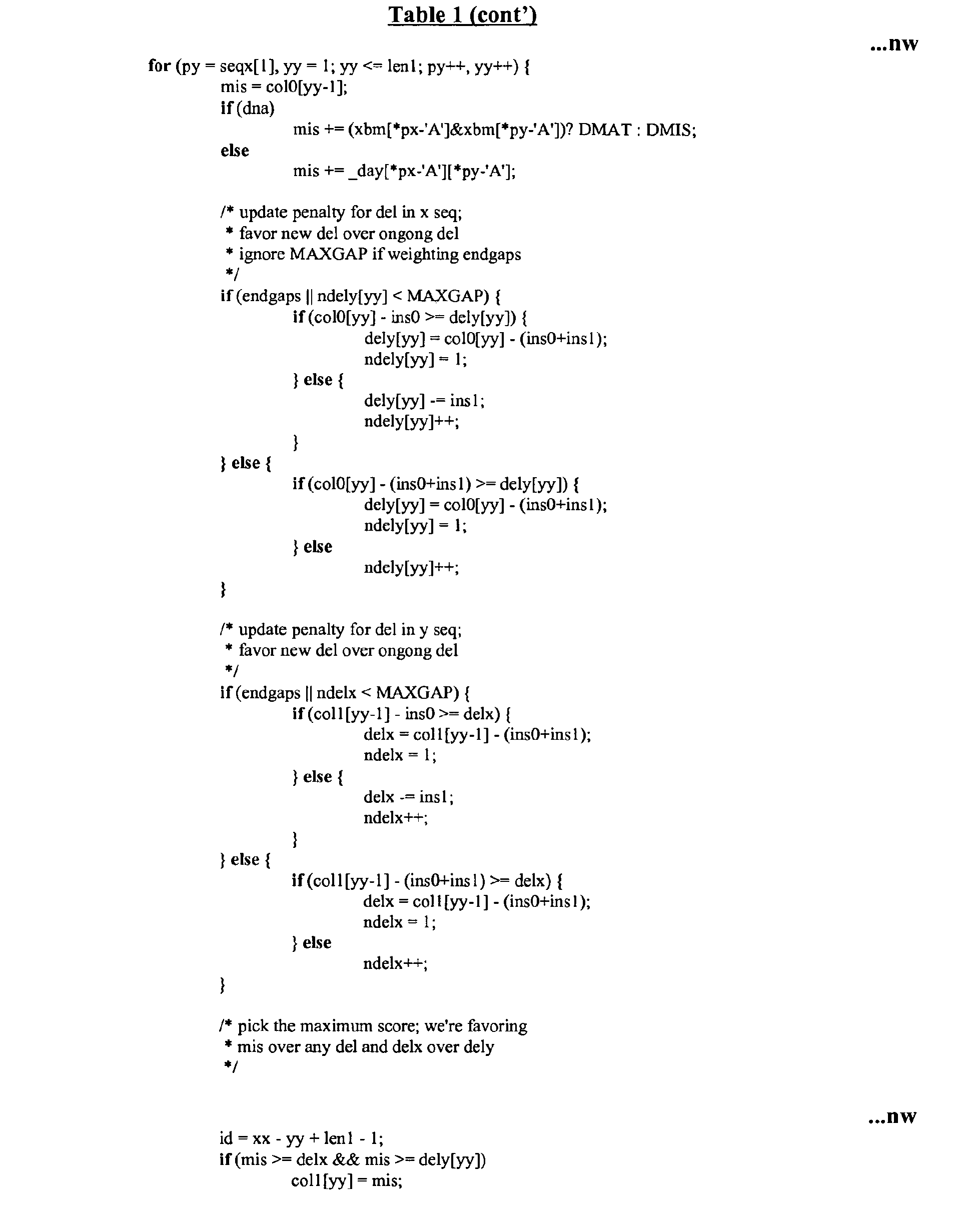

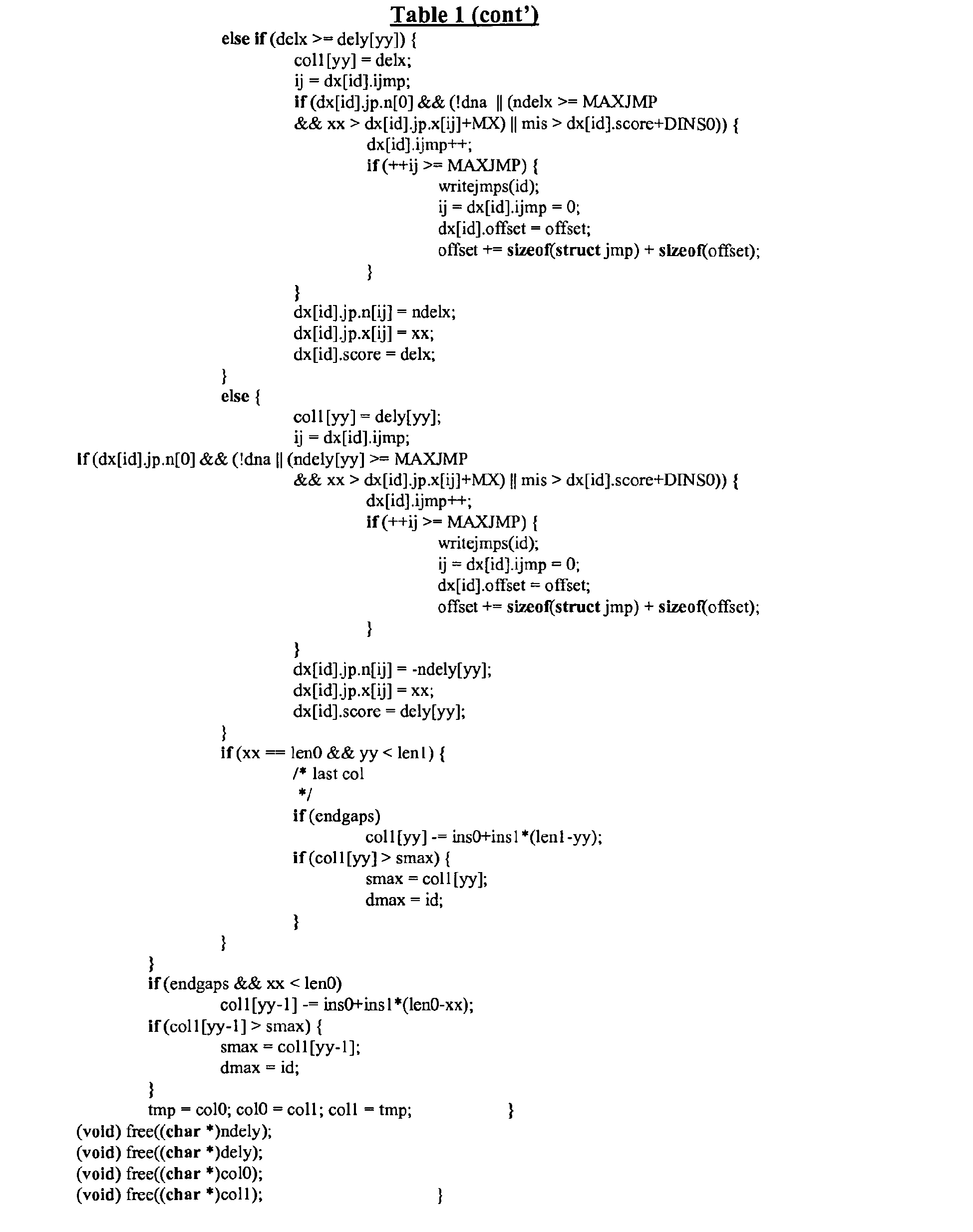

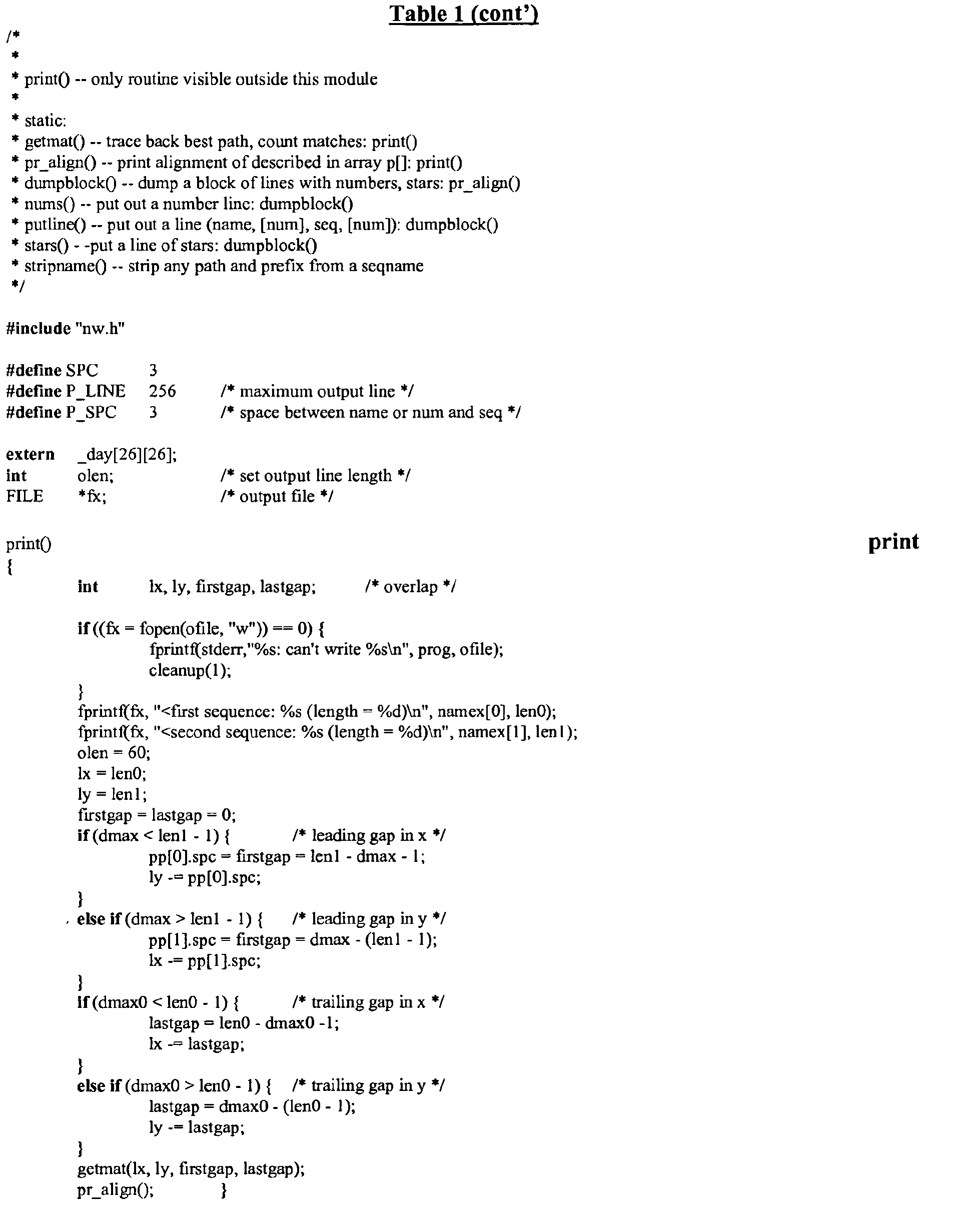

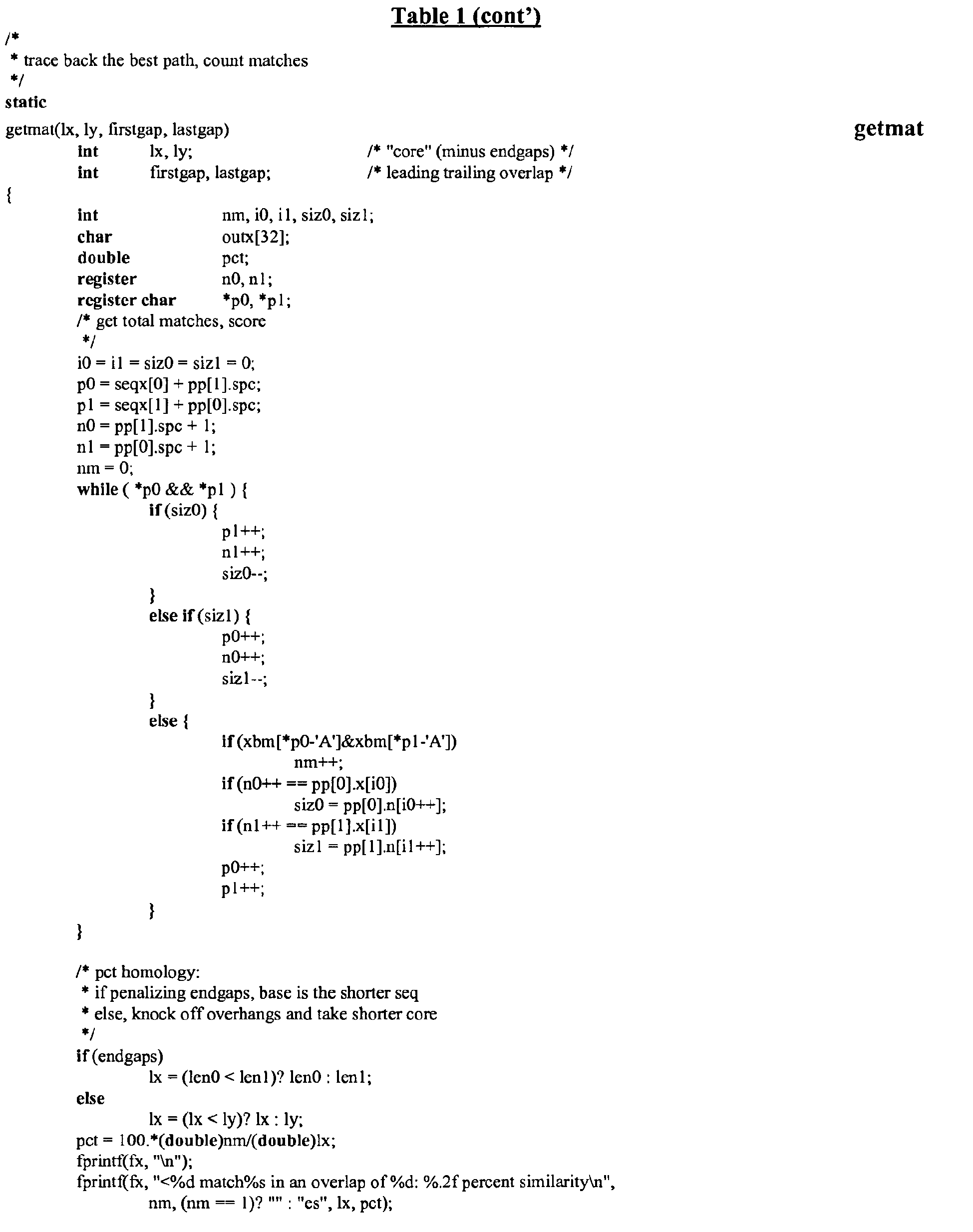

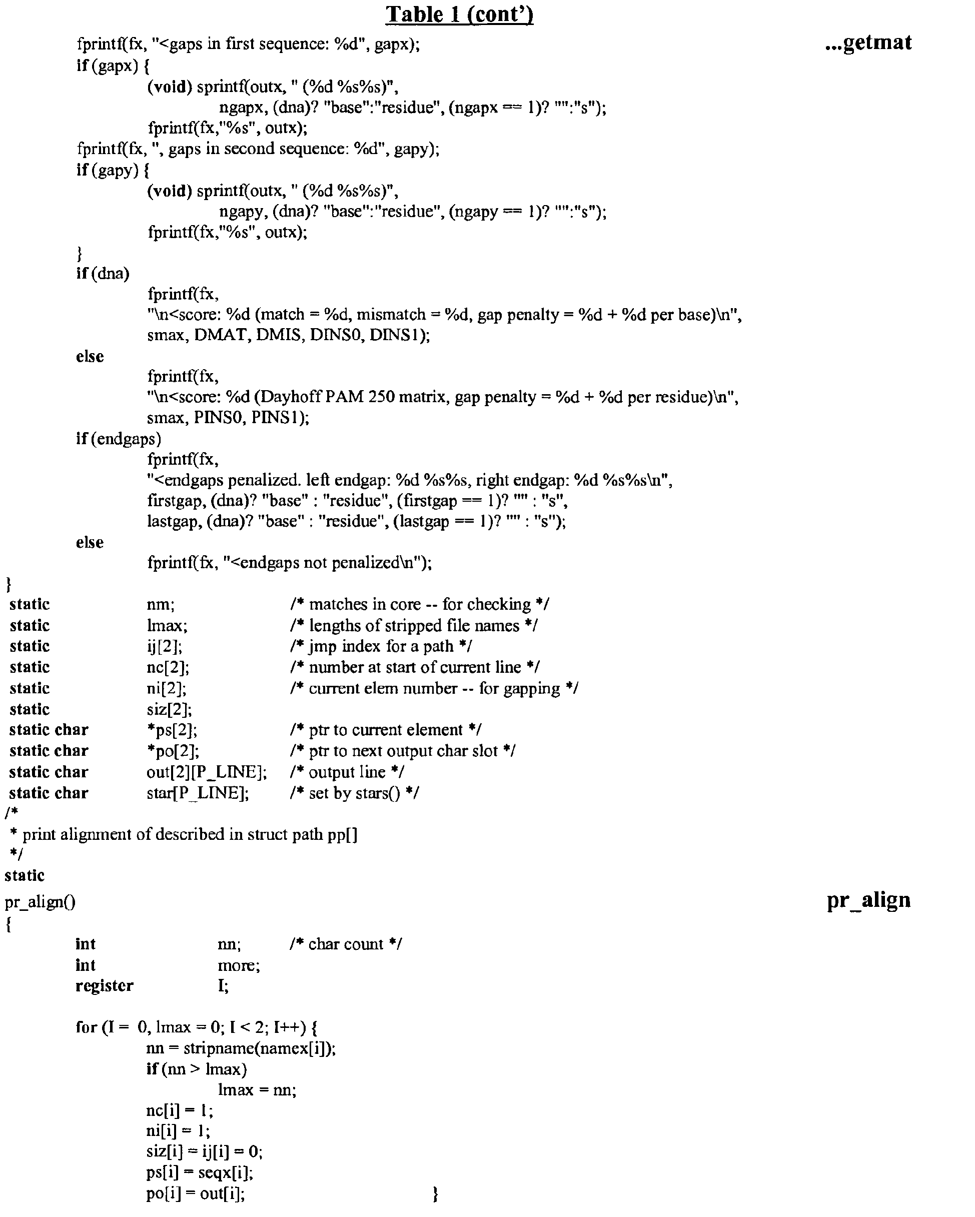

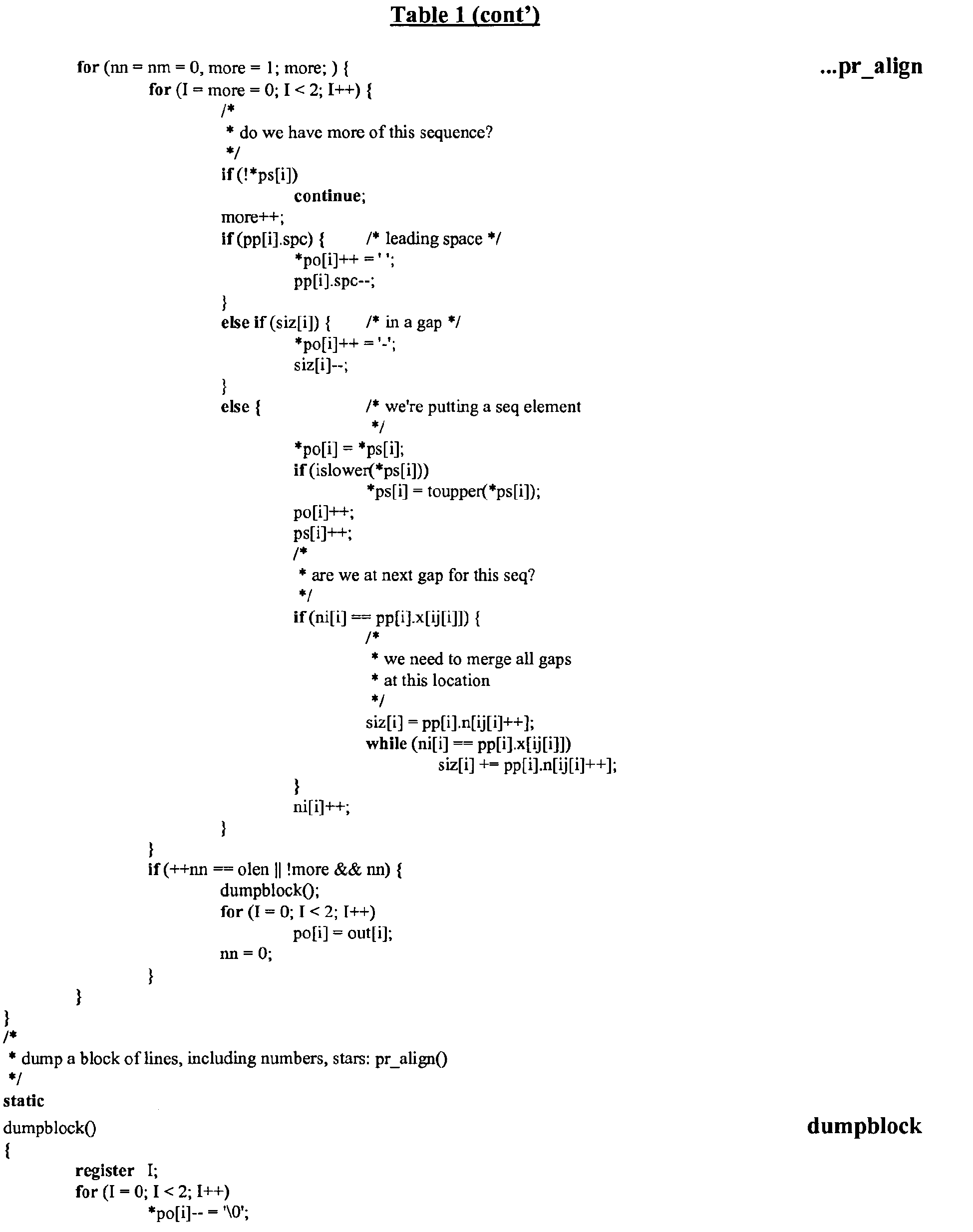

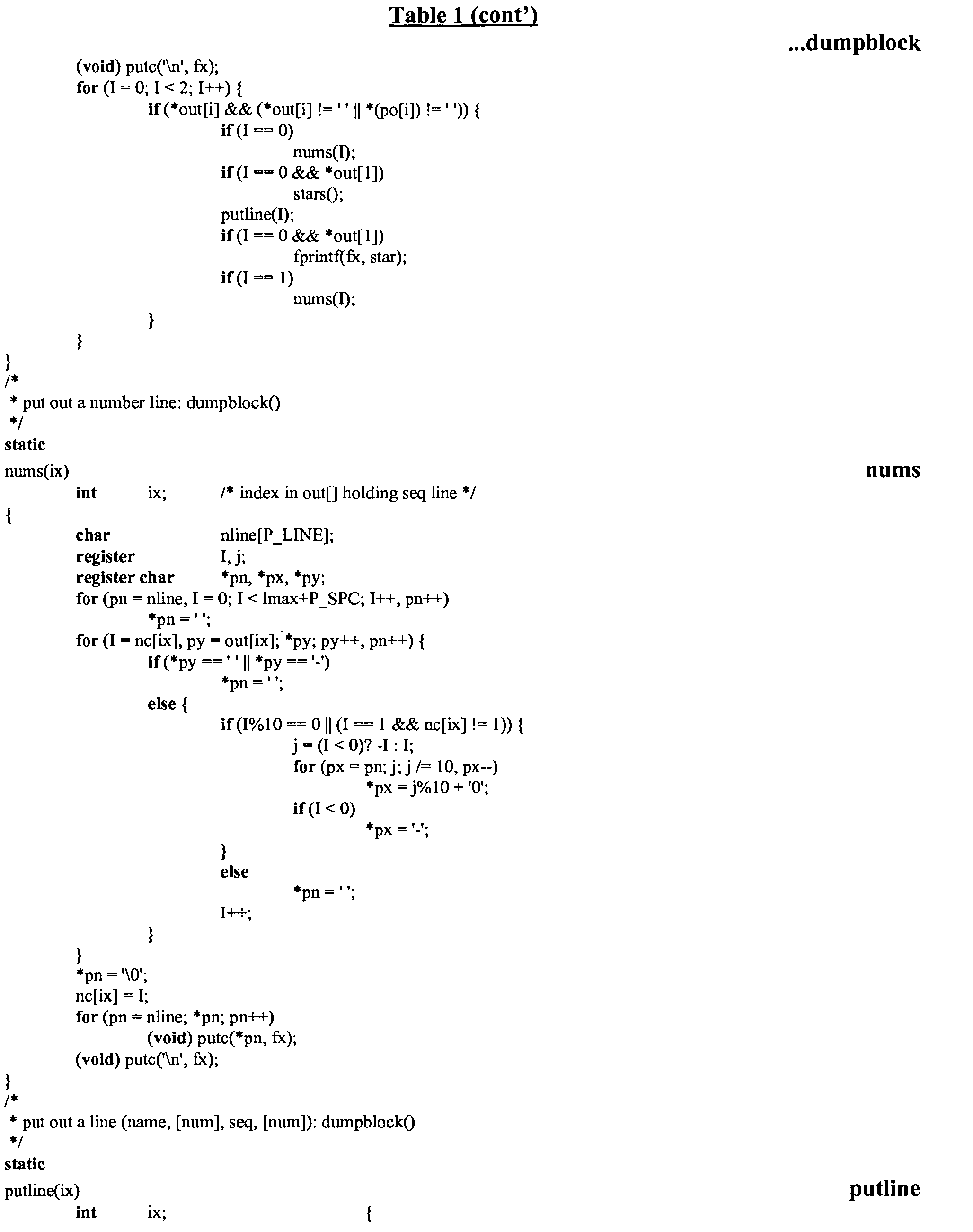

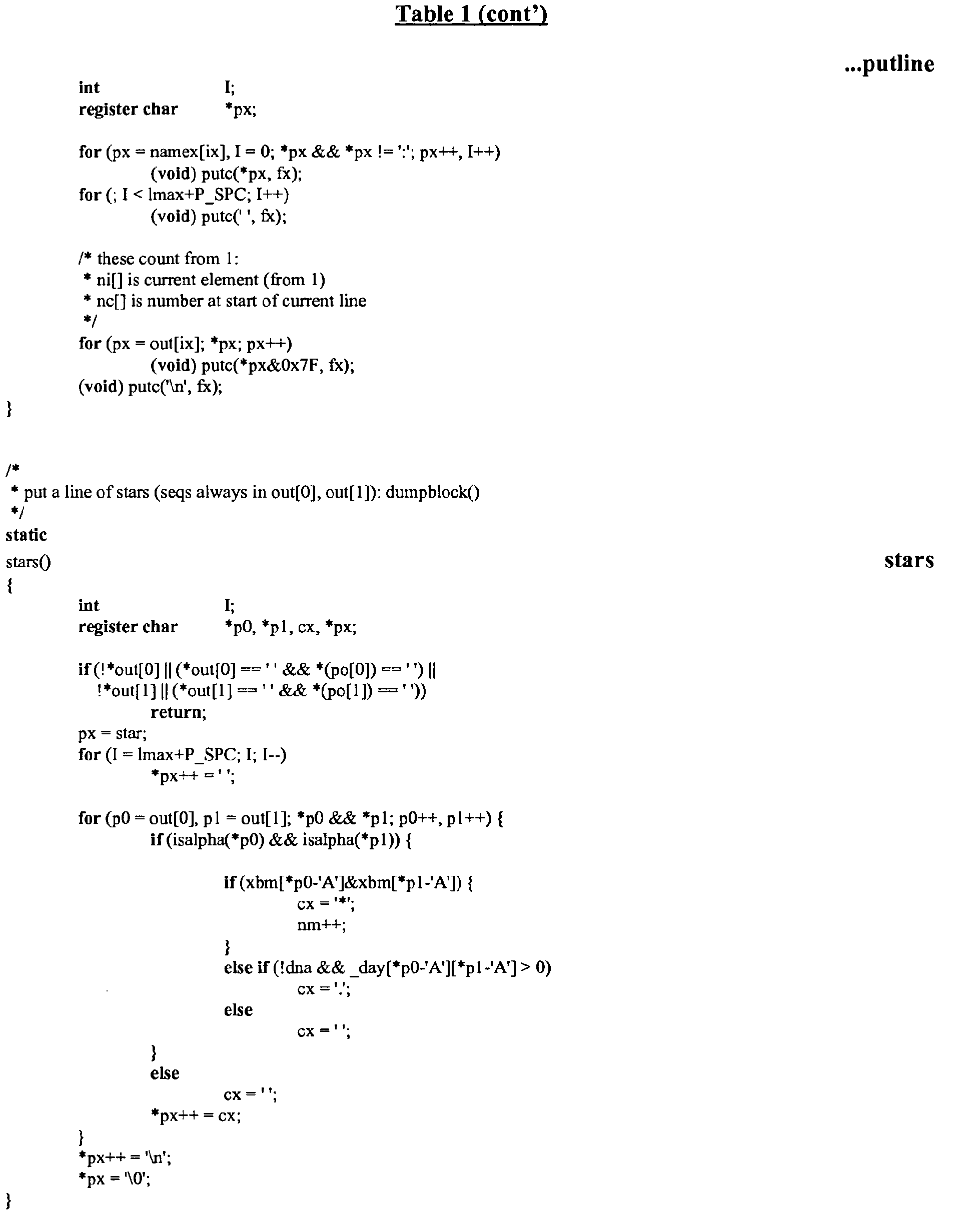

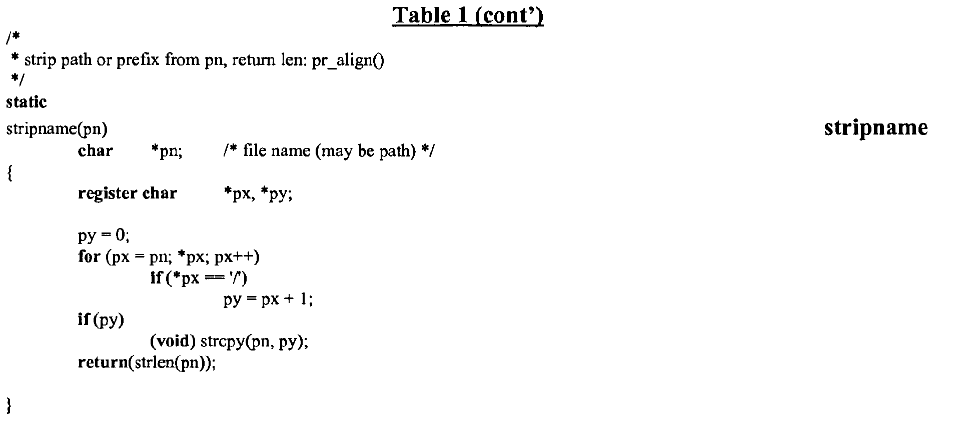

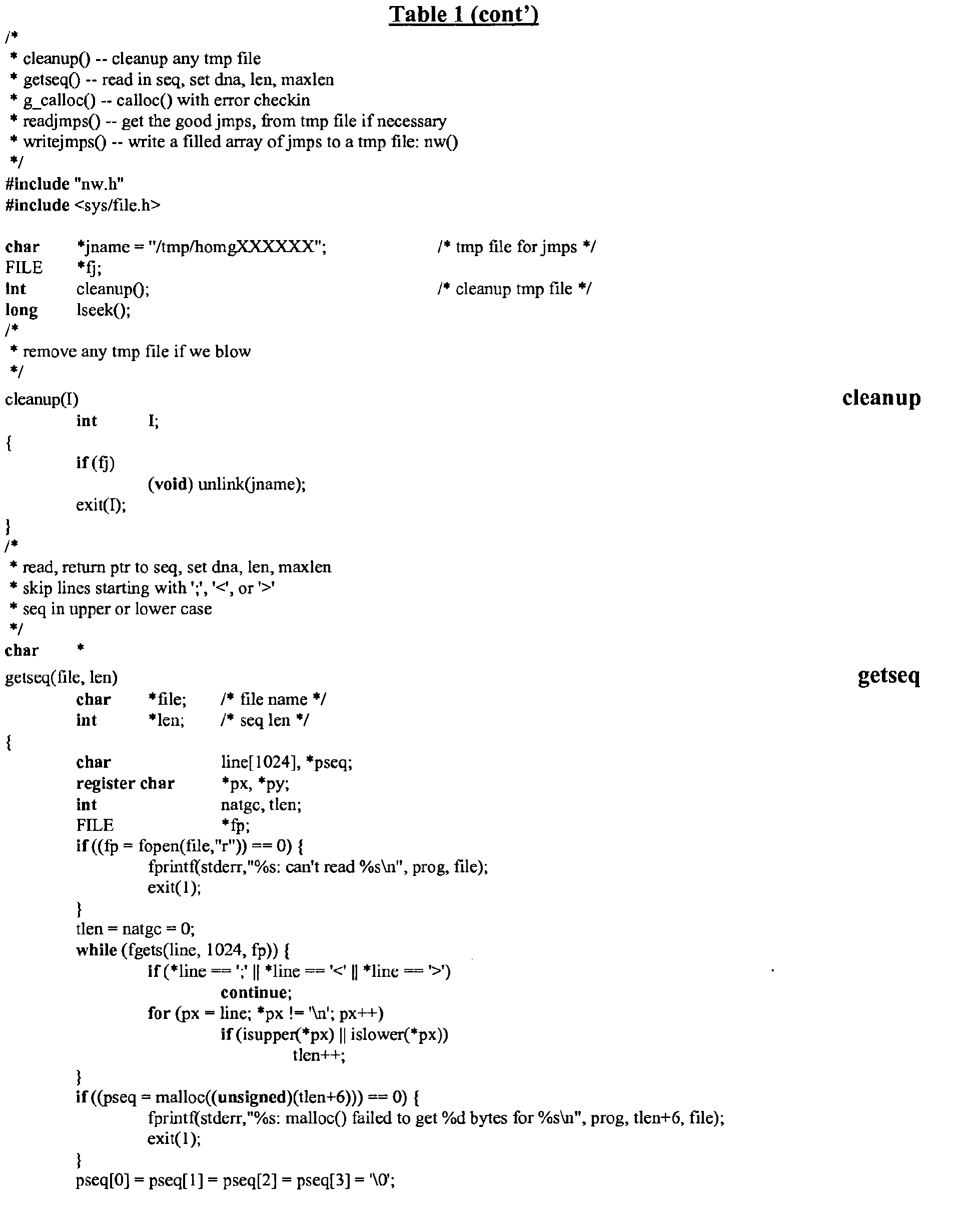

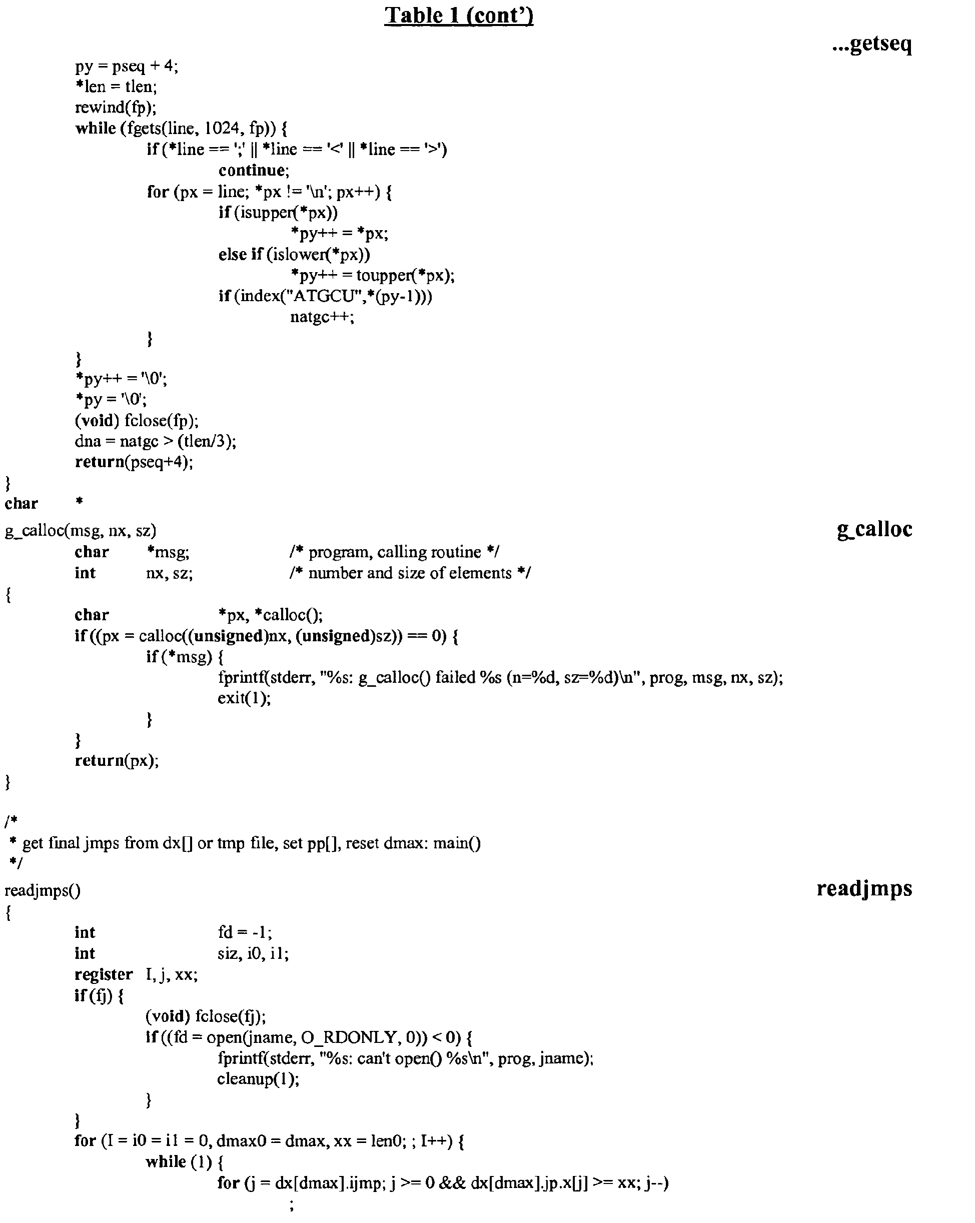

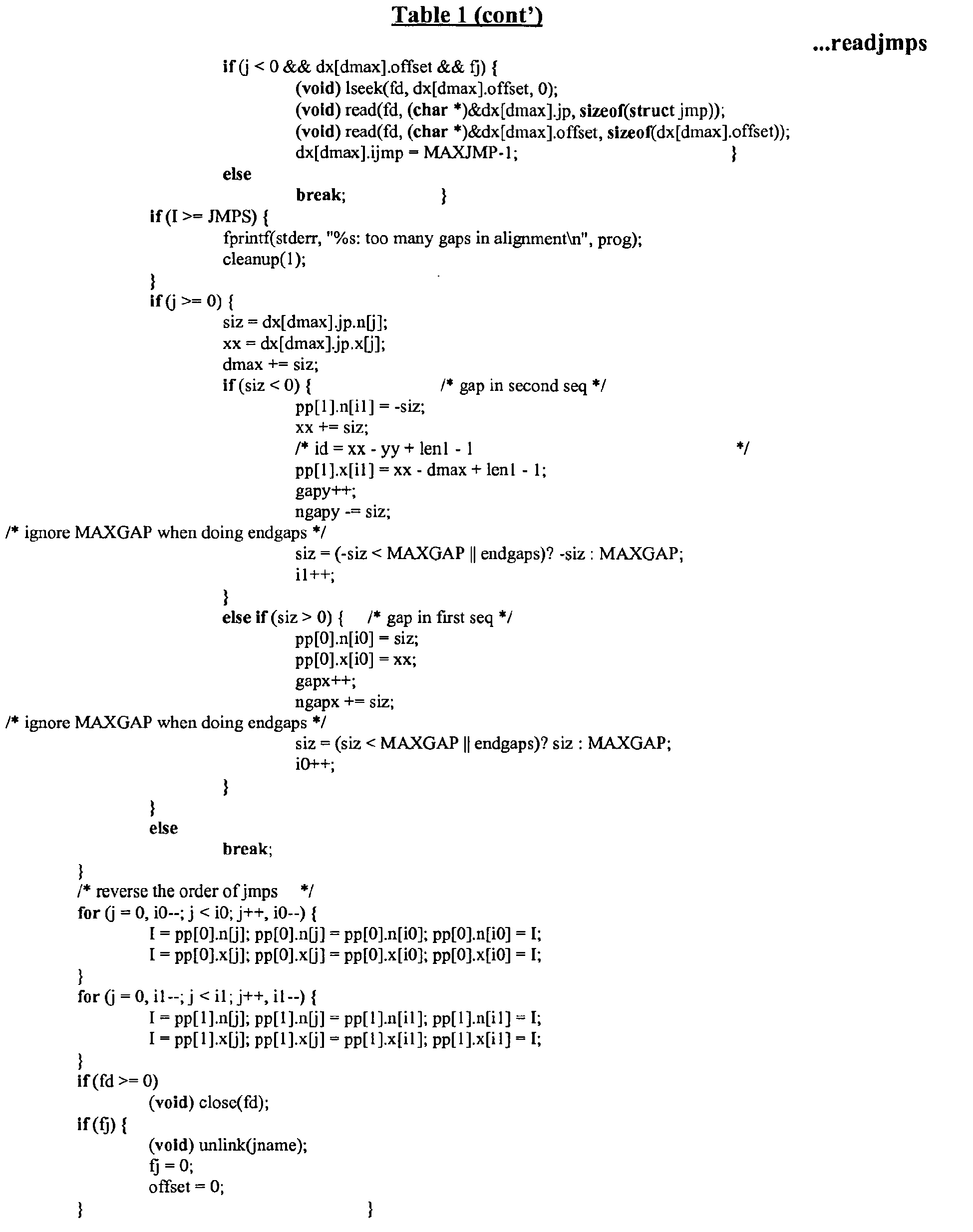

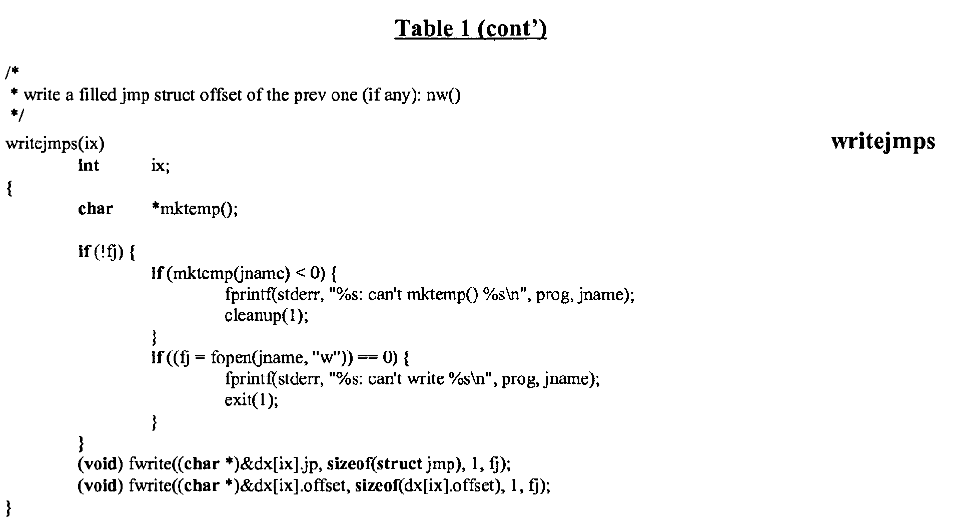

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity =
(the number of identically matching amino acid residues between the two polypeptide sequences as determined by ALIGN-2) divided by (the total number of amino acid residues of the PRO polypeptide) = 5 divided by 15 = 33.3%

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |

% amino acid sequence identity =
(the number of identically matching amino acid residues between the two polypeptide sequences as determined by ALIGN-2) divided by (the total number of amino acid residues of the PRO polypeptide) = 5 divided by 10 = 50%

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) =
6 divided by 14 = 42.9%

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) =
4 divided by 12 = 33.3%

### II. Compositions and Methods of the Invention

### A. Full-Length PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PR038465 or PR0346 Polypeptides

The present invention provides newly identified and isolated nucleotide sequences encoding polypeptides referred to in the present application as PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides. In particular, cDNAs encoding various PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides have been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been deposited with the ATCC. The actual nucleotide sequences of those clones can readily be determined by the skilled artisan by sequencing of the deposited clone using routine methods in the art. The predicted amino acid sequence can be determined from the nucleotide sequence using routine skill. For the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides and encoding nucleic acids described herein, Applicants have identified what is believed to be the reading frame best identifiable with the sequence information available at the time.

### B. PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PR05238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 Polypeptide Variants

In addition to the full-length native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides described herein, it is contemplated that PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variants can be prepared. PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variants can be prepared by introducing appropriate nucleotide changes into the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 DNA, and/or by synthesis of the desired PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. Those skilled in the art will appreciate that amino acid changes may alter post-translational processes of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, such as changing the number or position of glycosylation sites or altering the membrane anchoring characteristics.

Variations in the native full-length sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide or in various domains of the PR0218, PR0228, PR0271, PR0273, PR0295, PR0302, PR0305, PRO326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide described herein, can be made, for example, using any of the techniques and guidelines for conservative and non-conservative mutations set forth, for instance, in U.S. Patent No. 5,364,934. Variations may be a substitution, deletion or insertion of one or more codons encoding the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide that results in a change in the amino acid sequence of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PRO50332, PR038465 or PR0346 polypeptide as compared with the native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. Optionally the variation is by substitution of at least one amino acid with any other amino acid in one or more of the domains of the PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. Guidance in determining which amino acid residue may be inserted, substituted or deleted without adversely affecting the desired activity may be found by comparing the sequence of the PR0218, PR0228, PR0271, PR0273, PR0295, PR0302, PR0305, PR0326, PR0386, PR0655, PR0162, PR0788, PR0792, PR0940, PR0941, PRO1004, PR01012, PR01016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR0523 8, PRO1069, PRO1111, PRO1113, PRO1130, PR01195, PR01271, PRO1865, PRO1879, PR03446, PR03543, PR04329, PR04352, PR05733, PRO9859, PR09864, PRO9904, PRO9907, PRO10013, PRO90948, PR028694, PRO16089, PRO19563, PRO19675, PR020084, PR021434, PR050332, PR038465 or PR0346 polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.

PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR0523 8, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 fragments may be prepared by any of a number of conventional techniques. Desired peptide fragments may be chemically synthesized. An alternative approach involves generating PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 polypeptide fragments share at least one biological and/or immunological activity with the native PR0218, PR0228, PR0271, PR0273, PR0295, PR0302, PR0305, PR0326, PR0386, PR0655, PRO162, PR0788, PR0792, PRO940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide disclosed herein.

Conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are preferably introduced and the products screened.

**Table 6**

| **Original Residue** | **Exemplary Substitutions** | **Preferred Substitutions** |
|---|---|---|
| Ala (A) | Val; Leu; Ile | Val |
| Arg ®) | Lys; Gln; Asn | Lys |
| Asn (N) | Gln; His; Asp, Lys; Arg | Gln |
| Asp (D) | Glu; Asn | Glu |
| Cys ©) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in function or immunological identity of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
Amino acids may be grouped according to similarities in the properties of their side chains (in A. L. Lehninger, in Biochemistry, second ed., pp. 73-75, Worth Publishers, New York (1975)):
   (1) non-polar: Ala (A), Val (V), Leu (L), Ile (I), Pro (P), Phe (F), Trp (W), Met (M)
   (2) uncharged polar: Gly (G), Ser (S), Thr (T), Cys (C), Tyr (Y), Asn (N), Gln (Q)
   /(3) acidic: Asp (D), Glu (E)
   (4) basic: Lys (K), Arg (R), His(H)
Alternatively, naturally occurring residues may be divided into groups based on common side-chain properties:
   (1) hydrophobic: Norleucine, Met, Ala, Val, Leu, Ile;
   (2) neutral hydrophilic: Cys, Ser, Thr, Asn, Gln;
   (3) acidic: Asp, Glu;
   (4) basic: His, Lys, Arg;
   (5) residues that influence chain orientation: Gly, Pro;
   (6) aromatic: Trp, Tyr, Phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the mainchain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 Polypeptides

Covalent modifications of PRO218, PRO228, PRO271, PRO273, PRO295, PRO302, PRO305, PRO326, PR0386, PR0655, PRO162, PRO788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides are included within the scope of this invention. One type of covalent modification includes reacting targeted amino acid residues of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide with an organic derivatizing agent that is capable of reacting with selected side chains or the N- or C- terminal residues ofthe PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PRO9864, PRO9904, PRO9907, PRO10013, PR090948, PR028694, PRO16089, PRO19563, PRO19675, PR020084, PR021434, PR050332, PR038465 or PR0346 polypeptide. Derivatization with bifunctional agents is useful, for instance, for crosslinking PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides to a water-insoluble support matrix or surface for use in the method for purifying anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PRO346antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as metbyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide included within the scope of this invention comprises altering the native glycosylation pattern ofthe polypeptide. "Altering the native glycosylation pattern" is intended for purposes herein to mean deleting one or more carbohydrate moieties found in native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PR01865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides (either by removing the underlying glycosylation site or by deleting the glycosylation by chemical and/or enzymatic means), and/or adding one or more glycosylation sites that are not present in the native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. In addition, the phrase includes qualitative changes in the glycosylation of the native proteins, involving a change in the nature and proportions of the various carbohydrate moieties present.

Additionofglycosylation sites to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide may be accomplished by altering the amino acid sequence. The alteration may be made, for example, by the addition of, or substitution by, one or more serine or threonine residues to the native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 (for O-linked glycosylation sites). The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PRO9907, PRO10013, PR090948, PR028694, PRO16089, PRO19563, PRO19675, PR020084, PR021434, PR050332, PR038465 or PR0346 amino acid sequence may optionally be altered through changes at the DNA level, particularly by mutating the DNA encoding the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PR01113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide at preselected bases such that codons are generated that will translate into the desired amino acids.

Another means of increasing the number of carbohydrate moieties on the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PR0218, PR0228, PR0271, PR0273, PR0295, PR0302, PR0305, PR0326, PR0386, PR0655, PRO162, PR0788, PR0792, PRO940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides comprises linking the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PR0218, PR0228, PR0271, PR0273, PR0295, PR0302, PR0305, PR0326, PR0386, PR0655, PRO162, PRO788, PR0792, PRO940, PRO941, PRO1004, PRO1012, PRO1016, PR0474, PR05238, PRO1069, PRO1111, PR01113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptides of the present invention may also be modified in a way to form a chimeric molecule comprising the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide fused to another, heterologous polypeptide or amino acid sequence.

Such a chimeric molecule comprises a fusion of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PRO5733, PR09859, PR09864, PR09904, PR09907, PRO10013, PR090948, PR028694, PRO16089, PRO19563, PRO19675, PR020084, PR021434, PR050332, PR03 8465 or PR0346 polypeptide with a tag polypeptide which provides an epitope to which an anti-tag antibody can selectively bind. The epitope tag is generally placed at the amino- or carboxyl- terminus of the PR0218, PR0228, PR0271, PR0273, PR0295, PR0302, PR0305, PR0326, PR0386, PR0655, PRO162, PRO788, PRO792, PRO940, PRO941, PRO1004, PRO1012, PRO1016, PRO474, PRO5238, PRO1069, PRO1111, PRO1113, PRO1130, PRO1195, PR01271, PRO1865, PRO1879, PR03446, PR03543, PR04329, PR04352, PR05733, PR09859, PR09864, PR09904, PR09907, PRO10013, PR090948, PR028694, PRO16089, PR019563, PR019675, PR020084, PR021434, PR050332, PR038465 or PR0346 polypeptide. The presence of such epitope-tagged forms ofthe PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PR010013, PR090948, PR028694, PRO16089, PRO19563, PRO19675, PR020084, PR021434, PR050332, PR038465 or PR0346 polypeptide can be detected using an antibody against the tag polypeptide. Also, provision of the epitope tag enables the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

The chimeric molecule may comprise a fusion of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PRO28694, PRO16089, PRO19563, PRO19675, PR020084, PR021434, PR050332, PR038465 or PR0346 polypeptide with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide in place of at least one variable region within an Ig molecule. In a particularly preferred aspect of the invention, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

### D. Preparation of PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PR03446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 Polypeptides

The description below relates primarily to production of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PR01879, PR03446, PR03543, PR04329, PR04352, PRO5733, PR09859, PR09864, PR09904, PR09907, PRO10013, PR090948, PR028694, PRO16089, PRO19563, PRO19675, PR020084, PR021434, PR050332, PR038465 or PR0346 polypeptides by culturing cells transformed or transfected with a vector containing PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PRO3543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 nucleic acid. It is, of course, contemplated that alternative methods, which are well known in the art, may be employed to prepare PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides. For instance, the PR0218, PR0228, PR0271, PR0273, PR0295, PR0302, PR0305, PRO326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide may be chemically synthesized separately and combined using chemical or enzymatic methods to produce the full-length PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.

### 1. Isolation of DNA Encoding PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 Polypeptides

DNA encoding PR0218, PR0228, PR0271, PR0273, PR0295, PR0302, PR0305, PR0326, PR0386, PR0655, PRO162, PR0788, PR0792, PRO940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides may be obtained from a cDNA library prepared from tissue believed to possess the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 mRNA and to express it at a detectable level. Accordingly, human PR0218-, PR0228-, PR0271-, PR0273-, PR0295-, PR0302-, PR0305-, PR0326-, PR0386-, PR0655-, PRO162-, PR0788-, PR0792-, PR0940-, PR0941-, PRO1004-, PRO1012-, PRO1016-, PR0474-, PR05238-, PRO1069-, PRO1111-, PRO1113-, PRO1130-, PRO1195-, PRO1271-, PRO1865-, PRO1879-, PR03446-, PR03543-, PRO4329-, PR04352-, PR05733-, PR09859-, PR09864-, PR09904-, PR09907-, PRO10013-, PR090948-, PR028694-, PRO16089-, PRO19563-, PR019675-, PR020084-, PR021434-, PR050332-, PR038465- or PRO346-DNA can be conveniently obtained from a cDNA library prepared from human tissue, such as described in the Examples. The PR0218-, PR0228-, PR0271-, PR0273-, PR0295-, PR0302-, PR0305-, PR0326-, PR0386-, PR0655-, PRO162-, PR0788-, PR0792-, PR0940-, PR0941-, PRO1004-, PRO1012-, PRO1016-, PRO474-, PRO5238-, PRO1069-, PRO1111-, PRO1113-, PRO1130- , PRO1195-, PRO1271-, PRO1865-, PRO1879-, PR03446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PRO346-encoding gene may also be obtained from a genomic library or by known synthetic procedures (e.g., automated nucleic acid synthesis).

Libraries can be screened with probes (such as antibodies to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strainMM294 (ATCC 31,446); *E. coli* X 1776 (ATCC 31,537); E. *coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3; E. coli* W3110 strain 27C7 (ATCC 55,244), which has the complete genotype *tonA ptr3 phoA E15 (argF-lac) 169 degP ompT kan^{r}; E. coli* W3110 strain 37D6, which has the complete genotype *tonA ptr3 phoA E15 (argF-lac)169 degP ompT rbs7 ilvG kan^{r}*; *E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PR0218-, PR0228-, PR0271-, PR0273-, PR0295-, PR0302-, PR0305-, PR0326-, PR0386-, PR0655-, PRO162-, PR0788-, PR0792-, PR0940-, PR0941-, PRO1004-, PRO1012-, PRO1016-, PR0474-, PR05238-, PRO1069-, PRO1111-, PRO1113-, PR01130-, PRO1195-, PRO1271-, PRO1865-, PRO1879-, PR03446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PRO346-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A*. *niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W 138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (e.g., cDNA or genomic DNA) encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PRO9859, PR09864, PRO9904, PRO9907, PRO10013, PRO90948, PR028694, PR016089, PRO19563, PRO19675, PR020084, PR021434, PR050332, PR038465 or PR0346 polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO218- , PR0228-, PR0271-, PR0273-, PR0295-, PR0302-, PR0305-, PR0326-, PR0386-, PR0655-, PRO162-, PRO788-, PR0792-, PR0940-, PR0941-, PRO1004-, PRO1012-, PRO1016-, PR0474-, PR05238-, PRO1069-, PRO1111-, PRO1113-, PRO1130-, PRO1195-, PRO1271-, PRO1865-, PRO1879-, PR03446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PRO346-encoding DNA that is inserted into the vector. The signal sequence may be a prokaryotic signal sequence selected, for example, from the group of the alkaline phosphatase, penicillinase, Ipp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PRO346- encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PR0218-, PR0228-, PR0271-, PR0273-, PR0295-, PR0302-, PR0305-, PR0326-, PR0386-, PR0655-, PRO162-, PR0788-, PR0792-, PR0940-, PR0941-, PRO1004-, PRO1012-, PRO1016-, PR0474-, PR05238-, PRO1069-, PRO1111-, PRO1113-, PRO1130-, PRO1195-, PRO1271-, PRO1865-, PRO1879-, PR03446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PRO346-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PRO38465 or PR0346 polypeptides.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PR01271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PR0218, PR0228, PR0271, PR0273, PR0295, PRO302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side ofthe replication origin, and adenovirus enhancers. The enhancer may be spliced into the vector at a position 5' or 3'to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides.

Still other methods, vectors, and host cells suitable for adaptation to the synthesis of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide or against a synthetic peptide based on the DNA sequences provided herein or against exogenous sequence fused to PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 DNA and encoding a specific antibody epitope.

### 5. Purification of Polypeptide

Forms of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides may be recovered from culture medium or from host cell lysates. If membrane-bound, it can be released from the membrane using a suitable detergent solution (e.g. Triton-X 100) or by enzymatic cleavage. Cells employed in expression of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptides can be disrupted by various physical or chemical means, such as freeze-thaw cycling, sonication, mechanical disruption, or cell lysing agents.

It may be desired to purify PR0218, PR0228, PR0271, PR0273, PR0295, PR0302, PR0305, PR0326, PR0386, PRO655, PRO162, PRO788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide produced.

### E. Uses for PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 Polypeptides

Nucleotide sequences (or their complement) encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides have various applications in the art of molecular biology, including uses as hybridization probes, in chromosome and gene mapping and in the generation of anti-sense RNA and DNA. PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 nucleic acid will also be useful for the preparation of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PRO38465 or PR0346 polypeptides by the recombinant techniques described herein.

The full-length native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene, or portions thereof, may be used as hybridization probes for a cDNA library to isolate the full-length PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 cDNAorto isolate still other cDNAs (for instance, those encoding naturally-occurring variants of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PRO21434, PRO50332, PR038465 or PR0346 polypeptides or PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides from other species) which have a desired sequence identity to the native PR0218, PR0228, PR0271, PR0273, PR0295, PR0302, PR0305, PR0326, PR0386, PR0655, PRO162, PRO788, PRO792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PRO9904, PR09907, PRO10013, PR090948, PR028694, PRO16089, PRO19563, PRO19675, PR020084, PR021434, PR050332, PR038465 or PR0346 sequence disclosed herein. Optionally, the length of the probes will be about 20 to about 50 bases. The hybridization probes may be derived from at least partially novel regions of the full length native nucleotide sequence wherein those regions may be determined without undue experimentation or from genomic sequences including promoters, enhancer elements and introns of native sequence PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346. By way of example, a screening method will comprise isolating the coding region of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene using the known DNA sequence to synthesize a selected probe of about 40 bases. Hybridization probes may be labeled by a variety of labels, including radionucleotides such as ³²P or ³⁵S, or enzymatic labels such as alkaline phosphatase coupled to the probe via avidin/biotin coupling systems. Labeled probes having a sequence complementary to that of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene ofthe present invention can be used to screen libraries of human cDNA, genomic DNA or mRNA to determine which members of such libraries the probe hybridizes to. Hybridization techniques are described in further detail in the Examples below.

Any EST sequences disclosed in the present application may similarly be employed as probes, using the methods disclosed herein.

Other useful fragments of the PR0218, PR0228, PR0271, PRO273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 nucleic acids include antisense or sense oligonucleotides comprising a singe-stranded nucleic acid sequence (either RNA or DNA) capable of binding to target PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 mRNA (sense) or PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 DNA (antisense) sequences. Antisense or sense oligonucleotides, according to the present invention, comprise a fragment of the coding region of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 DNA. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. The antisense oligonucleotides thus may be used to block expression of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, CaPO₄-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either in *vivo* or *ex vivo.* Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 bases in length, about 10 bases in length, about 15 bases in length, about 20 bases in length, about 25 bases in length, about 30 bases in length, about 35 bases in length, about 40 bases in length, about 45 bases in length, about 50 bases in length, about 55 bases in length, about 60 bases in length, about 65 bases in length, about 70 bases in length, about 75 bases in length, about 80 bases in length, about 85 bases in length, about 90 bases in length, about 95 bases in length, about 100 bases in length, or more.

The probes may also be employed in PCR techniques to generate a pool of sequences for identification of closely related PR0218, PR0228, PR0271, PR0273, PR0295, PR0302, PR0305, PR0326, PR0386, PR0655, PRO162, PR0788, PRO792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 coding sequences.

Nucleotide sequences encoding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide can also be used to construct hybridization probes for mapping the gene which encodes that PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PRO4352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

When the coding sequences for PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 encode a protein which binds to another protein (for example, where the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 is a receptor), the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide can be used in assays to identify the other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Also, the receptor PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PRO50332, PR038465 or PR0346 can be used to isolate correlative ligand(s). Screening assays can be designed to find lead compounds that mimic the biological activity of a native PR0218, PR0228, PR0271, PR0273, PR0295, PR0302, PR0305, PR0326, PR0386, PRO655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or a receptor for PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PR019563, PRO19675, PR020084, PR021434, PR050332, PR038465 or PR0346 polypeptides. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. The invention provides cDNA encoding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide which can be used to clone genomic DNA encoding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide in accordance with established techniques and the genomic sequences used to generate transgenic animals that contain cells which express DNA encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PRO4329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides. Any technique known in the art may be used to introduce a target gene transgene into animals to produce the founder lines of transgenic animals. Such techniques include, but are not limited to pronuclear microinjection (U.S. Pat. Nos. 4,873,191, 4,736,866 and 4,870,009); retrovirus mediated gene transfer into germ lines (Van der Putten, et al., Proc. Natl. Acad. Sci.,USA, 82:6148-6152 (1985)); gene targeting in embryonic stem cells (Thompson, et al., Cell, 56:313-321 (1989)); nonspecific insertional inactivation using a gene trap vector (U.S. Pat. No. 6,436,707); electroporation of embryos (Lo, Mol. Cell. Biol., 3:1803-1814 (1983)); and sperm-mediated gene transfer (Lavitrano, et al., Cell, 57:717-723 (1989)); etc. Typically, particular cells would be targeted for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 transgene incorporation with tissue-specific enhancers. Transgenic animals that include a copy of a transgene encoding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide introduced into the germ line of the animal at an embryonic stage can be used to examine the effect of increased expression of DNA encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PRO9859, PR09864, PR09904, PR09907, PRO10013, PR090948, PR028694, PRO16089, PRO19563, PRO19675, PR020084, PR021434, PR050332, PR038465 or PR0346 polypeptides. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition. Alternatively, non-human homologues of PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides can be used to construct a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 "knock out" animal which has a defective or altered gene encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 proteins as a result of homologous recombination between the endogenous gene encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PR01271, PR01865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides and altered genomic DNA encoding PR0218, PR0228, PR0271, PRO273, PRO295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides introduced into an embryonic stem cell of the animal. Preferably the knock out animal is a mammal. More preferably, the mammal is a rodent such as a rat or mouse. For example, cDNA encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides can be used to clone genomic DNA encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides in accordance with established techniques. A portion of the genomic DNA encoding the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the gene encoding the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.

In addition, knockout mice can be highly informative in the discovery of gene function and pharmaceutical utility for a drug target, as well as in the determination of the potential on-target side effects associated with a given target. Gene function and physiology are so well conserved between mice and humans., since they are both mammals and contain similar numbers of genes, which are highly conserved between the species. It has recently been well documented, for example, that 98% of genes on mouse chromosome 16 have a human ortholog (Mural et al., Science 296:1661-71 (2002)).

Although gene targeting in embryonic stem (ES) cells has enabled the construction of mice with null mutations in many genes associated with human disease, not all genetic diseases are attributable to null mutations. One can design valuable mouse models of human diseases by establishing a method for gene replacement (knock-in) which will disrupt the mouse locus and introduce a human counterpart with mutation, Subsequently one can conduct *in vivo* drug studies targeting the human protein (Kitamoto et. Al., Biochemical and Biophysical Res. Commun., 222:742-47 (1996)).

Nucleic acid encoding the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane. (Zamecnik et al., Proc. Natl. Acad. Sci. USA 83:4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use ofliposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau et al., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem. 262, 4429-4432 (1987); and Wagner et al., Proc. Natl. Acad. Sci. USA 87, 3410-3414 (1990). For review of gene marking and gene therapy protocols see Anderson et al., Science 256, 808-813 (1992).

The PR0218, PR0228, PR0271, PR0273, PR0295, PR0302, PR0305, PR0326, PR0386, PR0655, PRO162, PRO788, PRO792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PR016089, PRO19563, PR019675, PR020084, PR021434, PR050332, PR038465 or PR0346 polypeptides described herein may also be employed as molecular weight markers for protein electrophoresis purposes and the isolated nucleic acid sequences may be used for recombinantly expressing those markers.

The nucleic acid molecules encoding the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides or fragments thereof described herein are useful for chromosome identification. In this regard, there exists an ongoing need to identify new chromosome markers, since relatively few chromosome marking reagents, based upon actual sequence data are presently available. Each PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PRO38465 or PR0346 nucleic acid molecule of the present invention can be used as a chromosome marker.

The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PRO5238, PRO1069, PRO1111, PRO1113, PRO1130, PRO1195, PRO1271, PRO1865, PRO1879, PR03446, PR03543, PR04329, PRO4352, PRO5733, PR09859, PR09864, PRO9904, PR09907, PRO10013, PRO90948, PRO28694, PRO16089, PRO19563, PRO19675, PR020084, PR021434, PR050332, PR038465 or PRO346 polypeptides and nucleic acid molecules of the present invention may also be used diagnostically for tissue typing, wherein the PR0218, PR0228, PR0271, PR0273, PRO295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PR01271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides of the present invention may be differentially expressed in one tissue as compared to another, preferably in a diseased tissue as compared to a normal tissue of the same tissue type. PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 nucleic acid molecules will find use for generating probes for PCR, Northern analysis, Southern analysis and Western analysis.

The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR03 8465 or PRO346 polypeptides described herein may also be employed as therapeutic agents. The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides of the present invention can be formulated according to known methods to prepare pharmaceutically useful compositions, whereby the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 product hereof is combined in admixture with a pharmaceutically acceptable carrier vehicle. Therapeutic formulations are prepared for storage by mixing the active ingredient having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, PLURONICS^{™} or PEG.

The formulations to be used for *in vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal, intracerebral, intramuscular, intraocular, intraarterial or intralesional routes, topical administration, or by sustained release systems.

Dosages and desired drug concentrations of pharmaceutical compositions of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi et al., Eds., Pergamon Press, New York 1989, pp. 42-96.

When in *vivo* administration of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or agonist or antagonist thereof is employed, normal dosage amounts may vary from about 10 ng/kg to up to 100 mg/kg of mammal body weight or more per day, preferably about 1 µg/kg/day to 10 mg/kg/day, depending upon the route ofadministration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

Where sustained-release administration of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 polypeptide is desired in a formulation with release characteristics suitable for the treatment of any disease or disorder requiring administration of the PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 polypeptide, microencapsulation of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 polypeptide is contemplated. Microencapsulation ofrecombinant proteins for sustained release has been successfully performed with human growth hormone (rhGH), interferon-(rhIFN- ), interleukin-2, and MN rgp120. Johnson et al., Nat. Med., 2:795-799 (1996); Yasuda, Biomed. Ther., 27:1221-1223 (1993); Hora et al., Bio/Technology, 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-462; WO 97/03692, WO 96/40072, WO 96/07399; and U.S. Pat. No. 5,654,010.

The sustained-release formulations of these proteins were developed using poly-lactic-coglycolic acid (PLGA) polymer due to its biocompatibility and wide range of biodegradable properties. The degradation products of PLGA, lactic and glycolic acids, can be cleared quickly within the human body. Moreover, the degradability ofthis polymer can be adjusted from months to years depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41.

This invention encompasses methods of screening compounds to identify those that mimic the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide (agonists) or prevent the effect of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PRO4329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide (antagonists). Agonists that mimic a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide would be especially valuable therapeutically in those instances where a negative phenotype is observed based on findings with the non-human transgenic animal whose genome comprises a disruption of the gene which encodes for the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PR01113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. Antagonists that prevent the effects of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide would be especially valuable therapeutically in those instances where a positive phenotype is observed based upon observations with the non-human transgenic knockout animal. Screening assays for antagonist drug candidates are designed to identify compounds that bind or complex with the PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide encoded by the genes identified herein, or otherwise interfere with the interaction of the encoded polypeptide with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide encoded by a nucleic acid identified herein under conditions and for a time sufficient to allow these two components to interact.

In binding assays, the interaction is binding and the complex formed can be isolated or detected in the reaction mixture. The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide encoded by the gene identified herein or the drug candidate is immobilized on a solid phase, e.g., on a microtiter plate, by covalent or non-covalent attachments. Non-covalent attachment generally is accomplished by coating the solid surface with a solution of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide and drying. Alternatively, an immobilized antibody, e.g., a monoclonal antibody, specific for the PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide encoded by a gene identified herein, its interaction with that polypeptide can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL 1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113. PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide may be added to a cell along with the compound to be screened for a particular activity and the ability of the compound to inhibit the activity of interest in the presence of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide indicates that the compound is an antagonist to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. Alternatively, antagonists may be detected by combining the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide and a potential antagonist with membrane-bound PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PRO5733, PR09859, PR09864, PR09904, PR09907, PRO10013, PR090948, PR028694, PRO16089, PRO19563, PRO19675, PR020084, PR021434, PR050332, PR038465 or PR0346 polypeptide receptors orrecombinant receptors under appropriate conditions for a competitive inhibition assay. The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide can be labeled, such as by radioactivity, such that the number of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PRO1865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide and a cDNA library created from this RNA is divided into pools and used to transfect COS cells or other cells that are not responsive to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PRO5238, PRO1069, PRO1111, PR01113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. Transfected cells that are grown on glass slides are exposed to labeled PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, the labeled PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PR01069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

Another approach in assessing the effect of an antagonist to a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, would be administering a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 antagonist to a wild-type mouse in order to mimic a known knockout phenotype. Thus, one would initially knockout the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene of interest and observe the resultant phenotype as a consequence of knocking out or disrupting the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene. Subsequently, one could then assess the effectiveness of an antagonist to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide by administering an antagonist to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide to a wild-type mouse. An effective antagonist would be expected to mimic the phenotypic effect that was initially observed in the knockout animal.

Likewise, one could assess the effect of an agonist to a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, by administering a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 agonist to a non-human transgenic mouse in order to ameliorate a known negative knockout phenotype. Thus, one would initially knockout the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PR01271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene of interest and observe the resultant phenotype as a consequence of knocking out or disrupting the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 gene. Subsequently, one could then assess the effectiveness of an agonist to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide by administering an agonist to the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PR01879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide to a the non-human transgenic mouse. An effective agonist would be expected to ameliorate the negative phenotypic effect that was initially observed in the knockout animal.

In another assay for antagonists, mammalian cells or a membrane preparation expressing the receptor would be incubated with a labeled PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide in the presence of the candidate compound. The ability of the compound to enhance or block this interaction could then be measured.

More specific examples of potential antagonists include an oligonucleotide that binds to the fusions of immunoglobulin with the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PRO20084, PR021434, PR050332, PR038465 or PR0346 polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide that recognizes the receptor but imparts no effect, thereby competitively inhibiting the action of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.

Another potential PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PRO9907, PRO10013, PR090948, PR028694, PRO16089, PRO19563, PRO19675, PR020084, PR021434, PR050332, PR038465 or PR0346 polypeptides herein, is used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al., Science, 241: 456 (1988); Dervan et al., Science, 251:1360 (1991)), thereby preventing transcription and the production of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. The antisense RNA oligonucleotide hybridizes to the mRNA in *vivo* and blocks translation of the mRNA molecule into the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the PR0218, PRO228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. When antisense DNA is used, oligodeoxyribonucleotides derived from the translation-initiation site, e.g., between about -10 and +10 positions of the target gene nucleotide sequence, are preferred.

Potential antagonists include small molecules that bind to the active site, the receptor binding site, or growth factor or other relevant binding site of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PRO5733, PR09859, PR09864, PR09904, PR09907, PRO10013, PRO90948, PR028694, PRO16089, PRO19563, PRO19675, PR020084, PR021434, PR050332, PR038465 or PR0346 polypeptide, thereby blocking the normal biological activity of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO 97/33551, *supra.*

These small molecules can be identified by any one or more of the screening assays discussed hereinabove and/or by any other screening techniques well known for those skilled in the art.

Diagnostic and therapeutic uses of the herein disclosed molecules may also be based upon the positive functional assay hits disclosed and described below.

### F. Anti-PRO218, Anti-PRO228, Anti-PRO271, Anti-PRO273, Anti-PRO295, Anti-PRO302, Anti-PRO305, Anti-PRO326, Anti-PRO386, Anti-PRO655, Anti-PRO162, Anti-PRO788, Anti-PRO792, Anti-PRO940. Anti-PRO941, Anti-PRO1004. Anti-PRO1012, Anti-PRO1016, Anti-PRO474, Anti-PRO5238, Anti-PRO1069, Anti-PRO1111, Anti-PRO1113, Anti-PRO1130, Anti-PRO1195, Anti-PRO1271, Anti-PRO1865, Anti-PRO1879, Anti-PRO3446, Anti-PRO3543, Anti-PRO4329, Anti-PRO4352, Anti-PRO5733, Anti-PRO9859, Anti-PRO9864, Anti-PRO9904, Anti-PRO9907, Anti-PRO10013, Anti-PRO90948, Anti-PRO28694. Anti-PRO16089, Anti-PRO19563, Anti-PRO19675, Anti-PRO20084, Anti-PR021434, Anti-PR050332, Anti-PR038465 or Anti-PR0346 Antibodies

The present invention provides anti-PR0218, anti-PR0228, anti-PR0271, anti-PR0273, anti-PR0295, anti-PR0302, anti-PR0305, anti-PR0326, anti-PRO386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PRO19563, anti-PRO19675, anti-PR020084, anti-PR021434, anti-PR050332, anti-PR038465 or anti-PR0346 antibodies which may find use herein as therapeutic and/or diagnostic agents. Exemplary antibodies include polyclonal, monoclonal, humanized, bispecific, and heteroconjugate antibodies.

### 1. Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen (especially when synthetic peptides are used) to a protein that is immunogenic in the species to be immunized. For example, the antigen can be conjugated to keyhole limpet hemocyanin (KLH), serum albumin, bovine thyroglobulin, or soybean trypsin inhibitor, using a bifunctional or derivatizing agent, e.g., maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R' are different alkyl groups.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later, the animals are boosted with 1/5 to 1/10 the original amount of peptide or conjugate in Freund's complete adjuvant by subcutaneous injection at multiple sites. Seven to 14 days later, the animals are bled and the serum is assayed for antibody titer. Animals are boosted until the titer plateaus. Conjugates also can be made in recombinant cell culture as protein fusions. Also, aggregating agents such as alum are suitably used to enhance the immune response.

### 2. Monoclonal Antibodies

Monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium which medium preferably contains one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

Preferred fusion partner myeloma cells are those that fuse efficiently, support stable high-level production of antibody by the selected antibody-producing cells, and are sensitive to a selective medium that selects against the unfused parental cells. Preferred myeloma cell lines are murine myeloma lines, such as those derived from MOPC-21 and MPC-11 mouse tumors available from the Salk Institute Cell Distribution Center, San Diego, California USA, and SP-2 and derivatives e.g., X63-Ag8-653 cells available from the American Type Culture Collection, Manassas, Virginia, USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980).

Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown *in vivo* as ascites tumors in an animal e.g,, by i.p. injection of the cells into mice.

The monoclonal antibodies secreted by the subclones are suitably separated from the culture medium, ascites fluid, or serum by conventional antibody purification procedures such as, for example, affinity chromatography (e.g., using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs. 130:151-188 (1992).

Monoclonal antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain (C_{H} and C_{L}) sequences for the homologous murine sequences (U.S. PatentNo. 4,816,567; and Morrison, et al., Proc. Natl Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### 3. Human and Humanized Antibodies

The anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PRO788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PRO9907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibodies ofthe invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)).

It is further important that antibodies be humanized with retention of high binding affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PR019563, anti-PRO19675, anti-PR020084, anti-PR021434, anti-PR050332, anti-PR038465 or anti-PR0346 antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgG1 antibody.

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggemann et al., Year in Immuno. 7:33 (1993); U.S. Patent Nos. 5,545,806, 5,569,825, 5,591,669 (all ofGenPharm); 5,545,807; and WO 97/17852.

Alternatively, phage display technology (McCafierty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M 13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, e.g., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patents 5,567,610 and 5,229,275).

### 4. Antibody fragments

In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from *E. coli,* thus allowing the facile production of large amounts of these fragments. Antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Fab and F(ab')₂ fragment with increased in vivo half-life comprising a salvage receptor binding epitope residues are described in U.S. Patent No. 5,869,046. Other techniques for the production of antibody fragments will be apparent to the skilled practitioner. The antibody of choice is a single chain Fv fragment (scFv). See WO 93/16185; U.S. Patent No. 5,571,894; and U.S. Patent No. 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Patent 5,641,870 for example. Such linear antibody fragments may be monospecific or bispecific.

### 5. Bispecific Antibodies

Bispecific antibodies are antibodies that have binding specificities for at least two different epitopes. Exemplary bispecific antibodies may bind to two different epitopes of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 protein as described herein. Other such antibodies may combine a PR0218, PR0228, PR0271, PR0273, PR0295, PR0302, PR0305, PR0326, PR0386, PR0655, PRO162, PR0788, PR0792, PR0940, PRO941, PRO1004, PRO1012, PRO1016, PRO474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PR016089, PRO19563, PRO19675, PR020084, PR021434, PR050332, PR038465 or PR0346 binding site with a binding site for another protein. Alternatively, ananti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PR01069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PR019563, anti-PRO19675, anti-PR020084, anti-PR021434, anti-PR050332, anti-PR038465 or anti-PR0346 arm may be combined with an arm which binds to a triggering molecule on a leukocyte such as a T-cell receptor molecule (e.g. CD3), or Fc receptors for IgG (FcyR), such as FcγRI (CD64), FcγRII (CD32) and FcγRIII (CD16), so as to focus and localize cellular defense mechanisms to the PR0218-, PR0228-, PR0271-, PR0273-, PRO295- , PR0302-, PR0305-, PR0326-, PR0386-, PR0655-, PRO162-, PR0788-, PR0792-, PR0940-, PR0941-, PRO1004-, PRO1012-, PRO1016-, PR0474-, PR05238-, PRO1069-, PRO1111-, PRO1113-, PRO1130-, PRO1195-, PRO1271-, PRO1865-, PRO1879-, PR03446-, PR03543-, PRO4329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR03 8465- or PR0346-expressing cell. Bispecific antibodies may also be used to localize cytotoxic agents to cells which express a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide. These antibodies possess a PR0218-, PR0228-, PR0271-, PR0273-, PR0295-, PR0302-, PR0305-, PR0326-, PR0386-, PR0655-, PRO162-, PR0788-, PRO792-, PRO940-, PR0941-, PRO1004-, PRO1012-,PRO1016-,PRO474-,PRO5238-,PRO1069-, PRO1111-, PRO1113-, PRO1130-, PRO1195-, PRO1271-, PRO1865-, PRO1879-, PR03446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PR0346-binding arm and an arm which binds the cytotoxic agent (e.g., saporin, anti-interferon-α, vinca alkaloid, ricin A chain, methotrexate or radioactive isotope hapten). Bispecific antibodies can be prepared as full length antibodies or antibody fragments (e.g., F(ab')₂ bispecific antibodies).

WO 96/16673 describes a bispecific anti-ErbB2/anti-FcγRIII antibody and U.S. Patent No. 5,837,234 discloses a bispecific anti-ErbB2/anti-FcγRI antibody. A bispecific anti-ErbB2/Fcα antibody is shown in WO98/02463. U.S. Patent No. 5,821,337 teaches a bispecific anti-ErbB2/anti-CD3 antibody.

Methods for making bispecific antibodies are known in the art. Traditional production of full length bispecific antibodies is based on the co-expression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J. 10:3655-3659 (1991).

According to a different approach, antibody variable domains with the desired binding specificity (antibody-antigen combining sites) are fused to immunoglobulin constant domain sequences. Preferably, the fusion is with an Ig heavy chain constant domain, comprising at least part of the hinge, C_{H}2, and C_{H}3 regions. It is preferred to have the first heavy-chain constant region (C_{H}1) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination.

The invention provides bispecific antibodies which are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the C_{H}3 domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al., J. Exp. Med. 175: 217-225 (1992) describe the production of a fully humanized bispecific antibody F(ab')₂molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a V_{H} connected to a V_{L} by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al., J. Immunol. 147:60 (1991).

### 6. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 7. Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-(X1)ₙ-VD2-(X2)ₙ-Fc, wherein VD1 is a first variable domain, VD2 is a second variable domain, Fc is one polypeptide chain of an Fc region, X 1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 8. Effector Function Engineering

It may be desirable to modify the antibody of the invention with respect to effector function, e.g., so as to enhance antigen-dependent cell-mediated cytotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolffet al., Cancer Research 53:2560-2565 (1993). Alternatively, an antibody can be engineered which has dual Fc regions and may thereby have enhanced complement lysis and ADCC capabilities. See Stevenson et al., Anti-Cancer Drug Design 3:219-230 (1989). To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (e.g., IgG₁, IgG₂, IgG₃, or IgG₄) that is responsible for increasing the *in vivo* serum half-life of the IgG molecule.

### 9. Immunoconjugates

The invention also pertains to immunoconjugates comprising an antibody conjugated to a cytotoxic agent such as a chemotherapeutic agent, a growth inhibitory agent, a toxin (*e.g.*, an enzymatically active toxin of bacterial, fungal, plant, or animal origin, or fragments thereof), or a radioactive isotope (*i.e*., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

Conjugates of an antibody and one or more small molecule toxins, such as a calicheamicin, maytansinoids, a trichothene, and CC1065, and the derivatives of these toxins that have toxin activity, are also contemplated herein.

### Maytansine and maytansinoids

The invention provides an anti-PR0218, anti-PR0228, anti-PR0271, anti-PR0273, anti-PR0295, anti-PR0302, anti-PR0305, anti-PR0326, anti-PRO386, anti-PR0655, anti-PRO162, anti-PR0788, anti-PR0792, anti-PR0940, anti-PR0941, anti-PRO1004, anti-PRO1012, anti-PR01016, anti-PR0474, anti-PR05238, anti-PRO1069, anti-PRO1111, anti-PRO1113, anti-PR01130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PR019563, anti-PRO19675, anti-PR020084, anti-PR021434, anti-PR050332, anti-PR038465 or anti-PRO346 antibody (full length or fragments) which is conjugated to one or more maytansinoid molecules.

Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533, the disclosures of which are hereby expressly incorporated by reference.

### Maytansinoid-antibody conjugates

In an attempt to improve their therapeutic index, maytansine and maytansinoids have been conjugated to antibodies specifically binding to tumor cell antigens. Immunoconjugates containing maytansinoids and their therapeutic use are disclosed, for example, in U.S. Patent Nos. 5,208,020, 5,416,064 and European Patent EP 0 425 235 B1, the disclosures of which are hereby expressly incorporated by reference. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an *in vivo* tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/*neu* oncogene. The cytotoxicity of the TA.1-maytansonoid conjugate was tested *in vitro* on the human breast cancer cell line SK-BR-3, which expresses 3 x 10⁵ HER-2 surface antigens per cell. The drug conjugate achieved a degree of cytotoxicity similar to the free maytansonid drug, which could be increased by increasing the number of maytansinoid molecules per antibody molecule. The A7-maytansinoid conjugate showed low systemic cytotoxicity in mice.

### Anti-PRO218, Anti-PRO228, Anti-PRO271, Anti-PRO273, Anti-PRO295, Anti-PRO302, Anti-PRO305, Anti-PRO326, Anti-PRO386, Anti-PRO655, Anti-PRO162, Anti-PRO788, Anti-PRO792, Anti-PRO940, Anti-PRO941, Anti-PRO1004, Anti-PRO1012, Anti-PRO1016, Anti-PRO474, Anti-PRO5238, Anti-PRO1069, Anti-PRO1111, Anti-PRO1113, Anti-PRO1130, Anti-PRO1195, Anti-PRO1271. Anti-PRO1865, Anti-PRO1879, Anti-PRO3446, Anti-PRO3543, Anti-PRO4329, Anti-PRO4352, Anti-PRO5733, Anti-PRO9859, Anti-PRO9864, Anti-PRO9904. Anti-PRO9907, Anti-PRO10013, Anti-PRO90948, Anti-PRO28694, Anti-PRO16089, Anti-PRO19563, Anti-PRO19675, Anti-PRO20084, Anti-PRO21434, Anti-PRO50332, Anti-PRO38465 or Anti-PRO346 Antibody-Maytansinoid Conjugates (Immunoconjugates)

Anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PRO9907, anti-PRO10013, anti-PR090948, anti-PR028694, anti-PRO16089, anti-PRO19563, anti-PR019675, anti-PR020084, anti-PR021434, anti-PR050332, anti-PR038465 or anti-PR0346 antibody-maytansinoid conjugates are prepared by chemically linking an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PR01016, anti-PR0474, anti-PR05238, anti-PRO1069, anti-PRO1111, anti-PRO1113, anti-PRO1130, anti-PRO1195, anti-PR01271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PR019563, anti-PRO19675, anti-PR020084, anti-PR021434, anti-PR050332, anti-PR038465 or anti-PR0346 antibody to a maytansinoid molecule without significantly diminishing the biological activity of either the antibody or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5,208,020 or EP Patent 0 425 235 B1, and Chari et al., Cancer Research 52:127-131 (1992). The linking groups include disufide groups, thioether groups, acid labile groups, photolabile groups, peptidase labile groups, or esterase labile groups, as disclosed in the above-identified patents, disulfide and thioether groups being preferred.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 [1978]) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. The linkage is formed at the C-3 position of maytansinol or a maytansinol analogue.

### Calicheamicin

Another immunoconjugate of interest comprises an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PRO788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, γ₁^{I}, α₂^{I}, α₃^{I}, N-acetyl-γ₁^{I}, PSAG and θ^{I}₁ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

### Other cytotoxic agents

Other antitumor agents that can be conjugated to the anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PRO90948, anti-PR028694, anti-PRO16089, anti-PRO19563, anti-PRO19675, anti-PR020084, anti-PR021434, anti-PR050332, anti-PR038465 or anti-PR0346 antibodies of the invention include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PR019563, anti-PR019675, anti-PR020084, anti-PR021434, anti-PR050332, anti-PR038465 or anti-PR0346 antibodies. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the conjugate is used for diagnosis, it may comprise a radioactive atom for scintigraphic studies, for example tc^{99m} or I¹²³, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as tc^{99m} or I¹²³, Re¹⁸⁶, Re¹⁸⁸ and In¹¹¹ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

Alternatively, a fusion protein comprising the anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PR01130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PR019563, anti-PRO19675, anti-PR020084, anti-PR021434, anti-PR050332, anti-PR038465 or anti-PR0346 antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

The invention provides that the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

### 10. Immunoliposomes

The anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PRO9907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PR0346 antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Pat. Nos. 4,485,045 and 4,544,545; and WO97/38731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

### 11. Pharmaceutical Compositions of Antibodies

Antibodies specifically binding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide identified herein, as well as other molecules identified by the screening assays disclosed hereinbefore, can be administered for the treatment of various disorders in the form of pharmaceutical compositions.

If the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PRO20084, PR021434, PR050332, PR038465 or PR0346 polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g.,* Marasco et al., Proc. Natl. Acad. Sci. USA, 90: 7889-7893 (1993). The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g.,* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### G. Uses for Anti-PRO218, Anti-PRO228, Anti-PRO271, Anti-PRO273, Anti-PRO295, Anti-PRO302, Anti-PRO305, Anti-PR0326, Anti-PRO386, Anti-PRO655, Anti-PRO162, Anti-PR0788, Anti-PR0792, Anti-PRO940, Anti-PR0941, Anti-PRO1004, Anti-PRO1012, Anti-PRO1016, Anti-PRO474, Anti-PRO5238, Anti-PR01069. Anti-PRO1111, Anti-PRO1113, Anti-PRO1130, Anti-PRO1195, Anti-PRO1271, Anti-PRO1865,Anti-PRO1879, Anti-PRO3446, Anti-PRO3543, Anti-PRO4329, Anti-PRO4352, Anti-PR05733, Anti-PRO9859, Anti-PR09864, Anti-PR09904, Anti-PRO9907, Anti-PRO10013, Anti-PRO90948, Anti-PRO28694, Anti-PRO16089, Anti-PRO19563, Anti-PRO19675, Anti-PRO20084, Anti-PRO21434, Anti-PRO50332, Anti-PRO38465 or Anti-PR0346 Antibodies

The anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PRO941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PRO9907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibodies of the invention have various therapeutic and/or diagnostic utilities for a neurological disorder; a cardiovascular, endothelial or angiogenic disorder; an immunological disorder; an oncological disorder; an embryonic developmental disorder or lethality, or a metabolic abnormality. For example, anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibodies may be used in diagnostic assays for PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346, *e.g.*, detecting its expression (and in some cases, differential expression) in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014 (1974); Pain et al., J. Immunol. Meth., 40:219 (1981); and Nygren, J. Histochem. and Cytochem., 30:407 (1982).

Anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PRO788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PRO9907, anti-PRO10013, anti-PR090948, anti-PR028694, anti-PRO16089, anti-PR019563, anti-PRO19675, anti-PR020084, anti-PR021434, anti-PR050332, anti-PR038465 or anti-PR0346 antibodies also are useful for the affinity purification of PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides from recombinant cell culture or natural sources. In this process, the antibodies against PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides are immobilized on a suitable support, such a Sephadex resin or filter paper, using methods well known in the art. The immobilized antibody then is contacted with a sample containing the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide to be purified, and thereafter the support is washed with a suitable solvent that will remove substantially all the material in the sample except the PR0218, PR0228, PR0271, PR0273, PR0295, PR0302, PRO305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, which is bound to the immobilized antibody. Finally, the support is washed with another suitable solvent that will release the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide from the antibody.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (*e.g.,* Dayhoff, GenBank), and proprietary databases (e.g. LIFESEQ^{™}, Incyte Pharmaceuticals, Palo Alto, CA). The search was performed using the computer program BLAST or BLAST-2 (Altschul et al., Methods in Enzymology, 266:460-480 (1996)) as a comparison ofthe ECD protein sequences to a 6 frame translation of the EST sequences. Those comparisons with a BLAST score of 70 (or in some cases 90) or greater that did not encode known proteins were clustered and assembled into consensus DNA sequences with the program "phrap" (Phil Green, University of Washington, Seattle, WA).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel *et al.,* Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI sites.

### EXAMPLE 2: Isolation of cDNA clones by Amylase Screening

### 1. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

### 2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

### 3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, *e.g.* CsCl-gradient. The purified DNA was then carried on to the yeast protocols below.

The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11, his3-15, MAL⁺, SUC⁺, GAL⁺. Preferably, yeast mutants can be employed that have deficient post-translational pathways. Such mutants may have translocation deficient alleles in *sec*71*, sec*72*, sec*62, with truncated *sec*71 being most preferred. Alternatively, antagonists (including antisense nucleotides and/or ligands) which interfere with the normal operation of these genes, other proteins implicated in this post translation pathway (e.g., SEC61p, SEC72p, SEC62p, SEC63p, TDJ1p or SSA1p-4p) or the complex formation of these proteins may also be preferably employed in combination with the amylase-expressing yeast.

Transformation was performed based on the protocol outlined by Gietz et al., Nucl. Acid. Res., 20:1425 (1992). Transformed cells were then inoculated from agar into YEPD complex media broth (100 ml) and grown overnight at 30°C. The YEPD broth was prepared as described in Kaiser et al., Methods in Yeast Genetics, Cold Spring Harbor Press, Cold Spring Harbor, NY, p. 207 (1994). The overnight culture was then diluted to about 2 x 10⁶ cells/ml (approx. OD₆₀₀=0.1) into fresh YEPD broth (500 ml) and regrown to 1 x 10⁷ cells/ml (approx. OD₆₀₀=0.4-0.5).

The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM Li₂OOCCH₃), and resuspended into LiAc/TE (2.5 ml).

Transformation took place by mixing the prepared cells (100 µl) with freshly denatured single stranded salmon testes DNA (Lofstrand Labs, Gaithersburg, MD) and transforming DNA (1 µg, vol. < 10 µl) in microfuge tubes. The mixture was mixed briefly by vortexing, then 40% PEG/TE (600 µl, 40% polyethylene glycol-4000, 10 mM Tris-HCl, 1 mM EDTA, 100 mM Li₂OOCCH₃, pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 µl, 10 mM Tris-HCI, 1 mM EDTA pH 7.5) followed by recentrifugation. The cells were then diluted into TE (1 ml) and aliquots (200 µl) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press, Cold Spring Harbor, NY, p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15% (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation ofred starch into buffered SCD-Ura agar. Positive colonies were determined by their ability to break down starch resulting in a clear halo around the positive colony visualized directly.

### 4. Isolation of DNA bv PCR Amplification

When a positive colony was isolated, a portion of it was picked by a toothpick and diluted into sterile water (30 µl) in a 96 well plate. At this time, the positive colonies were either frozen and stored for subsequent analysis or immediately amplified. An aliquot of cells (5 µl) was used as a template for the PCR reaction in a 25 µl volume containing: 0.5 µl Klentaq (Clontech, Palo Alto, CA); 4.0 µl 10 mM dNTP's (Perkin Elmer-Cetus); 2.5 µl Kentaq buffer (Clontech); 0.25 µl forward oligo 1; 0.25 µl reverse oligo 2; 12.5 µl distilled water. The sequence of the forward oligonucleotide 1 was:
5'-TGTAAAACGACGGCCAGTTAAATAGACCTGCAATTATTAATCT-3' (SEQ ID NO:97)
The sequence of reverse oligonucleotide 2 was:
5'-CAGGAAACAGCTATGACCACCTGCACACCTGCAAATCCATT-3' (SEQ ID NO:98)
PCR was then performed as follows:

| | | | | |
|---|---|---|---|---|
| a. | | Denature | 92°C, | 5 minutes |
| | | | | |
| b. | 3 cycles of: | Denature | 92°C, | 30 seconds |
| | | Anneal | 59°C, | 30 seconds |
| | | Extend | 72°C, | 60 seconds |
| | | | | |
| c. | 3 cycles of: | Denature | 92°C, | 30 seconds |
| | | Anneal | 57°C, | 30 seconds |
| | | Extend | 72°C, | 60 seconds |
| | | | | |
| d. | 25 cycles of: | Denature | 92°C, | 30 seconds |
| | | Anneal | 55°C, | 30 seconds |
| | | Extend | 72°C, | 60 seconds |
| | | | | |
| e. | | Hold | 4°C | |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR, an aliquot of the reaction (5 µl) was examined by agarose gel electrophoresis in a 1 % agarose gel using a Tris-Borate-EDTA (TBE) buffering system as described by Sambrook *et al., supra.* Clones resulting in a single strong PCR product larger than 400 bp were further analyzed by DNA sequencing after purification with a 96 Qiaquick PCR clean-up column (Qiagen Inc., Chatsworth, CA).

### EXAMPLE 3: Isolation of cDNA Clones Using Signal Algorithm Analysis

Various polypeptide-encoding nucleic acid sequences were identified by applying a proprietary signal sequence finding algorithm developed by Genentech, Inc. (South San Francisco, CA) upon ESTs as well as clustered and assembled EST fragments from public (*e.g.*, GenBank) and/or private (LIFESEQ®, Incyte Pharmaceuticals, Inc., Palo Alto, CA) databases. The signal sequence algorithm computes a secretion signal score based on the character ofthe DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

Using the techniques described in Examples 1 to 3 above, numerous full-length cDNA clones were identified as encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides as disclosed herein. These cDNAs were then deposited under the terms of the Budapest Treaty with the American Type Culture Collection, 10801 University Blvd., Manassas, VA 20110-2209, USA (ATCC) as shown in Table 7 below. In addition, the sequence ofDNA257845 encoding PR05238 polypeptides was identified from GenBank accession no.: AF369794; the sequence of DNA82343 encoding PR05733 polypeptides was identified from GenBank accession no.: BC017089; the sequence ofDNA336882 encoding PRO90948polypeptides was identified from GenBank accession no.: AK045869; the sequence of DNA184073 encoding PR028694 polypeptides was identified from GenBank accession no.: AX281784; the sequence of DNA255255 encoding PR050332 polypeptides was identified from GenBank accession no.: AB040120; and the sequence of DNA228002 encoding PR038465 polypeptides was identified from GenBank accession no.: AF142409.

**Table 7**

| Material | ATCC Dep. No. | Deposit Date |
|---|---|---|
| DNA30867-1335 | 209807 | April 28, 1998 |
| DNA33092-1202 | 209420 | October 18, 1998 |
| DNA39423-1182 | 209387 | October 17, 1997 |
| DNA39523-1192 | 209424 | October 31, 1997 |
| DNA38268-1188 | 209421 | October 28, 1997 |
| DNA40370-1217 | 209485 | November 21, 1997 |
| DNA40619-1220 | 209525 | December 10, 1997 |
| DNA37140-1234 | 209489 | November 12, 1997 |
| DNA45415-1318 | 209810 | April 28, 1998 |
| DNA50960-1224 | 209509 | December 3, 1997 |
| DNA56965-1356 | 209842 | May 6, 1998 |
| DNA56405-1357 | 209849 | May 6, 1998 |
| DNA56352-1358 | 209846 | May 6, 1998 |
| DNA54002-1367 | 209754 | April 7, 1998 |
| DNA53906-1368 | 209747 | April 7, 1998 |
| DNA57844-1410 | 203010 | June 23, 1998 |
| DNA56439-1376 | 209864 | May 14, 1998 |
| DNA56113-1378 | 203049 | July 1, 1998 |
| DNA56045-1380 | 209865 | May 14, 1998 |
| DNA59211-1450 | 209960 | June 9, 1998 |
| DNA58721-1475 | 203110 | August 11, 1998 |
| DNA57254-1477 | 203289 | September 29, 1998 |
| DNA59814-1486 | 203359 | October 20, 1998 |
| DNA65412-1523 | 203094 | August 4, 1998 |
| DNA66309-1538 | 203235 | September 15, 1998 |
| DNA81757-2512 | 203543 | December 15, 1998 |
| DNA54009-2517 | 203574 | January 12, 1999 |
| DNA92219-2541 | 203663 | February 9, 1999 |
| DNA86571-2551 | 203660 | February 9, 1999 |
| DNA77629-2573 | 203850 | March 16, 1999 |
| DNA87976-2593 | 203888 | March 30, 1999 |
| DNA125170-2780 | PTA-953 | November 16, 1999 |
| DNA125151-2784 | PTA-1029 | December 7, 1999 |
| DNA129549-2798 | PTA-1099 | December 22, 1999 |
| DNA142392-2800 | PTA-1092 | December 22, 1999 |
| DNA125181-2804 | PTA-1096 | December 22, 1999 |
| DNA150163-2842 | PTA-1533 | March 21, 2000 |
| DNA96861-2844 | PTA-1436 | March 2, 2000 |
| DNA131658-2875 | PTA-1671 | April 11, 2000 |
| DNA168061-2897 | PTA-1600 | March 30, 2000 |
| DNA147253-2983 | PTA-2405 | August 22, 2000 |
| DNA44167-1243 | 209434 | November 7, 1997 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposits will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

### EXAMPLE 4: Isolation of cDNA clones Encoding Human PRO218 Polypeptides [UNQ192]

A consensus sequence was obtained relative to a variety of EST sequences as described in Example 1 above, wherein the consensus sequence obtained is herein designated DNA17411. Two proprietary Genentech EST sequences were employed in the consensus assembly. Based on the DNA17411 consensus sequence, oligonucleotides were synthesized: 1) to identify by PCR a cDNA library that contained the sequence of interest, and 2) for use as probes to isolate a clone of the full-length coding sequence for PR0218.

A pair of PCR primers (forward and reverse) were synthesized:
forward PCR primer 5'-AAGTGGAGCCGGAGCCTTCC-3' (SEQ ID NO:99);
reverse PCR primer 5'-TCGTTGTTTATGCAGTAGTCGG-3' (SEQ ID NO:100).
Additionally, a synthetic oligonucleotide hybridization probe was constructed from the consensus DNA17411 sequence which had the following nucleotide sequence:
hybridization probe
5'-ATTGTTTAAAGACTATGAGATACGTCAGTATGTTGTACAGG-3' (SEQ ID NO:101).

In order to screen several libraries for a source of a full-length clone, DNA from the libraries was screened by PCR amplification with the PCR primer pair identified above. A positive library was then used to isolate clones encoding the PR0218 gene using the probe oligonucleotide and one of the PCR primers. RNA for construction of the cDNA libraries was isolated from human fetal kidney tissue (LIB28).

DNA sequencing of the clones isolated as described above gave the full-length DNA sequence for PR0218 [herein designated as UNQ192 (DNA30867-1335)] (SEQ ID NO:1) and the derived protein sequence for PR0218.

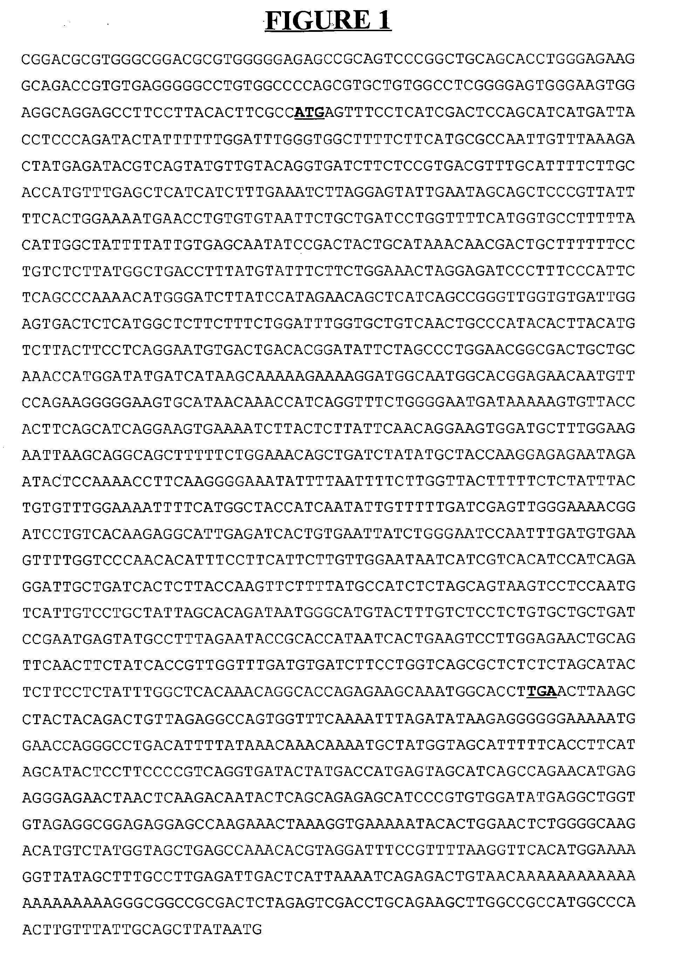

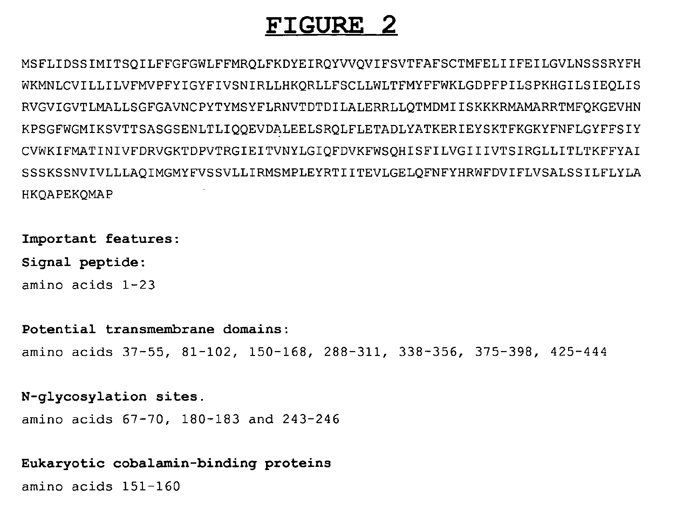

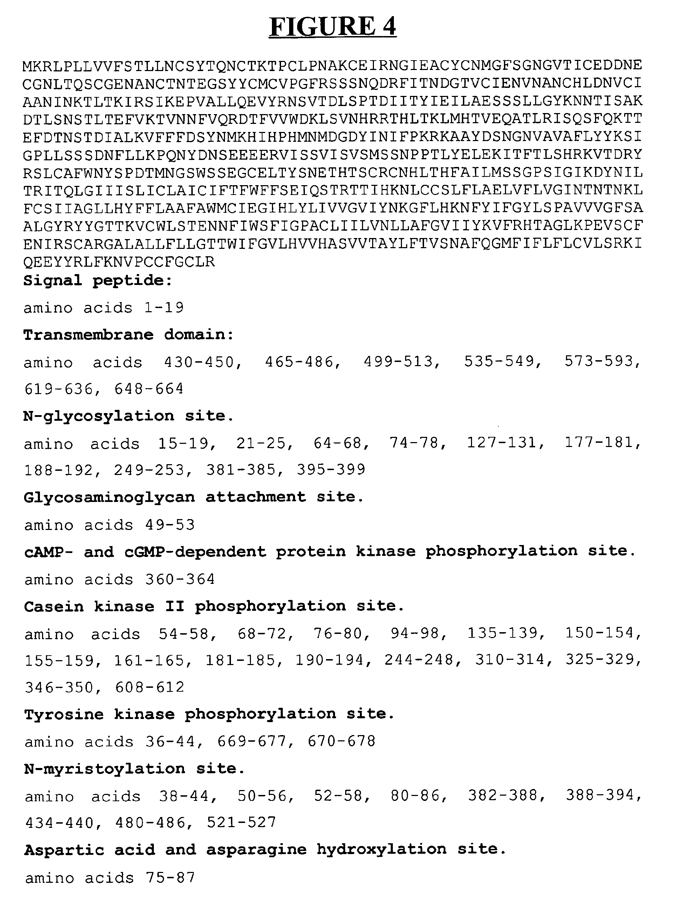

The entire nucleotide sequence of UNQ192 (DNA30867-1335) is shown in Figure 1 (SEQ ID NO:1). Clone UNQ 192 (DNA30867-1335) contains a single open reading frame with an apparent translational initiation site at nucleotide positions 150-152 and ending at the stop codon at nucleotide positions 1515-1517 (Figure 1). The predicted polypeptide precursor is 455 amino acids long (Figure 2; SEQ ID NO:2). The full-length PR0218 protein shown in Figure 4 has an estimated molecular weight of about 52,917 daltons and a pI of about 9.5. Clone UNQ192 (DNA30867-1335) has been deposited with the ATCC on April 28, 1998 with ATCC deposit number 209807. Regarding the sequence, it is understood that the deposited clone contains the correct sequence, and the sequences provided herein are based on known sequencing techniques.

Analysis of the amino acid sequence of the full-length PR0218 polypeptide suggests that PR0218 may be a novel transmembrane protein.

Still analyzing the amino acid sequence of SEQ ID NO:2, the putative signal peptide is at about amino acids 1 through 23 of SEQ ID NO:2. Transmembrane domains are potentially at about amino acids 37-55, 81-102, 150-168, 288-311, 338-356, 375-398, and 425-444 of SEQ ID NO:2. N-glycosylation sites are at about amino acids 67, 180, and 243 of SEQ ID NO:2. Eukaryotic cobalamin-binding protein is at about amino acids 151-160 of SEQ ID NO:2. The corresponding nucleotides can be routinely determined given the sequences provided herein.

### EXAMPLE 5: Isolation of cDNA clones Encoding Human PRO228 Polypeptides [UNQ202]

A consensus DNA sequence was assembled relative to other EST sequences using phrap as described in Example 1 above. This consensus sequence is herein designated DNA28758. An EST proprietary to Genentech was employed in the consensus assembly. This EST is herein designated as DNA21951.

Based on the DNA28758 consensus sequence, oligonucleotides were synthesized: 1) to identify by PCR a cDNA library that contained the sequence of interest, and 2) for use as probes to isolate a clone of the full-length coding sequence for PR0228.

PCR primers (forward and reverse) were synthesized:
forward PCR primer 5'-GGTAATGAGCTCCATTACAG-3' (SEQ ID NO:102)
forward PCR primer 5'-GGAGTAGAAAGCGCATGG-3' (SEQ ID NO:103)
forward PCR primer 5'-CACCTGATACCATGAATGGCAG-3' (SEQ ID NO:104)
reverse PCR primer 5'-CGAGCTCGAATTAATTCG-3' (SEQ ID NO:105)
reverse PCR primer 5'-GGATCTCCTGAGCTCAGG-3' (SEQ ID NO:106)
reverse PCR primer 5'-CCTAGTTGAGTGATCCTTGTAAG-3' (SEQ ID NO:107)
Additionally, a synthetic oligonucleotide hybridization probe was constructed from the consensus DNA28758
sequence which had the following nucleotide sequence
hybridization probe
5'-ATGAGACCCACACCTCATGCCGCTGTAATCACCTGACACATTTTGCAATT-3' (SEQ ID NO:108)

In order to screen several libraries for a source of a full-length clone, DNA from the libraries was screened by PCR amplification with the PCR primer pairs identified above. A positive library was then used to isolate clones encoding the PR0228 gene using the probe oligonucleotide and one of the PCR primers.

RNA for construction of the cDNA libraries was isolated from human fetal kidney tissue.

DNA sequencing of the clones isolated as described above gave the full-length DNA sequence for PR0228 [herein designated as DNA33092-1202] (SEQ ID NO:3) and the derived protein sequence for PR0228.

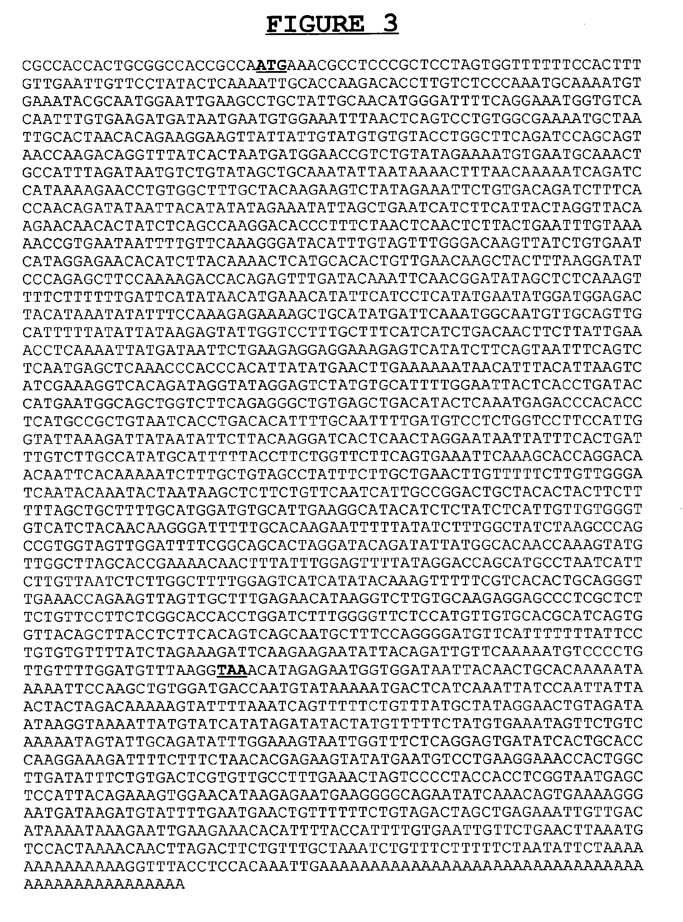

The entire nucleotide sequence of DNA33092-1202 is shown in Figure 3 (SEQ ID NO:3). Clone DNA33092-1202 contains a single open reading frame with an apparent translational initiation site at nucleotide positions 24-26 of SEQ ID NO:3 and ending at the stop codon after nucleotide position 2093 of SEQ ID NO:3. The predicted polypeptide precursor is 690 amino acids long (Figure 4; SEQ ID NO:4). Clone DNA33092-1202 has been deposited with ATCC on October 18, 1997 and is assigned ATCC deposit no. ATCC 209420.

Analysis of the amino acid sequence of the full-length PR0228 polypeptide suggests that portions of it possess significant homology to the secretin-related proteins CD97 and EMR1 as well as the secretin member, latrophilin, thereby indicating that PR0228 may be a new member of the secretin related proteins.

### EXAMPLE 6: Isolation of cDNA clones Encoding Human PRO271 Polypeptides [UNQ238]

A consensus DNA sequence was assembled relative to other EST sequences using phrap as described in Example 1 above. This consensus sequence is herein designated DNA35737. Based on the DNA35737 consensus sequence, oligonucleotides were synthesized: 1) to identify by PCR a cDNA library that contained the sequence of interest, and 2) for use as probes to isolate a clone of the full-length coding sequence for PR0271.

Forward and reverse PCR primers were synthesized:
forward PCR primer 1 5'-TGCTTCGCTACTGCCCTC-3' (SEQ ID NO:109)
forward PCR primer 2 5'-TTCCCTTGTGGGTTGGAG-3' (SEQ ID NO:110)
forward PCR primer 3 5'-AGGGCTGGAAGCCAGTTC-3' (SEQ ID NO:111)
reverse PCR primer 1 5'-AGCCAGTGAGGAAATGCG-3' (SEQ ID NO:112)
reverse PCR primer 2 5'-TGTCCAAAGTACACACACCTGAGG-3' (SEQ ID NO:113)
Additionally, a synthetic oligonucleotide hybridization probe was constructed from the consensus DNA35737 sequence which had the following nucleotide sequence
hybridization probe
5'-GATGCCACGATCGCCAAGGTGGGACAGCTCTTTGCCGCCTGGAAG-3' (SEQ ID NO:114)

In order to screen several libraries for a source of a full-length clone, DNA from the libraries was screened by PCR amplification with the PCR primer pair identified above. A positive library was then used to isolate clones encoding the PR0271 gene using the probe oligonucleotide and one of the PCR primers.

RNA for construction of the cDNA libraries was isolated from human fetal brain tissue.

DNA sequencing of the clones isolated as described above gave the full-length DNA sequence for PR0271 [herein designated as DNA39423-1182] (SEQ ID NO:5) and the derived protein sequence for PR0271.

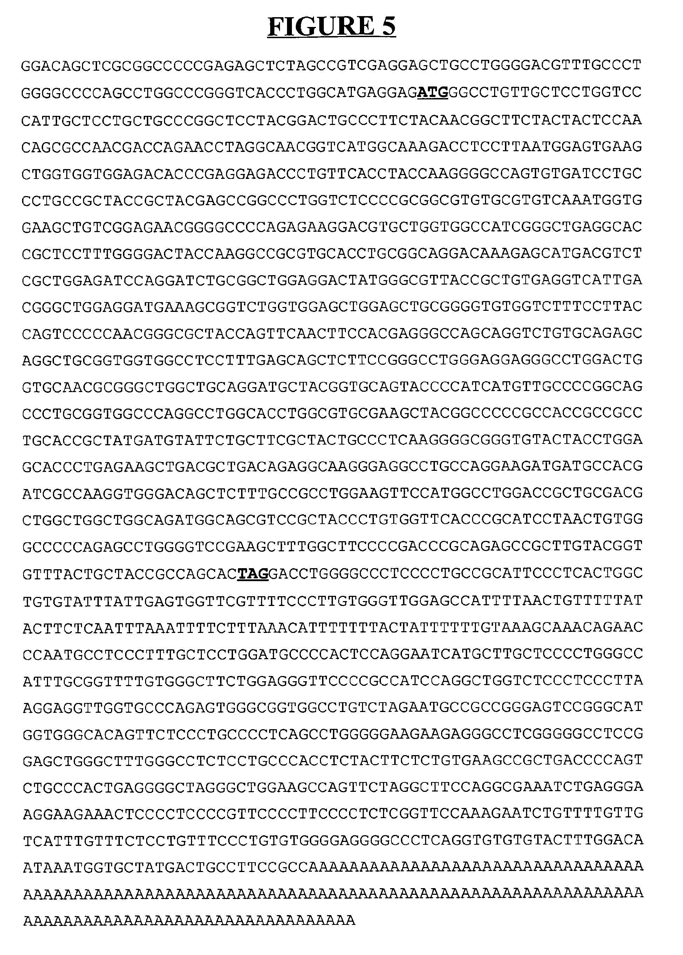

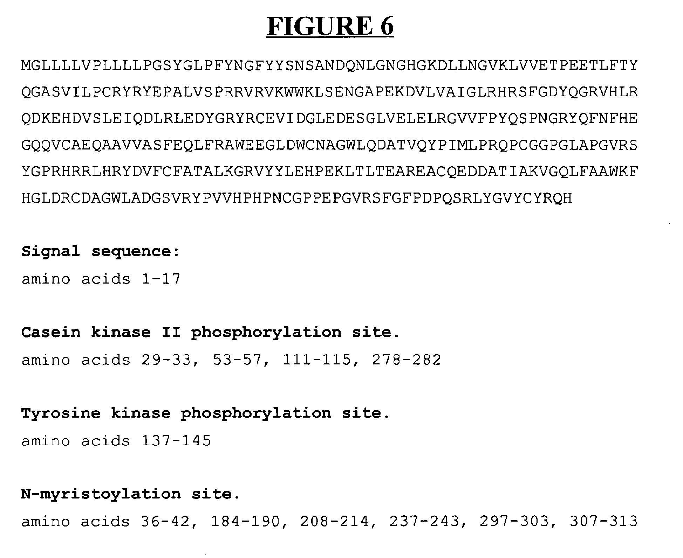

The entire nucleotide sequence of DNA39423-1182 is shown in Figure 5 (SEQ ID NO:5). Clone DNA39423-1182 contains a single open reading frame with an apparent translational initiation site at nucleotide positions 101-103 and ending at the stop codon at nucleotide positions 1181-1183 (Figure 5). The predicted polypeptide precursor is 360 amino acids long (Figure 6; SEQ ID NO:6). Clone DNA39423-1182 has been deposited with ATCC and on October 17, 1997 and is assigned ATCC deposit no. ATCC 209387.

Analysis of the amino acid sequence of the full-length PR0271 polypeptide suggests that it possess significant homology to the proteoglycan link protein, thereby indicating that PRO271 may be a link protein homolog.

### EXAMPLE 7: Isolation of cDNA clones Encoding Human PRO273 Polypeptides [UNQ240]

A consensus sequence was obtained relative to a variety of EST sequences as described in Example 1 above, wherein the consensus sequence obtained is herein designated DNA36465. Based on the DNA36465 consensus sequence, oligonucleotides were synthesized: 1) to identify by PCR a cDNA library that contained the sequence of interest, and 2) for use as probes to isolate a clone of the full-length coding sequence for PR0273.

A pair of PCR primers (forward and reverse) were synthesized:
forward PCR primer 5'-CAGCGCCCTCCCCATGTCCCTG-3' (SEQ ID NO:115)
reverse PCR primer 5'-TCCCAACTGGTTTGGAGTTTTCCC-3' (SEQ ID NO:116)
Additionally, a synthetic oligonucleotide hybridization probe was constructed from the consensus DNA36465 sequence which had the following nucleotide sequence
hybridization probe
5'-CTCCGGTCAGCATGAGGCTCCTGGCGGCCGCTGCTCCTGCTGCTG-3' (SEQ ID NO:117)

In order to screen several libraries for a source of a full-length clone, DNA from the libraries was screened by PCR amplification with the PCR primer pair identified above. A positive library was then used to isolate clones encoding the PR0273 gene using the probe oligonucleotide and one of the PCR primers. RNA for construction of the cDNA libraries was isolated from human fetal kidney tissue.

DNA sequencing of the clones isolated as described above gave the full-length DNA sequence for PR0273 [herein designated as UNQ240 (DNA39523-1192)] (SEQ ID NO:7) and the derived protein sequence for PR0273.

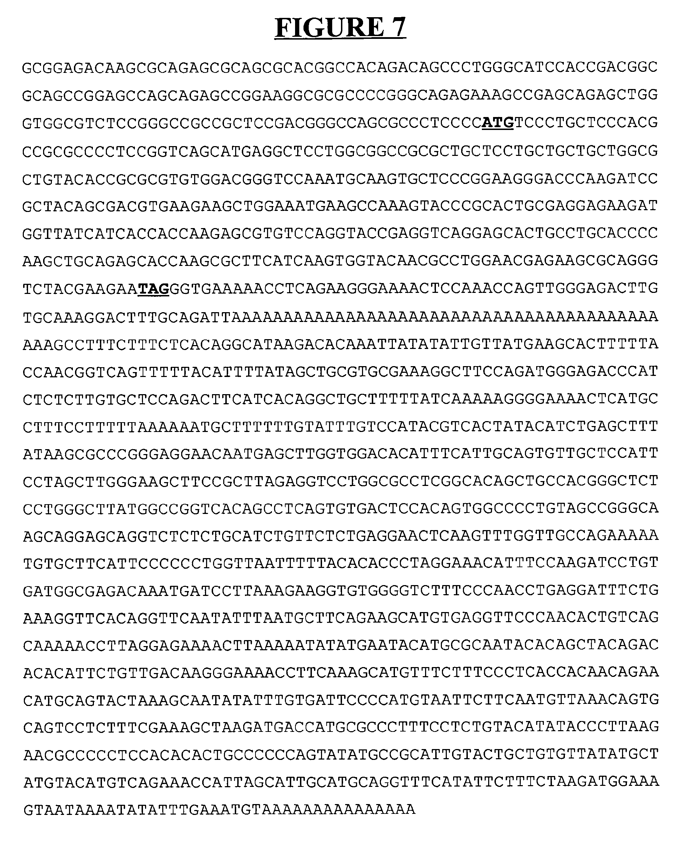

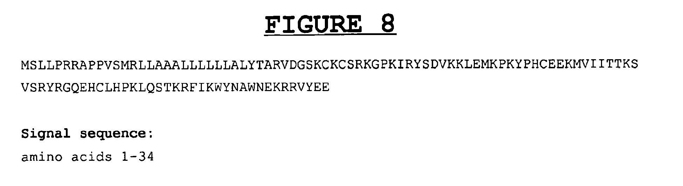

The entire nucleotide sequence of UNQ240 (DNA39523-1192) is shown in Figure 7 (SEQ ID NO:7). Clone UNQ240 (DNA39523-1192) contains a single open reading frame with an apparent translational initiation site at nucleotide positions 167-169 and ending at the stop codon at nucleotide positions 500-502 (Figure 7). The predicted polypeptide precursor is 111 amino acids long (Figure 8; SEQ ID NO:8). Clone UNQ240 (DNA39523-1192) has been deposited with the ATCC on October 31, 1997 and is assigned ATCC number 209424. It is understood that the deposited clone contains the actual sequence and that the sequences provided herein are merely representative based on current sequencing techniques. Moreover, given the sequences provided herein and knowledge of the universal genetic code, the corresponding nucleotides for any given amino acid can be routinely identified and vice versa.

Analysis of the amino acid sequence of the full-length PR0273 polypeptide suggests that portions of it possess sequence identity with human macrophage inflammatory protein-2, cytokine-induced neutrophil chemoattractant 2, and neutrophil chemotactic factor 2-beta, thereby indicating that PR0273 is a novel chemokine.

As discussed further below, the cDNA was subcloned into a baculovirus vector and expressed in insect cells as a C-terminally tagged IgG fusion protein. N-terminal sequencing of the resultant protein identified the signal sequence cleavage site, yielding a mature polypeptide of 77 amino acids. The mature sequence, showing 31-40% identity to other human CXC chemokines, includes the four canonical cysteine residues but lacks the ELR motif. Northern analysis demonstrates expression at least in the small intestine, colon, spleen, lymph node and kidney. By in situ hybridization, also described in detail below, mRNA is localized to the lamina propria of intestinal villi and to renal tubules.

### EXAMPLE 8: Isolation of cDNA clones Encoding Human PRO295 Polypeptides [UNQ258]

A consensus DNA sequence was assembled relative to other EST sequences using phrap as described in Example 1 above. This consensus sequence is herein designated DNA35814. Based on the DNA35814 consensus sequence, oligonucleotides were synthesized: 1) to identify by PCR a cDNA library that contained the sequence of interest, and 2) for use as probes to isolate a clone of the full-length coding sequence for PR0295.

Forward and reverse PCR primers were synthesized:
forward PCR primer (.f1) 5'-GCAGAGCGGAGATGCAGCGGCTTG-3' (SEQ ID NO:118)
forward PCR primer (.f2) 5'-CCCAGCATGTACTGCCAG-3' (SEQ ID NO:119)
forward PCR primer (.f3) 5'-TTGGCAGCTTCATGGAGG-3' (SEQ ID NO:120)
forward PCR primer (.f4) 5'-CCTGGGCAAAAATGCAAC-3' (SEQ ID NO:121)
reverse PCR primer (.r1) 5'-CTCCAGCTCCTGGCGCACCTCCTC-3' (SEQ ID NO:122)
Additionally, a synthetic oligonucleotide hybridization probe was constructed from the consensus DNA35814 sequence which had the following nucleotide sequence
hybridization probe
5'-GGCTCTCAGCTACCGCGCAGGAGCGAGGCCACCCTCAATGAGATG-3'
(SEQ ID NO:123)

In order to screen several libraries for a source of a full-length clone, DNA from the libraries was screened by PCR amplification with the PCR primer pairs identified above. A positive library was then used to isolate clones encoding the PR0295 gene using the probe oligonucleotide and one of the PCR primers.

RNA for construction of the cDNA libraries was isolated from human fetal lung tissue.

DNA sequencing of the clones isolated as described above gave the full-length DNA sequence for PR0295 [herein designated as DNA38268-1188] (SEQ ID NO:9) and the derived protein sequence for PR0295.

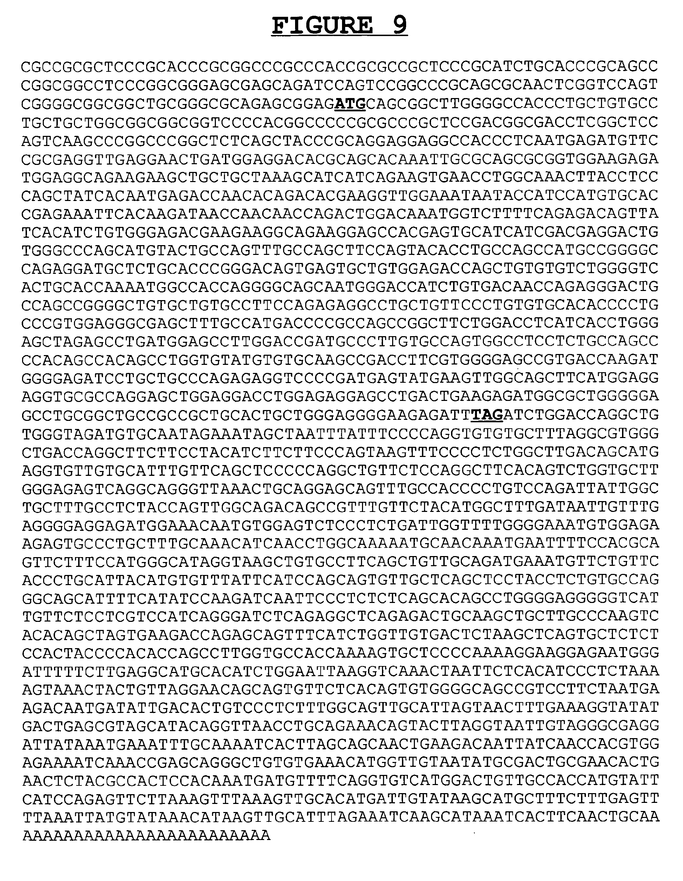

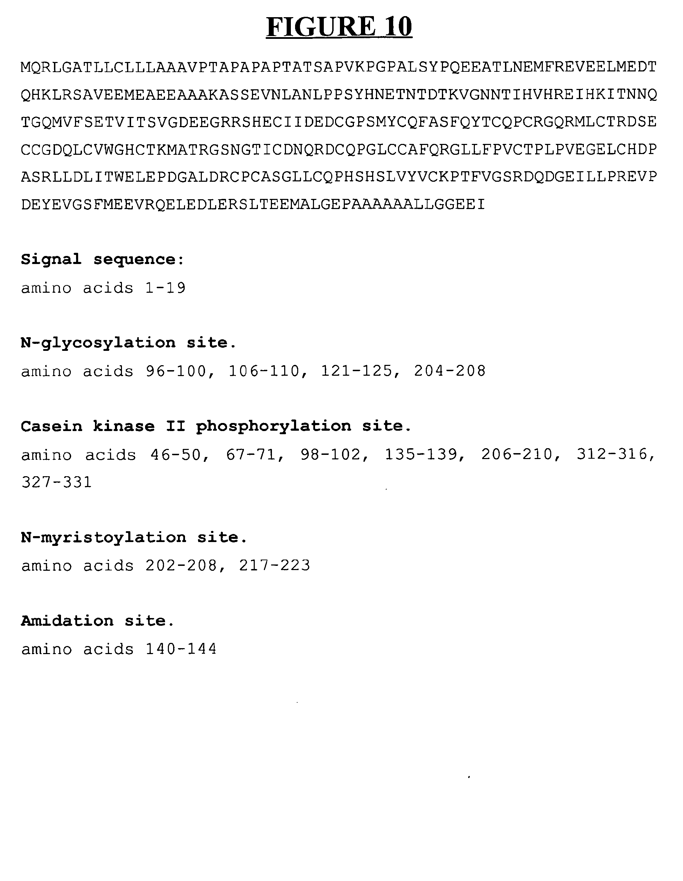

The entire nucleotide sequence of DNA38268-1188 is shown in Figure 9 (SEQ ID NO:9). Clone DNA38268-1188 contains a single open reading frame with an apparent translational initiation site at nucleotide positions 153-155 and ending at the stop codon at nucleotide positions 1202-1204 (Figure 9). The predicted polypeptide precursor is 350 amino acids long (Figure 10; SEQ ID NO:10). Clone DNA38268-1188 has been deposited with ATCC on October 28,1997 and is assigned ATCC deposit no. 209421.

Analysis of the amino acid sequence of the full-length PR0295 polypeptide suggests that portions of it possess significant homology to the integrin proteins, thereby indicating that PR0295 may be a novel integrin.

### EXAMPLE 9: Isolation of cDNA clones Encoding Human PRO302 Polypeptides [UNQ265]

Consensus DNA sequences were assembled relative to other EST sequences using phrap as described in Example 1 above. These consensus sequences are herein designated DNA35953. Based on the DNA35953 consensus sequence, oligonucleotides were synthesized: 1) to identify by PCR a cDNA library that contained the sequence of interest, and 2) for use as probes to isolate a clone of the full-length coding sequence for PR0302.

PCR primers (forward and reverse) were synthesized:
forward PCR primer 1 5'-GTCCGCAAGGATGCCTACATGTTC-3' (SEQ ID NO:124)
forward PCR primer 2 5'-GCAGAGGTGTCTAAGGTTG-3' (SEQ ID NO:125)
reverse PCR primer 5'-AGCTCTAGACCAATGCCAGCTTCC-3' (SEQ ID NO:126)
Also, a synthetic oligonucleotide hybridization probe was constructed from the consensus DNA35953 sequence which had the following nucleotide sequence
hybridization probe
5'-GCCACCAACTCCTGCAAGAACTTCTCAGAACTGCCCCTGGTCATG-3' (SEQ ID NO:127)

In order to screen several libraries for a source of a full-length clone, DNA from the libraries was screened by PCR amplification with the PCR primer pairs identified above. A positive library was then used to isolate clones encoding the PR0302 gene using the probe oligonucleotide and one of the PCR primers.

RNA for construction of the cDNA libraries was isolated from human fetal kidney tissue (LIB228).

DNA sequencing of the clones isolated as described above gave the full-length DNA sequence for PR0302 [herein designated as DNA40370-1217] (SEQ ID NO:11) and the derived protein sequence for PR0302.

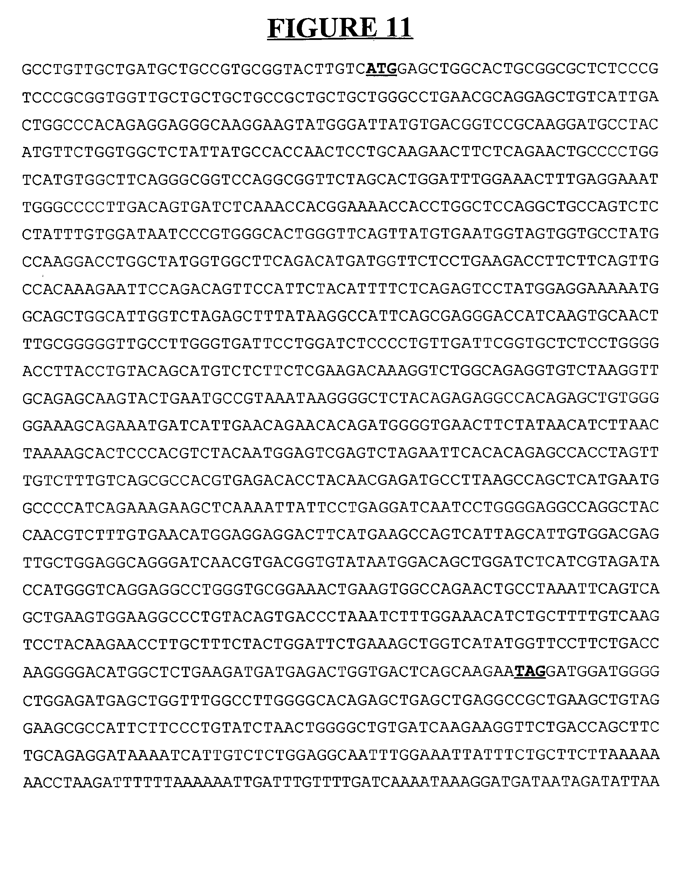

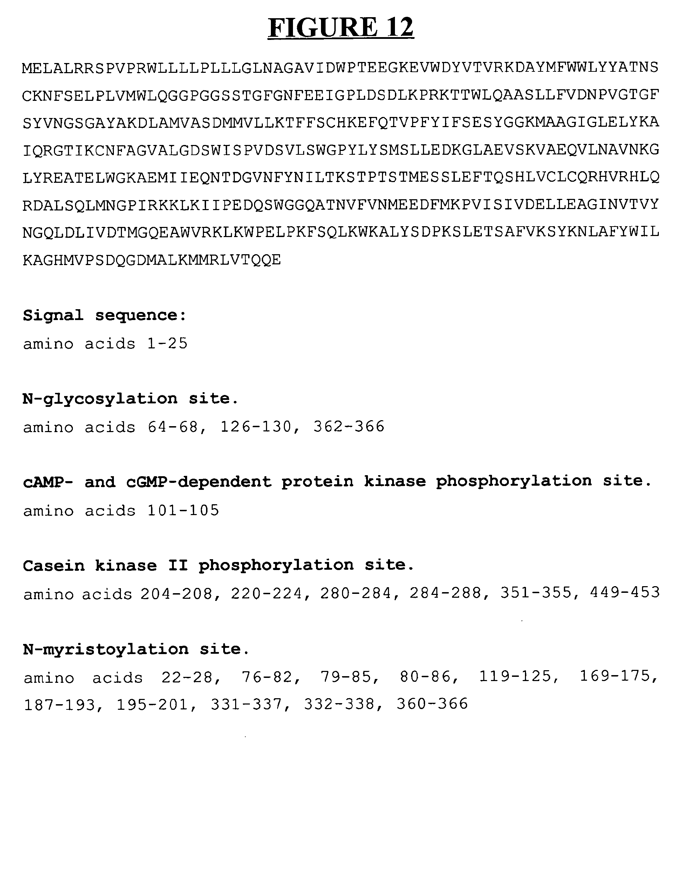

The entire nucleotide sequence of DNA40370-1217 is shown in Figure 11 (SEQ ID NO:11). Clone DNA40370-1217 contains a single open reading frame with an apparent translational initiation site at nucleotide positions 34-36 and ending at the stop codon at nucleotide positions 1390-1392 (Figure 11). The predicted polypeptide precursor is 452 amino acids long (Figure 12; SEQ ID NO:12). Various unique aspects of the PRO302 protein are shown in Figure 12. Clone DNA40370-1217 has been deposited with the ATCC on November 21, 1997 and is assigned ATCC deposit no. ATCC 209485.

### EXAMPLE 10: Isolation of cDNA clones Encoding Human PRO305 Polypeptides [UNQ268]

The extracellular domain (ECD) sequences (including the secretion signal, if any) of from about 950 known secreted proteins from the Swiss-Prot public protein database were used to search expressed sequence tag (EST) databases. The EST databases included public EST databases (e.g., GenBank) and a proprietary EST DNA database (LIFESEQTM, Incyte Pharmaceuticals, Palo Alto, CA). The search was performed using the computer program BLAST or BLAST2 (Altshul et al., Methods in Enzymology 266:460-480 (1996)) as a comparison of the ECD protein sequences to a 6 frame translation of the EST sequence. Those comparisons resulting in a BLAST score of 70 (or in some cases 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap. This consensus sequence is herein designated DNA36440-from dna. In some cases, the consensus DNA sequence was extended using repeated cycles of BLAST and phrap to extend the consensus sequence as far as possible using the sources of EST sequences discussed above (the initial sequence used is designated DNA36440.init).

Based on the DNA3 6440-from dna consensus sequence, oligonucleotides were synthesized: 1) to identify by PCR a cDNA library that contained the sequence of interest, and 2) for use as probes to isolate a clone of the full-length coding sequence for PR0305. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, with the PCR primer pair. A positive library was then used to isolate clones encoding the gene of interest by the in vivo cloning procedure using the probe oligonucleotide and one of the primer pairs.

A pair of PCR primers (forward and reverse) were synthesized:
forward PCR primer 5'-TGCGACGGCTGCTGGTTTTGAAAC-3' (SEQ ID NO:128)
reverse PCR primer 5'-AAAGCATTCATGGCCATTGTGAAG-3' (SEQ ID NO:129)
Additionally, a synthetic oligonucleotide hybridization probe was constructed from the consensus DNA36440-from dna sequence which had the following nucleotide sequence
hybridization probe
5'-CGCTCGTCCTGGCTGCCTTTTGCTTGGGAATAGCCTCCGCTGTTC-3' (SEQ ID NO:130)

In order to screen several libraries for a source of a full-length clone, DNA from the libraries was screened by PCR amplification with the PCR primer pair identified above. A positive library was then used to isolate clones encoding the PR0305 gene using the probe oligonucleotide and one of the PCR primers.

RNA for construction of the cDNA libraries was isolated from human fetal lung tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

DNA sequencing of the clones isolated as described above gave the full-length DNA sequence for PR0305 [herein designated as UNQ268 (DNA40619-seqmin)] (SEQ ID NO:13) and the derived protein sequence for PR0305.

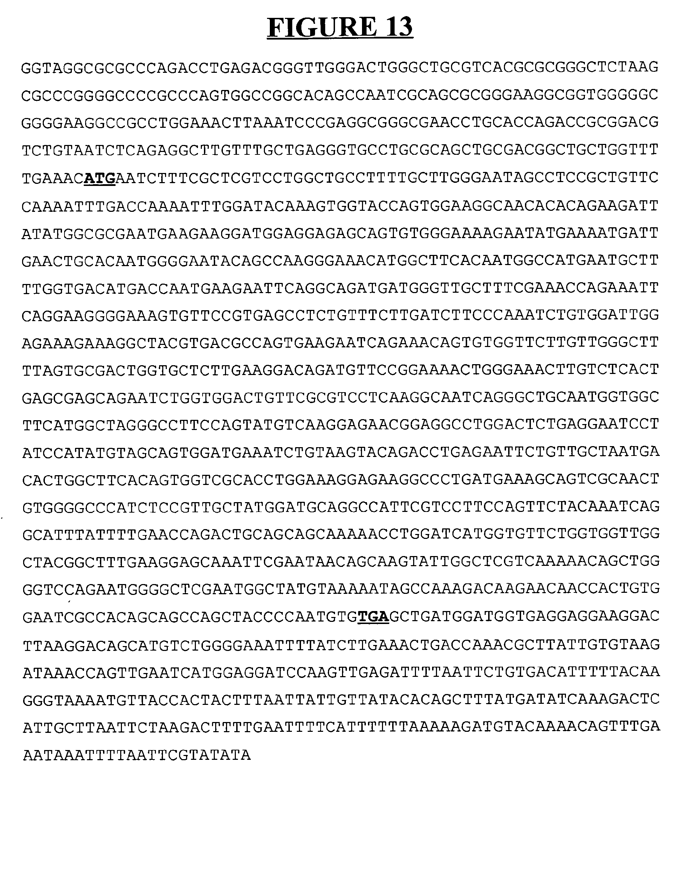

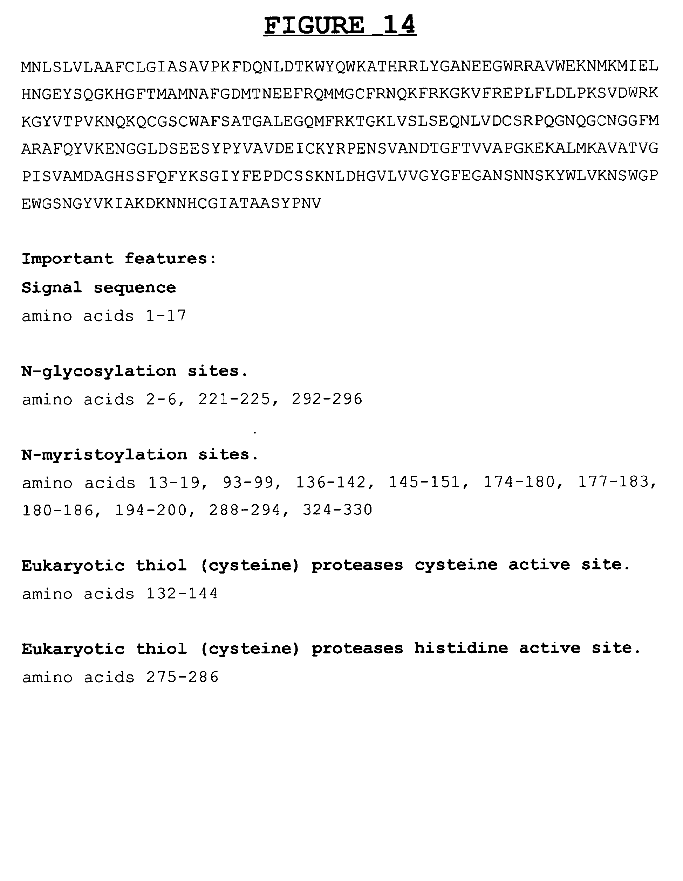

The entire nucleotide sequence of UNQ268 (DNA40619-seqmin) is shown in Figure 13 (SEQ ID NO:13). Clone UNQ268 (DNA40619-seqmin) contains a single open reading frame with an apparent translational initiation site at nucleotide positions 251-253 and ending at the stop codon at nucleotide positions 1253-1255 (Figure 13). The predicted polypeptide precursor is 334 amino acids long (Figure 14; SEQ ID NO:14). Clone UNQ268 (DNA40619-seqmin) has been deposited with ATCC on December 10, 1997and is assigned ATCC deposit no. 209525.

Analysis of the amino acid sequence of the full-length PR0305 polypeptide suggests that portions of it possess significant homology to the human procathepsin L protein thereby indicating that PR0305 is a novel member of the cathepsin family.

Analysis of the amino acid sequence of Figure 14 (SEQ ID NO:14) shows the following characteristics. The signal peptide is from amino acids 1 through 17. The start of the mature peptide begins with amino acid 18. The cysteine proteases cysteine active site is from amino acids 132 through 143. The cysteine proteases histidine active site is from amino acids 275 through 285. Potential N-glycosylation sites are at amino acids 221 and 292. The active site by homology to "CATL-PIG" is from amino acids 301 through 334.

### EXAMPLE 11: Isolation of cDNA clones Encoding Human PRO326 Polypeptides [UNQ287]

A consensus DNA sequence was assembled relative to other EST sequences using phrap as described in Example 1 above. This consensus sequence is herein designated DNA3 6685. Based on the DNA36685 consensus sequence, and Incyte EST sequence no. 2228990, oligonucleotides were synthesized: 1) to identify by PCR a cDNA library that contained the sequence of interest, and 2) for use as probes to isolate a clone of the full-length coding sequence for PR0326.

Forward and reverse PCR primers were synthesized for the determination of PR0326:
forward PCR primer 5'-ACTCCAAGGAAATCGGATCCGTTC-3' (SEQ ID NO:131)
reverse PCR primer 5'-TTAGCAGCTGAGGATGGGCACAAC-3' (SEQ ID NO:132)
Additionally, a synthetic oligonucleotide hybridization probe was constructed for the determination of PRO331 which had the following nucleotide sequence
hybridization probe
5'-GCCTTCACTGGTTTGGATGCATTGGAGCATCTAGACCTGAGTGACAACGC-3'
(SEQ ID NO:133)

In order to screen several libraries for a source of a full-length clone, DNA from the libraries was screened by PCR amplification with the PCR primer pairs identified above. A positive library was then used to isolate clones encoding the PR0326 gene using the probe oligonucleotide and one of the PCR primers.

RNA for construction of the cDNA libraries was isolated from human fetal kidney tissue.

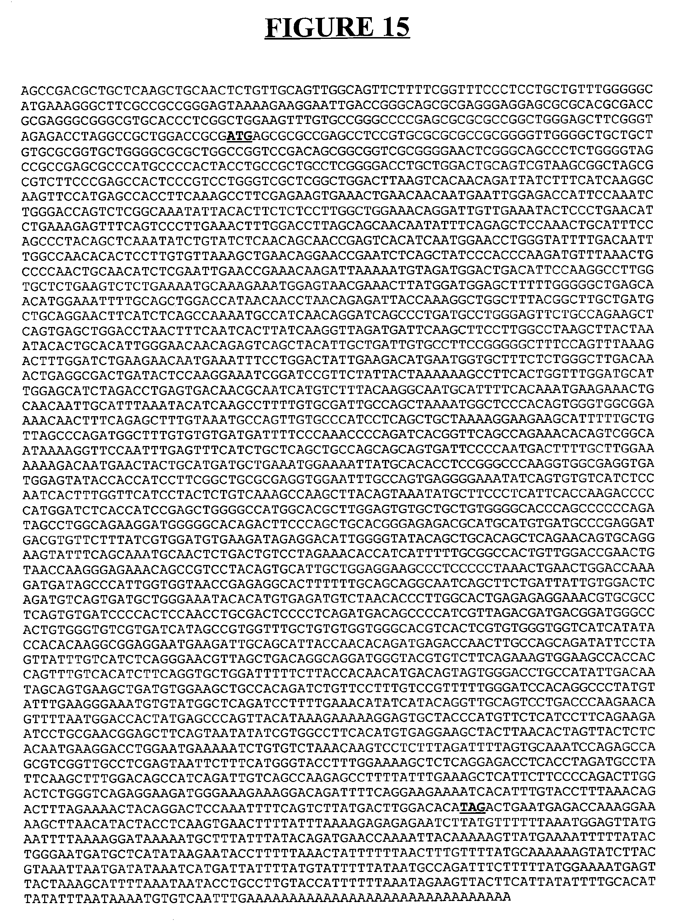

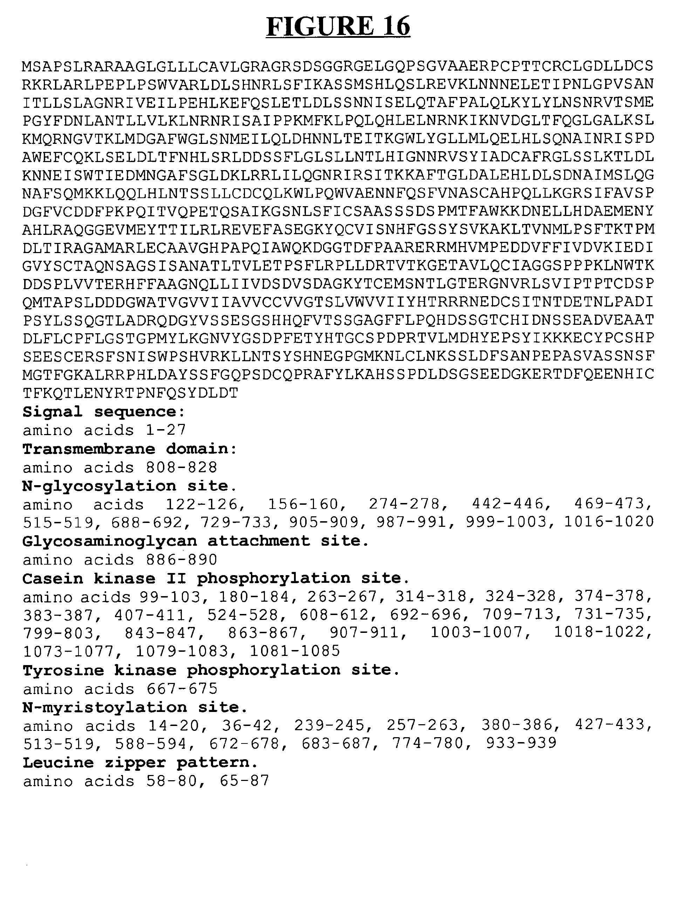

DNA sequencing of the clones isolated as described above gave the full-length DNA sequence for PR0326 [herein designated as SEQ ID NO:15; see Figure 15], and the derived protein sequence for PR0326 (see Figure 16; SEQ ID NO:16).

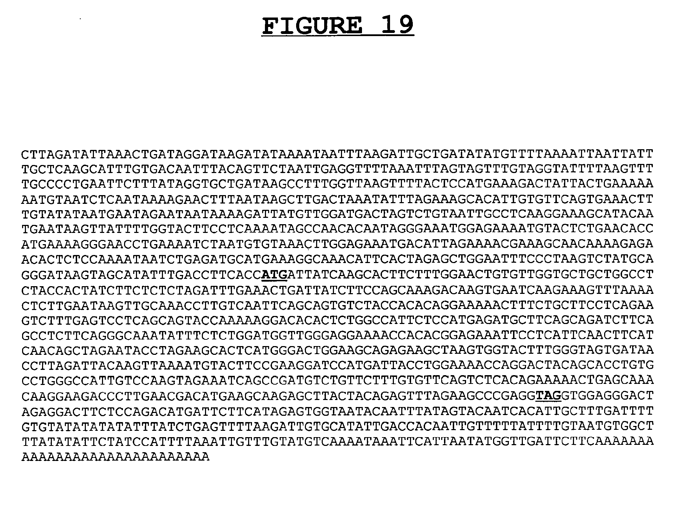

The entire nucleotide sequences is shown in Figure 19, deposited with the ATCC on November 21, 1997 and is assigned ATCC deposit number 209489.

Analysis of the amino acid sequence of the full-length PR0326 polypeptide suggests that portions of it possess significant homology to the LIG-1 protein, thereby indicating that PR0326 may be a novel LIG-1-related protein.

### EXAMPLE 12: Isolation of cDNA clones Encoding Human PRO386 Polypeptides [UNQ326]

A consensus sequence was obtained relative to a variety of EST sequences as described in Example 1 above, wherein the consensus sequence obtained is herein designated DNA40674. Two proprietary Genentech EST sequences were employed in the consensus sequence assembly, wherein those EST sequences are herein designated DNA23350 and DNA23536. Based on the DNA40674 consensus sequence, oligonucleotides were synthesized: 1) to identify by PCR a cDNA library that contained the sequence of interest, and 2) for use as probes to isolate a clone of the full-length coding sequence for PR0386.

A pair of PCR primers (forward and reverse) were synthesized:
forward PCR primer 5'-ACGGAGCATGGAGGTCCACAGTAC-3' (SEQ ID NO:134)
reverse PCR primer 5'-GCACGTTTCTCAGCATCACCGAC-3' (SEQ ID NO:135)
Additionally, a synthetic oligonucleotide hybridization probe was constructed from the consensus DNA40674 sequence which had the following nucleotide sequence
hybridization probe
5'-CGCCTGCCCTGCACCTTCAACTCCTGCTACACAGTGAACCACAAACAGTT-3' (SEQ ID NO:136)

In order to screen several libraries for a source of a full-length clone, DNA from the libraries was screened by PCR amplification with the PCR primer pair identified above. A positive library was then used to isolate clones encoding the PR0386 gene using the probe oligonucleotide and one of the PCR primers. RNA for construction of the cDNA libraries was isolated from human fetal brain tissue (LIB 153).

DNA sequencing of the clones isolated as described above gave the full-length DNA sequence for PR0386 [herein designated as UNQ326 (DNA45415-1318)] (SEQ ID NO:17) and the derived protein sequence for PR0386.

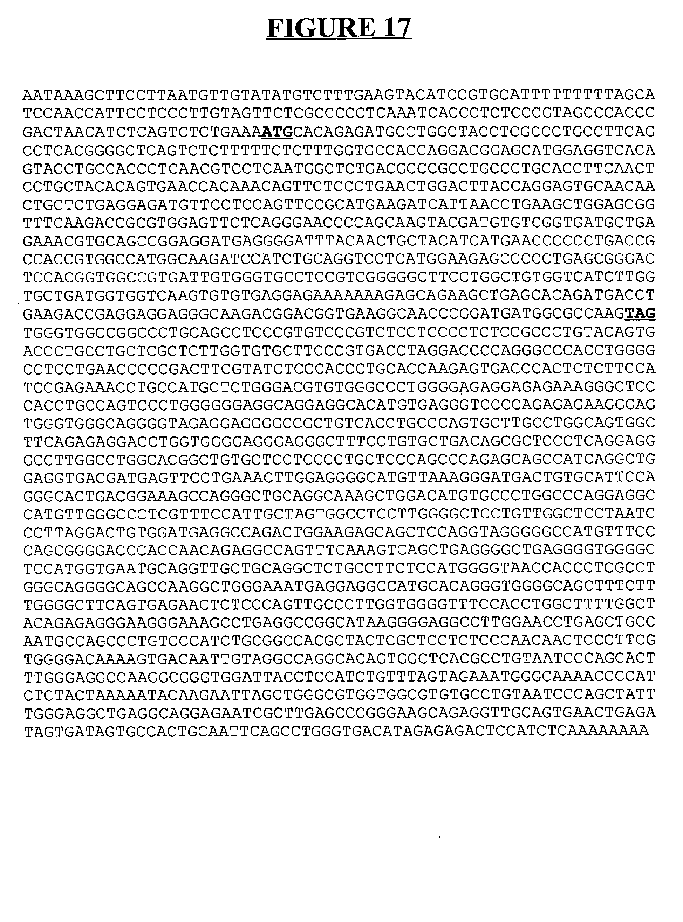

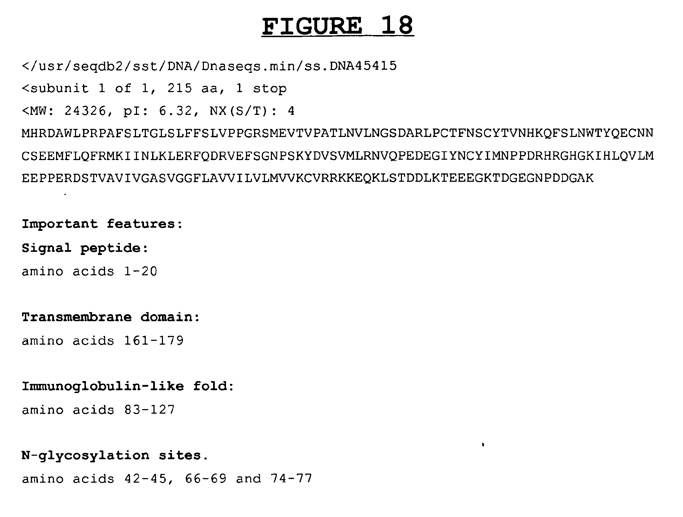

The entire nucleotide sequence of UNQ326 (DNA45415-1318) is shown in Figure 17 (SEQ ID NO:17). Clone UNQ326 (DNA45415-1318) contains a single open reading frame with an apparent translational initiation site at nucleotide positions 146-148 and ending at the stop codon at nucleotide positions 791-793 (Figure 17). The predicted polypeptide precursor is 215 amino acids long (Figure 18; SEQ ID NO:18). The full-length PR0386 protein shown in Figure 18 has an estimated molecular weight of about 24,326 daltons and a pI of about 6.32. Analysis of the full-length PR0386 sequence shown in Figure 18 (SEQ ID NO:18) evidences the presence of the following: a signal peptide from about amino acid 1 to about amino acid 20, a transmembrane domain from about amino acid 161 to about amino acid 179, an immunoglobulin-like fold from about amino acid 83 to about amino acid 127 and potential N-glycosylation sites from about amino acid 42 to about amino acid 45, from about amino acid 66 to about amino acid 69 and from about amino acid 74 to about amino acid 77. Clone UNQ326 (DNA45415-1318) has been deposited with ATCC on April 28, 1998 and is assigned ATCC deposit no. 209810.

Analysis of the amino acid sequence of the full-length PR0386 polypeptide suggests that it possesses significant sequence similarity to the sodium channel beta-2 subunit, thereby indicating that PR0386 is a novel homolog thereof. More specifically, an analysis of the Dayhoff database (version 35.45 SwissProt 35) evidenced significant homology between the PR0386 amino acid sequence and the following Dayhoff sequences, A57843, MYPO_HUMAN, GEN14531, JC4024, HS46KDA_1, HSU90716_1, D86996_2, MUSIGLVD_1, DMU42768_1 and S 19247.

### EXAMPLE 13: Isolation of cDNA clones Encoding Human PRO655 Polypeptides [UNQ360]

An expressed sequence tag (EST) DNA database (LIFESEQTM, Incyte Pharmaceuticals, Palo Alto, CA) was searched and an EST was identified which showed homology to interferon-e. Possible homology was noted between Incyte EST 3728969 (subsequently renamed as DNA49668) and mammalian alpha interferons, in particular IFN- 14. The homology was confirmed by inspection.

The following PCR primers and oligonucleotide probe were synthesized:
49668.r1:
TCTCTGCTTCCAGTCCCATGAGTGC (SEQ ID NO:137)
49668.r2:
GCTTCCAGTCCCATGAGTGCTTCTAGG (SEQ ID NO:138)
49668.p1:
GGCCATTCTCCATGAGATGCTTCAGCAGATCTTCAGCCTCTTCAGGGCAA (SEQ ID NO:139)

In order to screen several libraries for a source of a full-length clone, DNA from the libraries was screened using the r1 and r2 probes identified above. A positive library was then used to isolate clones encoding the IFN-∈-encoding gene using the probe oligonucleotide.

Three million clones from a size selected (500-4000 bp) oligo dT primed cDNA library from human small intestine (LIB 99) constructed in a pRK5-based vector screened by hybridization. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites. Only one positive clone was found out of 3.6 x 106 cfu. The clone was sequenced in both directions and was found to cover the entire reading frame (ORF). A BAC clone (F480) was identified by screening a BAC array panel (Research Genetics) with PCR primers generated from the sequence of IFN-∈. DNA sequencing of the clone isolated as described above gave the full-length DNA sequence for DNA50960 and the derived protein sequence for IFN-∈ (PR0655).

The entire nucleotide sequence of DNA50960 is shown in Figure 19 (SEQ ID NO:19). Clone DNA50960 contains a single open reading frame with an apparent translational initiation site at nucleotide positions 621-623 (Fig. 19; SEQ ID NO:19). The predicted polypeptide precursor is 208 amino acids long, of which 21 N-terminal amino acid residues represent a putative signal sequence. Clone DNA50960-1224 (clone F480) has been deposited with ATCC and is assigned ATCC deposit no. 209509, deposited on December 3, 1997.

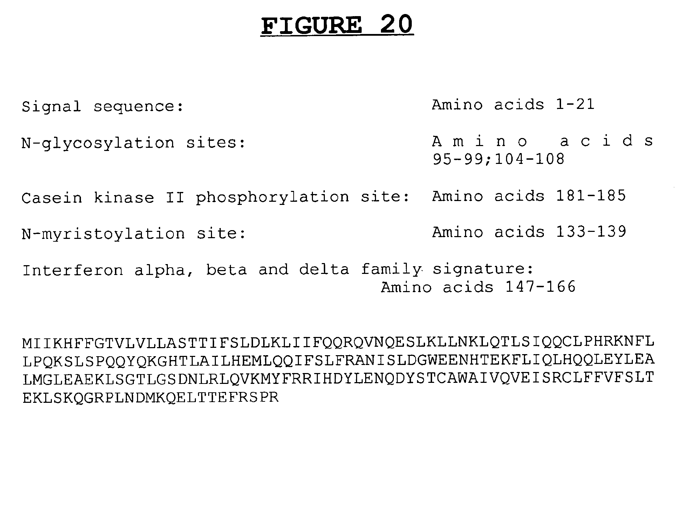

Using BLAST and FastA sequence alignment computer programs, it was found that PR0655 (shown in Fig. 20 and SEQ ID NO:20) has about 35-40% amino acid sequence identity with the sequence of various human IFN-∈ species. The homology is highest within the 22-189 amino acid region of the sequence of Fig. 20 (SEQ ID NO:20). At the nucleotide level, the homology with the coding sequence of IFN-∈ is about 60%. Based upon these data as well as the presence of a characteristic sequence beginning at amino acid 147 that is typical of type I interferons ([FYH][FY].[GNRC][LIVM]. {1} [FY]L. {7} [CY]AW), this molecule was identified as a member of the type I IFN family. The sequence of IFN-∈ is nearly as divergent from IFN-α as it is from IFN-β family members (33% and 37% sequence identity to IFN- α2a and IFN-β, respectively) and thus defines a new branch on the type 1 interferon family tree. Molecular modeling suggests that IFN-∈ displays similar tertiary structure compared to IFN-α (L. Presta, data not shown).

### EXAMPLE 14: Isolation of cDNA clones Encoding Human PRO162 Polypeptides [UNQ429]

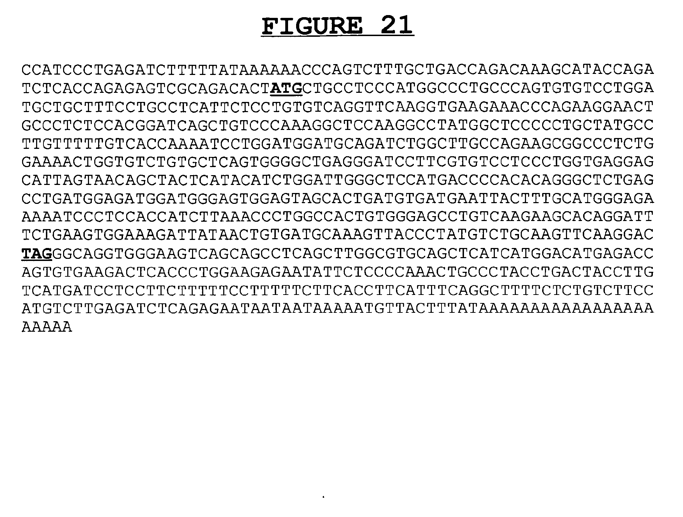

An expressed sequence tag (EST) DNA database (Merck/Washington University) was searched and an EST AA397543 was identified which showed homology to human pancreatitis-associated protein. The EST AA397543 cole was purchased and its insert obtained and sequenced and the sequence obtained is shown in Figure 21 (SEQ ID NO:21).

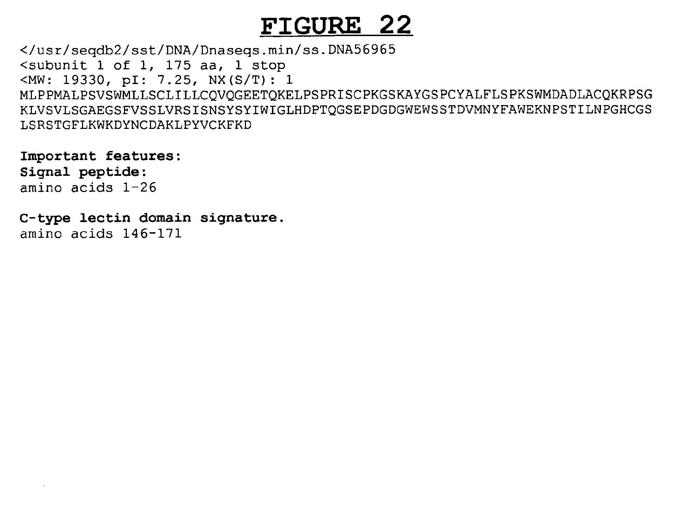

The entire nucleotide sequence of PRO162 is shown in Figure 21 (SEQ ID NO:21). DNA sequencing of the clone gave the full-length DNA sequence for PRO162 [herein designated as UNQ429 (DNA56965-1356)] (SEQ ID NO:21) and the derived protein sequence for PRO162. Clone UNQ429 (DNA56965-1356) contains a single open reading frame with an apparent translational initiation site at nucleotide positions 86-88 and ending at the stop codon at nucleotide positions 611-613 (Figure 21). The predicted polypeptide precursor is 175 amino acids long (Figure 22; SEQ ID NO:22). The full-length PRO162 protein shown in Figure 22 has an estimated molecular weight of about 19,330 daltons and a pI of about 7.25. Clone UNQ429 (DNA56965-1356) has been deposited with the ATCC on May 6, 1998 and is assigned ATCC number 209842. Regarding the sequence, it is understood that the deposited clone contains the correct sequence, and the sequences provided herein are based on known sequencing techniques.

Analysis of the amino acid sequence of the full-length PRO162 polypeptide suggests that portions of it possess significant homology to the human pancreatitis-associated protein, thereby indicating that PRO162 may be a novel pancreatitis-associated protein.

Still analyzing the amino acid sequence of SEQ ID NO:22, the putative signal peptide is at about amino acids 1-26 of SEQ ID NO:22. A C-type lectin domain signature is at about amino acids 146-171 of SEQ ID NO:22. The corresponding nucleotides can be routinely determined given the sequences provided herein.

### EXAMPLE 15: Isolation of cDNA clones Encoding Human PRO788 Polypeptides [UNQ430]

A consensus DNA sequence (designated herein as DNA49308) was assembled relative to other EST sequences using phrap as described in Example 1 above. Based upon an observed homology between the DNA49308 consensus sequence and the Incyte EST clo0ne no. 2777282, the Incyte EST clone no. 2777282 was purchased and its insert obtained and sequenced, which gave the full-length DNA sequence for PRO788 [herein designated as UNQ430 (DNA56405-1357)] (SEQ ID NO:23) and the derived protein sequence for PR0788.

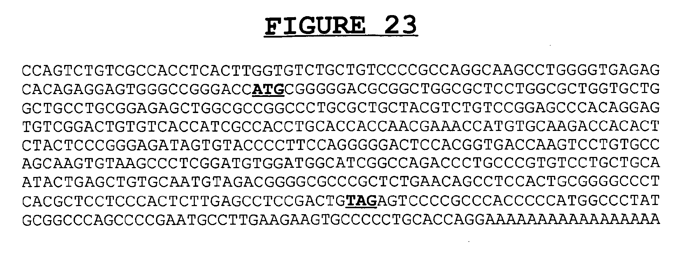

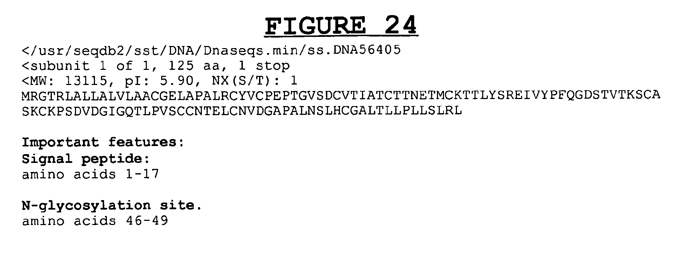

Clone UNQ430 (DNA56405-1357) contains a single open reading frame with an apparent translational initiation site at nucleotide positions 84-86 and ending at the stop codon at nucleotide positions 459-461 (Figure 23; SEQ ID NO:23). The predicted polypeptide precursor is 125 amino acids long (Figure 24, SEQ ID NO:24). The full-length PRO788 protein shown in Figure 24 has an estimated molecular weight of about 13,115 daltons and a pI of about 5.90. Clone UNQ430 (DNA56405-1357) has been deposited with the ATCC on May 6, 1998 and is assigned ATCC number 209849. Regarding the sequence, it is understood that the deposited clone contains the correct sequence, and the sequences provided herein are based on known sequencing techniques.

Still analyzing Figure 24, a signal peptide is shown at about amino acids 1-17 of SEQ ID NO:24. An N-glycosylation site is at about amino acids 46-49 of SEQ ID NO:24.

### EXAMPLE 16: Isolation of cDNA clones Encoding Human PRO792 Polypeptides [UNQ431]

A consensus sequence was obtained relative to a variety of EST sequences as described in Example 1 above, wherein the consensus sequence obtained is herein designated DNA38106. Based on the DNA38106 consensus sequence, oligonucleotides were synthesized: 1) to identify by PCR a cDNA library that contained the sequence of interest, and 2) for use as probes to isolate a clone of the full-length coding sequence for PR0792.

A pair of PCR primers (forward and reverse) were synthesized:
forward PCR primer 5'-GCGAGAACTGTGTCATGATGCTGC-3' (SEQ ID NO:140)
reverse PCR primer 5'-GTTTCTGAGACTCAGCAGCGGTGG-3' (SEQ ID NO:141)
Additionally, a synthetic oligonucleotide hybridization probe was constructed from the consensus DNA38106 sequence which had the following nucleotide sequence
hybridization probe
5'-CACCGTGTGACAGCGAGAAGGACGGCTGGATCTGTGAGAAAAGGCACAAC-3' (SEQ ID NO:142)

In order to screen several libraries for a source of a full-length clone, DNA from the libraries was screened by PCR amplification with the PCR primer pair identified above. A positive library was then used to isolate clones encoding the PR0792 gene using the probe oligonucleotide and one of the PCR primers. RNA for construction of the cDNA libraries was isolated from human bone marrow tissue (LIB255).

DNA sequencing of the clones isolated as described above gave the full-length DNA sequence for PR0792 [herein designated as UNQ431 (DNA56352-1358)] (SEQ ID NO:25) and the derived protein sequence for PR0792.

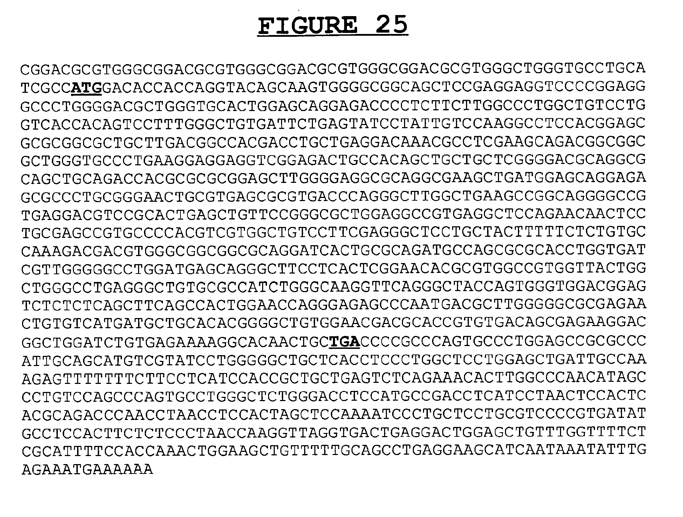

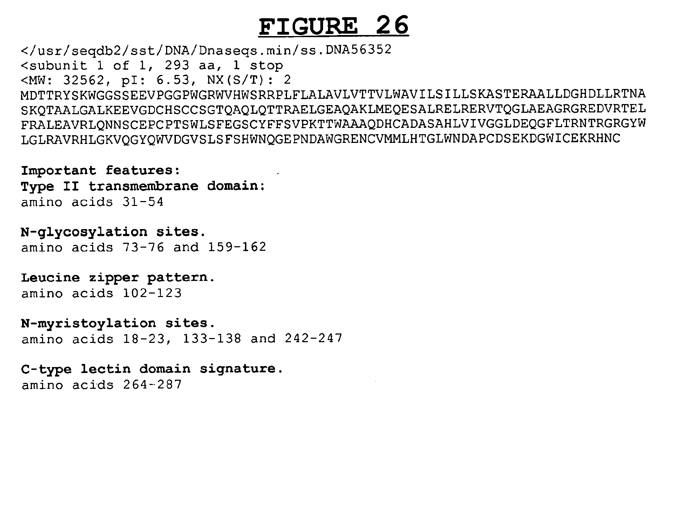

The entire nucleotide sequence ofUNQ431 (DNA56352-1358) is shown in Figure 25 (SEQ ID NO:25). Clone UNQ431 (DNA56352-1358) contains a single open reading frame with an apparent translational initiation site at nucleotide positions 67-69 and ending at the stop codon at nucleotide positions 946-948 (Figure 25). The predicted polypeptide precursor is 293 amino acids long (Figure 26; SEQ ID NO:26). The full-length PR0792 protein shown in Figure 26 has an estimated molecular weight of about 32,562 daltons and a pI of about 6.53. Analysis of the full-length PR0792 sequence shown in Figure 26 (SEQ ID NO:26) evidences the presence of the following: a type II transmembrane domain from about amino acid 31 to about amino acid 54, potential N-glycosylation sites from about amino acid 73 to about amino acid 76 and from about amino acid 159 to about amino acid 162, a leucine zipper amino acid sequence pattern from about amino acid 102 to about amino acid 123, potential N-myristolation sites from about amino acid 18 to about amino acid 23, from about amino acid 133 to about amino acid 138 and from about amino acid 242 to about amino acid 247 and a C-type lectin domain signature block from about amino acid 264 to about amino acid 287. Clone UNQ431 (DNA56352-1358) has been deposited with ATCC on May 6, 1998 and is assigned ATCC deposit no. 209846.

Analysis of the amino acid sequence of the full-length PR0792 polypeptide suggests that it possesses significant sequence similarity to the CD23 protein, thereby indicating that PR0792 may be a novel CD23 homolog. More specifically, an analysis of the Dayhoff database (version 35.45 SwissProt 35) evidenced significant homology between the PR0792 amino acid sequence and the following Dayhoff sequences, S34198, A07100_1, A05303_1, P_R41689, P_P82839, A10871_1, P_R12796, P_R47199, A46274 and P_R32188.

### EXAMPLE 17: Isolation of cDNA clones Encoding Human PRO940 Polypeptides [UNQ477]

A consensus sequence was obtained relative to a variety of EST sequences as described in Example 1 above, wherein the consensus sequence obtained is herein designated DNA47442. Based on the DNA47442 consensus sequence, oligonucleotides were synthesized: 1) to identify by PCR a cDNA library that contained the sequence of interest, and 2) for use as probes to isolate a clone of the full-length coding sequence for PR0940.

A pair of PCR primers (forward and reverse) were synthesized:
forward PCR primer 5'-CAAAGCCTGCGCCTGGTCTGTG-3' (SEQ ID NO:143)
reverse PCR primer 5'-TTCTGGAGCCCAGAGGGTGCTGAG-3' (SEQ ID NO:144)
Additionally, a synthetic oligonucleotide hybridization probe was constructed from the consensus DNA47442 sequence which had the following nucleotide sequence
hybridization probe
5'-GGAGCTGCCACCCATTCAAATGGAGCACGAAGGAGAGTTCACCTG-3' (SEQ ID NO:145)

In order to screen several libraries for a source of a full-length clone, DNA from the libraries was screened by PCR amplification with the PCR primer pair identified above. A positive library was then used to isolate clones encoding the PR0940 gene using the probe oligonucleotide and one of the PCR primers. RNA for construction of the cDNA libraries was isolated from human fetal liver tissue (LIB229).

DNA sequencing of the clones isolated as described above gave the full-length DNA sequence for PR0940 [herein designated as UNQ477 (DNA54002-1367)] (SEQ ID NO:27) and the derived protein sequence for PR0940.

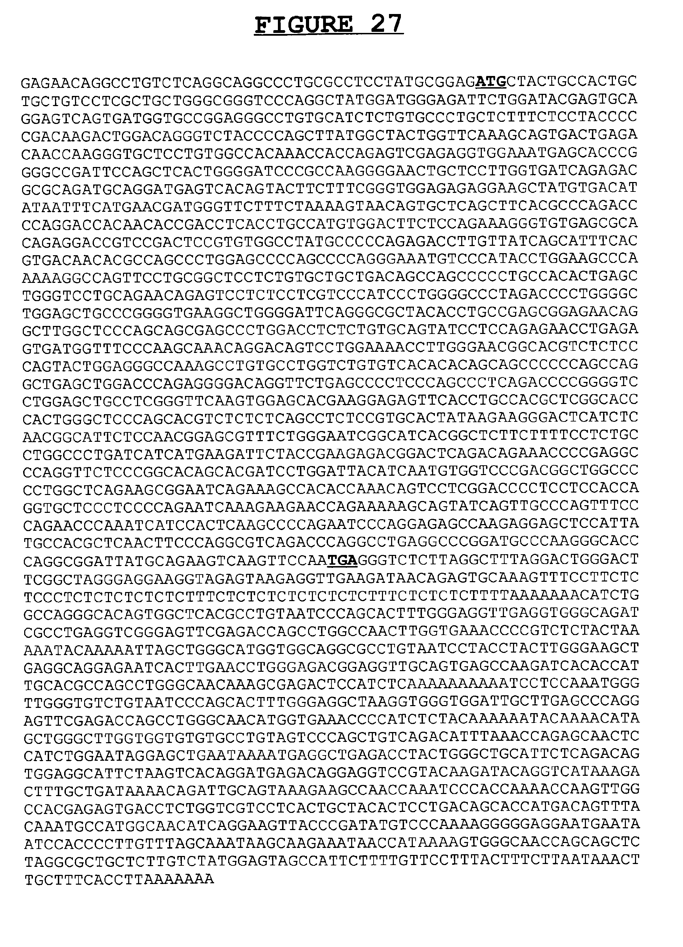

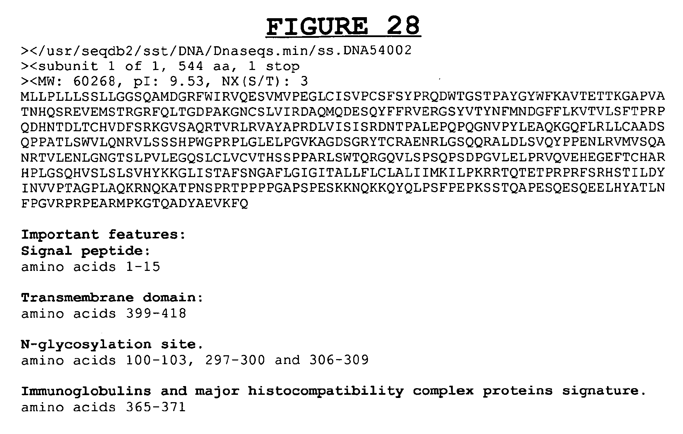

The entire nucleotide sequence of UNQ477 (DNA54002-1367) is shown in Figure 27 (SEQ ID NO:27). Clone UNQ477 (DNA54002-1367) contains a single open reading frame with an apparent translational initiation site at nucleotide positions 46-48 and ending at the stop codon at nucleotide positions 1678-1680 (Figure 27). The predicted polypeptide precursor is 544 amino acids long (Figure 28; SEQ ID NO:28). The full-length PR0940 protein shown in Figure 28 has an estimated molecular weight of about 60,268 daltons and a pI of about 9.53. Analysis of the full-length PR0940 sequence shown in Figure 28 (SEQ ID NO:28) evidences the presence of the following: a signal peptide from about amino acid 1 to about amino acid 15, potential N-glycosylation sites from about amino acid 100 to about amino acid 103, from about amino acid 297 to about amino acid 300 and from about amino acid 306 to about amino acid 309 and an immunoglobulin and major histocompatibility complex signature sequence block from about amino acid 365 to about amino acid 371. Clone UNQ477 (DNA54002-1367) has been deposited with ATCC on April 7, 1998 and is assigned ATCC deposit no. 209754.

Analysis of the amino acid sequence of the full-length PR0940 polypeptide suggests that it possesses significant sequence similarity to CD33 and the OB binding protein-2. More specifically, an analysis of the Dayhoffdatabase (version 35.45 SwissProt 35) evidenced significant homology between the PR0940 amino acid sequence and the following Dayhoff sequences, CD33_HUMAN, HSU71382_1, HSU71383_1, D86359_1, PGBM_HUMAN, MAGS_MOUSE, D86983_1, C22B_HUMAN, P_W01002 and HVU24116_1.

### EXAMPLE 18: Isolation of cDNA clones Encoding Human PRO941 Polypeptides [UNQ478]

A consensus sequence was obtained relative to a variety of EST sequences as described in Example 1 above, wherein the consensus sequence obtained is herein designated DNA35941. An EST sequence proprietary to Genentech was employed in the assembly and is herein designated DNA6415. Based on the DNA35941 consensus sequence, oligonucleotides were synthesized: 1) to identify by PCR a cDNA library that contained the sequence of interest, and 2) for use as probes to isolate a clone of the full-length coding sequence for PR0941.

A pair of PCR primers (forward and reverse) were synthesized:
forward PCR primer 5'-CTTGACTGTCTCTGAATCTGCACCC-3' (SEQ ID NO:146)
reverse PCR primer 5'-AAGTGGTGGAAGCCTCCAGTGTGG-3' (SEQ ID NO:147)
Additionally, a synthetic oligonucleotide hybridization probe was constructed from the consensus DNA35941 sequence which had the following nucleotide sequence
hybridization probe
5'-CCACTACGGTATTAGAGCAAAAGTTAAAAACCATCATGGTTCCTGGAGCAGC-3' (SEQ ID NO:148)

In order to screen several libraries for a source of a full-length clone, DNA from the libraries was screened by PCR amplification with the PCR primer pair identified above. A positive library was then used to isolate clones encoding the PR0941 gene using the probe oligonucleotide and one of the PCR primers. RNA for construction of the cDNA libraries was isolated from human fetal kidney tissue (LIB227).

DNA sequencing of the clones isolated as described above gave the full-length DNA sequence for PR0941 [herein designated as UNQ478 (DNA53906-1368)] (SEQ ID NO:29) and the derived protein sequence for PR0941.

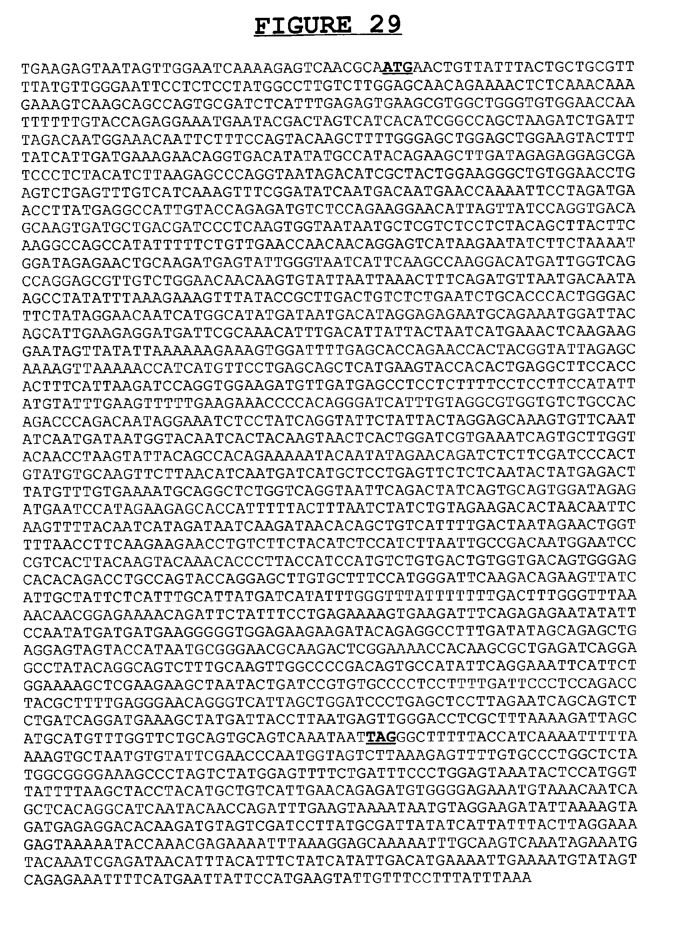

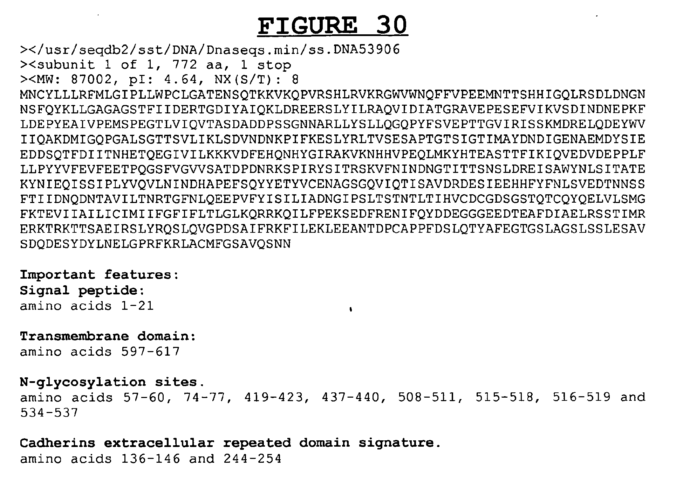

The entire nucleotide sequence of UNQ478 (DNA53906-1368) is shown in Figure 29 (SEQ ID NO:29). Clone UNQ478 (DNA53906-1368) contains a single open reading frame with an apparent translational initiation site at nucleotide positions 37-39 and ending at the stop codon at nucleotide positions 2353-2355 (Figure 29). The predicted polypeptide precursor is 772 amino acids long (Figure 30; SEQ ID NO:30). The full-length PR0941 protein shown in Figure 30 has an estimated molecular weight of about 87,002 daltons and a pI of about 4.64. Analysis of the full-length PRO941 sequence shown in Figure 30 (SEQ ID NO:30) evidences the presence of the following: a signal peptide from about amino acid 1 to about amino acid 21, potential N-glycosylation sites from about amino acid 57 to about amino acid 60, from about amino acid 74 to about amino acid 77, from about amino acid 419 to about amino acid 422, from about amino acid 437 to about amino acid 440, from about amino acid 508 to about amino acid 511, from about amino acid 515 to about amino acid 518, from about amino acid 516 to about amino acid 519 and from about amino acid 534 to about amino acid 537, and cadherin extracellular repeated domain signature sequences from about amino acid 136 to about amino acid 146 and from about amino acid 244 to about amino acid 254. Clone UNQ478 (DNA53906-1368) has been deposited with ATCC on April 7,1998 and is assigned ATCC deposit no. 209747.

Analysis of the amino acid sequence of the full-length PR0941 polypeptide suggests that it possesses significant sequence similarity to a cadherin protein, thereby indicating that PR0941 may be a novel cadherin protein family member. More specifically, an analysis of the Dayhoff database (version 35.45 SwissProt 35) evidenced significant homology between the PR0941 amino acid sequence and the following Dayhoff sequences, I50180, CADA_CHICK, I50178, GEN12782, CADC_HUMAN, P_W25637, A38992, P_R49731, D38992 and G02678.

### EXAMPLE 19: Isolation of cDNA clones Encoding Human PRO1004 Polypeptides [UNQ488]

Use of the signal sequence algorithm described in Example 3 above allowed identification of a single Incyte EST cluster sequence, Incyte cluster sequence No. 73681. This EST cluster sequence was then compared to a variety of expressed sequence tag (EST) databases which included public EST databases (e.g., GenBank) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, Univ. of Washington, Seattle, Washington). The consensus sequence obtained therefrom is herein designated as DNA56516.

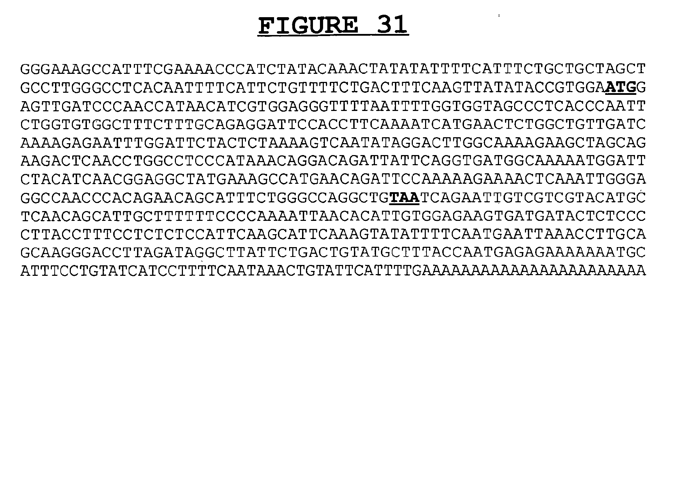

In light of an observed sequence homology between the DNA56516 consensus sequence and an EST sequence encompassed within the Merck EST clone no. H43837, the Merck EST clone H43837 was purchased and the cDNA insert was obtained and sequenced. It was found that this insert encoded a full-length protein. The sequence of this cDNA insert is shown in Figure 31.

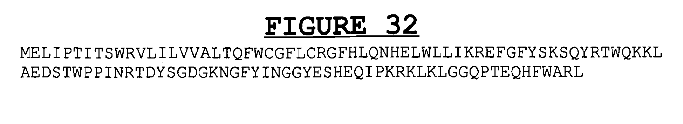

The full length clone shown in Figure 31 contained a single open reading frame with an apparent translational initiation site at nucleotide positions 119-121 and ending at the stop codon at nucleotide positions 464-466 (Figure 31; SEQ ID NO:31). The predicted polypeptide precursor is 115 amino acids long (Figure 32; SEQ ID NO:32). The full-length PRO1004 protein shown in Figure 32 has an estimated molecular weight of about 13,649 daltons and a pI of about 9.58. Analysis of the full-length PRO1004 sequence shown in Figure 32 (SEQ ID NO:32) evidences the presence of the following features: a signal peptide at about amino acids 1-24, a microbodies C-terminal targeting signal at about amino acids 113-115, a potential N-glycosylation site at about amino acids 71-74, and a domain having sequence identity with dihydrofolate reductase proteins at about amino acids 22-48.

Analysis of the amino acid sequence of the full-length PRO1004 polypeptide using the Dayhoff database (version 35.45 SwissProt 35) evidenced homology between the PRO1004 amino acid sequence and the following Dayhoff sequences: CELR02D3_7,LECI_MOUSE, AF006691_3, SSZ97390_1, SSZ97395_1, and SSZ97400_1.

Clone DNA57844-1410 was deposited with the ATCC on June 23, 1998, and is assigned ATCC deposit no. 203010.

### EXAMPLE 20: Isolation of cDNA clones Encoding Human PRO1012 Polypeptides [UNQ495]

A consensus DNA sequence was assembled relative to other EST sequences using phrap as described in Example 1 above, wherein the consensus sequence is herein designated DNA49313. Based on the DNA49313 consensus sequence, oligonucleotides were synthesized: 1) to identify by PCR a cDNA library that contained the sequence of interest, and 2) for use as probes to isolate a clone of the full-length coding sequence for PRO1012.

A pair of PCR primers (forward and reverse) were synthesized:
forward PCR primer 5'-ACTCCCCAGGCTGTTCACACTGCC-3' (SEQ ID N0:149);
reverse PCR primer 5'-GATCAGCCAGCCAATACCAGCAGC-3' (SEQ ID NO:150).
Additionally, a synthetic oligonucleotide hybridization probe was constructed from the DNA49313 consensus sequence which had the following nucleotide sequence:
hybridization probe
5'-GTGGTGATGATAGAATGCTTTGCCGAATGAAAGGAGTCAACAGCTATCCC-3' (SEQ ID NO:151).

In order to screen several libraries for a source of a full-length clone, DNA from the libraries was screened by PCR amplification with the PCR primer pair identified above. A positive library was then used to isolate clones encoding the PRO1012 gene using the probe oligonucleotide and one of the PCR primers. RNA for construction of the cDNA libraries was isolated from human fetal kidney tissue (LIB227).

DNA sequencing of the clones isolated as described above gave the full-length DNA sequence for PRO1012 [herein designated as UNQ495 (DNA56439-1376)] (SEQ ID NO:33) and the derived protein sequence for PRO1012.

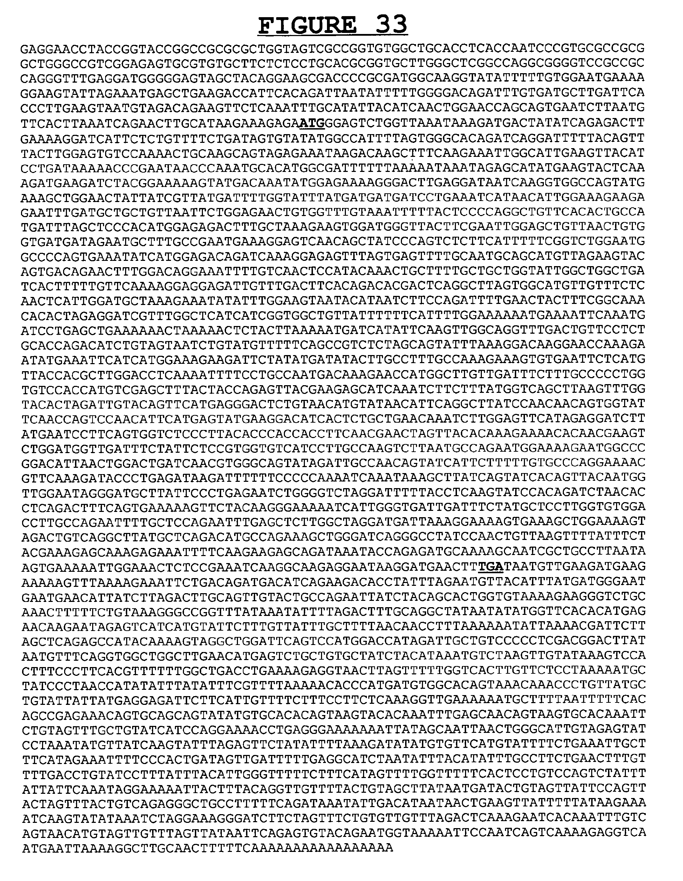

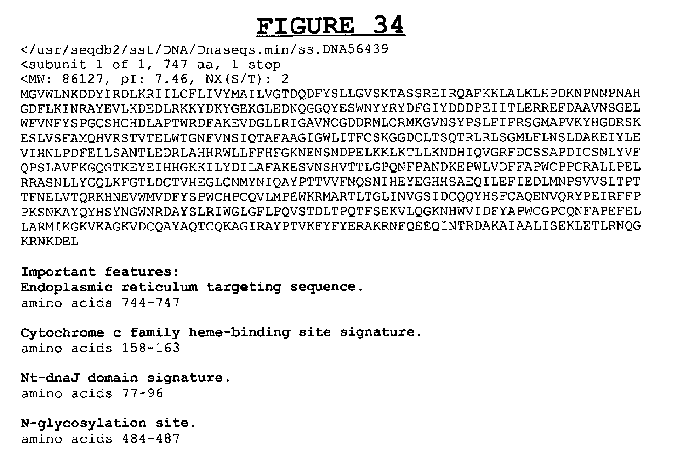

The entire nucleotide sequence of UNQ495 (DNA56439-1376) is shown in Figure 33 (SEQ ID NO:33). Clone UNQ495 (DNA56439-1376) contains a single open reading frame with an apparent translational initiation site at nucleotide positions 404-406 and ending at the stop codon at nucleotide positions 2645-2647 (Figure 33). The predicted polypeptide precursor is 747 amino acids long (Figure 34; SEQ ID NO:34). The full-length PRO1012 protein shown in Figure 34 has an estimated molecular weight of about 86,127 daltons and a pI of about 7.46. Clone UNQ495 (DNA56439-1376) has been deposited with ATCC on May 14, 1998 and is assigned ATCC number 209864. Regarding the sequence, it is understood that the deposited clone contains the correct sequence, and the sequences provided herein are based on known sequencing techniques.

Analysis of the amino acid sequence of the full-length PRO1012 polypeptide suggests that portions of it possess sequence identity with disulfide isomerase thereby indicating that PRO1012 may be a novel disulfide isomerase related protein.

Still analyzing the amino acid sequence of SEQ ID NO:34, the cytochrome C family heme-binding site signature is at about amino acids 158-163 of SEQ ID NO:34. The Nt-DNAJ domain signature is at about amino acids 77-96 of SEQ ID NO:34. An N-glycosylation site is at about amino acids 484-487 of SEQ ID NO:34. The ER targeting sequence is at about amino acids 744-747 of SEQ ID NO:34. It is understood that the polypeptide and nucleic acids disclosed can be routinely formed with or without, these portions as desired, in alternative embodiments. For example, it may be desirable to produce PRO1012 without the ER targeting sequence. The corresponding nucleotides can be routinely determined given the sequences provided herein.

### EXAMPLE 21: Isolation of cDNA clones Encoding Human PRO1016 Polypeptides [UNQ499]

A consensus DNA sequence was assembled relative to other EST sequences using phrap as described in Example 1 above. The consensus sequence obtained is herein designated DNA53502.

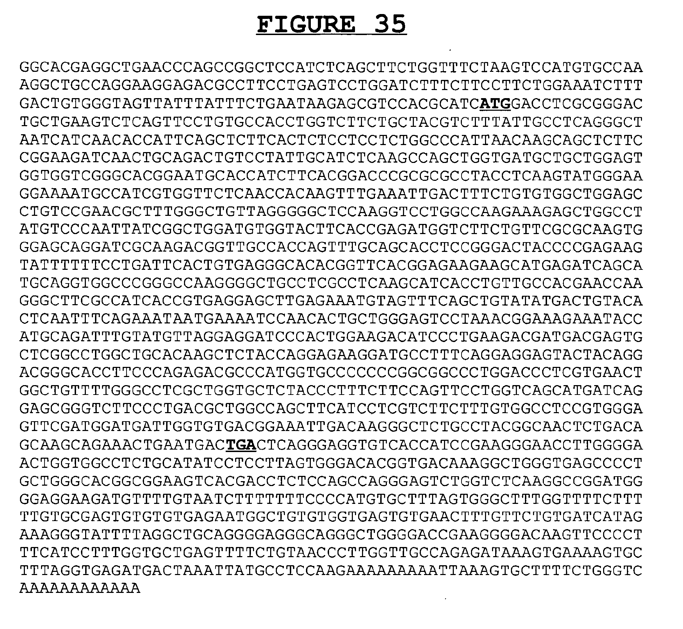

In light of an observed sequence homology between the DNA53502 consensus sequence and an EST sequence encompassed within the Merck EST clone no. 38680, the Merck EST clone 38680 was purchased and the cDNA insert was obtained and sequenced. It was found that this insert encoded a full-length protein. The sequence of this cDNA insert is shown in Figure 35.

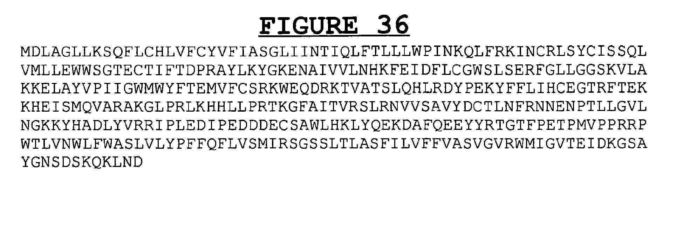

The entire nucleotide sequence of DNA56113-1378 is shown in Figure 35 (SEQ ID NO:35). Clone DNA56113-1378 contains a single open reading frame with an apparent translational initiation site at nucleotide positions 168-170 and ending at the stop codon at nucleotide positions 1302-1304 (Figure 35). The predicted polypeptide precursor is 378 amino acids long (Figure 36; SEQ ID NO:36). The full-length PRO1016 protein shown in Figure 36 has an estimated molecular weight of about 44,021 daltons and a pI of about 9.07. Clone DNA56113-1378 has been deposited with the ATCC on July 1, 1998 and is assigned ATCC number 203049. Regarding the sequence, it is understood that the deposited clone contains the correct sequence, and the sequences provided herein are based on known sequencing techniques.

Analysis of the amino acid sequence of the full-length PRO1016 polypeptide suggests that portions of it possess sequence identity with acyltransferase, thereby indicating that PRO1016 may be a novel acyltransferase.

Still analyzing the amino acid sequence of SEQ ID NO:36, the putative signal peptide is at about amino acids 1-18 of SEQ ID NO:36. The transmembrane domain(s) are at about amino acids 332-352 and 305-330 of SEQ ID NO:36. The fructose-bisphospbate aldolase class-II protein homology sequence is at about amino acids 73-90 of SEQ ID NO:36. The extradiol ring-cleavage dioxygenase protein is at about amino acids 252-275 of SEQ ID NO:36. The corresponding nucleotides can be routinely determined given the sequences provided herein.

The specific Dayhoff database designation names of sequences to which PRO1016 has sequence identity with include the following: S52645, P_R59712, P_R99249, P_R59713, BNAGPATRF_1, CELT05H4_15 and CELZK40_1.

### EXAMPLE 22: Isolation of cDNA clones Encoding Human PRO474 Polypeptides [UNQ502]

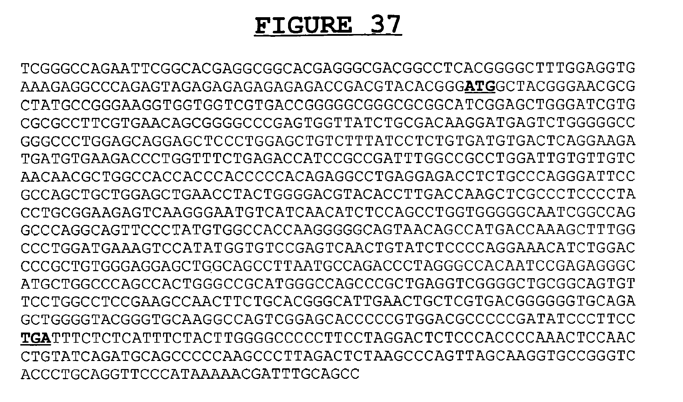

A consensus DNA sequence was assembled relative to other EST sequences using phrap as described in Example 1 above, wherein the consensus sequence obtained is herein designated DNA49818. Based upon an observed homology between the DNA49818 consensus sequence and the Merck EST clone no. H77889, the Merck EST clone no. H77889 was purchased and its insert obtained and sequenced, wherein the sequence obtained is herein shown in Figure 37 (SEQ ID NO:37). DNA sequencing gave the full-length DNA sequence for PR0474 [herein designated as UNQ502 (DNA56045-1380)] (SEQ ID NO:37) and the derived protein sequence for PR0474.

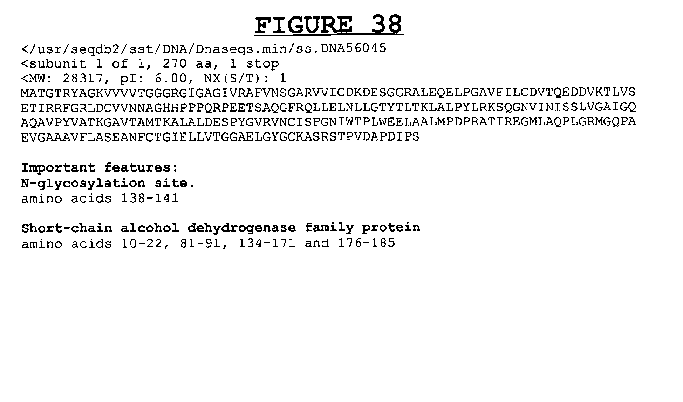

The entire nucleotide sequence of UNQ502 (DNA56045-1380) is shown in Figure 37 (SEQ ID NO:37). Clone UNQ502 (DNA56045-1380) contains a single open reading frame with an apparent translational initiation site at nucleotide positions 106-108 and ending at the stop codon at nucleotide positions 916-918 (Figure 3 7). The predicted polypeptide precursor is 270 amino acids long (Figure 38; SEQ ID NO:38). The full-length PR0474 protein shown in Figure 38 has an estimated molecular weight of about 28,317 daltons and a pI of about 6.0. Clone UNQ502 (DNA56045-1380) has been deposited with the ATCC on May 14, 1998 and is assigned ATCC number 209865. Regarding the sequence, it is understood that the deposited clone contains the correct sequence, and the sequences provided herein are based on known sequencing techniques.

Still analyzing the amino acid sequence of SEQ ID NO:38, an N-glycosylation site is at about amino acids 138-141 of SEQ ID NO:38. Short-chain alcohol dehydrogenase family proteins are at about amino acids 10-22, 81-91, 134-171 and 176-185 of SEQ ID NO:38. The corresponding nucleotides can be routinely determined given the sequences provided herein.

### EXAMPLE 23: Isolation of cDNA clones Encoding Human PR01069 Polypeptides [UNQ526]

Use of the signal sequence algorithm described in Example 3 above allowed identification of a single Incyte EST sequence designated herein as 100727. This sequence was then compared to a proprietary EST DNA database (LIFESEQTM, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, Univ. of Washington, Seattle, Washington). The consensus sequence obtained therefrom is herein designated DNA56001.

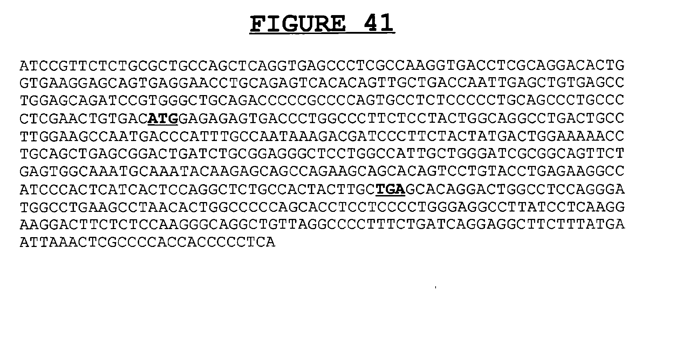

In light of an observed sequence homology between the DNA56001 consensus sequence and an EST sequence encompassed within the Incyte EST clone no. 3533881, the Incyte EST clone 3533881 was purchased and the cDNA insert was obtained and sequenced. It was found that this insert encoded a full-length protein. The sequence of this cDNA insert is shown in Figure 41 and is the full-length DNA sequence for PRO1069. Clone DNA59211-1450 was deposited with the ATCC on June 9, 1998, and is assigned ATCC deposit no. 209960.

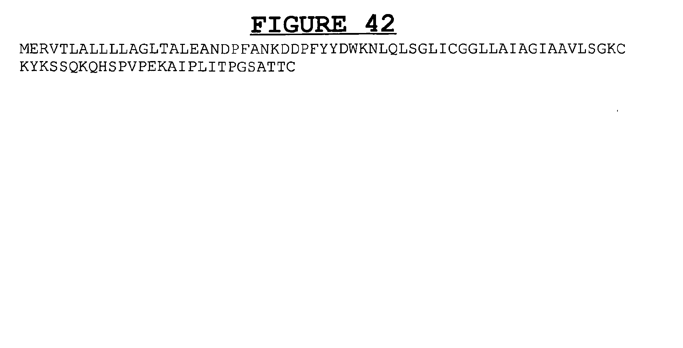

The entire nucleotide sequence of DNA59211-1450 is shown in Figure 41 (SEQ ID NO:41). Clone DNA59211-1450 contains a single open reading frame with an apparent translational initiation site at nucleotide positions 197-199 and ending at the stop codon at nucleotide positions 464-466. The predicted polypeptide precursor is 89 amino acids long (Figure 42; SEQ ID NO:42). The full-length PRO1069 protein shown in Figure 42 has an estimated molecular weight of about 9,433 daltons and a pI of about 8.21. Analysis of the full-length PRO1069 sequence shown in Figure 42 (SEQ ID NO:42) evidences the presence of the following features: a signal peptide sequence at amino acid 1 to about 16; a transmembrane domain at about amino acids 36 to about 59; potential N-myristoylation sites at about amino acids 41-46, 45-50, and 84-89; and homology with extracellular proteins SCP/Tpx-1/Ag5/PR-1/Sc7 at about amino acids 54 to about 66.

Analysis of the amino acid sequence of the full-length PRO1069 polypeptide suggests that it possesses significant sequence similarity to CHIF, thereby indicating that PRO1069 may be a member of the CHIF family of polypeptides. More particularly, analysis of the amino acid sequence of the full-length PRO1069 polypeptide using the Dayhoff database (version 35.45 SwissProt 35) evidenced homology between the PRO1069 amino acid sequence and the following Dayhoffsequences: CHIF_RAT, A55571, PLM_HUMAN, A40533, ATNG_BOVIN, RIC_MOUSE, PETD_SYNY3, VTB1_XENLA, A05009, and S75086.

Clone DNA59211-1450 was deposited with the ATCC on June 9, 1998, and is assigned ATCC deposit no. 209960.

### EXAMPLE 24: Isolation of cDNA clones Encoding Human PRO1111 Polypeptides [UNQ554]

An expressed sequence tag (EST) DNA database (LIFESEQ®, Incyte Pharmaceuticals, Palo Alto, CA) was searched and an EST was identified which had homology to insulin-like growth factor binding protein.

RNA for construction of cDNA libraries was isolated from human fetal brain. The cDNA libraries used to isolate the cDNA clones encoding human PRO1111 were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI.

The human fetal brain cDNA libraries (prepared as described above), were screened by hybridization with a synthetic oligonucleotide probe based upon the Incyte EST sequence described above:
5'-CCACCACCTGGAGGTCCTGCAGTTGGGCAGGAACTCCATCCGGCAGATTG-3' (SEQ ID NO:152).

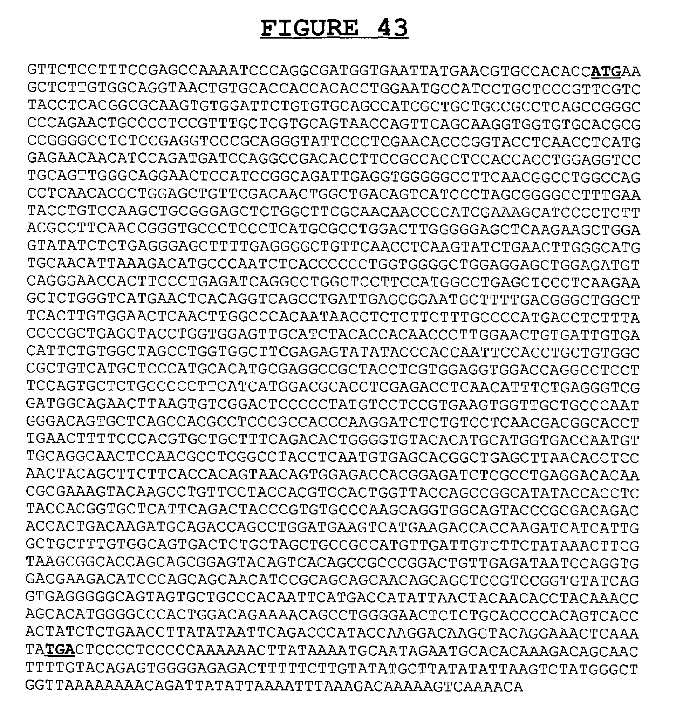

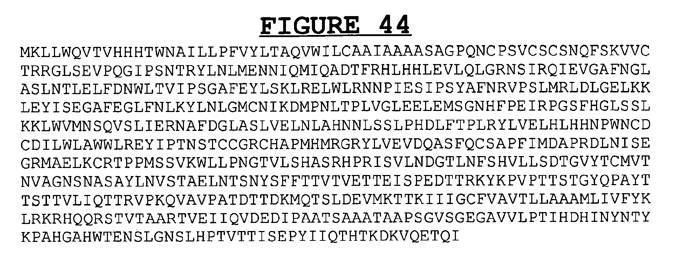

An identified cDNA clone was sequenced in entirety. The entire nucleotide sequence of PRO1111 is shown in Figure 43 (SEQ ID NO:43). Clone DNA58721-1475 contains a single open reading frame with an apparent translational initiation site at nucleotide positions 57-59 and a stop codon at nucleotide positions 2016-2018 (Figure 43; SEQ ID NO:43). The predicted polypeptide precursor is 653 amino acids long (Figure 44; SEQ ID NO:44). The transmembrane domains are at positions 21-40 (type II) and 528-548. Clone DNA58721-1475 has been deposited with ATCC on August 11, 1998and is assigned ATCC deposit no. 203110. The full-length PRO1111 protein shown in Figure 44 has an estimated molecular weight of about 72,717 daltons and a pI of about 6.99.

An analysis of the Dayhoff database (version 35.45 SwissProt 35), using a WU-BLAST2 sequence alignment analysis of the full-length sequence shown in Figure 44 (SEQ ID NO:44), revealed some sequence identity between the PRO1111 amino acid sequence and the following Dayhoff sequences: A58532, D86983_1, RNPLGPV_1, PGS2_HUMAN, AF038127_1, ALS_MOUSE, GPV_HUMAN, PGS2_BOVIN, ALS_PAPPA and 147020.

### EXAMPLE 25: Isolation of cDNA clones Encoding Human PRO1113 Polypeptides [UNQ556]

A consensus DNA sequence was assembled relative to other EST sequences using phrap as described in Example 1 above. This consensus sequence is designated herein "DNA34025". Based on the DNA34025 consensus sequence, oligonucleotides were synthesized: 1) to identify by PCR a cDNA library that contained the sequence of interest, and 2) for use as probes to isolate a clone of the full-length coding sequence for PRO1113.

PCR primers (forward and reverse) were synthesized:
forward PCR primer 5'GAGGACTCACCAATCTGGTTCGGC3' (SEQ ID NO:153); and
reverse PCR primer 5'AACTGGAAAGGAAGGCTGTCTCCC3' (SEQ ID NO:154).

Additionally, a synthetic oligonucleotide hybridization probe was constructed from the consensus DNA34025 sequence which had the following nucleotide sequence:
hybridization probe 5'GTAAAGGAGAAGAACATCACGGTACGGGATACCAGGTGTGTTTATCCTAA3' (SEQ ID NO:155).

In order to screen several libraries for a source of a full-length clone, DNA from the libraries was screened by PCR amplification with the PCR primer pair identified above. A positive library was then used to isolate clones encoding the PRO1113 gene using the probe oligonucleotide and one of the PCR primers. RNA for construction of the cDNA libraries was isolated from human fetal kidney.

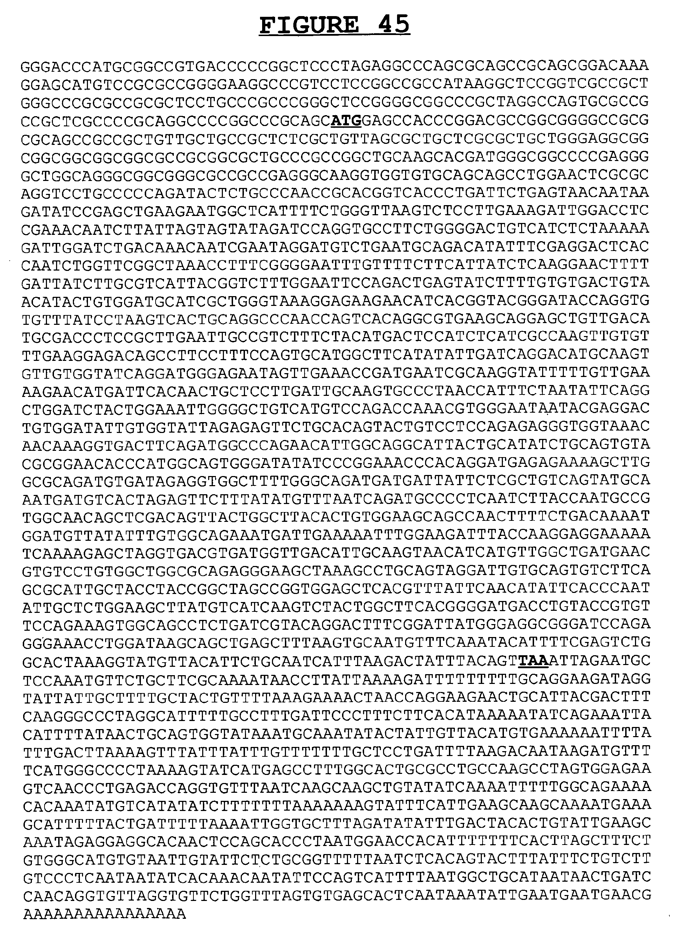

DNA sequencing of the clones isolated as described above gave the full-length DNA sequence for PRO1113 (designated herein as DNA57254-1477 [Figure 45, SEQ ID NO:45]; and the derived protein sequence for PRO1113.

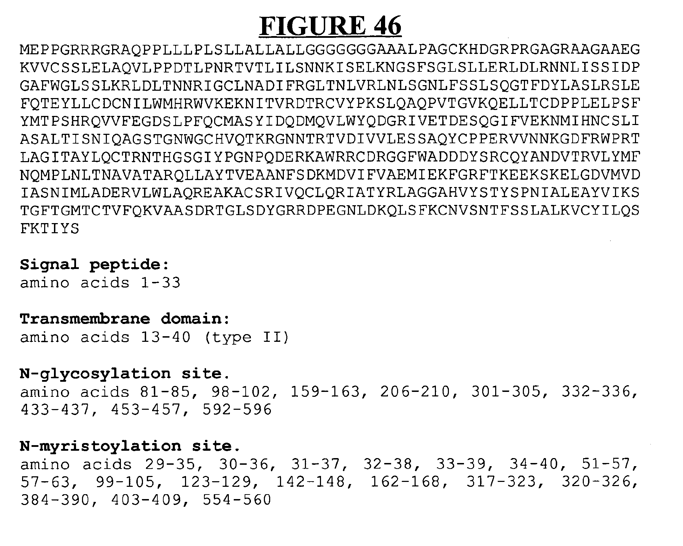

The entire coding sequence of PRO1113 is shown in Figure 45 (SEQ ID NO:45). Clone DNA57254-1477 contains a single open reading frame with an apparent translational initiation site at nucleotide positions 214-216, and an apparent stop codon at nucleotide positions 2062-2064 of SEQ ID NO:45. The predicted polypeptide precursor is 616 amino acids long [Figure 46; SEQ ID NO:46]. The transmembrane domain (type II) is believed to be at about amino acids 13-40 of SEQ ID NO:46. The N-glycosylation sites and N-myristoylation sites are indicated in Figure 46. Clone DNA57254-1477 has been deposited with the ATCC on September 29, 1998and is assigned ATCC deposit no. 203289. The full-length PRO1113 protein shown in Figure 46 has an estimated molecular weight of about 68,243 daltons and a pI of about 8.66.

An analysis of the Dayhoff database (version 35.45 SwissProt 35), using a WU-BLAST2 sequence alignment analysis of the full-length sequence shown in Figure 46 (SEQ ID NO:46), revealed sequence identity between the PRO1113 amino acid sequence and the following Dayhoff sequences (data incorporated herein): D86983_1, A58532, SLIT_DROME, AB007865_1, AC004142_1, CELT21D12_8, AB003184_1, DMU42767_1, MUSLRRP_1 and GPCR_LYMST.

### EXAMPLE 26: Isolation of cDNA clones Encoding Human PRO1130 Polypeptides [UNQ567]

A consensus DNA sequence was assembled relative to other EST sequences using phrap as described in Example 1 above. This consensus sequence is herein designatedDNA34360. Based on the DNA34360 consensus sequence, oligonucleotides were synthesized: 1) to identify by PCR a cDNA library that contained the sequence of interest, and 2) for use as probes to isolate a clone of the full-length coding sequence for PRO1130.

PCR primers (forward and reverse) were synthesized:
forward PCR primer (34360.f1) 5'-GCCATAGTCACGACATGGATG-3' (SEQ ID NO:156)
forward PCR primer (34360.f2) 5'-GGATGGCCAGAGCTGCTG-3' (SEQ ID NO:157)
forward PCR primer (34360.f3) 5'-AAAGTACAAGTGTGGCCTCATCAAGC-3' (SEQ ID NO:158)
reverse PCR primer (34360.r1) 5'-TCTGACTCCTAAGTCAGGCAGGAG-3' (SEQ ID NO:159)
reverse PCR primer (34360.r2) 5'-ATTCTCTCCACAGACAGCTGGTTC'3' (SEQ ID NO:160)
Additionally, a synthetic oligonucleotide hybridization probe was constructed from the consensus DNA34360 sequence which had the following nucleotide sequence
hybridization probe (34360.pl)
5'-GTACAAGTGTGGCCTCATCAAGCCCTGCCCAGCCAACTACTTTGCG-3' (SEQ ID NO:161)

In order to screen several libraries for a source of a full-length clone, DNA from the libraries was screened by PCR amplification with the PCR primer pair identified above. A positive library was then used to isolate clones encoding the PRO1130 gene using the probe oligonucleotide and one of the PCR primers. RNA for construction of the cDNA libraries was isolated from human aortic endothelial cell tissue.

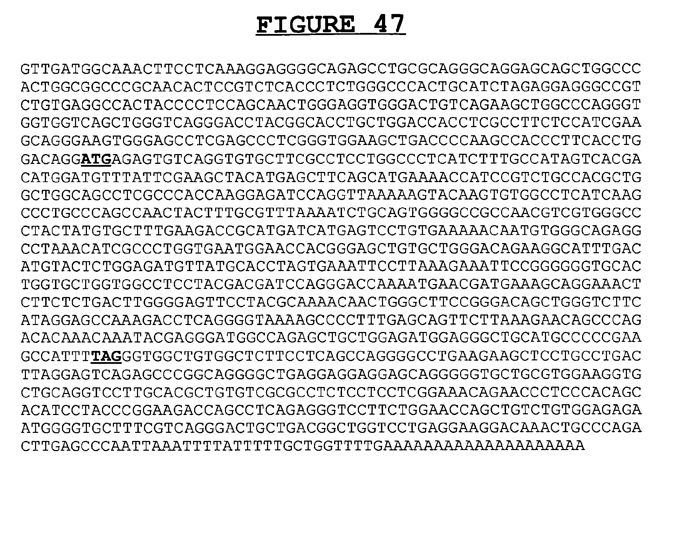

DNA sequencing of the clones isolated as described above gave the full-length DNA sequence for PRO1130 (designated herein as DNA59814-1486 [Figure 47, SEQ ID NO:47]; and the derived protein sequence for PRO1130.

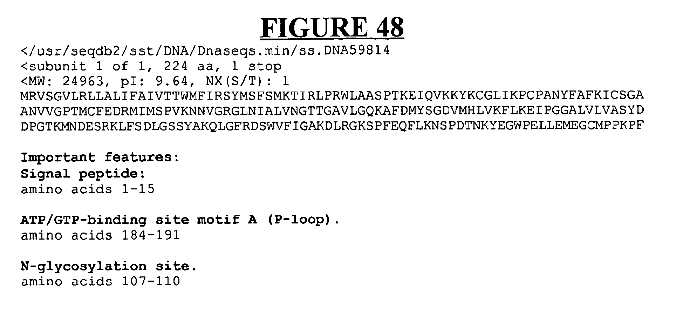

The entire nucleotide sequence of DNA59814-1486 is shown in Figure 47 (SEQ ID NO:47). Clone DNA59814-1486 contains a single open reading frame with an apparent translational initiation site at nucleotide positions 312-314 and ending at the stop codon at nucleotide positions 984-986 (Figure 47). The predicted polypeptide precursor is 224 amino acids long (Figure 48; SEQ ID NO:48). The full-length PRO1130 protein shown in Figure 48 has an estimated molecular weight of about 24,963 daltons and a pI of about 9.64. Analysis of the full-length PRO1130 sequence shown in Figure 48 (SEQ ID NO:48), evidences the presence of the following: a signal peptide from about amino acid 1 to about amino acid 15, an ATP/GTP-binding site motif A from about amino acid 184 to about amino acid 191 and a potential N-glycosylation site from about amino acid 107 to about amino acid 110. Clone DNA59814-1486 has been deposited with ATCC on October 20,1998 and is assigned ATCC deposit no. 203359.

An analysis of the Dayhoff database (version 35.45 SwissProt 35), using a WU-BLAST2 sequence alignment analysis of the full-length sequence shown in Figure 48 (SEQ ID NO:48), evidenced significant homology between the PRO1130 amino acid sequence and the following Dayhoff sequences: P_W06547, 216_HUMAN, D87120_1, MMU72677_1, LAU04889_1, and D69319.

### EXAMPLE 27: Isolation of cDNA clones Encoding Human PRO1195 Polypeptides [UNQ608]

Use ofthe signal sequence algorithm described in Example 3 above allowed identification of a single EST cluster sequence from the Incyte database. This EST cluster sequence was then compared to a variety ofexpressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA55716.

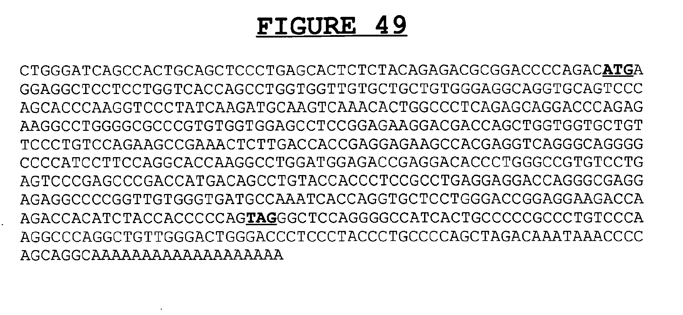

In light of an observed sequence homology between the DNA55716 consensus sequence and an EST sequence encompassed within the Incyte EST clone no. 3252980, the Incyte EST clone 3252980 was purchased and the cDNA insert was obtained and sequenced. It was found that this insert encoded a full-length protein. The sequence of this cDNA insert is shown in Figure 49 and is herein designated as DNA65412-1523.

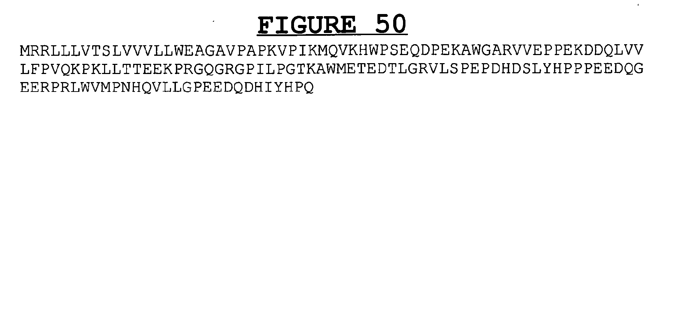

The full length clone shown in Figure 49 contained a single open reading frame with an apparent translational initiation site at nucleotide positions 58-60 and ending at the stop codon found at nucleotide positions 511-513 (Figure 49; SEQ ID NO:49). The predicted polypeptide precursor (Figure 50, SEQ ID NO:50) is 151 amino acids long. The signal sequence is at about amino acids 1-22 of SEQ ID NO:50. PRO1195 has a calculated molecular weight of approximately 17,277 daltons and an estimated pI of approximately 5.33. Clone DNA65412-1523 was deposited with the ATCC on August 4, 1998 and is assigned ATCC deposit no. 203094.

An analysis of the Dayhoff database (version 35.45 SwissProt 35), using a WU-BLAST2 sequence alignment analysis of the full-length sequence shown in Figure 50 (SEQ ID NO:50), revealed some sequence identity between the PRO1195 amino acid sequence and the following Dayhoff sequences: MMU28486_1, AF044205_1, P_W31186, CELK03C7_1, F69034, EF1A_METVA, AF024540_1, SSU90353_1, MRSP_STAAU and P_R97680.

### EXAMPLE 28: Isolation of cDNA clones Encoding Human PRO1271 Polypeptides [UNQ641]

Use of the signal sequence algorithm described in Example 3 above allowed identification of a single EST cluster sequence from the Incyte database. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA57955.

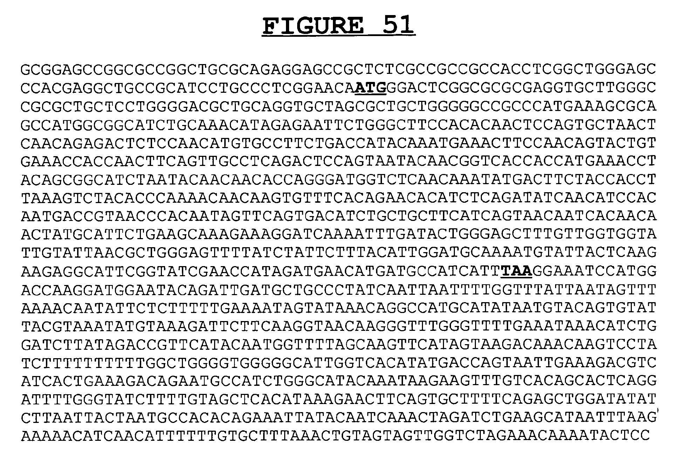

In light of an observed sequence homology between the DNA57955 consensus sequence and an EST sequence encompassed within the Merck EST clone no. AA625350, the Merck EST clone AA625350 was purchased and the cDNA insert was obtained and sequenced. It was found that this insert encoded a full-length protein. The sequence of this cDNA insert is shown in Figure 51 and is herein designated as DNA66309-1538.

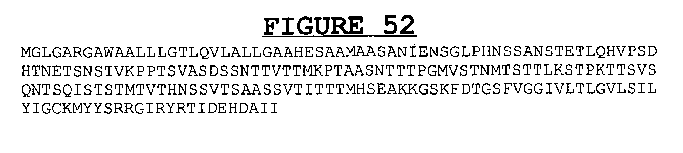

Clone DNA66309-1538 contains a single open reading frame with an apparent translational initiation site at nucleotide positions 94-96 and ending at the stop codon at nucleotide positions 718-720 (Figure 51; SEQ ID NO:51). The predicted polypeptide precursor is 208 amino acids long (Figure 52; SEQ ID NO:52). The full-length PRO1271 protein shown in Figure 52 has an estimated molecular weight of about 21,531 daltons and a pI of about 8.99. Analysis of the full-length PRO1271 sequence shown in Figure 52 (SEQ ID NO:52) evidences the presence of the following: a signal peptide from about amino acid 1 to about amino acid 31 and a transmembrane domain from about amino acid 166 to about amino acid 187. Clone DNA66309-1538 has been deposited with ATCC on September 15, 1998 and is assigned ATCC deposit no. 203235.

An analysis of the Dayhoff database (version 35.45 SwissProt 35), using a WU-BLAST2 sequence alignment analysis of the full-length sequence shown in Figure 52 (SEQ ID NO:52), evidenced significant homology between the PRO1271 amino acid sequence and the following Dayhoff sequences: S57180, S63257, AGA1_YEAST, BPU43599_1, YS8A_CAEEL, S67570, LSU54556_2, S70305, VGLX_HSVEB, and D88733_1.

### EXAMPLE 29: Isolation of cDNA clones Encoding Human PRO1865 Polypeptides [UNQ856]

The extracellular domain (ECD) sequences (including the secretion signal sequence, if any) from about 950 known secreted proteins from the Swiss-Prot public database were used to search EST databases. The EST databases included public EST databases (e.g., GenBank), and a proprietary EST database (LIFESEQ®, Incyte Pharmaceuticals, Palo Alto, CA). The search was performed using the computer program BLAST or BLAST2 [Altschul et al., Methods in Enzymology, 266:460-480 (1996)] as a comparison of the ECD protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap. This consensus sequence is herein designated DNA34023. Based on the DNA34023 consensus sequence oligonucleotides were synthesized: 1) to identify by PCR a cDNA library that contained the sequence of interest, and 2) for use as probes to isolate a clone of the full-length coding sequence for PRO1865. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

PCR primers (forward and reverse) were synthesized:
forward PCR primer (34023.f1) 5'-TAACCTAAGTAATTTACCTCAGGG-3' (SEQ ID NO:162)
reverse PCR primer (34023.r1) 5'-ATTGAGATCCTTATAGCCATCCC-3' (SEQ ID NO:163)
Additionally, a synthetic oligonucleotide hybridization probe was constructed from the consensus DNA34023 sequence which had the following nucleotide sequence
hybridization probe (34023.p1)
5'-ACCTGTGAAGGTCAACGTGCGTGGGCTCATGTGCCAAGCCCCAGAAAAGG-3' (SEQ ID NO:164)

RNA for construction of the cDNA libraries was isolated from human fetal lung tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

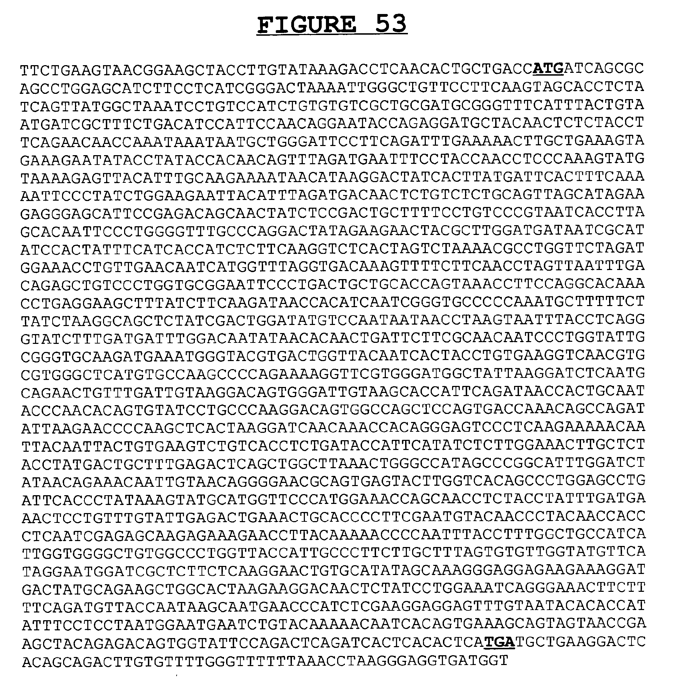

DNA sequencing of the clones isolated as described above gave the full-length DNA sequence for PRO1865 (designated herein as DNA81757-2512 [Figure 53, SEQ ID NO: 53]; (UNQ856) and the derived protein sequence for PRO1865.

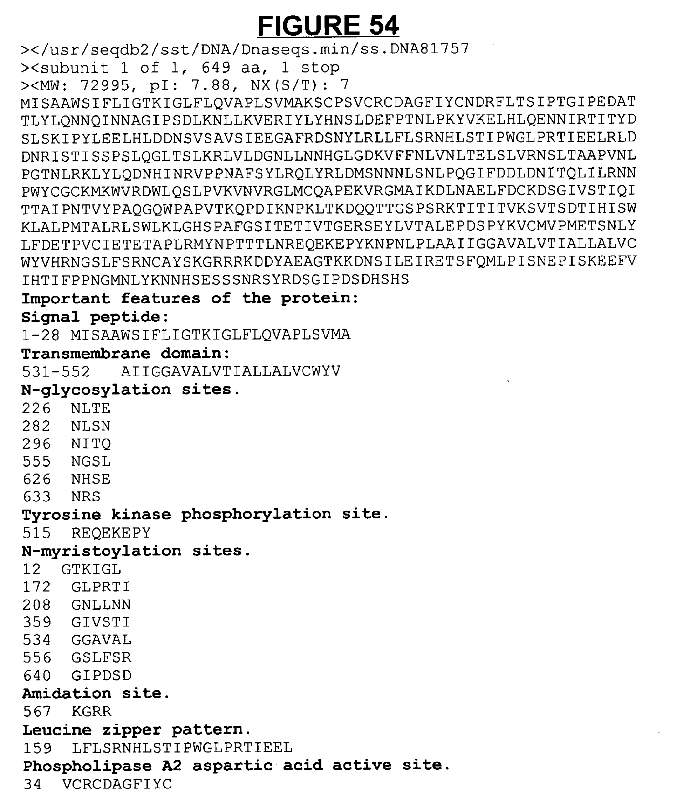

The entire nucleotide sequence of UNQ856 (DNA81757-2512) is shown in Figure 53 (SEQ ID NO:53). Clone UNQ856 (DNA81757-2512) contains a single open reading frame with an apparent translational initiation site at nucleotide positions 51-53 and ending at the stop codon at nucleotide positions 1998-2000 (Figure 53). The predicted polypeptide precursor is 649 amino acids long (Figure 54; SEQ ID NO:54). The full-length PRO1865 protein shown in Figure 54 has an estimated molecular weight of about 72,995 daltons and a pI of about 7.88. Analysis of the full-length PRO1865 sequence shown in Figure 54 (SEQ ID NO:54) evidences the presence of the following: a signal peptide from about amino acid 1 to about amino acid 28, a transmembrane domain from about amino acid 531 to about amino acid 552, potential N-glycosylation sites from about amino acid 226 to about amino acid 229, from about amino acid 282 to about amino acid 285, from about amino acid 296 to about amino acid 299, from about amino acid 555 to about amino acid 558, from about amino acid 626 to about amino acid 629 and from about amino acid 633 to about amino acid 636, a tyrosine kinase phosphorylation site from about amino acid 515 to about amino acid 522, potential N-myristolation sites from about amino acid 12 to about amino acid 17, from about amino acid 172 to about amino acid 177, from about amino acid 208 to about amino acid 213, from about amino acid 359 to about amino acid 364, from about amino acid 534 to about amino acid 539, from about amino acid 556 to about amino acid 561 and from about amino acid 640 to about amino acid 645, an amidation site from about amino acid 567 to about amino acid 570, a leucine zipper pattern sequence from about amino acid 159 to about amino acid 181 and a phospholipase A2 aspartic acid active site from about amino acid 34 to about amino acid 44. Clone UNQ856 (DNA81757-2512) has been deposited with ATCC on December 15, 1998 and is assigned ATCC deposit no. 203543.

An analysis of the Dayhoff database (version 35.45 SwissProt 35), using a WU-BLAST2 sequence alignment analysis of the full-length sequence shown in Figure 54 (SEQ ID NO:54), evidenced significant homology between the PR01865 amino acid sequence and the following Dayhoff sequences: AB007865_1, AF007139_1, GEN12302, PGS2_HUMAN, P_R89439, FMOD_HUMAN, AB000114_1, LUM_CHICK, SLIT_DROME and P_R05160.

### EXAMPLE 30: Isolation of cDNA clones Encoding Human PRO1879 Polypeptides [UNQ863]

An initial DNA sequence, referred to herein as DNA45564, was identified using a yeast screen, in a human fetal liver cDNA library that preferentially represents the 5' ends of the primary cDNA clones. DNA45565 was compared to ESTs from public databases (e.g., GenBank), and a proprietary EST database (LIFESEQ®, Incyte Pharmaceuticals, Palo Alto, CA), using the computer program BLAST or BLAST2 [Altschul et al., Methods in Enzymology, 266:460-480 (1996)]. The ESTs were clustered and assembled into a consensus DNA sequence using the computer program "phrap" (Phil Green, University of Washington, Seattle, Washington). This consensus sequence is designated herein as "DNA46964". Based on the DNA46964 consensus sequence, the following oligonucleotides were synthesized for use as primers and/or probes to isolate a clone of the full-length coding sequence for PRO1879:
forward (SEQ ID NO:165): 5'TGTTAACACCAGTCTCAGTTGGAGGG3';
reverse (SEQ ID NO:166): 5'GCCACAATACTAGCAGAATGACGCC3'; and
plasmid(SEQ ID NO:167):
5'CCTTATTGGTATCTGTGCCTTTAGCCATGCCCATAGCCATGCCCATGGAG3'.

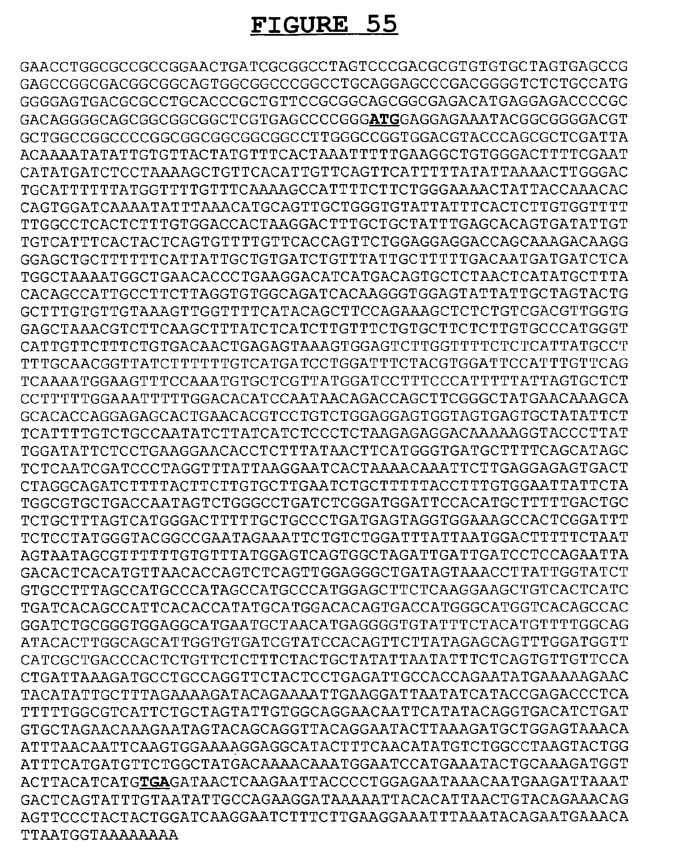

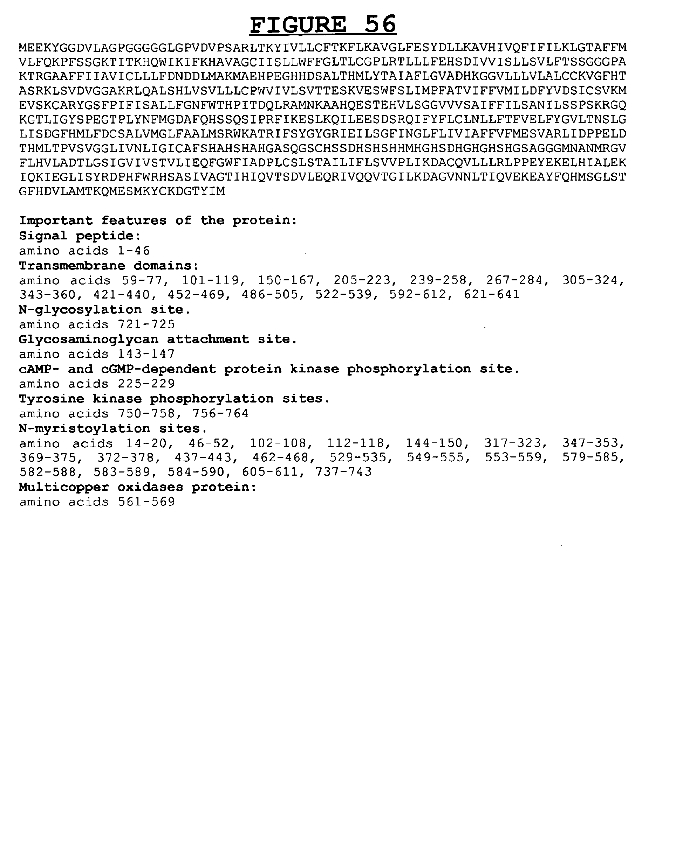

The full length DNA54009-2517 clone shown in Figure 55 contained a single open reading frame with an apparent translational initiation site at nucleotide positions 219-221 and ending at the stop codon found at nucleotide positions 2514-2516 (Figure 55; SEQ ID NO:55). The predicted polypeptide precursor (Figure 56, SEQ ID NO:56) is 765 amino acids long. PRO1879 has a calculated molecular weight of approximately 83974 daltons and an estimated pI of approximately 7.29.

An analysis of the Dayhoff database (version 35.45 SwissProt 35), using a WU-BLAST2 sequence alignment analysis ofthe full-length sequence shown in Figure 56 (SEQ ID NO:56), revealed significant homology between the PRO1879 amino acid sequence and the following Dayhoff sequences: S61568; D89239_1; P_RO4584; ZRC1_YEAST; STU60071_1, S54303; P_R46087; MMZNT4S4_1; CEH13N06_5; and S76964.

Clone DNA54009-2517 (UNQ863), designated as DNA54009-2517 has been deposited with the ATCC on January 12, 1999 and is assigned ATCC deposit no. 203574.

### EXAMPLE 31: Isolation of cDNA clones Encoding Human PRO3446 Polypeptides [UNQ1833]

DNA92219-2541 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

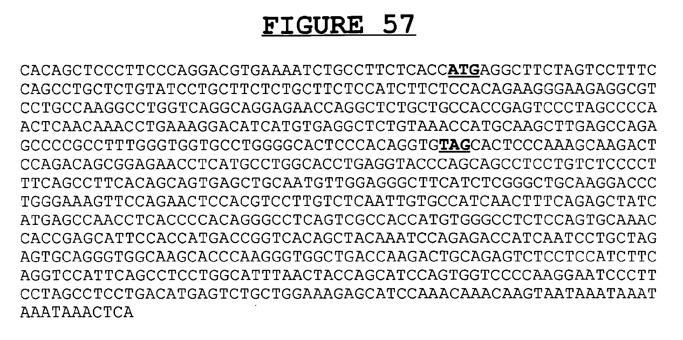

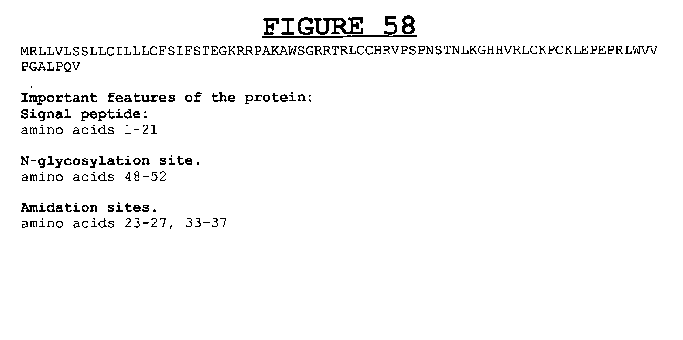

Use of the above described signal sequence algorithm allowed identification of an EST cluster sequence from the Incyte database. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). One or more of the ESTs used in the assembly was derived from a epidermal keratinocyte library. The consensus sequence obtained therefrom is herein designated DNA79199. In light of the sequence homology between DNA79199 and the EST 4564202H1, the clone containing this EST was purchased and the cDNA insert was obtained and sequenced herein identified as DNA92219-2541 (SEQ ID NO:57, Figure 57).The full length clone shown in Figure 57 contained a single open reading frame with an apparent translational initiation site at nucleotide positions 42-44 and ending at the stop codon found at nucleotide positions 285-287 (Figure 57; SEQ ID NO:57). The predicted polypeptide precursor (Figure 58, SEQ ID NO:58) is 81 amino acids long. PR03446 has a calculated molecular weight of approximately 9173 daltons and an estimated pI of approximately 10.8.

Clone DNA92219-2541 (UNQ1833), designated as DNA92219-2541 was deposited with the ATCC on February 9, 1999and is assigned ATCC deposit no. 203663.

### EXAMPLE 32: Isolation of cDNA clones Encoding Human PR03543 Polypeptides [UNQ1835]

DNA86571-2551 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence. The clone 743044 which includes this EST sequence was purchased and the cDNA insert was obtained and sequenced.

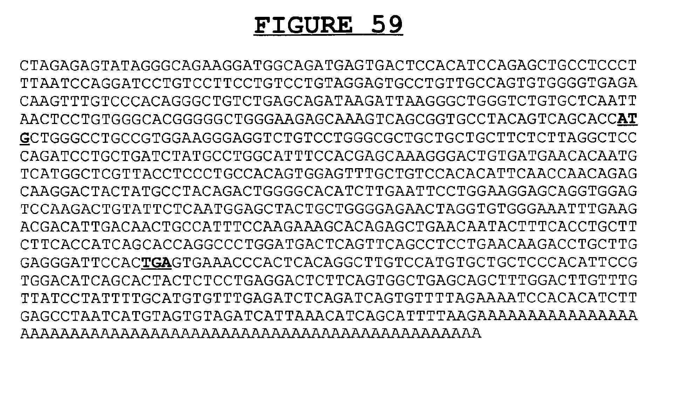

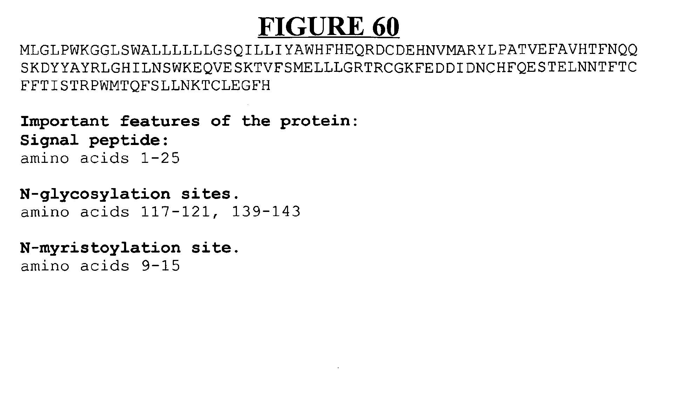

The full length clone shown in Figure 59 contained a single open reading frame with an apparent translational initiation site at nucleotide positions 243-245 and ending at the stop codon found at nucleotide positions 684-686 (Figure 59; SEQ ID NO:59). The predicted polypeptide precursor (Figure 60, SEQ ID NO:60) is 147 amino acids long. PR03543 has a calculated molecular weight of approximately 17276 daltons and an estimated pI of approximately 6.31.

An analysis of the Dayhoff database (version 35.45 SwissProt 35), using a WU-BLAST2 sequence alignment analysis of the full-length sequence shown in Figure 60 (SEQ ID NO:60), revealed homology between the PR02543 amino acid sequence and the following Dayhoff sequences: MMU18243_1, ABO17157_1, SCCL54M_1, CYTT_HUMAN, CYTC_MACMU, CYTO_BOVIN, HGS_RF246, AF031825_1, AB015225_1, P_W15791.

Clone DNA86571-2551 (UNQ1835), designated as DNA86571-2551 was deposited with the ATCC on February 9, 1999and is assigned ATCC deposit no. 203660.

### EXAMPLE 33: Isolation of cDNA clones Encoding Human PRO4329 Polypeptides [UNQ1885]

The extracellular domain (ECD) sequences (including the secretion signal sequence, if any) from about 950 known secreted proteins from the Swiss-Prot public database were used to search EST databases. The EST databases included public EST databases (e.g., GenBank), and a proprietary EST database (LIFESEQ®, Incyte Pharmaceuticals, Palo Alto, CA). The search was performed using the computer program BLAST or BLAST2 [Altschul et al., Methods in Enzymology, 266:460-480 (1996)] as a comparison of the ECD protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

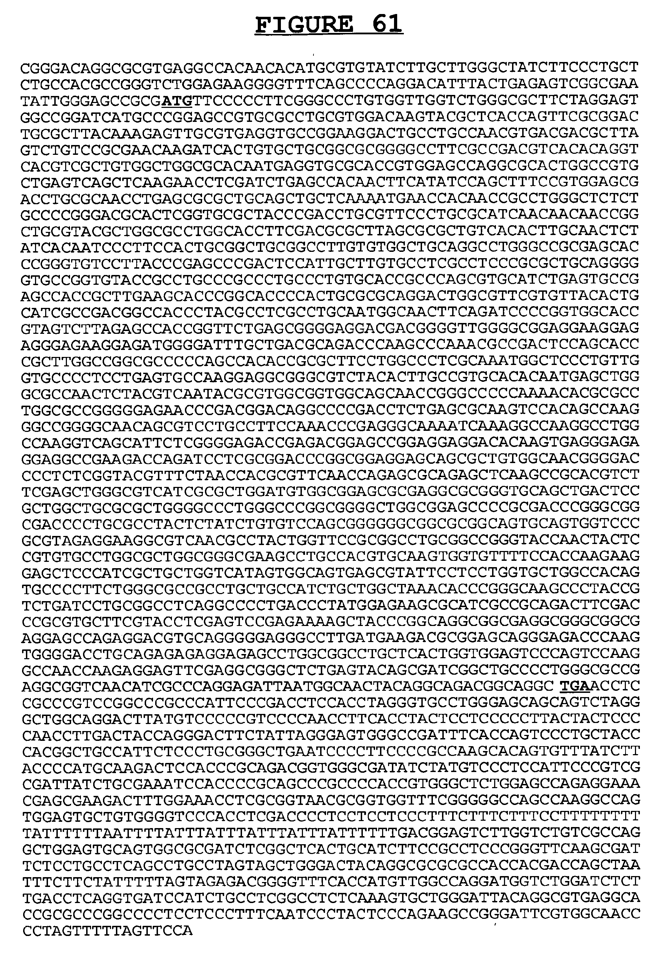

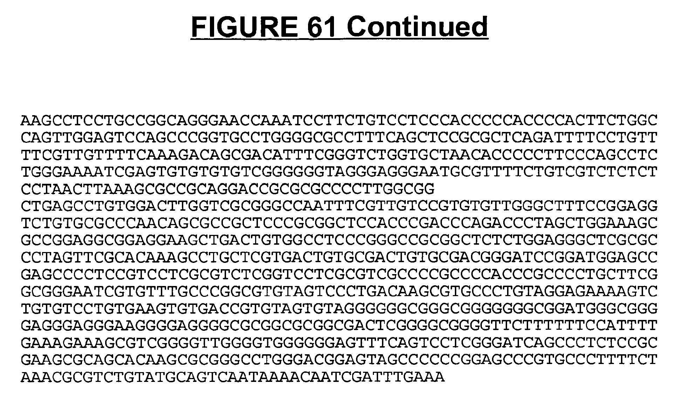

A consensus DNA sequence was assembled relative to other EST sequences using phrap. This consensus sequence is herein designated DNA52164. Based upon an observed homology between the DNA52164 consensus sequence and the Incyte EST clone no. 3350865, Incyte EST clone no. 3350865 was purchased and its insert obtained and sequenced, wherein the sequence obtained is shown in Figure 61.

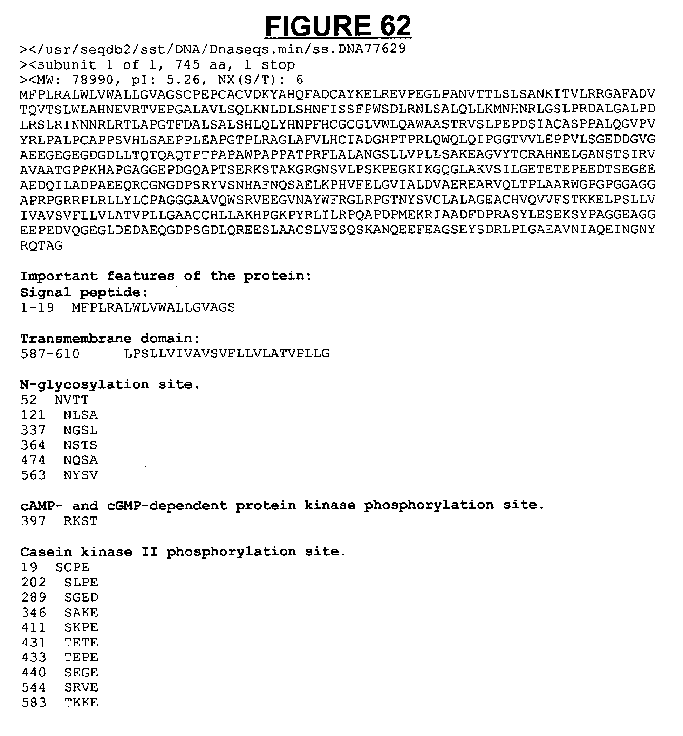

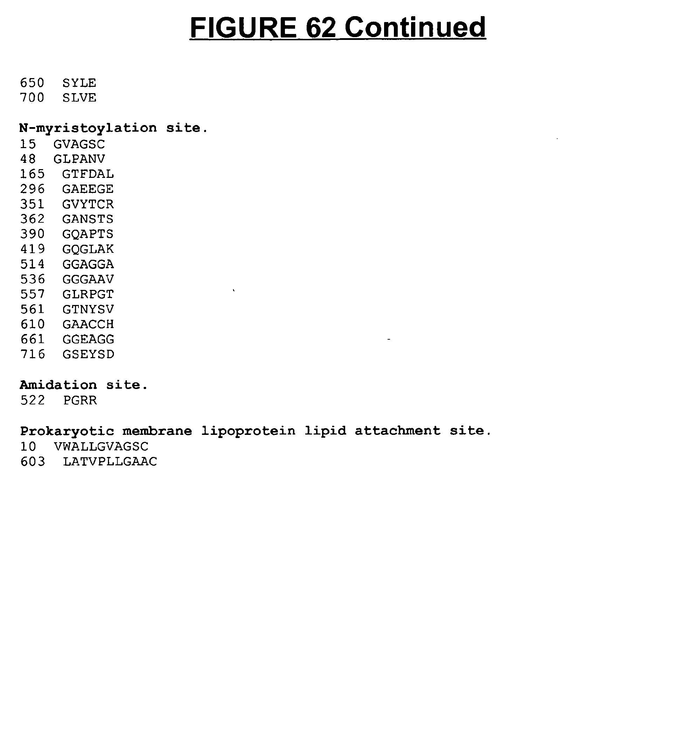

The entire nucleotide sequence of UNQ1885 (DNA77629-2573) is shown in Figure 61 (SEQ ID NO:61). Clone UNQ 1885 (DNA77629-2573) contains a single open reading frame with an apparent translational initiation site at nucleotide positions 137-139 and ending at the stop codon at nucleotide positions 2372-2374 (Figure 61). The predicted polypeptide precursor is 745 amino acids long (Figure 62; SEQ ID NO:62). The full-length PR04329 protein shown in Figure 62 has an estimated molecular weight of about 78,990 daltons and a pI of about 5.26. Analysis of the full-length PR04329 sequence shown in Figure 62 (SEQ ID NO:62) evidences the presence of a variety of important polypeptide domains as shown in Figure 62. Clone UNQ1885 (DNA77629-2573) has been deposited with ATCC on March 16, 1999 and is assigned ATCC deposit no. 203850.

An analysis of the Dayhoff database (version 35.45 SwissProt 35), using a WU-BLAST2 sequence alignment analysis of the full-length sequence shown in Figure 62 (SEQ ID NO:62), evidenced significant homology between the PR04329 amino acid sequence and the following Dayhoff sequences: AB003184_1, AB011530_1, AB017167_1, P_W46966, AB017167_1, SLIT_DROME, DMU11052_1, A53531, DROWHEELER_1 and A2GL_HUMAN.

### EXAMPLE 34: Isolation of cDNA clones Encoding Human PRO4352 Polypeptides [UNQ1906]

A consensus DNA sequence was assembled relative to other EST sequences using phrap as described in Example 1 above. This consensus sequence is designated herein "DNA83397". Based on the DNA83397 consensus sequence, oligonucleotides were synthesized: 1) to identify by PCR a cDNA library that contained the sequence of interest, and 2) for use as probes to isolate a clone of the full-length coding sequence for PR04352.

PCR primers (forward and reverse) were synthesized:
forward PCR primer: 5'-CTGGGGAGTGTCCTTGGCAGGTTC-3' (SEQ ID NO:168) and
reverse PCR primer: 5'-CAGCATACAGGGCTCTTTAGGGCACAC-3' (SEQ ID NO:169).

Additionally, a synthetic oligonucleotide hybridization probe was constructed from the consensus DNA83397 sequence which had the following nucleotide sequence:
hybridization probe: 5'-CGGTGACTGAGGAAACAGAGAAAGGATCCTTTGTGGTCAATCTGGC-3' (SEQ ID NO:170).

In order to screen several libraries for a source of a full-length clone, DNA from the libraries was screened by PCR amplification with the PCR primer pair identified above. A positive library was then used to isolate clones encoding the PR04352 gene using the probe oligonucleotide and one of the PCR primers. RNA for construction of the cDNA libraries was isolated from human fetal brain.

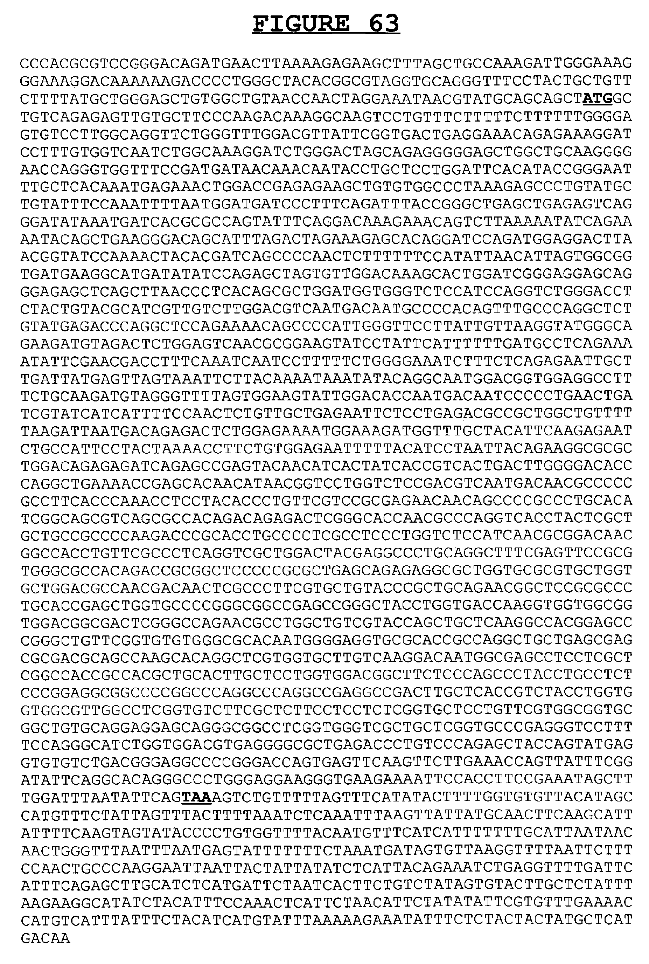

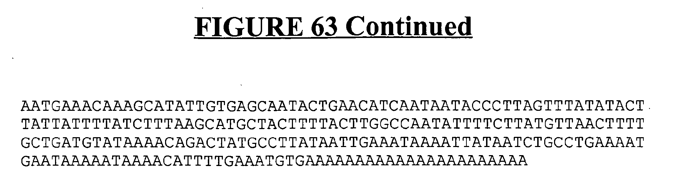

DNA sequencing of the clones isolated as described above gave the full-length DNA sequence for PR04352 (designated herein as DNA87976-2593 [Figure 63, SEQ ID NO:63]; and the derived protein sequence for PR04352.

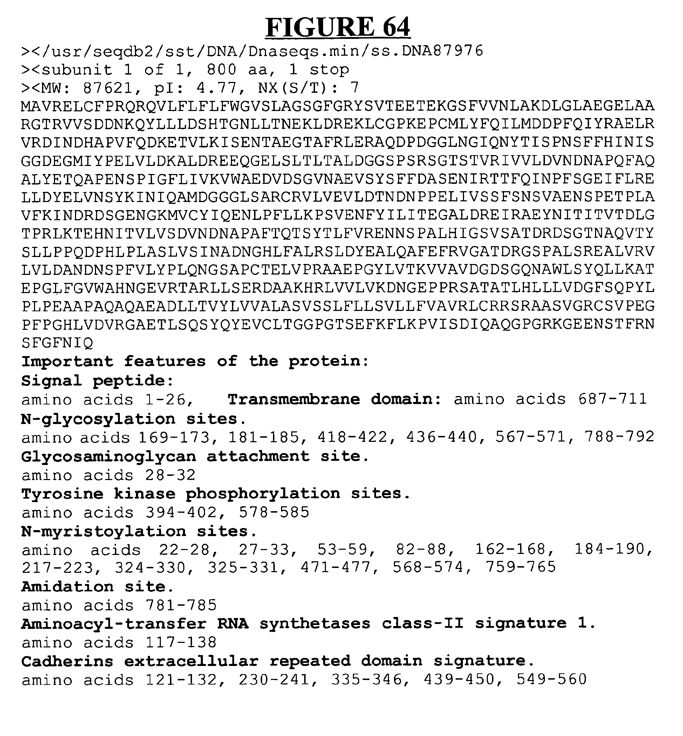

The entire coding sequence of PR04352 is shown in Figure 63 (SEQ ID NO:63). Clone DNA87976-2593 contains a single open reading frame with an apparent translational initiation site at nucleotide positions 179-181, and an apparent stop codon at nucleotide positions 2579-2581 of SEQ ID NO:63. The predicted polypeptide precursor is 800 amino acids long. Clone DNA87976-2593 has been deposited with ATCC on March 30, 1999 and is assigned ATCC deposit no. 203888. The full-length PR04352 protein shown in Figure 64 has an estimated molecular weight of about 87,621 daltons and a pI of about 4.77.

An analysis of the Dayhoff database (version 35.45 SwissProt 35), using a WU-BLAST2 sequence alignment analysis of the full-length sequence shown in Figure 64 (SEQ ID NO:64), revealed homology between the PR04352 amino acid sequence and the following Dayhoff sequences: P_R86865, P_R86866, RATPCDH_1, AB011160_1, MMU88549_1, D86917_1, AB008179_1, P_R58907, HSHFATPRO_1, and AF031572_1.

### EXAMPLE 35: Isolation of cDNA clones Encoding Human PRO9859 Polypeptides [UNQ3043]

DNA125170-2780 was identified by applying a proprietary signal sequence finding algorithm developed by Genentech, Inc. (South San Francisco, CA) upon ESTs as well as clustered and assembled EST fragments from public (e.g., Genbank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST cluster sequence from the LIFESEQ® database, Incyte Pharmaceuticals, Palo Alto, CA, designated herein as CLU35710. This EST cluster sequence was then compared to a variety of expressed sequence tag (EST) databases which included public EST databases (e.g., Genbank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA109258.

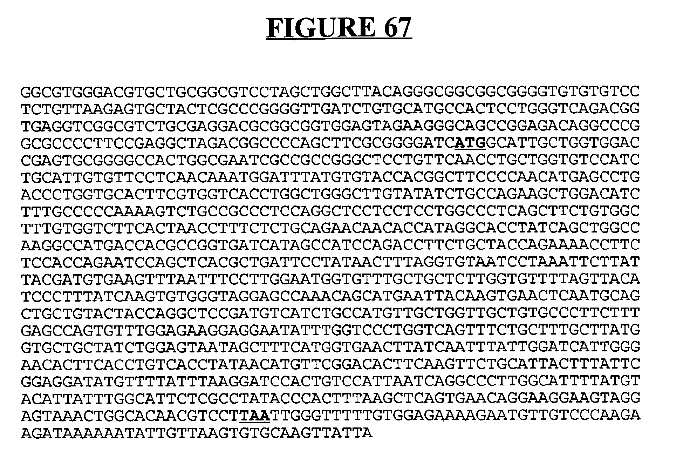

In light of an observed sequence homology between the DNA109258 sequence and an EST sequence encompassed within clone no. 3145532 from the LIFESEQ® database, Incyte Pharmaceuticals, Palo Alto, CA, clone no. 3145532 was purchased and the cDNA insert was obtained and sequenced. It was found herein that the cDNA insert encoded a full-length protein. The sequence of this cDNA insert is shown in Figure 67 and is herein designated as DNA125170-2780.

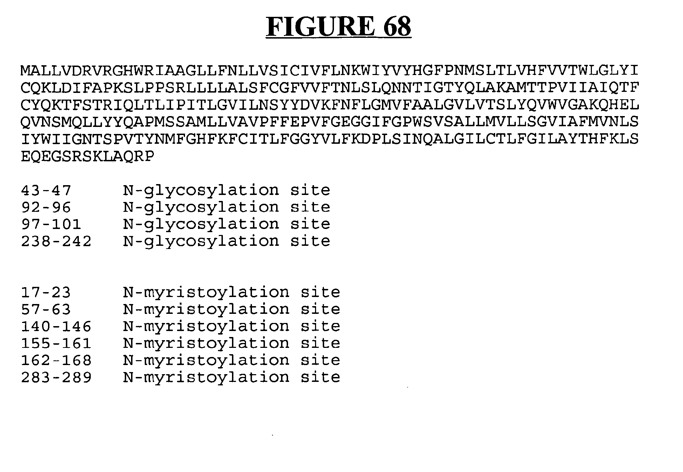

Clone DNA125170-2780 contains a single open reading frame with an apparent translational initiation site at nucleotide positions 223-225 and ending at the stop codon at nucleotide positions 1162-1164 (Figure 67; SEQ ID NO:67). The predicted polypeptide precursor is 313 amino acids long (Figure 68; SEQ ID NO:68). The full-length PR09859 protein shown in Figure 68 has an estimated molecular weight of about 35,066 daltons and a pI of about 9.39. Analysis of the full-length PR09859 sequence shown in Figure 68 (SEQ ID NO: 68) evidences the presence of a variety of important polypeptide domains as shown in Figure 68, wherein the locations given for those important polypeptide domains are approximate as described above. Clone DNA125170-2780 has been deposited with ATCC on November 16, 1999 and is assigned ATCC Deposit No. PTA-953.

An analysis of the Dayhoff database (version 35.45 SwissProt 35), using the ALIGN-2 sequence alignment analysis of the full-length sequence shown in Figure 68 (SEQ ID NO: 68), evidenced sequence identity between the PR09859 amino acid sequence and the following Dayhoff sequences: T04029, ATF23K16_3, C71612, SPBC83_11, P_W88504, D87449_1, CPT2_BRAOL, HSA005866_1, CPTR_SOLTU, and CPTR_FLAPR.

### EXAMPLE 36: Isolation of cDNA clones Encoding Human PRO9864 Polypeptides [UNQ3048]

DNA125151-2784 was identified by applying a proprietary signal sequence finding algorithm developed by Genentech, Inc. (South San Francisco, CA) upon ESTs as well as clustered and assembled EST fragments from public (e.g., Genbank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence from the LIFESEQ®, Incyte Pharmaceuticals, Palo Alto, CA database, designated herein as EST971407. This EST sequence was then compared to a variety of expressed sequence tag (EST) databases which included public EST databases (e.g., Genbank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA111248.

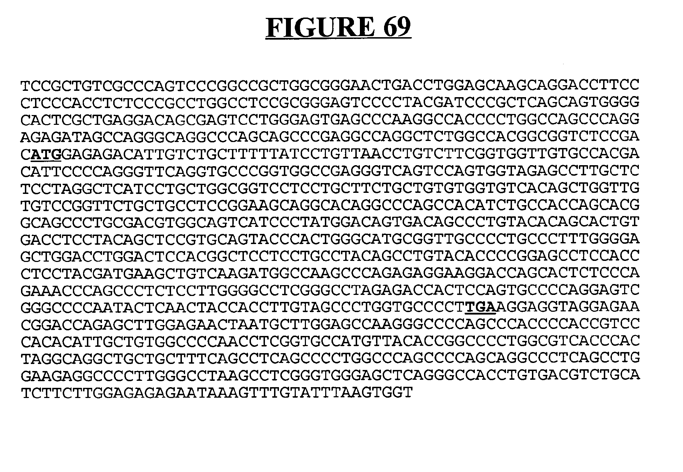

In light of an observed sequence homology between the DNA111248 sequence and an EST sequence encompassed within clone no. 971407 from the LIFESEQ®, Incyte Pharmaceuticals, Palo Alto, CA database, clone no. 971407 was purchased and the cDNA insert was obtained and sequenced. It was found herein that that cDNA insert encoded a full-length protein. The sequence of this cDNA insert is shown in Figure 69 and is herein designated as DNA125151-2784.

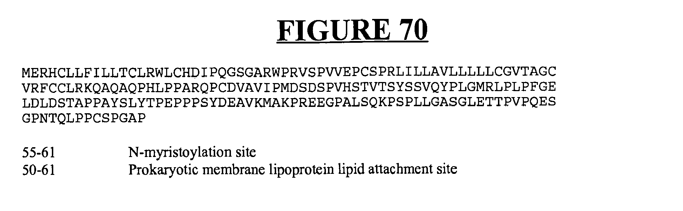

Clone DNA125151-2784 contains a single open reading frame with an apparent translational initiation site at nucleotide positions 242-244 and ending at the stop codon at nucleotide positions 824-826 (Figure 69; SEQ ID NO:69). The predicted polypeptide precursor is 194 amino acids long (Figure 70; SEQ ID NO:70). The full-length PR09864 protein shown in Figure 70 has an estimated molecular weight of about 20,882 daltons and a pI of about 6.44. Analysis of the full-length PR09864 sequence shown in Figure 70 (SEQ ID NO: 70) evidences the presence of a variety of important polypeptide domains as shown in Figure 70, wherein the locations given for those important polypeptide domains are approximate as described above. Clone DNA125151-2784 has been deposited with ATCC on December 7, 1999 and is assigned ATCC Deposit No. PTA-1029.

An analysis of the Dayhoff database (version 35.45 SwissProt 35), using the ALIGN-2 sequence alignment analysis of the full-length sequence shown in Figure 70 (SEQ ID NO: 70), evidenced sequence identity between the PR09864 amino acid sequence and the following Dayhoff sequences: DIA1_HUMAN, P_Y13464, HWP1_CANAL, AB022927_1, P_W76734, SRE1_RAT, MMHC188A7_6, S50755, FNU44091_1 and AF036334_1.

### EXAMPLE 37: Isolation of cDNA clones Encoding Human PRO9904 Polypeptides [UNQ3072]

DNA 129549-2798 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ , Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence from the LIFESEQ® database, designated herein as 1556012H 1. This EST sequence was then compared to a variety ofexpressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap (Phil Green, University of Washington, Seattle, Washington). The consensus sequence obtained therefrom is herein designated DNA115227.

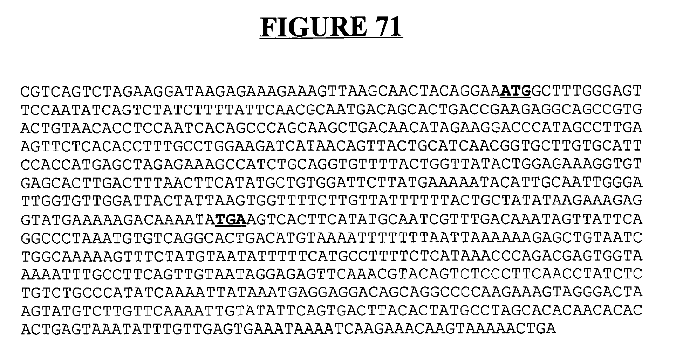

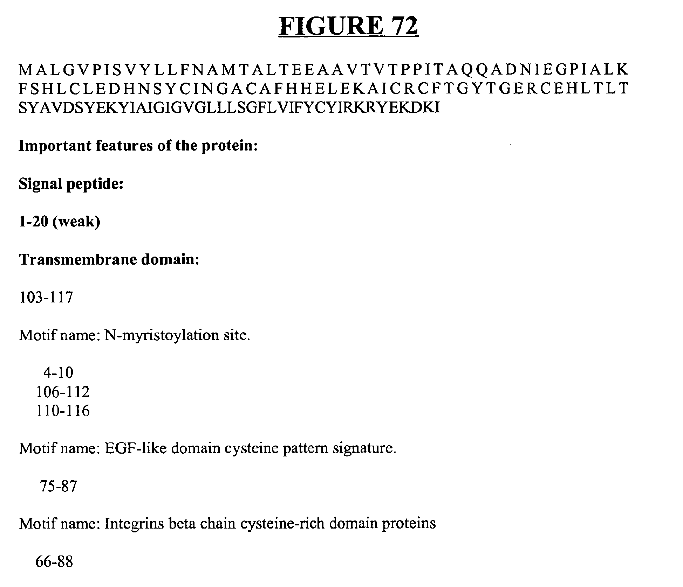

In light of an observed sequence homology between the DNA115227 sequence and an EST sequence encompassed within clone no. 1556012H1 from the LIFESEQ® database, clone no. 1556012H1 was purchased and the cDNA insert was obtained and sequenced. It was found herein that that cDNA insert encoded a full-length protein. The sequence of this cDNA insert is shown in Figure 71 and is herein designated as DNA129549-2798. Clone DNA129549-2798 contains a single open reading frame with an apparent translational initiation site at nucleotide positions 48-50 and ending at the stop codon at nucleotide positions 447-449 (Figure 71; SEQ ID NO:71). The predicted polypeptide precursor is 133 amino acids long (Figure 72; SEQ ID NO:72). The full-length PR09904 protein shown in Figure 72 has an estimated molecular weight of about 14792 daltons and a pI of about 5.97. Analysis of the full-length PR09904 sequence shown in Figure 72 (SEQ ID NO:72) evidences the presence of a variety of important polypeptide domains as shown in Figure 72, wherein the locations given for those important polypeptide domains are approximate as described above. Clone DNA129549-2798 has been deposited with ATCC on December 22, 1999 and is assigned ATCC deposit no. PTA-1099.

An analysis of the Dayhoff database (version 35.45 SwissProt 35), using the ALIGN-2 sequence alignment analysis of the full-length sequence shown in Figure 72 (SEQ ID NO:72), evidenced sequence identity between the PR09904 amino acid sequence and the following Dayhoff sequences: P_R82987, P_W09406, GRFA_VACCC, G01639, TMEFF1_1, D30782_1, XLEGFPR_1, P_W27536, P_R15350, HUMHERGC_1.

### EXAMPLE 38: Isolation of cDNA clones Encoding Human PRO9907 Polypeptides [UNQ3075]

The extracellular domain (ECD) sequences (including the secretion signal sequence, if any) from about 950 known secreted proteins from the Swiss-Prot public database were used to search EST databases. The EST databases included (1) a proprietary EST database LIFESEQ (Incyte Pharmaceuticals, Palo Alto, CA), and (2) a proprietary EST database from Genentech]. The search was performed using the computer program BLAST or BLAST2 [Altschul et al., Methods in Enzymology, 266:460-480 (1996)] as a comparison of the ECD protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington). A consensus DNA sequence was assembled relative to other EST sequences using phrap as described above. This consensus sequence is herein designated DNA130936. In some cases, the consensus sequence derives from an intermediate consensus DNA sequence which was extended using repeated cycles of BLAST and phrap to extend that intermediate consensus sequence as far as possible using the sources of EST sequences discussed above.

Based on the DNA130936 consensus sequence oligonucleotides were synthesized: 1) to identify by PCR a cDNA library that contained the sequence of interest, and 2) for use as probes to isolate a clone of the full-length coding sequence for PR09907. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5 kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.
PCR primers (forward and reverse) were synthesized:
forward PCR primer 5'-CACGTCTCTTCAACCTCCGCTC -3' (SEQ ID NO: 171)
reverse PCR primer 5'-GGATGTGCTTAGGTCCCGCAC -3' (SEQ ID NO: 172)
Additionally, a synthetic oligonucleotide hybridization probe was constructed from the consensus DNA130936 sequence which had the following nucleotide sequence
hybridization probe
5'-GGAACAGGATTCGCTCCATTAGCCAAGGTTTGACATGGACTTGGAG -3' (SEQ ID NO: 173)

RNA for construction of the cDNA libraries was isolated from human fetal brain tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

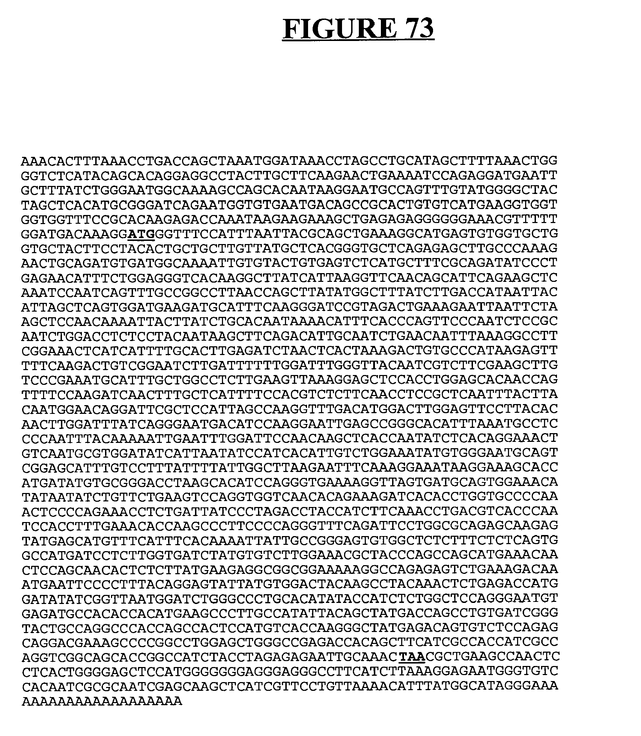

DNA sequencing of the clones isolated as described above gave the full-length DNA sequence for a full-length PR09907 polypeptide (designated herein as DNA142392-2800 [Figure 73, SEQ ID NO: 73]) and the derived protein sequence for that PR09907 polypeptide.

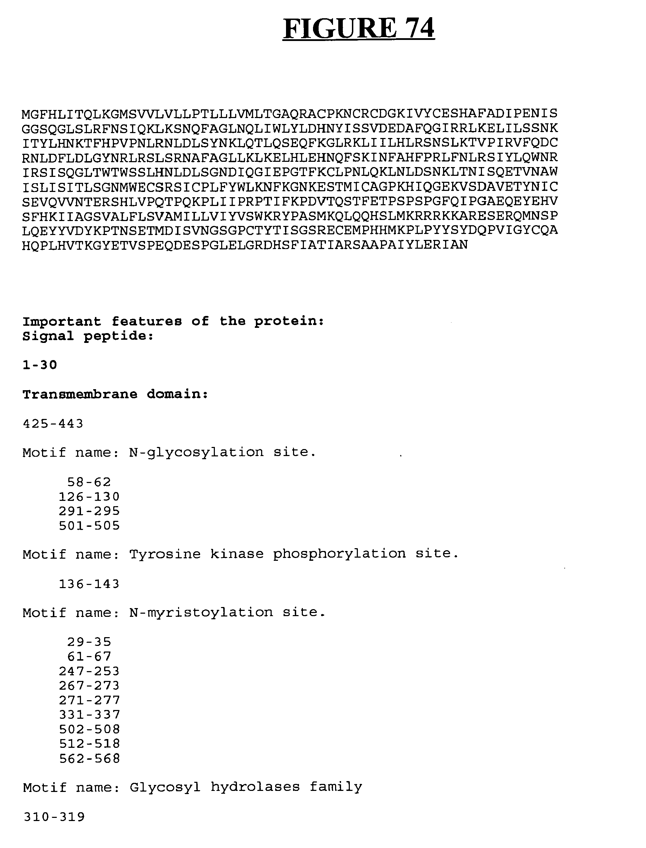

The full length clone identified above contained a single open reading frame with an apparent translational initiation site at nucleotide positions 325-327 and a stop signal at nucleotide positions 2095-2097 (Figure 73, SEQ ID NO:73). The predicted polypeptide precursor is 590 amino acids long, has a calculated molecular weight of approximately 67217 daltons and an estimated pI of approximately 9.26. Analysis of the full-length PR09907 sequence shown in Figure 74 (SEQ ID NO:74) evidences the presence of a variety of important polypeptide domains as shown in Figure 74, wherein the locations given for those important polypeptide domains are approximate as described above. Clone DNA142392-2800 has been deposited with ATCC on December 22, 1999 and is assigned ATCC deposit no. PTA-1092.

An analysis of the Dayhoff database (version 35.45 SwissProt 35), using the ALIGN-2 sequence alignment analysis of the full-length sequence shown in Figure 74 (SEQ ID NO:74), evidenced sequence identity between the PR09907 amino acid sequence and the following Dayhoff sequences: AB007876_1, P_Y08010, A58532, ALS_MOUSE, ALS_HUMAN, P_Y13394, CHAD_1, P_W93906, DMTARTAN_1 and GEN11209.

### EXAMPLE 39: Isolation of cDNA clones Encoding Human PRO10013 Polypeptides [UNQ3082]

DNA125181-2804 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ , Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST cluster sequence from the LIFESEQ (Incyte Pharmaceuticals, Palo Alto, CA) database, designated herein as 2692743H 1. This EST cluster sequence was then compared to a variety ofexpressed sequence tag (EST) databases which included public EST databases (e.g., GenBank) and a proprietary EST DNA database (LIFESEQ , Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA111265.

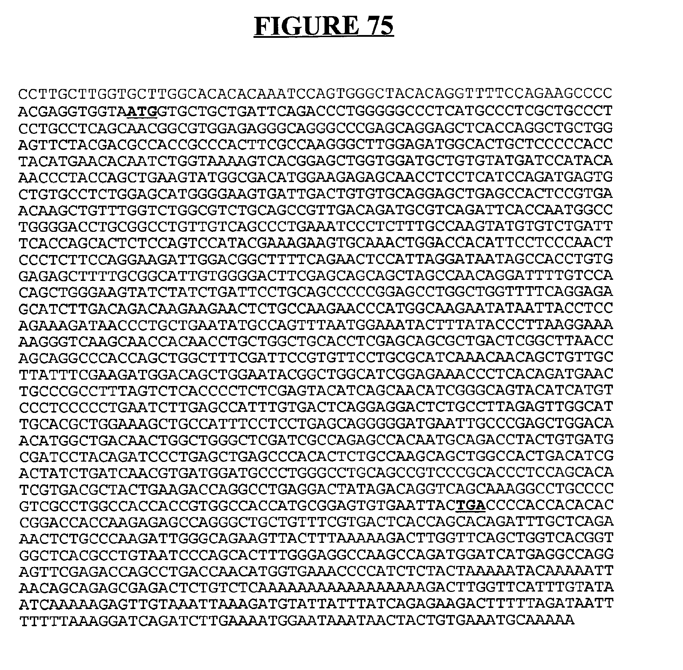

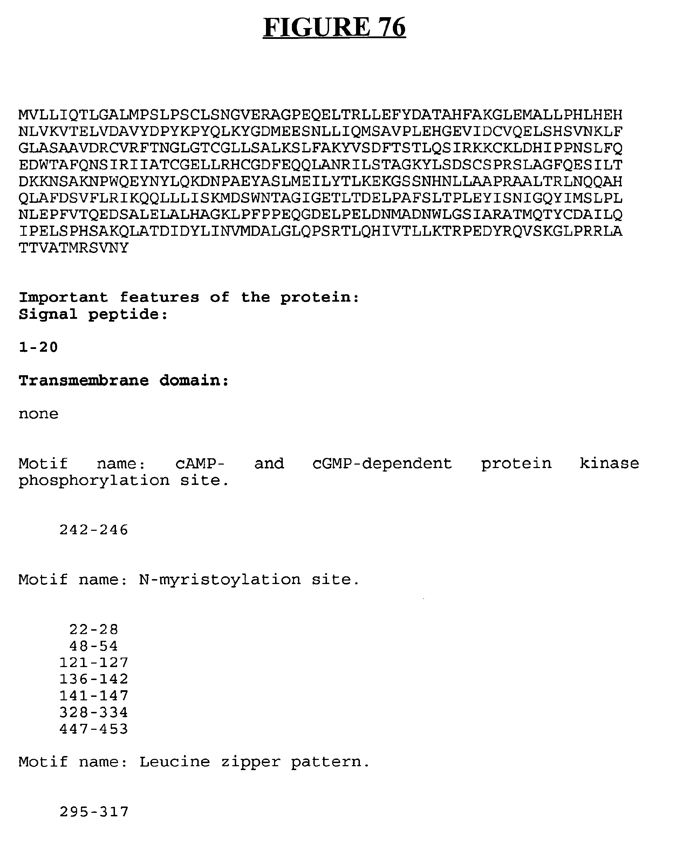

In light of an observed sequence homology between the DNA111265 sequence and an EST sequence encompassed within clone no. 2692743 from the LIFESEQ database, clone no. 2692743 was purchased and the cDNA insert was obtained and sequenced. It was found herein that the cDNA insert encoded a full-length protein. The sequence of this cDNA insert is shown in Figure 75 and is herein designated as DNA125181-2804. Clone DNA125181-2804 contains a single open reading frame with an apparent translational initiation site at nucleotide positions 72-74 and ending at the stop codon at nucleotide positions 1545-1547 (Figure 75; SEQ ID NO:75). The predicted polypeptide precursor is 491 amino acids long (Figure 76; SEQ ID NO:76). The full-length PRO10013 protein shown in Figure 76 has an estimated molecular weight of about 54759 daltons and a pI of about 5.61. Analysis of the full-length PRO10013 sequence shown in Figure 76 (SEQ ID NO: 76) evidences the presence of a variety of important polypeptide domains as shown in Figure 76, wherein the locations given for those important polypeptide domains are approximate as described above. Clone DNA125181-2804 has been deposited with ATCC on December 22, 1999 and is assigned ATCC deposit no. PTA-1096.

### EXAMPLE 40: Isolation of cDNA clones Encoding Human PRO16089 Polypeptides [UNQ5782]

### 1. Preparation of oligo dT primed cDNA library

mRNA was isolated from human testis tissue using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/Notl linkered cDNA was cloned into XhoI/NotI cleaved vector. pRK5D is a cloning vector that has an SP6 transcription initiation site followed by an SfiI restriction enzyme site preceding the XhoI/NotI cDNA cloning sites.

### 2. Preparation of random primed cDNA library

A secondary cDNA library was generated in order to preferentially represent the 5' ends of the primary cDNA clones. SP6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with the amylase sequence will lead to the secretion of amylase from appropriately transfected yeast colonies.

### 3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, e.g. CsCl-gradient. The purified DNA was then carried on to the yeast protocols below.

The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11, his3-15, MAL+, SUC+, GAL+. Preferably, yeast mutants can be employed that have deficient post-translational pathways. Such mutants may have translocation deficient alleles in sec71, sec72, sec62, with truncated sec71 being most preferred. Alternatively, antagonists (including antisense nucleotides and/or ligands) which interfere with the normal operation of these genes, other proteins implicated in this post translation pathway (e.g., SEC61p, SEC72p, SEC62p, SEC63p, TDJ1p or SSA1p-4p) or the complex formation of these proteins may also be preferably employed in combination with the amylase-expressing yeast.

Transformation was performed based on the protocol outlined by Gietz et al., Nucl. Acid. Res., 20:1425 (1992). Transformed cells were then inoculated from agar into YEPD complex media broth (100 ml) and grown overnight at 30 C. The YEPD broth was prepared as described in Kaiser et al., Methods in Yeast Genetics, Cold Spring Harbor Press, Cold Spring Harbor, NY, p. 207 (1994). The overnight culture was then diluted to about 2 x 106 cells/ml (approx. OD600=0.1) into fresh YEPD broth (500 ml) and regrown to 1 x 107 cells/ml (approx. OD600=0.4-0.5).

The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM Li200CCH3), and resuspended into LiAc/TE (2.5 ml).

Transformation took place by mixing the preared cells (100 1) with freshly denatured single stranded salmon testes DNA (Lofstrand Labs, Gaithersburg, MD) and transforming DNA (1 g, vol. < 10 l) in microfuge tubes. The mixture was mixed briefly by vortexing, then 40% PEG/TE (600 1, 40% polyethylene glycol-4000, 10 mM Tris-HCl, 1 mM EDTA, 100 mM Li200CCH3, pH 7.5) was added. This mixture was gently mixed and incubated at 30°C while agitating for 30 minutes. The cells were then heat shocked at 42°C for 15 minutes, and the reaction vessel contrifuged in a microfuge at 12,000 rpm for 5-10 seconds, decanted and resuspended into TE (500 l, 10 mM Tris-HCl, 1 mM EDTA pH 7.5) followed by recentrifugation. The cells were then diluted into TE (1 ml) and aliquots (200 l) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press, Cold Spring Harbor, NY, p. 208-210 (1994). Transformants were grown at 30 C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15% (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation ofred starch into buffered SCD-Ura agar. Positive colonies were determined by their ability to break down starch resulting in a clear halo around the positive colony visualized directly.

### 4. Isolation of DNA by PCR Amplification

When a positive colony was isolated, a portion of it was picked by a toothpick and diluted into sterile water (30 l) in a 96 well plate. At this time, the positive colonies were either frozen and stored for subsequent analysis or immediately amplified. An aliquot of cells (5 l) was used as a template for the PCR reaction in a 25 l volume containing: 0.5 l Klentaq (Clontech, Palo Aloto, CA); 4.0 l 10 mM dNTP's (Perkin Elmer-Cetus); 2.5 l Klentaq buffer (Clontech); 0.25 l forward oligo 1; 0.25 l reverse oligo 2; 12.5 l distilled water. The sequence of the forward oligonucleotide 1 was:
5'-TGTAAAACGACGGCCAGTTAAATAGACCTGCAATTATTAATCT-3'
(SEQ ID NO: 97)
The sequence of reverse oligonucleotide 2 was:
5'-CAGGAAACAGCTATGACCACCTGCACACCTGCAAATCCATT-3'
(SEQ ID NO: 98)

PCR was then performed as follows:

| | | | | |
|---|---|---|---|---|
| a. | | Denature | 92°C, | 5 minutes |
| | | | | |
| b. | 3 cycles of: | Denature | 92°C, | 30 seconds |
| | | Anneal | 59°C, | 30 seconds |
| | | Extend | 72°C, | 60 seconds |
| | | | | |
| c. | 3 cycles of: | Denature | 92°C, | 30 seconds |
| | | Anneal | 57°C, | 30 seconds |
| | | Extend | 72°C, | 60 seconds |

| | | | | |
|---|---|---|---|---|
| d. | 25 cycles of: | Denature | 92°C, | 30 seconds |
| | | Anneal | 55°C, | 30 seconds |
| | | Extend | 72°C, | 60 seconds |
| | | | | |
| e. | | Hold | 4°C | |

The underlined regions of the oligonucleotides disclosed above annealed to the ADH promoter region and the amylase region, respectively, and amplified a 307 bp region from vector pSST-AMY.0 when no insert was present. Typically, the first 18 nucleotides of the 5' end of these olignucleotides contained annealing sites for the sequencing primers. Thus, the total product of the PCR reaction from any empty vector was 343 bp. However, signal sequence-fused cDNA resulted in considerably longer nucleotide sequences.

Following the PCR, an aliquot of the reaction (5 l) was examined by agarose gel electrophoresis in a 1 % agarose gel using Tris-Borate-EDTA (TBE) buffering system as described by Sambrook et al., supra. Clones resulting in a single strong PCR product larger than 400 bp were further analyzed by DNA sequencing after purification with a 96 Qiaquick PCR clean-up column (Qiagen Inc., Chatsworth, CA).

### 5. Identification of Full-length Clone

A cDNA sequence isolated in the above screen is herein designated DNA65836. Probes were then generated from the sequence of the DNA65836 molecule and used to screen a human testis library prepared as described in paragraph 1 above. The cloning vector was pRK5B (pRK5B is a precursor of pRK5D that does not contain the SfiI site; see, Holmes et al., Science, 253:1278-1280 (1991)), and the cDNA size cut was less than 2800 bp. The oligonucleotides probes were synthesized: 1) to identify by PCR a cDNA library that contained the sequence of interest, and 2) for use as probes to isolate a clone of the full-length coding sequence for PRO16089. Forward and reverse PCR primers generally range from 20 to 30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

The oligonucleotide probes employed were as follows:
forward PCR primer 5' - CATCCAGCTCCATCTCCCATTTGG - 3' (SEQ ID NO: 174)
reverse PCR primer 5' - TGCAGTCCTTTCTACTGTTAGCCCAGG - 3' (SEQ ID NO: 175)
hybridization probe 5' - GTCATGCTGCTCTGGGTCTGGTAACTCTTTGCCTGATGTT - 3' (SEQ ID NO:176)

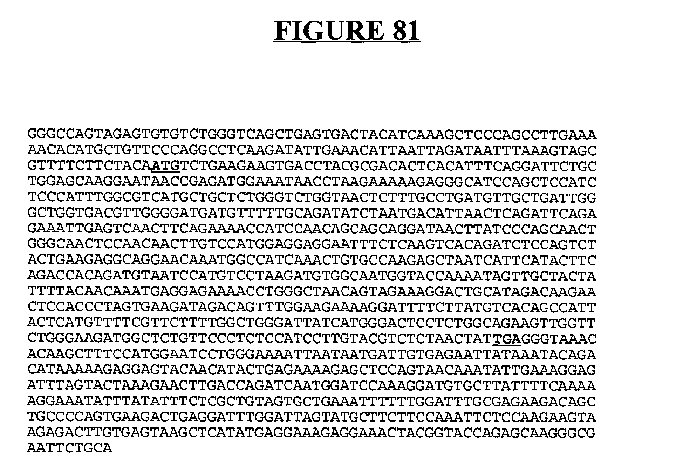

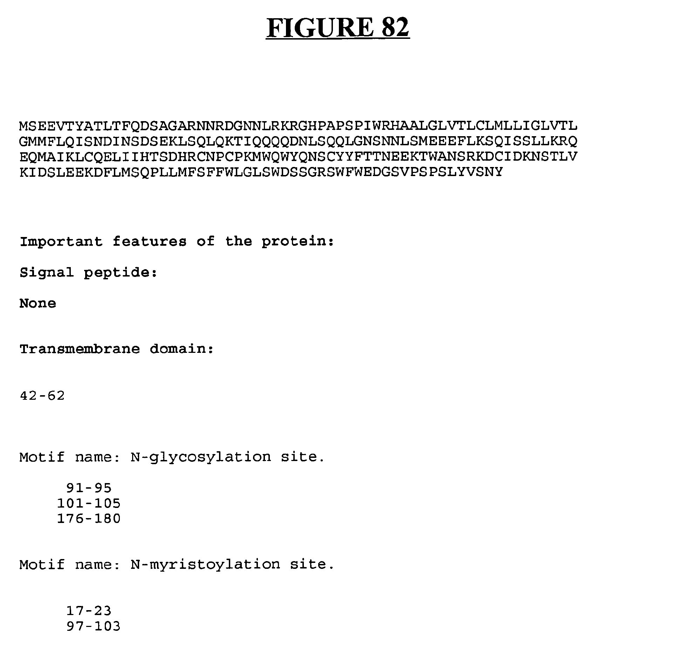

A full length clone was identified that contained a single open reading frame with an apparent translational initiation site at nucleotide positions 134-136 and a stop signal at nucleotide positions 830-832 (Figure 81, SEQ ID NO:81). The predicted polypeptide precursor is 232 amino acids long, has a calculated molecular weight of approximately 26754 daltons and an estimated pI of approximately 5.8. Analysis of the full-length PRO16089 sequence shown in Figure 82 (SEQ ID NO:82) evidences the presence of a variety of important polypeptide domains as shown in Figure 82, wherein the locations given for those important polypeptide domains are approximate as described above. Clone DNA150163-2842 has been deposited with ATCC on March 21, 2000 and is assigned ATCC deposit no. PTA-1533.

An analysis of the Dayhoff database (version 35.45 SwissProt 35), using the ALIGN-2 sequence alignment analysis of the full-length sequence shown in Figure 82 (SEQ ID NO:82), evidenced sequence identity between the PRO16089 amino acid sequence and the following Dayhoff sequences: P_Y02283, P_W40215, P_Y05317, P_Y31622, P_Y24151, AB010710_1, P_R99588, NKGD_HUMAN, P_W73889 and AB009597_1.

### EXAMPLE 41: Isolation of cDNA clones Encoding Human PRO19563 Polypeptides [UNQ5785]

DNA96861-2844 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence from the Incyte database, designated herein as 530612H1. This EST sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA79859.

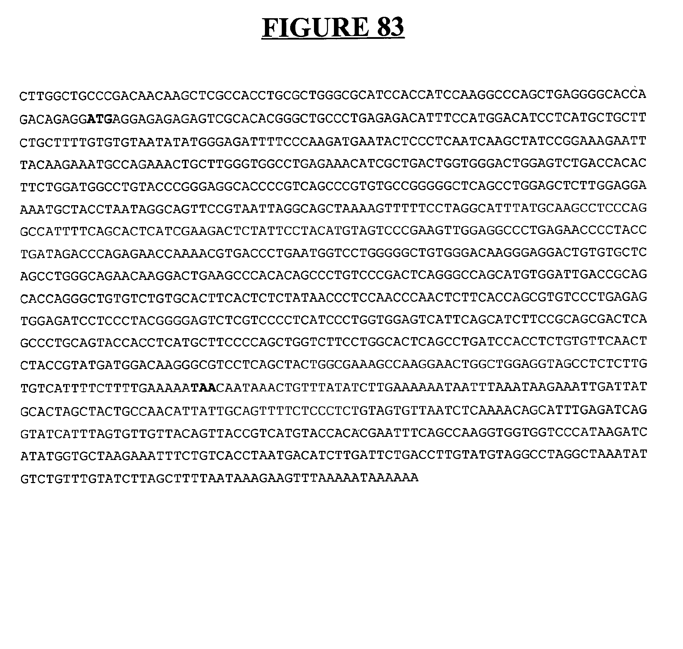

In light of an observed sequence homology between the DNA79859 sequence and an EST sequence encompassed within clone no. 530612H1 from the Incyte database, clone no. 530612H1 was purchased and the cDNA insert was obtained and sequenced. It was found herein that that cDNA insert encoded a full-length protein. The sequence of this cDNA insert is shown in Figure 83 and is herein designated as DNA96861-2844.

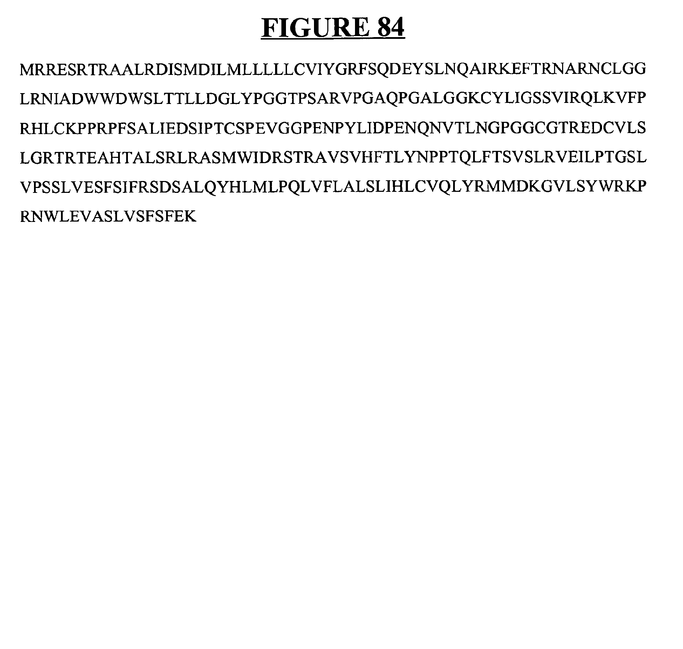

Clone DNA96861-2844 contains a single open reading frame with an apparent translational initiation site at nucleotide positions 83-85 and ending at the stop codon at nucleotide positions 983-985 (Figure 83; SEQ ID NO:83). The predicted polypeptide precursor is 300 amino acids long (Figure 84; SEQ ID NO:84). The full-length PRO19563 protein shown in Figure 84 has an estimated molecular weight of about 3364daltons and a pI of about 9.26. Analysis ofthe full-length PR019563 sequence shown in Figure 84 (SEQ ID NO:84) evidences the presence of a variety of important polypeptide domains as shown in Figure 84, wherein the locations given for those important polypeptide domains are approximate as described above. Clone DNA96861-2844 has been deposited with ATCC on March 2, 2000 and is assigned ATCC deposit no. PTA-1436.

An analysis of the Dayhoff database (version 35.45 SwissProt 35), using the ALIGN-2 sequence alignment analysis of the full-length sequence shown in Figure 84 (SEQ ID NO:84), evidenced sequence identity between the PRO19563 amino acid sequence and the following Dayhoff sequences: NM_000297_1.

### EXAMPLE 42: Isolation of cDNA clones Encoding Human PRO19675 Polypeptides [UNQ5835]

DNA 131658-2875 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence from the Incyte database, designated herein as 198190.2. This EST sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA128363.

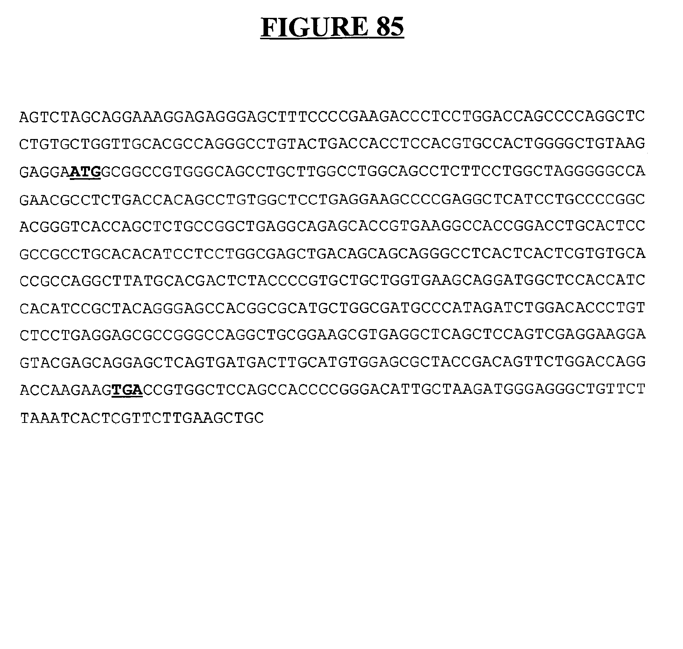

In light of an observed sequence homology between the DNA128363 sequence and an EST sequence encompassed within clone no. AI301403 from the Merck est database, clone no. AI301403 was purchased and the cDNA insert was obtained and sequenced. It was found herein that that cDNA insert encoded a full-length protein. The sequence of this cDNA insert is shown in Figure 85 and is herein designated as DNA131658-2875.

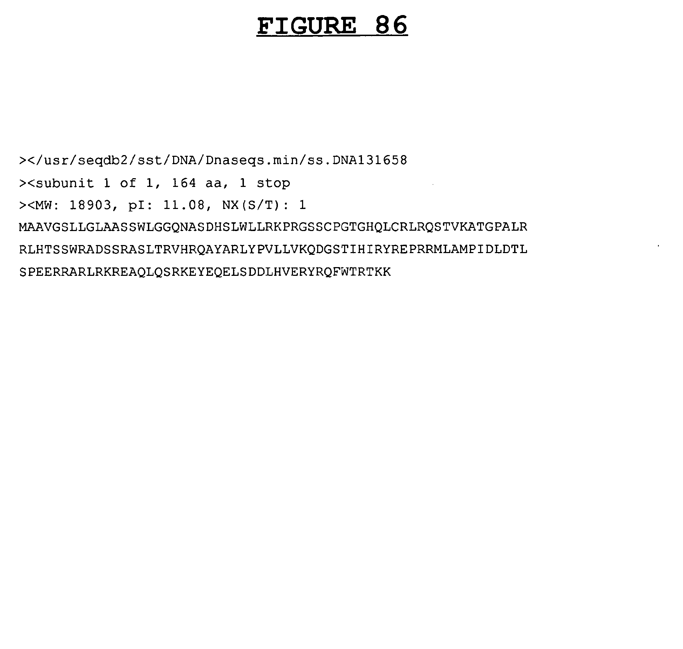

Clone DNA131658-2875 contains a single open reading frame with an apparent translational initiation site at nucleotide positions 128-130 and ending at the stop codon at nucleotide positions 620-622 (Figure 85; SEQ ID NO:85). The predicted polypeptide precursor is 164 amino acids long (Figure 86; SEQ ID NO:86). The full-length PRO19675 protein shown in Figure 86 has an estimated molecular weight of about 18,903 daltons and a pI of about 11.08. Analysis of the full-length PRO19675 sequence shown in Figure 86 (SEQ ID NO:86) evidences the presence of a variety of important polypeptide domains as shown in Figure 86, wherein the locations given for those important polypeptide domains are approximate as described above. Clone DNA131658-2875 has been deposited with ATCC on April 11, 2000 and is assigned ATCC deposit no. PTA-1671.

An analysis of the Dayhoff database (version 35.45 SwissProt 35), using the ALIGN-2 sequence alignment analysis of the full-length sequence shown in Figure 86 (SEQ ID NO:86), evidenced sequence identity between the PRO19675 amino acid sequence and the following Dayhoff sequences: T33928.

### EXAMPLE 43: Isolation of cDNA clones Encoding Human PRO20084 Polypeptides [UNQ6124]

A consensus sequence was obtained relative to a variety of EST sequences as described in Example 1 above, wherein the consensus sequence obtained is herein designated DNA167194. Two proprietary Genentech EST sequences were employed in the consensus assembly. Based on the DNA167194 consensus sequence, oligonucleotides were synthesized: 1) to identify by PCR a cDNA library that contained the sequence of interest, and 2) for use as probes to isolate a clone of the full-length coding sequence for PR020084.

A pair of PCR primers (forward and reverse) were synthesized:
forward PCR primer 5'-GCACCCTATCCCTGGACTGTAACTTACTGAA-3' (SEQ ID NO:177);
reverse PCR primer 5'-CAGCAATGAATATACCCATAAGGATCTCAAGC-3' (SEQ ID NO:178).
Additionally, a synthetic oligonucleotide hybridization probe was constructed from the consensus DNA167194 sequence which had the following nucleotide sequence:
hybridization probe
5'-ATGAGATGAAACCCTCAGAAGCCAGGGTCCCCCAGCTGAGC-3' (SEQ ID NO:179).

In order to screen several libraries for a source of a full-length clone, DNA from the libraries was screened by PCR amplification with the PCR primer pair identified above.

DNA sequencing of the clones isolated as described above gave the full-length DNA sequence for PR020084 [herein designated as UNQ6124 (DNA168061-2897)] (SEQ ID NO:87) and the derived protein sequence for PR020084.

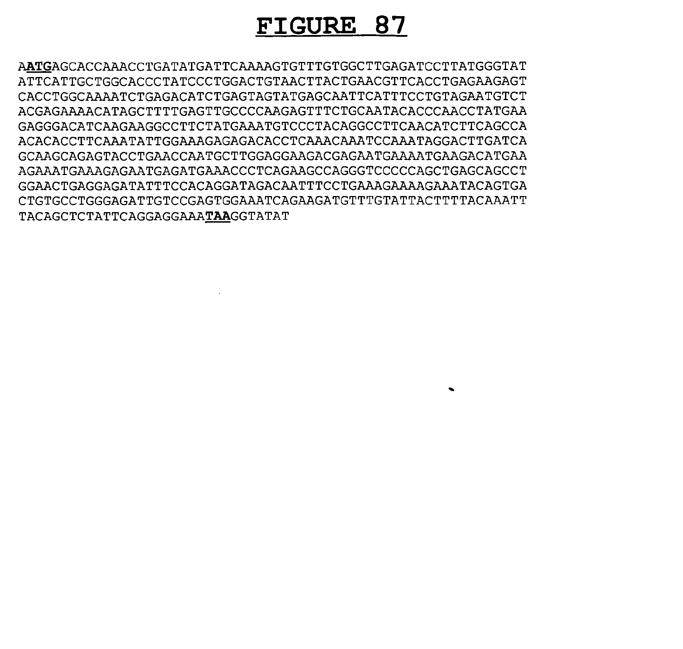

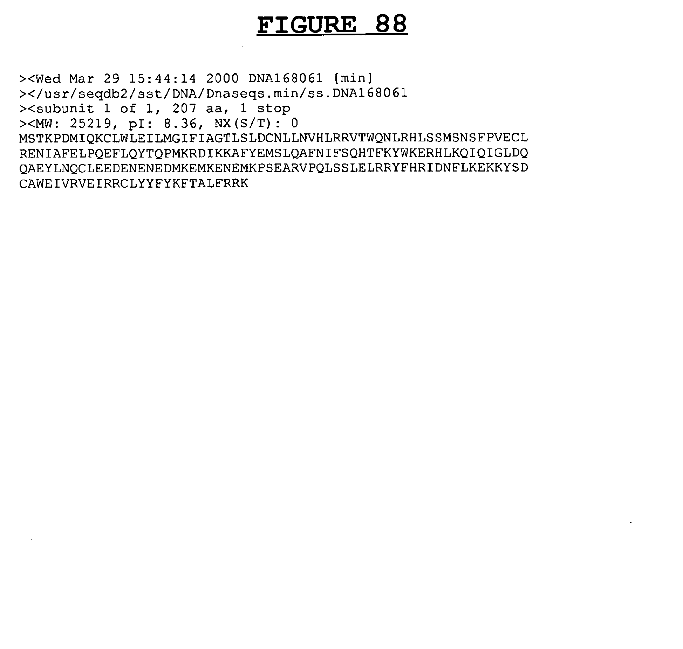

The entire nucleotide sequence ofUNQ6124 (DNA168061-2897) is shown in Figure 87 (SEQ ID NO:87). Clone UNQ6124 (DNA 168061-2897) contains a single open reading frame with an apparent translational initiation site at nucleotide positions 2-4 and ending at the stop codon at nucleotide positions 623-625 (Figure 87). The predicted polypeptide precursor is 207 amino acids long (Figure 88; SEQ ID NO:88). The full-length PR020084 protein shown in Figure 88 has an estimated molecular weight of about 25219 daltons and a pI of about 8.36. Clone UNQ6124 (DNA168061-2897) has been deposited with the ATCC on March 30, 2000 with ATCC deposit number PTA-1600. Regarding the sequence, it is understood that the deposited clone contains the correct sequence, and the sequences provided herein are based on known sequencing techniques.

Analysis of the amino acid sequence of the full-length PR020084 polypeptide using the Dayhoff database (version 35.45 SwissProt 35), using the ALIGN-2 sequence alignment analysis ofthe full-length sequence shown in Figure 88 (SEQ ID NO:88), evidenced sequence identity between the PR020084 amino acid sequence and the following Dayhoff sequence: INT1_BOVIN.

### EXAMPLE 44: Isolation of cDNA clones Encoding Human PRO21434 Polypeptides [UNQ6509]

DNA147253-2983 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence from the LIFESEQ® (Incyte Pharmaceuticals, Inc., Palo Alto, CA) database, designated herein as 221649.1. This EST sequence was then compared to a variety of expressed sequence tag (EST) databases which included public EST databases (e.g., GenBank) and proprietary EST DNA databases (Merck, Washington University; LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA130774.

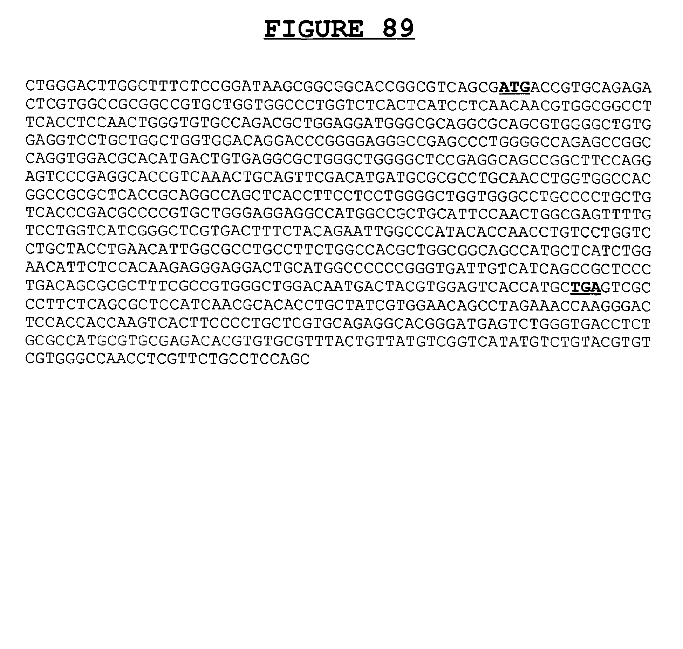

In light of an observed sequence homology between the DNA130774 sequence and an EST sequence encompassed within clone no. AI741157 from the Merck database, clone no. AI741157 was purchased from Merck and the cDNA insert was obtained and sequenced. It was found herein that that cDNA insert encoded a full-length protein. The sequence of this cDNA insert is shown in Figure 89 and is herein designated as DNA147253-2983.

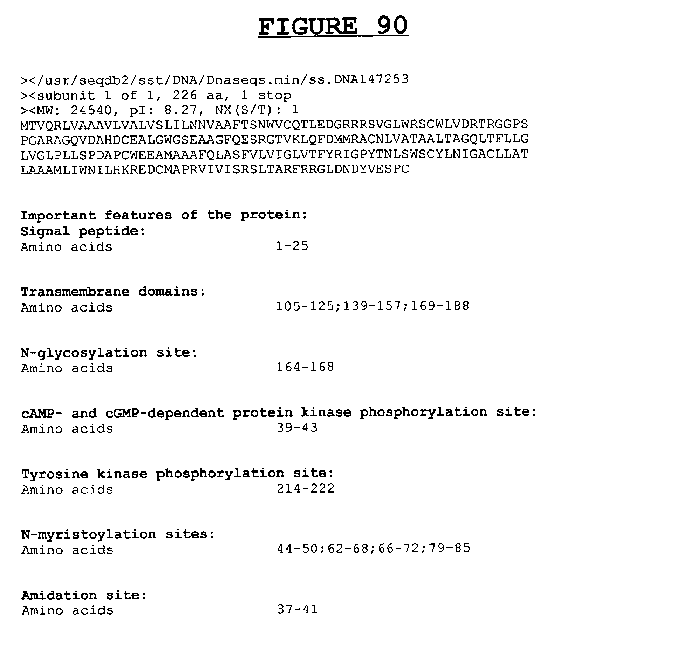

Clone DNA147253-2983 contains a single open reading frame with an apparent translational initiation site at nucleotide positions 47-49 and ending at the stop codon at nucleotide positions 725-727 (Figure 89; SEQ ID NO:89). The predicted polypeptide precursor is 226 amino acids long (Figure 90; SEQ ID NO:90). The full-length PR021434 protein shown in Figure 90 has an estimated molecular weight of about 24540 daltons and a pI of about 8.27. Analysis of the full-length PR021434 sequence shown in Figure 90 (SEQ ID NO:90) evidences the presence of a variety of important polypeptide domains as shown in Figure 90, wherein the locations given for those important polypeptide domains are approximate as described above. Clone DNA147253-2983 has been deposited with ATCC on August 22, 2000 and is assigned ATCC deposit no. PTA-2405.

An analysis of the Dayhoff database (version 35.45 SwissProt 35), using the ALIGN-2 sequence alignment analysis of the full-length sequence shown in Figure 90 (SEQ ID NO:90), evidenced no significant sequence identity between the PR021434 amino acid sequence and any Dayhoff sequences.

### EXAMPLE 45: Isolation of cDNA clones Encoding Human PRO346 Polypeptides [UNQ305]

A consensus DNA sequence was identified using phrap as described in Example 1 above. Specifically, this consensus sequence is herein designated DNA38240. Based on the DNA38240 consensus sequence, oligonucleotides were synthesized: 1) to identify by PCR a cDNA library that contained the sequence of interest, and 2) for use as probes to isolate a clone of the full-length PRO346 coding sequence.

RNA for construction of the cDNA libraries was isolated from human fetal liver. The cDNA libraries used to isolated the cDNA clones were constructed by standard methods using commercially available reagents (e.g., Invitrogen, San Diego, CA; Clontech, etc.) The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

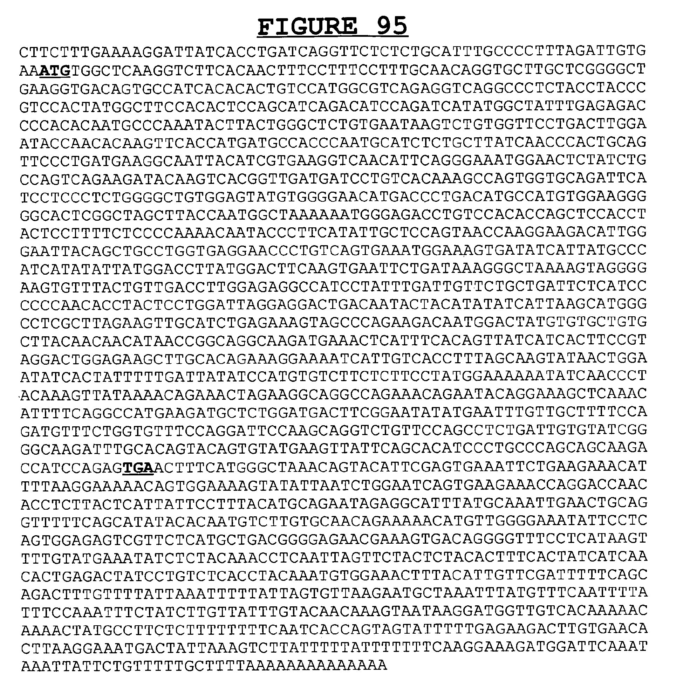

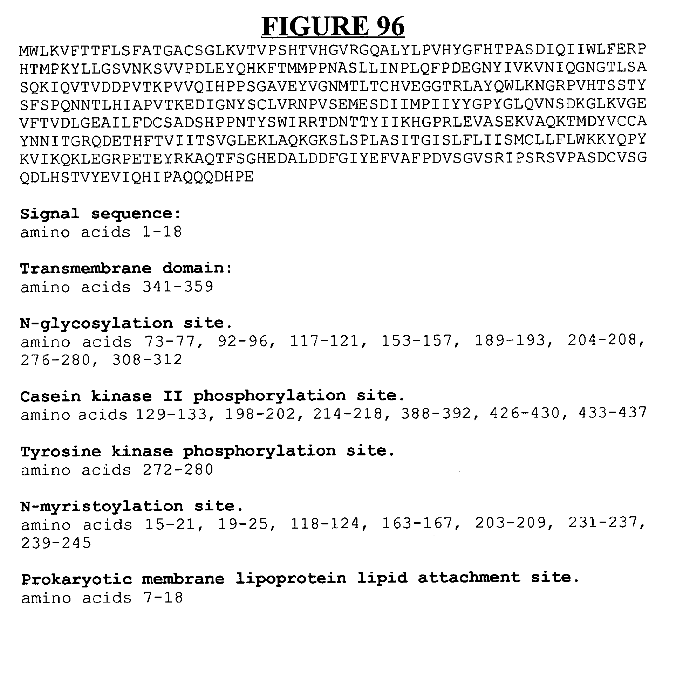

A cDNA clone was sequenced in entirety. The entire nucleotide sequence of DNA44167-1243 is shown in Figure 95 (SEQ ID NO:95). Clone DNA44167-1243 contains a single open reading frame with an apparent translational initiation site at nucleotide positions 64-66 (Fig. 95; SEQ ID NO:95). The predicted polypeptide precursor is 450 amino acids long (Fig. 96; SEQ ID NO:96). Clone DNA44167-1243 has been deposited with ATCC on November 7, 1997 and is assigned ATCC deposit no. ATCC 209434 (designation DNA44167-1243).

Based on a BLAST, BLAST-2 and FastA sequence alignment analysis (using the ALIGN computer program) of the full-length sequence, PR0346 shows amino acid sequence identity to carcinoembryonic antigen (28%).

The oligonucleotide sequences used in the above procedure were the following:
OLI2691 (38240.f1)
5'-GATCCTGTCACAAAGCCAGTGGTGC-3' (SEQ ID NO:180)
OLI2693 (38240.r1)
5'-CACTGACAGGGTTCCTCACCCAGG-3' (SEQ ID NO:181)
OLI2692 (38240.p1)
5'-CTCCCTCTGGGCTGTGGAGTATGTGGGGAACATGACCCTGACATG-3' (SEQ ID NO:182)

### EXAMPLE 46: Generation and Analysis of Mice Comprising PRO218, PRO228, PRO271, PRO273, PRO295, PRO302 PRO305 PRO326, PRO386, PRO655, PRO162, PRO788, PRO792, PRO940, PRO941, PRO1004, PRO1012 PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PR09904, PRO9907 PRO10013, PRO90948, PRO28694, PRO16089, PRO19563, PRO19675, PRO20084, PR021434, PRO50332, PRO38465 or PRO346 Gene Disruptions

To investigate the role of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides, disruptions in PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 genes were produced by homologous recombination or retroviral insertion techniques. Specifically, transgenic mice comprising disruptions in PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 genes (i.e., knockout mice) were created by either gene targeting or gene trapping. Mutations were confirmed by southern blot analysis to confirm correct targeting on both the 5' and 3' ends. Gene-specific genotyping was also performed by genomic PCR to confirm the loss of the endogenous native transcript as demonstrated by RT-PCR using primers that anneal to exons flanking the site of insertion. Targeting vectors were electroporated into 129 strain ES cells and targeted clones were identified. Targeted clones were microinjected into host blastocysts to produce chimeras. Chimeras were bred with C57 animals to produce F1 heterozygotes. Heterozygotes were intercrossed to produce F2 wild-type, heterozygote and homozygote cohorts which were used for phenotypic analysis. Rarely, if not enough F1 heterozygotes were produced, the F1 hets were bred to wild-type C57 mice to produce sufficient heterozygotes to breed for cohorts to be analyzed for a phenotype. All phenotypic analysis was performed from 12-16 weeks after birth.

### Overall Summary of Phenotypic Results:

### 46.1. Generation and Analysis of Mice Comprising DNA30867-1335 (UNQ192) Gene Disruptions

In these knockout experiments, the gene encoding PR0218 polypeptides (designated as DNA30867-1335) (UNQ192) was disrupted. The gene specific information for these studies is as follows: the mutated mouse gene corresponds to nucleotide reference: NM_026229 ACCESSION:NM_026229 NID: gi 21312913 ref NM_026229.1 Mus musculus RIKEN cDNA 4933412D19 gene (4933412D19Rik); protein reference: Q9D455 ACCESSION:Q9D455 NID: Mus musculus (Mouse). 4933412D19Rik protein (RIKEN cDNA 4933412D19 gene); the human gene sequence reference: NM_016334 Homo sapiens G protein-coupled receptor 89 (GPR89); the human protein sequence corresponds to reference: Q9Y302 ACCESSION:Q9Y302 NID: Homo sapiens (Human). CGI-13 protein (Putative G-protein coupled receptor) (Putative NFkB activating protein) (Putative MAPK activating protein).

The mouse gene of interest is Gpr89 (G protein-coupled receptor 89), ortholog of human GPR89. Aliases include 4933412D19Rik, SH120, and AL844549.1.

GPR89 is a putative integral plasma membrane protein that may function as a signal-transducing receptor. The protein contains nine transmembrane segments and appears to participate in nuclear factor-kappaB and mitogen-activated protein kinase signaling (Matsuda et al, Oncogene:22:3307-18 (2003); Lai et al, Genome Res. 10:703-13 (2000); Zhang et al, Genome Res.:10:1546-60 (2000)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 42 | 0 | 63 |
| Expected | 15.75 | 31.5 | 15.75 | 63 |

Chi-Sq.= 39.42 Significance= 2.7545848E-9 (hom/n)= 0.0 Avg. Litter Size= 8
Mutation Information
Mutation Type: Homologous Recombination (standard)
Description: Coding exons 2 and 3 were targeted (NCBI accession NM_026229.1).
WT Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.1.1. PHENOTYPIC ANALYSIS (for disrupted gene: DNA30867-1335 (UNQ192)

### (a) OVERALL PHENOTYPIC SUMMARY:

Mutation of the gene encoding the ortholog of human G protein-coupled receptor 89 (GPR89) resulted in lethality of (-/-) mutants. Gene disruption was confirmed by Southern blot.

### Discussion related to embryonic developmental abnormality of lethality:

Embryonic lethality in knockout mice usually results from various serious developmental problems including but not limited to neuro-degenerative diseases, angiogenic disorders, inflammatory diseases, or where the gene/protein has an important role in basic cell signaling processes in many cell types. In addition, embryonic lethals are useful as potential cancer models. Likewise, the corresponding heterozygous (+/-) mutant animals are particularly useful when they exhibit a phenotype and/or a pathology report which reveals highly informative clues as to the function of the knocked-out gene. For instance, EPO knockout animals were embryonic lethals, but the pathology reports on the embryos showed a profound lack ofRBCs.

### (b) Pathology

Microscopic: Due to embryonic lethality, microscopic analysis was not performed. At 12.5 days there were 42 embryos observed: 17 (+/-) embryos, 8 (+/+) embryos, and 17 resorption moles. Thus, lethality occurs after implantation.
Gene Expression: LacZ activity was not detected in the panel of tissues by immunohistochemical analysis.

### 46.2. Generation and Analysis of Mice Comprising DNA33092-1202 (UNQ202) Gene Disruptions

In these knockout experiments, the gene encoding PR0228 polypeptides (designated as DNA33092-1202) (UNQ202) was disrupted. The gene specific information for these studies is as follows: the mutated mouse gene corresponds to nucleotide reference: NM_133222 ACCESSION:NM_133222 NID: gi 18875377 ref NM_133222.1 Mus musculus ETL1 (ETL1); protein reference: Q923X1 ACCESSION:Q923X1 NID: Mus musculus (Mouse). ETL1; the human gene sequence reference: XM_371262 PREDICTED: Homo sapiens EGF, latrophilin and seven transmembrane domain containing 1 (ELTD1); the human protein sequence corresponds to reference: XP_371262 PREDICTED: EGF, latrophilin and seven transmembrane domain containing 1 [Homo sapiens].

The mouse gene of interest is Eltd1 (EGF, latrophilin seven transmembrane domain containing 1), ortholog ofhuman ELTD1 (EGF, latrophilin and seven transmembrane domain containing 1). Aliases include Etl, ETL1, and KPG_003.

ELTD 1 is a G-protein coupled receptor of the secretin family, containing a large extracellular N-terminal segment noncovalently linked to a seven-transmembrane segment. ELTD1 is expressed in cardiomyocytes, vascular smooth muscle cells, bronchiolar smooth muscle cells, kidney, brain, and liver. ELTD1 may play a role in development (Terskikh et al., Proc Natl Acad Sci U S A.:98:7934-9 (2001); Nechiporuk et al., J Biol Chem.:276:4150-7 (2001)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 33 | 13 | 64 |
| Expected | 16 | 32 | 16 | 64 |

Chi-Sq.= 0.83 Significance= 0.6603403 (hom/n)= 0.25 Avg. Litter Size= 8
Mutation Information
Mutation Type: Homologous Recombination (standard)
Description: Coding exons 4 through 6 were targeted (NCBI accession NM_133222.1).
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.2.1. PHENOTYPIC ANALYSIS (for disrupted gene: DNA33092-1202 (UNQ202)

### (a) OVERALL PHENOTYPIC SUMMARY:

Mutation of the gene encoding the ortholog of human EGF, latrophilin and seven transmembrane domain containing 1 (ELTD 1) resulted in the mutant (-/-) mice exhibiting decreased lean body mass, bone mineral content and bone mineral density measurements. The mutant (-/-) mice also exhibited an impaired glucose tolerance with increased serum glucose levels in both male and female (-/-) mice. Male infertility was also observed in the homozygous mice. Gene disruption was confirmed by Southern blot.

### (b) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

DEXA: The female (-/-) mice exhibited decreased mean lean body mass, bone mineral content and vertebrae bone mineral density measurements when compared with those of their gender-matched (+/+) littermates and the historical means.

Fertility: The male (-/-) mouse produced 3 pups after 40 days of breeding.

The (-/-) mice analyzed by DEXA exhibited decreased bone measurements and decreased body mass measurements when compared with their (+/+) littermates, suggestive of abnormal bone disorders. Thus, the (-/-) mice exhibited a negative bone phenotype. In addition, the decreased mean lean body mass is indicative of a metabolic disorder related to growth retardation and tissue wasting disorders. The negative bone phenotype indicates that PR0228 polypeptides or agonists thereof would be useful for maintaining bone homeostasis in addition to normal growth development. In addition, PR0228 polypeptides would be useful in bone healing or for the treatment of arthritis or osteoporosis, whereas antagonists (or inhibitors) of PR0228 polypeptides or its encoding gene would lead to abnormal or pathological bone disorders including inflammatory diseases associated with abnormal bone metabolism including arthritis, osteoporosis and osteopenia.

### (c) Blood Chemistry/Glucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of 2 wild type and 4 homozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Results:

Oral Glucose Tolerance: The (-/-) mice exhibited an impaired glucose tolerance when compared with that of their gender-matched (+/+) littermates and the historical mean. In line with this, increased blood glucose levels were seen in both male and female homozygous mice.

These studies indicated that (-/-) mice exhibit a decreased or impaired glucose tolerance in the presence of normal fasting glucose at all 3 intervals tested when compared with their gender-matched (+/+) littermates and the historical means. Thus, knockout mutant mice exhibited the phenotypic pattern of an impaired glucose homeostasis, and therefor PR0228 polypeptides (or agonists thereof) or its encoding gene would be useful in the treatment of conditions associated with an impaired glucose homeostasis and/or various cardiovascular diseases, including diabetes.

### 46.3. Generation and Analysis of Mice Comprising DNA39423-1182 (UNQ238) Gene Disruptions

In these knockout experiments, the gene encoding PR0271 polypeptides (designated as DNA39423-1182) (UNQ238) was disrupted. The gene specific information for these studies is as follows: the mutated mouse gene corresponds to nucleotide reference: NM_178255 Mus musculus hyaluronan and proteoglycan link protein 3 (Hapln3-pending); protein reference: Q80WM5 ACCESSION:Q80WM5 NID: Mus musculus (Mouse). Hyaluronan and proteoglycan link protein 3; the human gene sequence reference: NM_178232 Homo sapiens hyaluronan and proteoglycan link protein 3 (HAPLN3); the human protein sequence corresponds to reference: Q96S86 ACCESSION:Q96S86 NID: Homo sapiens (Human). PROTEOGLYCAN LINK PROTEIN.

The mouse gene of interest is Hapln3 (hyaluronan and proteoglycan link protein 3), ortholog of human HAPLN3. Aliases include Lpr3, 4930554N11Rik, and HsT19883.

HAPLN3 is a secreted protein that functions as a component of extracellular matrix. The protein contains a signal peptide, an immunoglobulin-like domain, and two link domains, which bind with hyaluronan. HAPLN3 may stabilize the interaction between chondroitin sulfate proteoglycans, such as versican (CSPG2), and hyaluronic acid of extracellular matrix (Spicer et al, J Biol Chem.:278:21083-91 (2003); Ogawa et al, Matrix Biol:23:287-98 (2004); Shi et al, J Biol Chem:279:12060-6 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 13 | 35 | 22 | 70 |
| Expected | 17.5 | 35 | 17.5 | 70 |

Chi-Sq.= 1.41 Significance= 0.4941086 (hom/n)= 0.26 Avg. Litter Size= 9
Mutation Information
Mutation Type: Homologous Recombination (standard)
Description: The gene consists of 5 exons, with the start codon located in exon 2 (NCBI accession NM_178255.3). Exons 2 and 3 were targeted.
WT Panel: Expression of the target gene was detected in embryonic stem (ES) cells and in all 13 adult tissue samples tested by RT-PCR, except skeletal muscle and adipose.
2. QC Expression: Disruption of the target gene was confirmed by Southern hybridization analysis.

### 46.3.1. PHENOTYPIC ANALYSIS (for disrupted gene: DNA39423-1182 (UNQ238)

### (a) OVERALL PHENOTYPIC SUMMARY:

Mutation of the gene encoding the ortholog of human hyaluronan and proteoglycan link protein 3 (HAPLN3) resulted in the mutant (-/-) mice exhibiting decreased bone-related measurements. GeneLogic analysis showed UNQ238 to be induced in activated T-cells and induced in activated NK cells and dendritic cells. Gene disruption was confirmed by Southern blot.

### (b) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The female (-/-) mice exhibited decreased bone mineral content (BMC) and total body and vertebrae bone mineral density (BMD) when compared with those of their gender-matched (+/+) littermates and the historical means.
micro CT: The male (-/-) mice exhibited a decreased mean femoral mid-shaft cross-sectional area when compared with that of their gender-matched (+/+) littermates and the historical mean.

The (-/-) mice analyzed by DEXA and bone micro CT analysis exhibited decreased bone measurements when compared with their (+/+) littermates, suggestive of abnormal bone disorders. Thus, the (-/-) mice exhibited a negative bone phenotype. The negative bone phenotype indicates that PR0271 polypeptides or agonists thereof would be useful for maintaining bone homeostasis. In addition, PR0271 polypeptides would be useful in bone healing or for the treatment of arthritis or osteoporosis, whereas antagonists (or inhibitors) of PRO271 polypeptides or its encoding gene would lead to abnormal or pathological bone disorders including inflammatory diseases associated with abnormal bone metabolism including arthritis, osteoporosis and osteopenia.

### (c) GeneLogic Analysis

GeneLogic analysis revealed that UNQ238 is induced in activated T cells. In addition, UNQ238 is induced in activated NK cells and dendritic cells.

### 46.4. Generation and Analysis of Mice Comprising DNA39523-1192 (UNQ240) Gene Disruptions

In these knockout experiments, the gene encoding PR0273 polypeptides (designated as DNA39523-1192) (UNQ240) was disrupted. The gene specific information for these studies is as follows: the mutated mouse gene corresponds to nucleotide reference: NM_019568 Mus musculus chemokine (C-X-C motif) ligand 14 (Cxc114); protein reference: Q9WUQ5 ACCESSION:Q9WUQ5 NID: Mus musculus (Mouse). Small inducible cytokine B14 precursor (CXCL14) (Chemokine BRAK) (Kidney-expressed chemokine CXC); the human gene sequence reference: NM_004887 Homo sapiens chemokine (C-X-C motif) ligand 14 (CXCL14); the human protein sequence corresponds to reference: Q9BTR1 ACCESSION:Q9BTR1 NID: Homo sapiens (Human). SMALL INDUCIBLE CYTOKINE SUBFAMILY B (CYS-X-CYS), MEMBER 14 (BRAK).

The mouse gene of interest is Cxc114 (chemokine (C-X-C motif) ligand 14), ortholog of human CXCL 14. Aliases include kidney-expressed chemokine CXC; musculus CXC chemokine MIP-2gamma; MGI:1888514; 1110031 L23Rik; 1200006I23Rik; BMAC; BRAK; KS1; Kec; MIP-2g; NJAC; Scyb14; bolekine; CXC chemokine in breast and kidney; small inducible cytokine B 14; small inducible cytokine subfamily B (Cys-X-Cys), member 14 (BRAK); HGNC:10640; and MGC10687.

CXCL14 is a secreted protein (cytokine) that likely functions as a signal-transducing ligand (Hromas et al, Biochem Biophys Res Commun: 255:703-6 (1999)). Although its cognate receptor is not known, CXCL14 binds specifically with B cells and macrophages as well as some B cell lines and monocyte cell lines (Sleeman et al, Int Immunol:12:677-89(2000)). Expression of CXCL14 appears to be ubiquitous but is often down regulated in various cancers (Frederick et al, Am J Pathol.: 156:1937-50 (2000)). CXCL14 potently inhibits angiogenesis in response to many different factors, including interleukin 8, basic fibroblast growth factor, and vascular endothelial growth factor. Thus, loss of CXCL14 expression in tumors may permit neovascularization (Shellenberger et al, Cancer Res.: 64:8262-70 (2004)). CXCL14 alone induces chemotaxis in neutrophils (Cao et al, J Immunol.:165:2588-95 (2000)), and CXCL14 in combination with prostaglandin E2 (PGE2) induces chemotaxis in monocytes (Kurth et al, J Exp Med: 194:855-61 (2001)). CXCL14 may play a role in macrophage recruitment and development (Kurth et al, J Exp Med: 194:855-61 (2001); Cao et at, J Immunol.:165:2588-95 (2000)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 20 | 28 | 12 | 60 |
| Expected | 15 | 30 | 15 | 60 |

Chi-Sq.= 0.87 Significance= 0.64726466 (hom/n)= 0.24 Avg. Litter Size= 7
Mutation Information
Mutation Type: Homologous Recombination (standard)
Description: Coding exons 1 through 3 were targeted (NCBI accession NM_019568.1).
WT Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.4.1. PHENOTYPIC ANALYSIS (for disrupted gene: DNA39523-1192 (UNQ240)

### (a) OVERALL PHENOTYPIC SUMMARY:

Mutation of the gene encoding the ortholog of human chemokine (C-X-C motif) ligand 14 (CXCL14) resulted in small male (-/-) mice. Male homozygous mutant mice were smaller than their gender-matched wild-type littermates, exhibiting decreased mean body weight and length, total tissue mass, lean body mass, bone mineral content (BMC), and vertebrae bone mineral density (BMD). The male (-/-) mice also showed decreased microCT bone density measurements. In addition, the mutant (-/-) demonstrated increased anxiety with an increased stress-induced hyperthermia response. The (-/-) mice also showed increased mean serum IgA levels. The homozygotes showed moderate kidney nephrosis. Nitrites were present in the urine of both homozygotes and heterozygotes but not in the wildtype littermate controls. Disruption of the target gene was confirmed by Southern hybridization analysis.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Serum Immunoglobulin Isotyping Assay:

The Serum Immunoglobulin Isotyping Assay is performed using a Cytometric Bead Array (CBA) kit. This assay is used to rapidly identify the heavy and light chain isotypes of a mouse monoclonal antibody in a single sample. The values expressed are "relative fluorescence units" and are based on the detection ofkappa light chains. Any value < 6 is not significant.

### Results:

Serum Imm. 2: The (-/-) mice exhibited increased mean serum IgA levels when compared with those of their (+/+) littermates and the (+/+) mice within the project run.

Mutant (-/-) mice exhibited increased mean serum IgA immunoglobulins compared to their gender-matched (+/+) littermates. IgA mainly functions as an epithelial cell protector which can neutralize bacterial toxins and viruses. Although no obvious disease susceptibility is associated with selective IgA defects, they are commoner in people with chronic lung disease than in the general population. This suggests that lack of IgA may result in a predisposition to lung infections with various pathogens and is consistent with the role of IgA in defense at the body surfaces. In this case, the phenotype observed for knockout mice resulted in an increase in IgA serum levels suggesting that inhibitors (antagonists) of PR0273 polypeptides would mimic these immunological effects.

The observed phenotype suggests that PR0273 polypeptides function as a negative regulator of IgA. These immunological abnormalities suggest that antagonists (inhibitors) of PR0273 polypeptides would be important agents which would stimulate the immune system.

### (c) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The male (-/-) mice exhibited decreased mean body weight when compared with that of their gender-matched (+/+) littermates and the historical mean, the difference being more notable in the males.
Length: The male (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean total tissue mass, lean body mass, bone mineral content and vertebrae bone mineral density measurements when compared with those of their gender-matched (+/+) littermates and the historical mean.
micro CT: The male (-/-) mice exhibited notably decreased mean vertebral trabecular bone volume, number, thickness, and connectivity density and decreased mean femoral mid-shaft cortical thickness and cross-sectional area when compared with those of their gender-matched (+/+) littermates and the historical means.

Mutant (-/-) mice deficient in the gene encoding PR0273 polypeptides show a phenotype consistent with growth retardation, marked by decreased body weight and length and decreased total tissue mass and lean body mass. Thus, antagonists or inhibitors of PR0273 polypeptides or its encoding gene would mimic these metabolic and growth related effects. On the other hand, PR0273 polypeptides or agonists thereof would be useful in the prevention and/or treatment of such metabolic disorders as diabetes or other tissue wasting diseases.

In addition, the (-/-) mice analyzed by DEXA and micro CT exhibited decreased bone measurements and decreased body mass measurements when compared with their (+/+) littermates, suggestive of abnormal bone disorders. Thus, the (-/-) mice exhibited a negative bone phenotype. In addition, the decreased mean total tissue mass and lean body mass is indicative of a metabolic disorder related to growth retardation and tissue wasting disorders. The negative bone phenotype indicates that PR0273 polypeptides or agonists thereof would be useful for maintaining bone homeostasis in addition to normal growth development. In addition, PR0273 polypeptides would be useful in bone healing or for the treatment of arthritis or osteoporosis, whereas antagonists (or inhibitors) of PR0273 polypeptides or its encoding gene would lead to abnormal or pathological bone disorders including inflammatory diseases associated with abnormal bone metabolism including arthritis, osteoporosis and osteopenia.

### (d) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Functional Observational Battery (FOB) Test - Stress-induced Hyperthermia:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Results:

Stress-Induced Hyperthermia: The (-/-) mice exhibited an increased sensitivity to stress-induced hyperthermia when compared with that of their gender-matched (+/+) littermates and the historical mean, suggesting an increased anxiety-like response in the mutants.

In summary, the functional observation testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PR0273 polypeptides or agonists thereof would be useful in the treatment of such neurological disorders.

### (e) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; Nitrites; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: Nitrites were observed in 1 of 4 heterozygotes (+/-) and 5 of 8 homozygotes (-/-) whereas no nitrites were present in the wildtype (+/+) littermates. These findings are consistent with histology observations which showed moderate kidney hydronephrosis in the mutant (-/-) mice.

### 46.5. Generation and Analysis of Mice Comprising DNA38268-1188 (UNQ258) Gene Disruptions

In these knockout experiments, the gene encoding PR0295 polypeptides (designated as DNA38268-1188) (UNQ258) was disrupted. The gene specific information for these studies is as follows: the mutated mouse gene corresponds to nucleotide reference: NM_015814 Mus musculus dickkopf homolog 3 (Xenopus laevis) (Dkk3); protein reference: Q9QUN9 ACCESSION:Q9QUN9 NID: Mus musculus (Mouse). Dickkopf related protein-3 precursor (Dkk-3) (Dickkopf-3) (mDkk-3); the human gene sequence reference: NM_013253 Homo sapiens dickkopf homolog 3 (Xenopus laevis) (DKK3); the human protein sequence corresponds to reference: Q9UBP4 ACCESSION:Q9UBP4 NID: Homo sapiens (Human). Dickkopf related protein-3 precursor (Dkk-3) (Dickkopf-3) (hDkk-3).

The mouse gene of interest is Dkk3 (dickkopfhomolog 3 [Xenopus laevis]), ortholog of human DKK3. Aliases include REIC, RIG-like 5-6, RIG-like 7-1, dickkopf (Xenopus laevis) homolog 3, and dickkopfhomolog 3.

DKK3 is a secreted protein belonging to the dickkopf (DKK) family of morphogens. DKK family members generally inhibit Wnt/beta-catenin signaling by binding with LRP, a coreceptor of Wnt receptor Frizzled. Association of DKKs with LRP antagonizes interaction of Wnt with Frizzled, inhibiting downstream signaling. DKKs can also bind with LRP and Kremen, an integral plasma membrane protein, forming a complex. This complex is internalized, lowering the concentration ofLRP on the cell surface and inhibiting Wnt signaling. Unlike DKK1, DKK2, and DKK4, DKK3 does not appear to interact with LRP or Kremen (Logan and Nusse, Annu Rev Cell Dev Biol.:20:781-810 (2004); Mao and Niehrs, Gene:302:179-83 (2003) but does appear to inhibit canonical Wnt signaling in some systems (Hoang et al, Cancer Res.:64:2734-9 (2004)). DKK3 may play a role in processes such as development, differentiation, tissue repair, and cancer (Logan and Nusse, Annu Rev Cell Dev Biol.:20:781-810 (2004); Beachy et al, Nature:432:324-31 (2004); Suwa et al, J Endocrinol.:178:149-58 (2003); Hsieh et al, Oncogene: 23:9183-9 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 38 | 21 | 77 |
| Expected | 19.25 | 38.5 | 19.25 | 77 |

Chi-Sq.= 1.3 Significance= 0.5220458 (hom/n)= 0.23 Avg. Litter Size= 8
Mutation Information
Mutation Type: Homologous Recombination (standard)
Description: Coding exon 1 was targeted (NCBI accession NM_015814.2).
WT Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.5.1. PHENOTYPIC ANALYSIS (for disrupted gene: DNA38268-1188 (UNQ258)

### (a) OVERALL PHENOTYPIC SUMMARY:

Mutation of the gene encoding the ortholog of human dickkopf homolog 3 (DKK3) resulted in the (-/-) mice exhibiting increased body fat and decreased bone mineral content and bone mineral density measurements. Gene disruption was confirmed by Southern blot.

### (b) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

DEXA: The female (-/-) mice exhibited increased mean total fat mass and increased percent total body fat as well as decreased mean bone mineral content (BMC) and bone mineral density (BMD) measurements when compared with those of their gender-matched (+/+) littermates and the historical means.

These studies suggest that mutant (-/-) non-human transgenic animals exhibit a negative phenotype that would be associated with obesity. Thus, PR0295 polypeptides or agonists thereof are essential for normal growth and metabolic processes and especially would be important in the prevention and/or treatment of obesity.

In addition, the (-/-) mice analyzed by DEXA analysis also exhibited decreased bone measurements when
compared with their (+/+) littermates, suggestive of abnormal bone disorders. The negative bone phenotype indicates that PR0295 polypeptides or agonists thereof would be useful for maintaining bone homeostasis. In addition, PR0295 polypeptides would be useful in bone healing or for the treatment of arthritis or osteoporosis, whereas antagonists (or inhibitors) of PR0295polypeptides or its encoding gene would lead to abnormal or pathological bone disorders including inflammatory diseases associated with abnormal bone metabolism including arthritis, osteoporosis and osteopenia.

### 46.6. Generation and Analysis of Mice Comprising DNA40370-1217 (UNQ265) Gene Disruptions

In these knockout experiments, the gene encoding PR0302 polypeptides (designated as DNA40370-1217) (UNQ265) was disrupted. The gene specific information for these studies is as follows: the mutated mouse gene corresponds to nucleotide reference: NM_029023 Mus musculus serine carboxypeptidase 1 (Scpep1); protein reference: Q920A5 ACCESSION:Q920A5 NID: Mus musculus (Mouse). RETINOID-INDUCIBLE SERINE CARBOXYPEPTIDASE PRECURSOR; the human gene sequence reference: NM_021626 ACCESSION:NM_021626 NID: gi 11055991 refNM_021626.1 Homo sapiens likely homolog of rat and mouse retinoid-inducible serine carboxypeptidase (RISC); the human protein sequence corresponds to reference: Q9HB40 ACCESSION:Q9HB40 NID: Homo sapiens (Human). SERINE CARBOXYPEPTIDASE 1 PRECURSOR PROTEIN (CDNA FLJ14467 FIS, CLONE MAMMA1000672, WEAKLY SIMILAR TO VITELLOGENIC CARBOXYPEPTIDASE PRECURSOR) (EC 3.4.16.-).

The mouse gene of interest is Scpep1 (serine carboxypeptidase 1), ortholog of human SCPEP1. Aliases include Risc, HSCP1, 2410018F01Rik, and 4833411K15Rik.

SCPEP1 is a secreted protein that likely functions as a serine carboxypeptidase, consisting of a signal peptide and a serine carboxypeptidase domain. The protein is expressed in aorta, bladder, and kidney and is localized primarily to medial smooth muscle or, in kidney, proximal convoluted tubule. Expression of SCPEP1 is induced in smooth muscle cells treated with retinoic acid, which inhibits smooth muscle cell proliferation and neointimal formation. SCPEP1 may be involved in vascular wall homeostasis and kidney function (Chen et al, J Biol Chem: 276:34175-81 (2001)).

Targeted or gene trap mutations are generated in strain 129SvEv^{Brd}-derived embryonic stem (ES) cells. The chimeric mice are bred to C57BL/6J albino mice to generate F1 heterozygous /animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 16 | 35 | 19 | 70 |
| Expected | 17.5 | 35 | 17.5 | 70 |

Chi-Sq.= 1.2 Significance= 0.5488116 (hom/n)= 0.25 Avg. Litter Size= 8
Mutation Information
Mutation Type: Homologous Recombination (standard)
Description: The gene consists of 13 exons, with the start codon located in exon 1 (NCBI accession NM_029023.2). Exons 1 and 2 were targeted.
1. WT Panel: Expression of the target gene was detected in embryonic stem (ES) cells and in all 13 adult tissue samples tested by RT-PCR, except skeletal muscle, bone, and adipose.
2. QC Expression: Disruption of the target gene was confirmed by Southern hybridization analysis.

### 46.6.1. PHENOTYPIC ANALYSIS (for disrupted gene: DNA40370-1217 (UNQ265)

### (a) OVERALL PHENOTYPIC SUMMARY:

Mutation of the gene encoding the ortholog of human serine carboxypeptidase 1 (SCPEP1) resulted in the female (-/-) mice showing decreased body length. GeneLogic studies showed UNQ265 to be expressed in myeloid B cells. Gene disruption was confirmed by Southern blot.

### (b) Bone Metabolism & Body Diagnostics

### Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection ofAvertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

Results: Female (-/-) mice exhibited decreased mean body length compared to their gender-matched (+/+) littermates. These results are consistent with growth related disorders.

### 46.7. Generation and Analysis of Mice Comprising DNA40619-1220 (UNQ268) Gene Disruptions

In these knockout experiments, the gene encoding PR0305 polypeptides (designated as DNA40619-1220) (UNQ268) was disrupted. The gene specific information for these studies is as follows: the mutated mouse gene corresponds to nucleotide reference: NM_009984 Mus musculus cathepsin L (Ctsl); protein reference: P06797 ACCESSION:P06797 NID: Mus musculus (Mouse). Cathepsin L precursor (EC 3.4.22.15) (Major excreted protein) (MEP); the human gene sequence reference: NM_001912 Homo sapiens cathepsin L (CTSL), transcript variant 1; the human protein sequence corresponds to reference:P07711 ACCESSION:P07711 NID: Homo sapiens (Human). Cathepsin L precursor (EC 3.4.22.15) (Major excreted protein) (MEP).

The mouse gene of interest is Ctsl (cathepsin L), ortholog of human CTSL. Aliases include fs, MEP, major excreted protein, nkt, 1190035F06Rik, and CATL.

CTSL is a cysteine protease that catalyzes the proteolysis of proteins in lysosomes. The protease contains a signal peptide, a propeptide domain, a cathepsin L heavy chain domain, a second propeptide domain, and a cathepsin L light chain domain. CTSL is synthesized as an inactive proenzyme. Upon activation, the mature peptide forms a heterodimer, with heavy and light chains linked by disulfide bonds (Coulombe et al, EMBO J: 15:5492-503 (1996)). Although 90% of CTSL is lysosomal, about 10% of the zymogen is secreted and activated, catalyzing the hydrolysis of extracellular matrix, prohormones, or other proteases (Stypmann et al, Proc Natl Acad Sci U S A: 99:6234-9 (2002)). CTSL is likely involved in processing IL-8 at sites of inflammation (Ohashi et al, Biochim Biophys Acta: 1649:30-9 (2003)), in degrading extracellular matrix for migration of antigen-presenting cells (Fiebiger et al, J Exp Med: 196:1263-9 (2002)), in generating MHC-bound peptides for antigen presentation (Honey et al, Nat Immunol: 3:1069-74 (2002), and in forming endostatin for inhibition of tumor angiogenesis (Felbor et al, EMBO J: 19:1187-94 2000)).

Several investigators have studied the physiological role of CTSL using knockout mice or mice with a naturally occurring CTSL mutation. Roth and colleagues [FASEB J: 14:2075-86 (2000)], Tobin and colleagues [Am J Pathol: 160:1807-21 (2002)], and Benavides and colleagues [Am J Pathol: 161:693 -703 (2002)] showed that disruption of the CTSL gene results in periodic hair loss due to defective hair follicle morphogenesis and cycling. The homozygous null mice also displayed several other skin-associated phenotypes, including epidermal hyperplasia, hair follicle canal dilatation, acanthosis, and hyperkeratosis. These investigators concluded that CTSL is essential for epidermal homeostasis and normal hair follicle function. Benavides and colleagues [Immunogenetics: 53:233-42 (2001)] showed that CD4 T cells in the thymus and peripheral lymphoid tissues were markedly lower in CTSL (-/-) mice than in wild-type mice. They concluded that CTSL may play a critical role in CD4 T cell selection in the thymus. Honey and coworkers [Nat Immunol: 3:1069-74(2002)] showed that the numbers of some natural killer T cell subsets were greatly reduced in CTSL (-/-) mice than in wild-type mice. They concluded that CTSL plays an important role in natural killer cell selection and major histocompatibility complex-mediated antigen presentation. Stypmann and colleagues [Proc Natl Acad Sci U S A: 99:6234-9 (2002)] reported that CTSL-deficient mice displayed significant ventricular and atrial enlargement, interstitial fibrosis, severely impaired myocardial contraction, and valvular defects and insufficiencies. They concluded that CTSL is critical for cardiac morphology and function. Nishimura and coworkers [Am J Pathol: 161:2047-52 (2002)] reported that CTSL-deficient mice displayed enlarged gingivae, which is frequently seen in patients treated with calcium channel antagonists for hypertension. They concluded that CTSL may play a role in skin and gingival abnormalities and proposed that reduced CTSL activity may be a factor in drug-induced gingival overgrowth. Potts and colleagues [Int J Exp Pathol: 85:85-96 (2004)] observed that trabecular bone volume but not cortical bone volume was significantly lower in CTSL (/-) and CTSL (-/-) mice than in wild-type mice. In contrast, trabecular bone loss in response to ovariectomy was significantly lower in CTSL (/-) and CTSL (-/-) mice than in wild-type mice. They concluded that CTSL may play a role in regulating bone turnover in normal development and in pathological states.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 22 | 33 | 24 | 79 |
| Expected | 19.75 | 39.5 | 19.75 | 79 |

Chi-Sq.= 0.34 Significance= 0.8436648 (hom/n)= 0.24 Avg. Litter Size= 8
Mutation Information
Mutation Type: Homologous Recombination (standard)
Description: Coding exons 1 through 6 were targeted (NCBI accession NM_009984.2).
1. WT Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.7.1. PHENOTYPIC ANALYSIS (for disrupted gene: DNA40619-1220 (UNQ268)

### (a) OVERALL PHENOTYPIC SUMMARY:

Mutation of the gene encoding the ortholog of human cathepsin L (CTSL) resulted in hyperplasia of the epidermis and sebaceous glands and granulocytopoiesis in the bone marrow of (-/-) mice. Microscopic analysis revealed epidermal and sebaceous gland hyperplasia and granulocytopoiesis in the bone marrow of the mutants. The homozygous mutant mice exhibited a thick oily coat and inflamed skin. By 12 weeks of age, the mutants exhibited excessive grooming around the eyes and face, resulting in the euthanization of those with skin lesions. In addition, the homozygous mutant mice exhibited a decreased mean heart rate and numerous immunological abnormalities when compared with those of their wild-type littermates and the historical means. The (-/-) mutant mice also exhibited decreased body fat and decreased bone mineral density measurements. Disruption of the target gene was confirmed by Southern hybridization analysis.

### (b) Pathology

Microscopic: The 6 (-/-) mice analyzed exhibited diffuse moderate-to-marked hyperplasia of sebaceous glands and multifocal hyperplasia of the epidermis (acanthosis and hyperkeratosis). The mutant (-/-) mice also exhibited focal mild to moderate dermatitis. Increased extramedullary hematopoeisis was observed in the mutant homozygous mice in the liver and spleen as well as myeloid hyperplasia of the bone marrow. Anagenic (growing) hair follicles were present in all sections of skin taken from the mutant mice, frequently involving more than 50% of the skin surface compared to less than 10% in their (+/+) littermates. Of the 6 (-/-) mice examined, 2 exhibited areas of serocellular crust formation overlying an inflammatory dermatitis on the facial skin. In affected areas, the dermis contained infiltrating inflammatory cells such as lymphocytes, mast cells, and neutrophils.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACSCalibur flow cytometer with CellQuest software.

### Results:

FACS3: The (-/-) mice exhibited an altered distribution of leukocyte subsets in peripheral blood, characterized by a decreased mean percentage of CD4 cells (SP thymocytes). Decreased percentages of CD4 cells in the periphery resulted in an increased percentage of B cells in lymph organs in the (-/-) mice. The CD4 cells also exhibited a more activated/memory phenotype (CD62Llow, CD44hi) when compared with those of their (+/+) littermates and the historical mean. Thus, the (-/-) mice showed a developmental defect in CD4+ cells. The (-/-) mice showed an impairment in CD4 T cell-dependent functions as observed in the lack of ova response (shown below). Thus, knocking out the gene which encodes PR0305 polypeptides causes a decrease in the T cell population. From these observations, PR0305 polypeptides or the gene encoding PR0305 appear to act as a regulator ofT cell proliferation. Thus, PR0305 polypeptides would be beneficial in enhancing T cell proliferation.

### Ovalbumin Challenge

Procedure: This assay was carried out on 7 wild types and 8 homozygotes. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immunodominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG 1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice failed to induce any ova-specific Ig titers when compared with that of their (+/+) littermates and the historical mean.

In summary, the ovalbumin challenge studies indicate that knockout mice deficient in the gene encoding PR0305 polypeptides exhibit immunological abnormalities when compared with their wild-type littermates. In particular, the mutant mice exhibited a decreased ability to elicit an immunological response when challenged with the T-cell dependent OVA antigen. Thus, PR0305 polypeptides or agonists thereof, would be useful for stimulating the immune system (such as T cell proliferation) and would find utility in the cases wherein this effect would be beneficial to the individual such as in the case of leukemia, and other types of cancer, and in immuno-compromised patients, such as AIDS sufferers. Accordingly, inhibitors (antagonists) of PR0305 polypeptides would be useful for inhibiting the immune response and thus would be useful candidates for suppressing harmful immune responses, e.g. in the case of graft rejection or graft-versus-host diseases.

### (d) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

Obvious: The (-/-) mice exhibited thick, oily coats and inflamed skin. The oiliness of the mutants' coats decreased with age. However, the (-/-) mice began to exhibit excessive grooming around the eyes and face by 12 weeks of age, and those with skin lesions were euthanized.
DEXA: The (-/-) mice exhibited decreased mean total fat mass, percent total body fat, and volumetric bone mineral density when compared with those of their gender-matched (+/+) littermates and the historical means.

Mutant (-/-) mice deficient in the gene encoding PRO305 polypeptides show a phenotype consistent with growth retardation, marked by decreased total body fat (%) and fat mass (g). Thus, antagonists or inhibitors of PR0305 polypeptides or its encoding gene would mimic these metabolic and growth related effects. On the other hand, PR0305 polypeptides or agonists thereof would be useful in the prevention and/or treatment of such metabolic disorders.

In addition, the (-/-) mice analyzed by DEXA exhibited decreased bone measurements in addition to the decreased body fat mass measurements when compared with their (+/+) littermates, suggestive of abnormal bone disorders. Thus, the (-/-) mice exhibited a negative bone phenotype. The negative bone phenotype indicates that PR0305 polypeptides or agonists thereof would be useful for maintaining bone homeostasis in addition to normal growth development. In addition, PR0305 polypeptides would be useful in bone healing or for the treatment of arthritis or osteoporosis, whereas antagonists (or inhibitors) of PR0305 polypeptides or its encoding gene would lead to abnormal or pathological bone disorders including inflammatory diseases associated with abnormal bone metabolism including arthritis, osteoporosis and osteopenia.

### (e) Cardiology - Heart Rate

### Description:

Heart rate is measured via a noninvasive tail-cuff method for four days on the Visitech BP-2000 Blood Pressure Analysis System. Heart rate is measured ten times each day for four days. The four days are then averaged to obtain a mouse's conscious heart rate.
Heart Rate: The (-/-) mice exhibited a decreased mean heart rate (1 standard deviation below the mean for both male and female (-/-) mice) when compared with that of their (+/+) littermates and the historical mean.

### 46.8. Generation and Analysis of Mice Comprising DNA37140-1234 (UNQ287) Gene Disruptions

In these knockout experiments, the gene encoding PR0326 polypeptides (designated as DNA37140-1234) (UNQ287) was disrupted. The gene specific information for these studies is as follows: the mutated mouse gene corresponds to nucleotide reference: NM_177152 Mus musculus leucine-rich repeats and immunoglobulin-like domains 3 (Lrig3); protein reference: Q6P1C6 ACCESSION:Q6P 1 C6 NID: Mus musculus (Mouse). Leucine-rich and immunoglobulin-like domains 3; the human gene sequence reference:NM_153377 Homo sapiens leucine-rich repeats and immunoglobulin-like domains 3 (LRIG3); the human protein sequence corresponds to reference: Q6UXM1 ACCESSION:Q6UXM1 NID: Homo sapiens (Human). SAPS287.

The mouse gene of interest is Lrig3 (leucine-rich repeats and immunoglobulin-like domains 3), ortholog of human LRIG3. Aliases include mKIAA3016, 9030421L11Rik, 9130004I02Rik, 9430095K15Rik, FLJ90440, and KIAA3016.

LRIG3 is a type I integral plasma membrane protein, containing a signal peptide, 15 tandem leucine-rich repeats flanked by cysteine-rich segments, 3 immunoglobulin-like domains, a transmembrane segment, and a short cytoplasmic tail. LRIG3 is a paralog of LRIG1 (Guo et al, Genomics: 84:157-65 (2004)), which functions as a negative regulator of receptor tyrosine kinase signaling. LRIG1 forms a complex with ErbB receptor family members and stimulates ErbB receptor ubiquitination and degradation (Laederich et al, J Biol Chem:279(45):47050-6 (2004)). LRIG3 expression is readily detected in a wide variety of tissues but is highest in stomach (Guo et al, Genomics: 84:157-65 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 28 | 39 | 26 | 93 |
| Expected | 23.25 | 46.5 | 23.25 | 93 |

Chi-Sq.= 1.06 Significance= 0.588605 (hom/n)= 0.28 Avg. Litter Size= 9
Mutation Information
Mutation Type: Homologous Recombination (standard)
Description: The gene consists of 19 exons, with the start codon located in exon 1 (NCBI accession NM_177152.4). Exon 1 was targeted.
1. Wild-type Expression Panel: Expression of the target gene was detected in embryonic stem (ES) cells and in all 13 adult tissue samples tested by RT-PCR, except bone and adipose.
2. QC Expression: Disruption of the target gene was confirmed by Southern hybridization analysis.

### 46.8.1. PHENOTYPIC ANALYSIS (for disrupted gene: DNA37140-1234 (UNQ287)

### (a) OVERALL PHENOTYPIC SUMMARY:

Mutation of the gene encoding the ortholog of human leucine-rich repeats and immunoglobulin-like domains 3 (LRIG3) resulted in impaired sensorimotor gating/attention in the (-/-) mice. The mutant (-/-) mice also exhibited decreased body weight and length. The mutant (-/-) mice exhibited numerous immunological abnormalities. Disruption of the target gene was confirmed by Southern hybridization analysis.

### (b) Bone Metabolism & Body Diagnostics

### Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The (-/-) mice exhibited decreased mean body weight when compared with that of their gender-matched (+/+) littermates and the historical mean.
Length: The (-/-) mice exhibited decreased mean body length when compared with that of their gender-matched (+/+) littermates and the historical mean.

Mutant (-/-) mice deficient in the gene encoding PR0326 polypeptides show a phenotype consistent with growth retardation, marked by decreased body weight and length. Thus, antagonists or inhibitors of PR0326 polypeptides or its encoding gene would mimic these metabolic and growth related effects. On the other hand, PR0326 polypeptides or agonists thereof would be useful in the prevention and/or treatment of such metabolic disorders as diabetes or other tissue wasting diseases.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Prepulse inhibition of the acoustic startle reflex

Prepulse inhibition of the acoustic startle reflex occurs when a loud 120 decibel (dB) startle-inducing tone is preceded by a softer (prepulse) tone. The PPI paradigm consists of six different trial types (70 dB background noise, 120 dB alone, 74dB + 120 dB - pp4, 78 dB + 120 dB - pp8, 82 dB + 120 dB - pp12, and 90 dB+ 120 dB - pp20) each repeated in pseudo random order six times for a total of 36 trials. The max response to the stimulus (V max) is averaged for each trial type. Animals with a 120 dB average value equal to or below 100 are excluded from analysis. The percent that the prepulse inhibits the animal's response to the startle stimulus is calculated and graphed.

### Results:

PPI: The (-/-) mice exhibited a decreased startle response during pp12 and pp20 when compared with those of their (+/+) littermates and the historical means, suggesting impaired sensorimotor gating/attention in the mutants.

### (d) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. HelperT cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Acute Phase Response:

Test Description: Bacterial lipopolysaccharide (LPS) is an endotoxin, and as such is a potent inducer of an acute phase response and systemic inflammation. The Level I LPS mice were injected intraperitoneally (i.p.) with a sublethal dose of LPS in 200 µL sterile saline using a 26 gauge needle. The doses were based on the average weight of the mice tested at 1 µg/g body weight 3 hours after injection; a 100ul blood sample was then taken and analyzed for the presence of TNFa, MCP-1, and IL-6 on the FACS Calibur instrument.

### Results:

Acute Phase Response: The (-/-) mice exhibited an increased MCP-1 response to LPS challenge when compared with that of their (+/+) littermates and the historical mean.

In summary, the LPS endotoxin challenge demonstrated that knockout mice deficient in the gene encoding PR0326 polypeptides exhibit immunological abnormalities when compared with their wild-type littermates. In particular, the mutant mice exhibited an increased ability to elicit an immunological response (MCP-1 production) when challenged with the LPS endotoxin indicating a proinflammatory response. MCP-1 plays a critical role in inducing the acute phase response and systemic inflammation. This suggests that inhibitors or antagonists to PR0326 polypeptides would stimulate the immune system and would find utility in the cases wherein this effect would be beneficial to the individual such as in the case of leukemia, and other types of cancer, and in immuno-compromised patients, such as AIDS sufferers. Accordingly, PR0326 polypeptides or agonists thereof would be useful in inhibiting the immune response and would be useful candidates for suppressing harmful immune responses, e.g. in the case of graft rejection or graft-versus-host diseases.

### Flourescence-activated cell-sorting (FACS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACSCalibur flow cytometer with CellQuest software.

### Results:

The mutant (-/-) mice exhibited a decreased percentage of CD4 cells and an increased percentage of B cells in the blood. A similar trend was observed in the tissues (decreased CD4 cells and increased B cells).

Thus, PR0326 polypeptides or agonists thereof appear to act as a negative regulator of B cell formation and maturation with an opposite effect on the T cell population.

### 46.9. Generation and Analysis of Mice Comprising DNA45415-1318 (UNQ326) Gene Disruptions

In these knockout experiments, the gene encoding PR0386 polypeptides (designated as DNA45415-1318) (UNQ326) was disrupted. The gene specific information for these studies is as follows: the mutated mouse gene corresponds to nucleotide reference: XM_134787 PREDICTED: Mus musculus similar to Sodium channel beta-2 subunit precursor (LOC214238); protein reference: XP_134787 similar to Sodium channel beta-2 subunit precursor [Mus musculus]; the human gene sequence reference: NM_004588 ACCESSION:NM_004588 NID:4759065 Homo sapiens Homo sapiens sodium channel, voltage-gated, type II, beta polypeptide (SCN2B); the human protein sequence corresponds to reference: 060939 ACCESSION:060939 NID: Homo sapiens (Human). SODIUM CHANNEL BETA-2 SUBUNIT PRECURSOR.

The mouse gene of interest is LOC214238 (similar to Sodium channel beta-2 subunit precursor), ortholog of human SCN2B (sodium channel, voltage-gated, type II, beta).

SCN2B is a type I integral plasma membrane protein that likely functions as a regulatory subunit of voltage-gated sodium channels. The 33-kDa glycoprotein consists of a signal peptide, an extracellular immunoglobulin-like domain, a transmembrane segment, and an intracellular C-terminal domain. SCN2B may play a role in assembly, expression, and modulation of heterotrimeric sodium channel complexes (Isom et al, Cell: 83:433-42 (1995); Jones et al, Genomics: 34:258-9 (1996); Eubanks et al, Neuroreport: 8:2775-9 (1997); Bolino et al, Eur J Hum Genet: 6:629-34 (1998); Haug et al, Neuroreport: 11:2687-9 (2000)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 24 | 33 | 18 | 75 |
| Expected | 18.75 | 37.5 | 18.75 | 75 |

Chi-Sq.= 0.04 Significance= 0.9801987 (hom/n)= 0.25 Avg. Litter Size= 9
Mutation Information
Mutation Type: Homologous Recombination (standard)
Description: Coding exons 2 through 4 were targeted (NCBI accession XM_134787.2).
1. Wild-type Expression Panel: Expression of the target gene was detected in embryonic stem (ES) cells and in all 13 adult tissue samples tested by RT-PCR, except skeletal muscle and bone.
2. QC Expression: Disruption of the target gene was confirmed by Southern hybridization analysis.

### 46.9.1. PHENOTYPIC ANALYSIS (for disrupted gene: DNA45415-1318 (UNQ326)

### (a) OVERALL PHENOTYPIC SUMMARY:

Mutation of the gene encoding the ortholog of human sodium channel, voltage-gated, type II, beta (SCN2B), resulted in an impaired glucose tolerance in male (-/-) mice. In addition, female homozygous mice showed a decreased skin fibroblast proliferation rate and a decrease in body weight and length. Gene disruption was confirmed by Southern blot.

### (b) Blood Chemistry/Glucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of 2 wild type and 4 homozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Results:

Oral Glucose Tolerance: The male (-/-) mice exhibited an impaired glucose tolerance when compared with that of their gender-matched (+/+) littermates and the historical mean (no available data for the female (-/-) mice).

These studies indicated that (-/-) mice exhibit a decreased or impaired glucose tolerance in the presence of normal fasting glucose at all 3 intervals tested when compared with their gender-matched (+/+) littermates and the historical means. Thus, knockout mutant mice exhibited the phenotypic pattern of an impaired glucose homeostasis, and therefor PR0386 polypeptides (or agonists thereof) or its encoding gene would be useful in the treatment of conditions associated with an impaired glucose homeostasis and/or various cardiovascular diseases, including diabetes.

### (c) Adult skin cell proliferation:

Procedure: Skin cells were isolated from 16 week old animals (2 wild type and 4 homozygotes). These were developed into primary fibroblast cultures and the fibroblast proliferation rates were measured in a strictly controlled protocol. The ability of this assay to detect hyper-proliferative and hypo-proliferative phenotypes has been demonstrated with p53 and Ku80. Proliferation was measured using Brdu incorporation.

Specifically, in these studies the skin fibroblast proliferation assay was used. An increase in the number of cells in a standardized culture was used as a measure of relative proliferative capacity. Primary fibroblasts were established from skin biopsies taken from wild type and mutant mice. Duplicate or triplicate cultures of 0.05 million cells were plated and allowed to grow for six days. At the end of the culture period, the number of cells present in the culture was determined using a electronic particle counter.

### Results:

Skin Proliferation: The female (-/-) mice exhibited a decreased mean skin fibroblast proliferation rate when compared with that of their gender-matched (+/+) littermates and the historical mean.

Thus, homozygous mutant mice demonstrated a hypo-proliferative phenotype. As suggested by these observations, antagonists or inhibitors of PR0386 polypeptides would mimic this hypo-proliferative phenotype and could function as tumor suppressors and would be useful in decreasing abnormal cell proliferation.

### (d) Bone Metabolism & Body Diagnostics

### Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The female (-/-) mice exhibited decreased mean body weight and length when compared with that of their gender-matched (+/+) littermates and the historical mean.

Mutant (-/-) mice deficient in the gene encoding PR0386 polypeptides show a phenotype consistent with growth retardation, marked by decreased body weight and length. Thus, antagonists or inhibitors of PR0386 polypeptides or its encoding gene would mimic these metabolic and growth related effects. On the other hand, PR0386 polypeptides or agonists thereof would be useful in the prevention and/or treatment of such metabolic disorders as diabetes or other tissue wasting diseases.

### 46.10. Generation and Analysis of Mice Comprising DNA50960-1224 (UNQ360) Gene Disruptions

In these knockout experiments, the gene encoding PR0655 polypeptides (designated as DNA50960-1224) (UNQ360) was disrupted. The gene specific information for these studies is as follows: the mutated mouse gene corresponds to nucleotide reference: NM_177348 Mus musculus interferon epsilon 1 (Ifne1); protein reference: Q80ZF2 ACCESSION:Q80ZF2 NID: Mus musculus (Mouse). Interferon epsilon-1; the human gene sequence reference: NM_176891 Homo sapiens interferon epsilon 1 (IFNE1); the human protein sequence corresponds to reference: Q86WN2 ACCESSION:Q86WN2 NID: Homo sapiens (Human). Interferon epsilon-1 (Interferon-epsilon).

The mouse gene of interest is Ifne1 (interferon epsilon 1), ortholog of human IFNE1. Aliases include Ifntl, Infel, Ifn-tau-1, and PR0655.

IFNE1 is a putative secreted protein that belongs to the type I interferon family. The protein contains a signal peptide and an interferon alpha, beta, and delta (IFabd) domain. Interferons generally produce antiviral and antiproliferative responses in cells (InterPro accession IPR000471). IFNE1 is expressed primarily in ovaries and uterus, suggesting that the protein plays a role in reproduction and host defense (Hardy et al, Genomics: 84:331-45 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 25 | 26 | 19 | 70 |
| Expected | 17.5 | 35 | 17.75 | 70 |

Chi-Sq.= 4.79 Significance= 0.09117268 (hom/n)= 0.23 Avg. Litter Size= 9
Mutation Information
Mutation Type: Homologous Recombination (standard)
Description: The gene consists of 1 exon (NCBI accession NM_177348.2). Exon 1 was targeted.
1. Wild-type Expression Panel: FPPA
   WT Panel: Expression of the target gene was detected in embryonic stem (ES) cells and in all 13 adult tissue samples tested by RT-PCR, except spleen, liver, skeletal muscle, bone, and heart.
2. QC Expression: Disruption of the target gene was confirmed by Southern hybridization analysis.

### 46.10.1. PHENOTYPIC ANALYSIS (for disrupted gene: DNA50960-1224 (UNQ360)

### (a) OVERALL PHENOTYPIC SUMMARY:

Mutation of the gene encoding the ortholog of human interferon epsilon 1 (IFNE1) resulted in immunological abnormalities in (-/-) mice. The homozygous mutant mice exhibited immunological abnormalities when compared with their wild-type littermates and the historical means, including an increased MCP-1 response to LPS challenge and an increased mean serum IgG2a response to ovalbumin challenge. The (-/-) mice also exhibited decreased mean body weight and length as well as decreased total tissue mass. The male (-/-) mice exhibited decreased trabecular bone volume, number and connectivity density as well as increased mean serum alkaline phosphatase levels. Disruption of the target gene was confirmed by Southern hybridization analysis.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis.of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Ovalbumin Challenge

Procedure: This assay was carried out on 7 wild types and 8 homozygotes. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immunodominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG 1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice exhibited an increased mean serum IgG2a response to ovalbumin challenge when compared with that of their (+/+) littermates and the historical mean.

In summary, the ovalbumin challenge studies indicate that knockout homozygous mice deficient in the gene encoding PR0655 polypeptides exhibit immunological abnormalities when compared with their wild-type littermates. In particular, the mutant (-/-) mice exhibited an increased ability to elicit an immunological response when challenged with the T-cell dependent OVA antigen. Thus, antagonists (inhibitors) of PR0655 polypeptides would be useful for stimulating the immune system (such as T cell proliferation) and would find utility in the cases wherein this effect would be beneficial to the individual such as in the case of leukemia, and other types of cancer, and in immuno-compromised patients, such as AIDS sufferers. Accordingly, PR0655 polypeptides or agonists thereof, would be useful for inhibiting the immune response and thus would be useful candidates for suppressing harmful immune responses, e.g. in the case of graft rejection or graft-versus-host diseases.

### Acute Phase Response:

Test Description: Bacterial lipopolysaccharide (LPS) is an endotoxin, and as such is a potent inducer of an acute phase response and systemic inflammation. The Level I LPS mice were injected intraperitoneally (i.p.) with a sublethal dose of LPS in 200 µL sterile saline using a 26 gauge needle. The doses were based on the average weight of the mice tested at 1 µg/g body weight 3 hours after injection; a 100ul blood sample was then taken and analyzed for the presence of TNFa, MCP-1, and IL-6 on the FACS Calibur instrument.

### Results:

Acute Phase Response: The (-/-) mice exhibited an increased MCP-1 response to LPS challenge when compared with that of their (+/+) littermates and the historical mean.

In summary, the LPS endotoxin challenge demonstrated that knockout mice deficient in the gene encoding PR0655 polypeptides exhibit immunological abnormalities when compared with their wild-type littermates. In particular, the mutant mice exhibited an increased ability to elicit an immunological response (MCP-1 production) when challenged with the LPS endotoxin indicating a proinflammatory response. MCP-1 plays a critical role in inducing the acute phase response and systemic inflammation. This suggests that inhibitors or antagonists to PR0655 polypeptides would stimulate the immune system and would find utility in the cases wherein this effect would be beneficial to the individual such as in the case of leukemia, and other types of cancer, and in immuno-compromised patients, such as AIDS sufferers. Accordingly, PR0655 polypeptides or agonists thereof would be useful in inhibiting the immune response and would be useful candidates for suppressing harmful immune responses, e.g. in the case of graft rejection or graft-versus-host diseases.

### Flourescence-activated cell-sorting (FACS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample oflysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACSCalibur flow cytometer with CellQuest software.

### Results:

Tissue Specific FACS: The (-/-) mice exhibited a decreased CD23 intensity in the spleen when compared with their (+/+) littermates. The (-/-) mice also exhibited increased mean percentages of B220 Med/CD23- cells and B220/CD11b- Low/CD23- cells in peritoneal lavage.

These observations indicate that there is a change of B cell subtypes in peritoneal lavage. Also, a decrease in CD23 in the spleen was observed. Thus, it appears that PR0655 polypeptides acts as a regulator for B cell production.

### (c) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: The male (-/-) mice exhibited an increased mean serum alkaline phosphatase level when compared with those of their gender-matched (+/+) littermates and the historical means. These results are consistent with decreased microCT bone related measurements (shown below).

### (d) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The (-/-) mice exhibited decreased mean body weight when compared with that of their gender-matched (+/+) littermates and the historical mean.
Length: The (-/-) mice exhibited decreased mean body length when compared with that of their gender-matched (+/+) littermates and the historical mean.

Mutant (-/-) mice deficient in the gene encoding PR0655 polypeptides show a phenotype consistent with growth retardation, marked by decreased body weight and length. Thus, antagonists or inhibitors of PR0655 polypeptides or its encoding gene would mimic these metabolic and growth related effects. On the other hand, PRO655 polypeptides or agonists thereof would be useful in the prevention and/or treatment of such metabolic disorders as diabetes or other tissue wasting diseases.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The female (-/-) mutant mice exhibited decreased mean total tissue mass when compared with that of their gender-matched (+/+) littermates and the historical means.
MicroCT: The (-/-) mice exhibited decreased trabecular bone volume, number, and connectivity density compared to their littermate controls (+/+ mice).

In summary, the (-/-) mice analyzed by DEXA and microCT exhibited decreased bone measurements and decreased body tissue mass measurements when compared with their (+/+) littermates, suggestive of abnormal bone disorders. Thus, the (-/-) mice exhibited a negative bone phenotype. In addition, the decreased mean total tissue mass is indicative of a metabolic disorder related to growth retardation and tissue wasting disorders. The negative bone phenotype indicates that PR0655 polypeptides or agonists thereof would be useful for maintaining bone homeostasis in addition to normal growth development. In addition, PR0655 polypeptides would be useful in bone healing or for the treatment of arthritis or osteoporosis, whereas antagonists (or inhibitors) of PR0655 polypeptides or its encoding gene would lead to abnormal or pathological bone disorders including inflammatory diseases associated with abnormal bone metabolism including arthritis, osteoporosis and osteopenia.

### 46.11. Generation and Analysis of Mice Comprising DNA56965-1356 (UNQ429) Gene Disruptions

In these knockout experiments, the gene encoding PRO162 polypeptides (designated as DNA56965-1356) (UNQ429) was disrupted. The gene specific information for these studies is as follows: the mutated mouse gene corresponds to nucleotide reference: NM_011036 Mus musculus pancreatitis-associated protein (Pap); protein reference: P35230ACCESSION:P35230NID: Mus musculus (Mouse). Pancreatitis-associated protein 1 precursor (REG III-beta); the human gene sequence reference: NM_138938 Homo sapiens pancreatitis-associated protein (PAP), transcript variant 2; the human protein sequence corresponds to reference: Q06141 ACCESSION:Q06141 NID: Homo sapiens (Human). Pancreatitis-associated protein 1 precursor.

The mouse gene of interest is Pap (pancreatitis-associated protein), ortholog of human REG3A (regenerating islet-derived 3 alpha). Aliases include HIP, PAP, PAP1, INGAP, REG3, RegIII (beta), Reg3b, REG-III, PAP-H, PBCGF, pancreatitis-associated protein, PAP homologous protein, hepatocarcinoma-intestine-pancreas, and pancreatic beta cell growth factor.

REG3A is a secreted protein that may function as a signal-transducing ligand, a defensive immune protein, an extracellular matrix component, or a cell adhesion molecule. The protein contains a signal peptide and a C-type lectin domain, which generally functions as calcium-dependent carbohydrate-binding module (SMART accession SM00034). REG3A is expressed by pancreatic acinar cells (Orelle et al, J Clin Invest: 90:2284-91 (1992); Christa et al, Am J Physiol: 271:G993-1002 (1996)), hepatic ductular cells (Simon et al, FASEB J: 17:1441-50 (2003)), intestinal neuroendocrine and Paneth cells (Christa et al, Am J Physiol: 271:G993-1002 (1996), and tumoral hepatocytes (Christa et al, Am J Physiol: 271:G993-1002 (1996)). Moreover, REG3A is upregulated in patients with pancreatitis (Orelle et al, J Clin Invest: 90:2284-91 (1992)). REG3A may be involved in proliferation of duct cells in the pancreas and liver (Rafaeloff et al, J Clin Invest: 99:2100-9 (1997); Simon et al, FASEB J: 17:1441-50 (2003); Christa et al, Am J Physiol: 271:G993-1002 (1996), in adhesion of hepatocytes (Christa et al, Am J Physiol: 271:G993-1002 (1996), in formation of extracellular matrix (Graf et al, J Biol Chem: 276:21028-38 (2001)), and in inhibition of inflammation (Vasseur et al, J Biol Chem: 279:7199-207 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 15 | 35 | 15 | 65 |
| Expected | 16.25 | 32.5 | 16.25 | 65 |

Chi-Sq.= 1.04 Significance= 0.59452057 (hom/n)= 0.23 Avg. Litter Size= 8
Mutation Information
Mutation Type: Homologous Recombination (standard)
Description: The gene consists of 6 exons, with the start codon located in exon 2 (NCBI accessionNM_011036.1). Exons 2 through 6 were targeted.
1. Wild-type Expression Panel: Expression of the target gene was detected in embryonic stem (ES) cells and in brain; spinal cord; eye; thymus; stomach, small intestine, and colon; and adipose among 13 adult tissue samples tested by RT-PCR.
2. QC Expression: Disruption of the target gene was confirmed by Southern hybridization analysis.

### 46.11.1. PHENOTYPIC ANALYSIS (for disrupted gene: DNA56965-1356 (UNQ429)

### (a) OVERALL PHENOTYPIC SUMMARY:

Mutation of the gene encoding the ortholog of human regenerating islet-derived 3 alpha (REG3A) resulted in an impaired glucose tolerance in male (-/-) mice. Gene disruption was confirmed by Southern blot.

### (b) Phenotypic Analysis: Metabolism -Blood Chemistry/Glucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of 2 wild type and 4 homozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Results:

### Blood Glucose Levels/Glucose Tolerance Test:

The male (-/-) mice exhibited an impaired glucose tolerance when compared with their gender-matched (+/+) littermates and the historical means. The (-/-) mice also exhibited an increased mean serum glucose level.

These studies indicated that homozygous (-/-) mice exhibit a decreased or impaired glucose tolerance in the presence of normal fasting glucose at all 3 intervals tested when compared with their gender-matched (+/+) littermates and the historical means. Thus, knockout mutant mice exhibited the phenotypic pattern of an impaired glucose homeostasis, and therefor PRO162 polypeptides (or agonists thereof) or its encoding gene would be useful in the treatment of conditions associated with an impaired glucose homeostasis and/or various cardiovascular diseases, including diabetes.

### 46.12. Generation and Analysis of Mice Comprising DNA56405-1357 (UNQ430) Gene Disruptions

In these knockout experiments, the gene encoding PR0788 polypeptides (designated as DNA56405-1357) (UNQ430) was disrupted. The gene specific information for these studies is as follows: the mutated mouse gene corresponds to nucleotide reference: AK002226 Mus musculus adult male kidney cDNA, RIKEN full-length enriched library, clone:0610005K03 product:hypothetical CD59 antigen containing protein, full insert sequence; protein reference: Q9DD23 ACCESSION:Q9DD23 NID: Mus musculus (Mouse). 0610005K03Rik protein; the human gene sequence reference: NM_205545 Homo sapiens LY6/PLAUR domain containing 2 (LYPDC2); the human protein sequence corresponds to reference: Q6UXB3 ACCESSION:Q6UXB3 NID: Homo sapiens (Human).

The mouse gene of interest is Lypdc2 (Ly6/Plaur domain containing 2), ortholog of human LYPDC2. Aliases include 0610005K03Rik, UNQ430, and RGTR430.

LYPDC2 is a putative secreted protein (Clark et al, Genome Res: 13:2265-70 (2003)), containing a signal peptide and an Ly-6 antigen/uPA receptor-like (LU) domain (SMART accession SM00134). Proteins with LU domains typically belong to the LU superfamily of receptor and secreted proteins, which participate in signal transduction, immune cell activation, or cellular adhesion. LYPDC2 is structurally similar to SLURP1 (secreted LY6/PLAUR domain containing 1). SLURP 1 is a secreted protein that functions as an epidermal modulator of alpha-7 nicotinic acetylcholine receptor in keratinocytes. SLURP1 plays a role in immune function, epidermal homeostasis, and wound healing (Chimienti et al, Hum Mol Genet: 12:3017-24 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 43 | 24 | 88 |
| Expected | 22 | 44 | 22 | 88 |

Chi-Sq.= 0.14 Significance= 0.93239385 (hom/n)= 0.26 Avg. Litter Size= 9
Mutation Information
Mutation Type: Homologous Recombination (standard)
Description: Coding exons 1 through 3 were targeted (NCBI accession AK002226).
1. Wild-type Expression Panel: Expression of the target gene was detected in all 13 adult tissue samples tested by RT-PCR, except spleen, liver, and bone.
2. QC Expression: Disruption of the target gene was confirmed by Southern hybridization analysis.

### 46.12.1. PHENOTYPIC ANALYSIS (for disrupted gene: DNA56405-1357 (UNQ430)

### (a) OVERALL PHENOTYPIC SUMMARY:

Mutation ofthe gene encoding the ortholog ofhuman Ly6/Plaur domain containing 2 (LYPDC2) resulted in small (-/-) mice. Both the male and female homozygous mutant mice were smaller than their gender-matched wild-type littermates, exhibiting decreased mean body weight and length, lean body mass, total tissue mass, total body fat and bone mineral content and density. The homozygous mice also exhibited decreased serum triglyceride and cholesterol levels. The (-/-) mice showed decreased ambulation (hypoactivity) during circadian rhythm testing. Disruption of the target gene was confirmed by Southern hybridization analysis.

### (b) Pathology

CATScan: The (-/-) mice exhibited generally decreased body size. However, no gross lesions were observed.

### (c) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements Body Length & Weight):

Body Measurements : A measurement of body length and weight was performed at approximately 16 weeks of age.

### Results:

Weight: Both the male and female (-/-) mice exhibited decreased mean body weight when compared with that of their gender-matched (+/+) littermates and the historical mean.
Length: Both the male and female (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: Both the male and female (-/-) mice exhibited decreased mean total tissue mass, lean body mass and bone mineral content and density measurements when compared with those of their gender-matched (+/+) littermates and the historical means. The male (-/-) mice also exhibited decreased total body fat and percent body fat consistent with decreased mean serum triglyceride and cholesterol levels shown below.
micro CT: The male (-/-) mice exhibited decreased mean vertebral trabecular bone volume, number, thickness, and connectivity density and decreased mean femoral mid-shaft cortical thickness when compared with that of their gender-matched (+/+) littermates and the historical means.

Mutant (-/-) mice deficient in the gene encoding PR0788 polypeptides show a phenotype consistent with growth retardation, marked by decreased body weight and length. Thus, antagonists or inhibitors of PR0788 polypeptides or its encoding gene would mimic these metabolic and growth related effects. On the other hand, PRO788 polypeptides or agonists thereof would be useful in the prevention and/or treatment of such metabolic disorders as diabetes or other tissue wasting diseases.

In addition, the (-/-) mice analyzed by DEXA and micro CT exhibited decreased bone measurements and decreased body mass measurements and total body fat when compared with their (+/+) littermates, suggestive of abnormal bone disorders. Thus, the (-/-) mice exhibited a negative bone phenotype. In addition, the decreased mean total tissue mass and lean body mass is indicative of a metabolic disorder related to growth retardation and tissue wasting disorders. The negative bone phenotype indicates that PR0788polypeptides or agonists thereof would be useful for maintaining bone homeostasis in addition to normal growth development. In addition, PR0788 polypeptides would be useful in bone healing or for the treatment of arthritis or osteoporosis, whereas antagonists (or inhibitors) of PRO788 polypeptides or its encoding gene would lead to abnormal or pathological bone disorders including inflammatory diseases associated with abnormal bone metabolism including arthritis, osteoporosis and osteopenia.

### (d) Phenotypic Analysis: Cardiology

In the area of cardiovascular biology, targets were identified herein for the treatment of hypertension, atherosclerosis, heart failure, stroke, various coronary artery diseases, dyslipidemias such as high cholesterol (hypercholesterolemia)and elevated serum triglycerides (hypertriglyceridemia), diabetes and/or obesity. The phenotypic tests included the measurement of serum cholesterol and triglycerides.

### Blood Lipids

Procedure: A cohort of 4 wild type, 4 heterozygotes and 8 homozygotes were tested in this assay. High cholesterol levels and increased triglyceride blood levels are recognized risk factors in the development of cardiovascular disease and/or diabetes. Measuring blood lipids facilitates the finding of biological switches that regulate blood lipid levels. Inhibition of factors which elevate blood lipid levels may be useful for reducing the risk for cardiovascular disease. In these blood chemistry tests, measurements were recorded using the COBAS Integra 400 (mfr: Roche).

### Results:

Blood Chemistry: The male (-/-) mice exhibited a decreased mean serum triglyceride and cholesterol level when compared with those of their gender-matched (+/+) littermates and the historical means.

In summary, these knockout mutant mice exhibited a decreased blood lipid phenotype with regards to lipid metabolism. These observations are consistent with decreased total body fat (DEXA results shown above). Thus, mutant mice deficient in the PR0788 gene can serve as a model for treatment of cardiovascular disease associated with dyslipidemia, hypertension, atherosclerosis, heart failure, stroke, or various coronary artery diseases.

### (e) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The female (-/-) mice exhibited decreased ambulatory counts (hypoactivity) during the 1- and 12-hour habituation periods and all light and dark periods when compared with those of their gender-matched (+/+) littermates and the historical means.

These results are consistent with lethargy or depressive disorders. Antagonists or inhibitors of PR0788 polypeptides or the PR0788 encoding gene would be expected to mimic this behavior. Likewise, PR0788 polypeptides or agonists thereof, would be useful in the treatment of such neurological disorders including depressive disorders or other decreased anxiety-like symptoms such as lethargy, cognitive disorders, hyperalgesia and sensory disorders.

### 46.13. Generation and Analysis of Mice Comprising DNA56352-1358 (UNQ431) Gene Disruptions

In these knockout experiments, the gene encoding PR0792 polypeptides (designated as DNA56352-1358) (UNQ431) was disrupted. The gene specific information for these studies is as follows: the mutated mouse gene corresponds to nucleotide reference: NM_029465 Mus musculus C-type lectin domain family 4, member g (Clec4g); protein reference: Q8BNX 1 ACCESSION:Q8BNX 1 NID: Mus musculus (Mouse). Hypothetical C-type lectin domain containing protein; the human gene sequence reference: NM_198492 ACCESSION:NM_198492 NID: gi 38348295 ref NM_198492.1 Homo sapiens liver and lymph node sinusoidal endothelial cell C-type lectin (LSECtin); the human protein sequence corresponds to reference: Q6UXB4 ACCESSION:Q6UXB4 NID: Homo sapiens (Human).

The mouse gene of interest is Clec4g (C-type lectin domain family 4, member g), ortholog of human CLEC4G. Aliases include LSECtin, 4930572L20Rik, and UNQ431.

CLEC4G is a type II integral plasma membrane protein that likely functions as an endocytic receptor or cell adhesion molecule. The protein contains a signal anchor and a C-terminal C-type lectin domain, which requires calcium for binding activity. CLEC4G binds with mannose, N-acetylglucosamine, and fucose but not with galactose. CLEC4G also binds with activated T cells in a calcium- and sugar-dependent manner. CLEC4G is expressed primarily in sinusoidal endothelial cells of liver and lymph node, possibly playing a role in antigen clearance and T cell trafficking from lymph nodes (Liu et al, J Biol Chem: 279:18748-58 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 40 | 26 | 84 |
| Expected | 21 | 42 | 21 | 84 |

Chi-Sq.= 2.03 Significance= 0.36240244 (hom/n)= 0.29 Avg. Litter Size= 9
Mutation Information
Mutation Type: Homologous Recombination (standard)
Description: Coding exons 1 through 9 were targeted (NCBI accession NM_029465.1).
1. Wild-type Expression Panel: Expression of the target gene was detected in all 13 adult tissue samples tested by RT-PCR, except eye; skeletal muscle; bone; stomach, small intestine, and colon; and adipose.
2. QC Expression: Disruption of the target gene was confirmed by Southern hybridization analysis.

### 46.13.1. PHENOTYPIC ANALYSIS (for disrupted gene: DNA56352-1358 (UNQ431)

### (a) OVERALL PHENOTYPIC SUMMARY:

Mutation of the gene encoding the ortholog of human C-type lectin domain family 4, member g (CLEC4G) resulted in immunological abnormalities in (-/-) mice when compared with their wild-type littermates and the historical means, including an increased mean serum IgG2a response to ovalbumin challenge. The (-/-) mice also showed a decreased mean systolic blood pressure. Disruption of the target gene was confirmed by Southern hybridization analysis.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Ovalbumin Challenge

Procedure: This assay was carried out on 7 wild types and 8 homozygotes. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immunodominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice exhibited an increased mean serum IgG2a response to ovalbumin challenge when compared with that of their (+/+) littermates and the historical mean.

In summary, the ovalbumin challenge studies indicate that knockout mice deficient in the gene encoding PR0792 polypeptides exhibit immunological abnormalities when compared with their wild-type littermates. In particular, the mutant mice exhibited an increased ability to elicit an immunological response when challenged with the T-cell dependent OVA antigen. Thus, antagonists (inhibitors) of PR0792 polypeptides would be useful for stimulating the immune system (such as T cell proliferation) and would find utility in the cases wherein this effect would be beneficial to the individual such as in the case of leukemia, and other types of cancer, and in immuno-compromised patients, such as AIDS sufferers. Accordingly, PR0792 polypeptides or agonists thereof, would be useful for inhibiting the immune response and thus would be useful candidates for suppressing harmful immune responses, e.g. in the case of graft rejection or graft-versus-host diseases.

### (c) Cardiology - Blood Pressure

### Description:

Systolic blood pressure is measured via a noninvasive tail-cuff method for four days on the Visitech BP-2000 Blood Pressure Analysis System. The blood pressure is measured ten times each day for four days. The four days are then averaged to obtain a mouse's conscious systolic blood pressure. The single (-/-) male mouse also exhibited a decreased heart rate (> two standard deviations below historic means).

### Results:

Blood Pressure: The (-/-) mice exhibited decreased mean systolic blood pressure (~ 1 SD below littermate controls for both male and females) when compared with that of their gender-matched (+/+) littermates.

### 46.14. Generation and Analysis of Mice Comprising DNA54002-1367 (UNQ477) Gene Disruptions

In these knockout experiments, the gene encoding PR0940 polypeptides (designated as DNA54002-1367) (UNQ477) was disrupted. The gene specific information for these studies is as follows: the mutated mouse gene corresponds to nucleotide reference: AY210400 Mus musculus Siglec-G; protein reference: Q80ZE3 ACCESSION:Q80ZE3 NID: Mus musculus (Mouse). Siglec-G; the human gene sequence reference: NM_033130 ACCESSION:NM_033130 NID: gi 15055512 ref NM_033130.1 Homo sapiens sialic acid binding Ig-like lectin 10 (SIGLEC10); the human protein sequence corresponds to reference: Q96LC7 ACCESSION:Q96LC7 NID: Homo sapiens (Human). Sialic acid binding Ig-like lectin 10 precursor (Siglec-10) (Siglec- like protein 2).

The mouse gene of interest is Siglec 10 (sialic acid binding Ig-like lectin 10), ortholog of human SIGLEC10. Aliases include mSiglec-G, 9830164H23, A630096C01Rik, SLG2, PR0940, and SIGLEC-10.

SIGLEC10 is a type I integral plasma membrane protein that likely functions as a signal-transducing receptor. The protein contains a signal peptide, five immunoglobulin (Ig)-like domains, a transmembrane segment, and a cytoplasmic C-terminal domain with two or three immune receptor tyrosine-based inhibitory motifs (ITIMs). SIGLEC10 can recruit protein tyrosine phosphatase PTPN6 (SHP-1), suggesting that SIGLEC10 functions as an inhibitory receptor. SIGLEC10 is capable of binding with sialic acid residues on erythrocytes, soluble sialoglycoconjugates, and GT1b ganglioside, suggesting that SIGLEC10 is involved in cell-cell recognition. SIGLEC10 is expressed primarily on peripheral blood leukocytes and is likely to play a role in negatively regulating immune cell function (Whitney et al, Eur J Biochem.: 268:6083-96 (2001); Li et al, J Biol Chem.: 276:28106-12 (2001); Munday et al, Biochem J.: 355:489-97 (2001); Rapoport et al, Bioorg Med Chem Lett.: 13:675-8 (2003); Kitzig et al, Biochem Biophys Res Commun.: 296:355-62 (2002)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 33 | 26 | 78 |
| Expected | 19.5 | 39 | 19.5 | 78 |

Chi-Sq.= 0.85 Significance= 0.6537698 (hom/n)= 0.27 Avg. Litter Size= 9
Mutation Information
Mutation Type: Homologous Recombination (standard)
Description: Coding exons 1 through 7 and the noncoding exon preceeding coding exon 1 were targeted (NCBI accession AK042488).
1. Wild-type Expression Panel: Expression of the target gene was detected in embryonic stem (ES) cells and in all 13 adult tissue samples tested by RT-PCR, except skeletal muscle, bone, and heart.
2. QC Expression: Disruption of the target gene was confirmed by Southern hybridization analysis.

### 46.14.1. PHENOTYPIC ANALYSIS (for disrupted gene: DNA54002-1367 (UNQ477)

### (a) OVERALL PHENOTYPIC SUMMARY:

Mutation of the gene encoding the ortholog of human sialic acid binding Ig-like lectin 10 (SIGLEC10) resulted in 3 (-/-) small mice that failed to thrive. The male (-/-) mice exhibited increased bone mineral content and bone mineral density measurements. Female (-/-) mice exhibited an altered sleep/wake cycle, and the male (-/-) mice exhibited an increased anxiety-related response with an increased stress-induced hyperthermia response. Gene disruption was confirmed by Southern blot.

### (b) Pathology

Obvious: Three of the (-/-) mice were small and failed to thrive but normal Mendelian ratios were present at genotyping. The rest were of normal size and appeared healthy.
Microscopic: Some (-/-) mice exhibited lymphocytic infiltrates in salivary glands, pancreas, and lungs; lesions rarely seen in mice of this age. Of the 3 mice examined at 3 weeks of age, 2 exhibited encephalitis due to Group B Streptococcus and 1 exhibited meningitis due to *E. coli* infection.
Gene Expression: LacZ activity was not detected in the panel of tissues by immunohistochemical analysis.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Functional Observational Battery (FOB) Test - Stress-induced Hyperthermia:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Results:

Stress-Induced Hyperthermia: The male (-/-) mice exhibited an increased sensitivity to stress-induced hyperthermia when compared with that of their gender-matched (+/+) littermates and the historical mean, suggesting an increased anxiety-like response in the mutants.

In summary, the functional observation testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PR0940 polypeptides or agonists thereof would be useful in the treatment of such neurological disorders.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The female (-/-) mice exhibited decreased ambulatory counts during the light periods when compared with that of their gender-matched (+/+) littermates and the historical mean. These results are consistent with an altered sleep wake cycle.

### (d) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

Male (-/-) mice exhibited increased bone mineral content and bone mineral density measurements compared to their gender-matched(+/+) littermates.

These results indicate that the knockout mutant phenotype can be associated with such bone abnormalities as osteopetrosis. Osteopetrosis is a condition characterized by abnormal thickening and hardening of bone and abnormal fragility of the bones. As such, PR0940 polypeptides or agonists thereof would be beneficial for the treatment of osteopetrosis or other osteo-related diseases. On the other hand, inhibitors or antagonists of PR0940 polypeptides would be useful in bone healing.

### 46.15. Generation and Analysis of Mice Comprising DNA53906-1368 (UNQ478) Gene Disruptions

In these knockout experiments, the gene encoding PR0941 polypeptides (designated as DNA53906-1368) (UNQ478) was disrupted. The gene specific information for these studies is as follows: the mutated mouse gene corresponds to nucleotide reference: XM_129984 Mus musculus similar to cadherin 19, type 2 preproprotein (LOC227485); protein reference: XP_129984 ACCESSION:XP_129984 NID: gi 51712173 ref XP_129984.3 similar to cadherin 19, type 2 preproprotein [Mus musculus]; the human gene sequence reference: NM_021153 ACCESSION:NM_021153 NID:16306535 Homo sapiens Homo sapiens cadherin 19, type 2 (CDH 19); the human protein sequence corresponds to reference: Q9H159 ACCESSION:Q9H159 NID: Homo sapiens (Human).

### CADHERIN-19 PRECURSOR.

The mouse gene of interest is LOC227485 (similar to cadherin 19, type 2 preproprotein), ortholog of human CDH19 (cadherin 19, type 2). Aliases include CDH7 and CDH7L2.

CDH19 is a type I integral plasma membrane protein that functions as a cell adhesion molecule. CDH19 likely interacts with other CDH19 molecules on different cells and plays a role in homophilic cell adhesion. CDH19 may suppress tumor invasion and metastasis (Kool et al, Genomics: 68:283-95 (2000); Blons et al, Oncogene: 21:5016-23 (2002); Hajra and Fearon, Genes Chromosomes Cancer: 34:255-68 (2002)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 41 | 20 | 80 |
| Expected | 20 | 40 | 20 | 80 |

Chi-Sq.= 1.72 Significance= 0.42316207 (hom/n)= 0.23 Avg. Litter Size= 8
Mutation Information
Mutation Type: Homologous Recombination (standard)
Description: Coding exon 3 was targeted (NCBI accession XM_129984.3).
1. Wild-type Expression Panel: Expression of the target gene was detected in all 13 adult tissue samples tested by RT-PCR.
2. QC Expression: Disruption of the target gene was confirmed by Southern hybridization analysis.

### 46.15.1. PHENOTYPIC ANALYSIS (for disrupted gene: DNA53906-1368 (UNQ478)

### (a) OVERALL PHENOTYPIC SUMMARY:

Mutation of the gene encoding the ortholog of human cadherin 19, type 2 (CDH 19) resulted in the mutant (-/-) exhibiting decreased serum glucose levels. Gene disruption was confirmed by Southern blot.

### (b) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes.

### Results:

### Blood Chemistry: The (-/-) mice exhibited a decreased mean serum glucose level when compared with that of their gender-matched (+/+) littermates and the historical mean.

In these studies the mutant (-/-) mice showed decreased serum glucose levels which could be due to an increased insulin sensitivity. Thus, antagonists (inhibitors) to PR0941 polypeptides or its encoding gene would be useful in the treatment of impaired glucose homeostasis.

### 46.16. Generation and Analysis of Mice Comprising DNA57844-1410 (UNQ488) Gene Disruptions

In these knockout experiments, the gene encoding PRO1004 polypeptides (designated as DNA57844-1410) (UNQ488) was disrupted. The gene specific information for these studies is as follows: the mutated mouse gene corresponds to nucleotide reference: NM_175650 Mus musculus ATPase type 13A5 (Atp13a5); protein reference: Q8BUP1 ACCESSION:Q8BUP1 NID: Mus musculus (Mouse). Mus musculus adult male hippocampus cDNA, RIKEN full-length enriched library, clone:C630015F21 product:hypothetical Microbodies C-terminal targeting signal/E1-E2 ATPases/Haloacid dehalogenase/epoxide hydrolase family/Cation transporter ATPase containing protein, full insert sequence; the human gene sequence reference: AK122613 Homo sapiens cDNA FLJ16025 fis, clone CTONG2004062, highly similar to ATPase subunit 6; the human protein sequence corresponds to reference: Q6ZWL0 ACCESSION:Q6ZWL0 NID: Homo sapiens (Human). Hypothetical protein FLJ16025.

The mouse gene of interest is Atp13a5 (ATPase type 13A5), ortholog of human ATP13A5. Aliases include C630015F21Rik and FLJ16025.

ATP13A5 is a putative integral plasma membrane protein that likely functions as a cation-transporting ATPase. The protein contains a "P-type ATPase of unknown specificity" domain (InterPro accession IPR006544) and at least 10 transmembrane segments. ATP13A5 is expressed primarily in brain and stomach (Schultheis et al, Biochem Biophys Res Commun.: 323:731-8 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 20 | 34 | 21 | 75 |
| Expected | 18.75 | 37.5 | 18.75 | 75 |

Chi-Sq.= 0.57 Significance= 0.7520143 (hom/n)= 0.26 Avg. Litter Size= 9
Mutation Information
Mutation Type: Homologous Recombination (standard)
Description: Coding exons 3 through 5 were targeted (NCBI accession NM_175650.2).
1. Wild-type Expression Panel: Expression of the target gene was detected in all 13 adult tissue samples tested by RT-PCR, except spleen, liver, bone, and heart.
2. QC Expression: Disruption of the target gene was confirmed by Southern hybridization analysis.

### 46.16.1. PHENOTYPIC ANALYSIS (for disrupted gene: DNA57844-1410 (UNQ488)

### (a) OVERALL PHENOTYPIC SUMMARY:

Mutation of the gene encoding the ortholog of human ATPase type 13A5 (ATP13A5) resulted in the mutant (-/-) mice exhibiting decreased bone-related measurements. Disruption of the target gene was confirmed by Southern hybridization analysis.

### (b) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean bone mineral content, femur bone mineral density and vertebrae bone mineral density measurements compared to their gender-matched littermates and the historical means.
micro CT: The male (-/-) mice exhibited decreased mean femoral mid-shaft cross-sectional area when compared with that of their gender-matched (+/+) littermates and the historical means.

The (-/-) mice analyzed by DEXA and bone micro CT analysis exhibited decreased bone measurements when compared with their (+/+) littermates, suggestive of abnormal bone disorders. Thus, the (-/-) mice exhibited a negative bone phenotype. The negative bone phenotype indicates that PRO1004 polypeptides or agonists thereof would be useful for maintaining bone homeostasis. In addition, PRO1004 polypeptides would be useful in bone healing or for the treatment of arthritis or osteoporosis, whereas antagonists (or inhibitors) of PRO1004 polypeptides or its encoding gene would lead to abnormal or pathological bone disorders including inflammatory diseases associated with abnormal bone metabolism including arthritis, osteoporosis and osteopenia.

### 46.17. Generation and Analysis of Mice Comprising DNA56439-1376 (UNQ495) Gene Disruptions

In these knockout experiments, the gene encoding PRO1012 polypeptides (designated as DNA56439-1376) (UNQ495) was disrupted. The gene specific information for these studies is as follows: the mutated mouse gene corresponds to nucleotide reference: NM_024181 ACCESSION:NM_024181 NID: gi 26190605 ref NM_024181.1 Mus musculus DnaJ (Hsp40) homolog, subfamily C, member 10 (Dnajc10); protein reference: Q8CH78 ACCESSION:Q8CH78 NID: Mus musculus (Mouse). ER-resident protein ERdj5; the human gene sequence reference: NM_018981 ACCESSION:NM_018981 NID: gi 24308126 ref NM_018981.1 Homo sapiens DnaJ (Hsp40) homolog, subfamily C, member 10 (DNAJC10); the human protein sequence corresponds to reference: Q96K44 ACCESSION:Q96K44 NID: Homo sapiens (Human). CDNA FLJ14741 FIS, CLONE NT2RP3002628, WEAKLY SIMILAR TO PROBABLE PROTEIN DISULFIDE ISOMERASE P5 PRECURSOR (EC 5.3.4.1).

The mouse gene of interest is Dnajc10 (DnaJ [Hsp40] homolog, subfamily C, member 10), ortholog of human DNAJC10. Aliases include JPDI, ERdj5, D2Ertd706e, 1200006L06Rik, and DKFZp434J1813.

DNAJC10 is a protein located in the endoplasmic reticulum that likely functions as a co-chaperone for protein folding and intramolecular disulfide bond formation. The protein contains a putative N-terminal translocation signal, a DNAJ domain, four thioredoxin-like domains, and a C-terminal KDEL endoplasmic reticulum (ER) retention signal. Enzymatic activity associated with DNAJC10 has not been detected. The DNAJ domain of DNAJC10 can bind with chaperone protein BiP, stimulating its ATPase activity. The DNAJC10-BiP complex likely associates with protein isomerases or other protein translocation components, playing a role in protein folding and trafficking. DNAJC10 is ubiquitously expressed but is particularly abundant in secretory tissue (Cunnea et al, J Biol Chem: 278:1059-66 (2003); Hosoda et al, J Biol Chem: 278:2669-76 (2003); Gu et al, Biochem Genet: 41:245-53 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 27 | 40 | 12 | 79 |
| Expected | 19.75 | 39.5 | 19.75 | 79 |

Chi-Sq.= 5.31 Significance= 0.07029884 (hom/n)= 0.19 Avg. Litter Size= 9
Mutation Information
Mutation Type: Homologous Recombination (standard)
Description: Coding exon 1 and the preceding noncoding exon were targeted (NCBI accession NM_024181.1).
1. Wild-type Expression Panel: Expression of the target gene was detected in embryonic stem (ES) cells and in all 13 adult tissue samples tested by RT-PCR, except bone and adipose.
2. QC Expression: Disruption of the target gene was confirmed by Southern hybridization analysis.

### 46.17.1. PHENOTYPIC ANALYSIS (for disrupted gene: DNA56439-1376 (UNQ495)

### (a) OVERALL PHENOTYPIC SUMMARY:

Mutation of the gene encoding the ortholog of human DnaJ (Hsp40) homolog, subfamily C, member 10 (DNAJC10) resulted in small female (-/-) mice. The homozygous mutant mice were smaller than their gender-matched wild-type littermates, exhibiting decreased mean body weight and length, mean total tissue mass, and lean body mass. The mutant (-/-) mice also showed decreased bone mineral content and total body bone mineral density. The male (-/-) mice showed decreased mean systolic blood pressure. Disruption of the target gene was confirmed by Southern hybridization analysis.

### (b) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The (-/-) mice exhibited decreased mean body weight when compared with that of their gender-matched (+/+) littermates and the historical mean.
Length: The (-/-) mice exhibited decreased mean body length when compared with that of their gender-matched (+/+) littermates and the historical mean.

Decreased mean body weight and length was more pronounced in the female (-/-) mice compared to the male homozygotes.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

DEXA: The (-/-) mice exhibited decreased mean total tissue mass, lean body mass, bone mineral content,and total body bone mineral density measurements when compared with those of their gender-matched (+/+) littermates and the historical means.

Mutant (-/-) mice deficient in the gene encoding PRO1012 polypeptides show a phenotype consistent with growth retardation, marked by decreased body weight and length. Thus, antagonists or inhibitors of PRO1012 polypeptides or its encoding gene would mimic these metabolic and growth related effects. On the other hand, PRO1012 polypeptides or agonists thereof would be useful in the prevention and/or treatment of such metabolic disorders as diabetes or other tissue wasting diseases.

In addition, the (-/-) mice analyzed by DEXA exhibited decreased bone measurements and decreased body mass measurements when compared with their (+/+) littermates, suggestive of abnormal bone disorders. Thus, the (-/-) mice exhibited a negative bone phenotype. In addition, the decreased mean total tissue mass and lean body mass is indicative of a metabolic disorder related to growth retardation and tissue wasting disorders. The negative bone phenotype indicates that PRO1012 polypeptides or agonists thereof would be useful for maintaining bone homeostasis in addition to normal growth development. In addition, PRO1012 polypeptides would be useful in bone healing or for the treatment of arthritis or osteoporosis, whereas antagonists (or inhibitors) of PRO1012 polypeptides or its encoding gene would lead to abnormal or pathological bone disorders including inflammatory diseases associated with abnormal bone metabolism including arthritis, osteoporosis and osteopenia.

### (c) Cardiology - Blood Pressure

### Description:

Systolic blood pressure is measured via a noninvasive tail-cuff method for four days on the Visitech BP-2000 Blood Pressure Analysis System. The blood pressure is measured ten times each day for four days. The four days are then averaged to obtain a mouse's conscious systolic blood pressure. The single (-/-) male mouse also exhibited a decreased heart rate (> two standard deviations below historic means.

### Results:

Blood Pressure: The male (-/-) mice exhibited slightly decreased mean systolic blood pressure when compared with that of their gender-matched (+/+) littermates and the historical mean.

### 46.18. Generation and Analysis of Mice Comprising DNA56113-1378 (UNQ499) Gene Disruptions

In these knockout experiments, the gene encoding PRO1016 polypeptides (designated as DNA56113-1378) (UNQ499) was disrupted. The gene specific information for these studies is as follows: the mutated mouse gene corresponds to nucleotide reference: NM_026644 ACCESSION:NM_026644 NID:21313141 Mus musculus Mus musculus 1-acylglycerol-3-phosphate O-acyltransferase 1 (lysophosphatidic acid acyltransferase, delta) (Agpat4); protein reference: Q8K4X7 ACCESSION:Q8K4X7 NID: Mus musculus (Mouse). Lysophosphatidic acid acyltransferase-delta(1-acylglycerol-3-phosphate O-acyttransferase 1) (Mus musculus adult male cerebellum cDNA, RIKEN full-length enriched library, clone:1500003P24 product:1- acylglycerol-3-phosphate O-acyltransferase 1 (lysophosphatidic acid acyltransferase, delta), full insert sequence); the human gene sequence reference:NM_020133 ACCESSION:NM_020133 NID:9910391 Homo sapiens Homo sapiens lysophosphatidic acid acyltransferase-delta (LPAAT-delta); the human protein sequence corresponds to reference: Q9NRZ5 ACCESSION:Q9NRZ5 NID: Homo sapiens (Human). 1-ACYL-SN-GLYCEROL-3-PHOSPHATE ACYLTRANSFERASE DELTA (EC 2.3.1.51) (1- AGP ACYLTRANSFERASE 4) (1-AGPAT 4) (LYSOPHOSPHATIDIC ACID ACYLTRANSFERASE-DELTA) (LPAAT-DELTA) (1-ACYLGLYCEROL-3-PHOSPHATE O- ACYLTRANSFERASE 4).

The mouse gene of interest is Agpat4 (1-acylglycerol-3-phosphate O-acyltransferase 1 [lysophosphatidic acid acyltransferase, delta]), ortholog of human AGPAT4. Aliases include 1500003P24Rik, dJ473J16.2, and LPAAT-delta.

AGPAT4 is a putative integral plasma membrane protein that likely functions as an enzyme, catalyzing the formation of phosphatidic acid from lysophosphatidic acid and acyl-coenzyme A. The protein contains an N-terminal transmembrane segment, a phosphate acyltransferase catalytic domain (SMART accession SM00563), and two C-terminal transmembrane segments. AGPAT4 likely plays a role in the biosynthesis of phospholipids (Lu et al, Biochem J: 385:469-77 (2005); Kume and Shimizu, Biochem Biophys Res Commun: 237:663-6 (1997); Kawaji et al, Genome Res: 12:367-78 (2002)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 15 | 41 | 16 | 72 |
| Expected | 18 | 36 | 18 | 72 |

Chi-Sq.= 0.94 Significance= 0.62500226 (hom/n)= 0.26 Avg. Litter Size= 9
Mutation Information
Mutation Type: Homologous Recombination (standard)
Description: Coding exons 3 through 5 were targeted (NCBI accession NM_026644.1).
1. Wild-type Expression Panel: Expression of the target gene was detected in embryonic stem (ES) cells and in all 13 adult tissue samples tested by RT-PCR, except stomach, small intestine, and colon.
2. QC Expression: Disruption of the target gene was confirmed by Southern hybridization analysis.

### 46.18.1. PHENOTYPIC ANALYSIS (for disrupted gene: DNA56113-1378 (UNQ499)

### (a) OVERALL PHENOTYPIC SUMMARY:

Mutation of the gene encoding the ortholog of human 1-acylglycerol-3-phosphate O-acyltransferase 1 (lysophosphatidic acid acyltransferase, delta) (AGPAT4) resulted in the (-/-) mice exhibiting decreased bone-related measurements. Gene disruption was confirmed by Southern blot.

### (b) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean lean body mass and femur bone mineral density when compared with those of their gender-matched (+/+) littermates and the historical means.
micro CT: The male (-/-) mice exhibited decreased mean femoral mid-shaft cross-sectional area when compared with that of their gender-matched (+/+) littermates and the historical means.

The (-/-) mice analyzed by DEXA and bone micro CT analysis exhibited decreased bone measurements and decreased lean body mass measurements when compared with their (+/+) littermates, suggestive of abnormal bone disorders. Thus, the (-/-) mice exhibited a negative bone phenotype. In addition, the decreased mean total tissue mass is indicative of a metabolic disorder related to tissue wasting disorders. The negative bone phenotype indicates that PRO1016 polypeptides or agonists thereof would be useful for maintaining bone homeostasis. In addition, PRO1016 polypeptides would be useful in bone healing or for the treatment of arthritis or osteoporosis, whereas antagonists (or inhibitors) of PRO1016polypeptides or its encoding gene would lead to abnormal or pathological bone disorders including inflammatory diseases associated with abnormal bone metabolism including arthritis, osteoporosis and osteopenia.

### 46.19. Generation and Analysis of Mice Comprising DNA56045-1380 (UNQ502) Gene Disruptions

In these knockout experiments, the gene encoding PR0474 polypeptides (designated as DNA56045-1380) (UNQ502) was disrupted. The gene specific information for these studies is as follows: the mutated mouse gene corresponds to nucleotide reference: NM_025330 ACCESSION:NM_025330 NID: gi 61098115 ref NM_025330.2 Mus musculus dehydrogenase/reductase (SDR family) member 10 (Dhrs10); protein reference: Q9CWL3 ACCESSION:Q9CWL3 NID: Mus musculus (Mouse). 0610039E24RIK PROTEIN; the human gene sequence reference: NM_016246 ACCESSION:NM_016246 NID: gi 59889577 refNM_016246.2 Homo sapiens dehydrogenase/reductase (SDR family) member 10 (DHRS10); the human protein sequence corresponds to reference: Q9BPX1 ACCESSION:Q9BPX1 NID: Homo sapiens (Human). UNKNOWN (PROTEIN FOR MGC:10539) (PROTEIN FOR MGC: 10685).

The mouse gene of interest is Dhrs10 (dehydrogenase/reductase [SDR family] member 10), ortholog of human DHRS 10. Aliases include 0610039E24Rik, retSDR3, and retinal short-chain dehydrogenase/reductase 3. DHRS10 is a hypothetical mitochondrial protein that contains a short-chain dehydrogenase domain (Pfam accession PF00106). Enzymes with this domain generally catalyze NAD- or NADP-dependent oxidoreductase reactions and include D-beta-hydroxybutyrate dehydrogenase, mitochondrial precursor (BDH); estradiol 17-beta-dehydrogenase 8 (HSD17B8); NADP-dependent retinol dehydrogenase (DHRS4); and dicarbonyl/L-xylulose reductase (DCXR).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 38 | 13 | 70 |
| Expected | 17.5 | 35 | 17.5 | 70 |

Chi-Sq.= 3.49 Significance= 0.17464499 (hom/n)= 0.2 Avg. Litter Size= 8
Mutation Information
Mutation Type: Homologous Recombination (standard)
Description: Coding exons 1 through 4 were targeted (NCBI accession NM_016246.1 [human]).
1. Wild-type Expression Panel: Expression of the target gene was detected in embryonic stem (ES) cells and in all 13 adult tissue samples tested by RT-PCR, except liver and bone.
2. QC Expression: Disruption of the target gene was confirmed by Southern hybridization analysis.

### 46.19.1. PHENOTYPIC ANALYSIS (for disrupted gene: DNA56045-1380 (UNQ502)

### (a) OVERALL PHENOTYPIC SUMMARY:

Mutation of the gene encoding the ortholog of human dehydrogenase/reductase (SDR family) member 10 (DHRS10) resulted in infertile male (-/-) mice. The male homozygous mutant mice exhibited vacuolar degeneration in the testes, resulting in decreased sperm production and infertility in the mutants. An enlarged liver was detected in one male (-/-) mouse. The female (-/-) mice also showed decreased weight and length. Bilateral white deposits were observed in the optic disc region of one of the mutant homozygous mice. Disruption of the target gene was confirmed by Southern hybridization analysis.

### (b) Pathology

Microscopic: Of the 3 male (-/-) mice examined, 2 exhibited vacuolar degeneration in the testes. Similar lesions were also noted in the epididymides of one mutant. These lesions resulted in notably decreased sperm production. CATScan: Of 3 (-/-) mice analyzed, 1 (M-151) exhibited a notably enlarged liver.
Gene Expression: LacZ activity was not detected in the panel of tissues by immunohistochemical analysis.

### (c) Bone Metabolism & Body Diagnostics

### Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The female (-/-) mice exhibited decreased mean body weight when compared with that of their gender-matched (+/+) littermates and the historical mean.
Length: The female (-/-) mice exhibited a decreased mean body length when compared with that of their gender-matched (+/+) littermates and the historical mean.

### Fertility:

Fertility: The male (-/-) mouse produced no pups after 40 days of breeding.
A single (-/-) mouse exhibited a decreased testes weight.

Mutant (-/-) mice deficient in the gene encoding PR0474 polypeptides show a phenotype consistent with growth retardation, marked by decreased body weight and length. Thus, antagonists or inhibitors of PR0474 polypeptides or its encoding gene would mimic these metabolic and growth related effects. On the other hand, PR0474 polypeptides or agonists thereof would be useful in the prevention and/or treatment of such metabolic disorders as diabetes or other tissue wasting diseases. In addition, the male (-/-) mice exhibited reproductive and infertility disorders.

### (d) Cardiovascular Phenotypic Analysis:

In the area of cardiovascular biology, phenotypic testing was performed to identify potential targets for the treatment of cardiovascular, endothelial or angiogenic disorders. One such phenotypic test included optic fundus photography and angiography to determine the retinal arteriovenous ratio (A/V ratio) in order to flag various eye abnormalities. An abnormal A/V ratio signals such systemic diseases or disorders that may be related to the vascular disease of hypertension (and any disease that causes hypertension, e.g. atherosclerosis), diabetes or other ocular diseases corresponding to ophthalmological disorders. Such eye abnormalities may include but are not limited to the following: retinal abnormality is retinal dysplasia, various retinopathies, restenosis, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

Procedure: A cohort of 4 wild type, 4 heterozygotes and 8 homozygotes were tested in this assay. Optic fundus photography was performed on conscious animals using a Kowa Genesis small animal fundus camera modified according to Hawes and coauthors (Hawes et al., 1999 Molecular Vision 1999; 5:22). Intra-peritoneal injection of fluorescein permitted the acquisition of direct light fundus images and fluorescent angiograms for each examination. In addition to direct ophthalmological changes, this test can detect retinal changes associated with systemic diseases such as diabetes and atherosclerosis or other retinal abnormalities. Pictures were provided of the optic fundus under normal light. The angiographic pictures allowed examination of the arteries and veins of the eye. In addition an artery to vein (A/V) ratio was determined for the eye.

Ophthalmology analysis was performed on generated F2 wild type, heterozygous, and homozygous mutant progeny using the protocol described above. Specifically, the A/V ratio was measured and calculated according to the fundus images with Kowa COMIT+ software. This test takes color photographs through a dilated pupil: the images help in detecting and classifying many diseases. The artery to vein ratio (A/V) is the ratio of the artery diameter to the vein diameter (measured before the bifurcation of the vessels). Many diseases will influence the ratio, i.e., diabetes, cardiovascular disorders, papilledema, optic atrophy or other eye abnormalities such as retinal degeneration (known as retinitis pigmentosa) or retinal dysplasia, vision problems or blindness. Thus, phenotypic observations which result in an increased artery-to-vein ratio in homozygous (-/-) and heterozygous (+/-) mutant progeny compared to wild-type (+/+) littermates would be indicative of such pathological conditions.

### Results:

Fundus: One (-/-) mouse exhibited bilateral multiple white deposits located around the optic disc region.

### 46.20. Generation and Analysis of Mice Comprising DNA257845 (UNQ503) Gene Disruptions

In these knockout experiments, the gene encoding PR05238 polypeptides (designated as DNA257845) (UNQ503) was disrupted. The gene specific information for these studies is as follows: the mutated mouse gene corresponds to nucleotide reference: NM_183222 Mus musculus Fc receptor-like protein 3 (Fcrh3); protein reference: Q80WN2 ACCESSION:Q80WN2 NID: Mus musculus (Mouse). BXMAS1-like protein 2; the human gene sequence reference: NM_031281 Homo sapiens immunoglobulin superfamily receptor translocation associated 2 (IRTA2); the human protein sequence corresponds to reference: Q5VYK9 ACCESSION:Q5VYK9 NID: Homo sapiens (Human). Immunoglobulin superfamily receptor translocation associated 2 (IRTA2).

The mouse gene of interest is Fcrl5 (Fc receptor-like protein 5), ortholog of human IRTA2 (immunoglobulin superfamily receptor translocation associated 2). Aliases include Fcrh3, BXMAS 1, FLJ00333, and mBXMH2.

IRTA2 is an integral plasma membrane protein expressed primarily on discrete B cell lineages. This protein likely functions as a receptor or cell adhesion molecule that mediates negative signaling. IRTA2 consists of a large extracellular domain, a transmembrane segment, and a short cytoplasmic domain. The extracellular domain contains a signal peptide and multiple immunoglobulin (Ig)-like domains, and the cytoplasmic domain contains 2 or 3 ITIM (immune-receptor tyrosine-based inhibition) motifs, which are capable of recruiting SH2 domain-containing inositol phosphatases. The IRTA2 transcript undergoes alternative splicing to yield at least 3 more variant isoforms. One variant is composed of a portion of the extracellular domain and is probably secreted. A second isoform contains the extracellular domain and a potential glycosylphosphatidylinositol (GPI)-anchoring site, suggesting that this isoform may be tethered to the extracellular surface of the plasma membrane. A third isoform encodes a protein of only 152 amino acids. IRTA2 may be involved in B cell development and lymphomagenesis (Hatzivassiliou et al, Immunity: 14:277-89 (2001); Nakayama et al, Biochem Biophys Res Commun: 285:830-7 (2001); Davis et al, Proc Natl Acad Sci U S A: 98:9772-7 (2001); Miller et al, Blood: 99:2662-9 (2002); Davis et al, Int Immunol: 16:1343-53 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 22 | 37 | 18 | 77 |
| Expected | 19.25 | 38.5 | 19.25 | 77 |

Chi-Sq.= 7.83 Significance= 0.01994055 (hom/n)= 0.23 Avg. Litter Size= 8
Mutation Information
Mutation Type: Homologous Recombination (standard)
Description: Coding exon 1 was targeted (NCBI accession NM_183222.2).
1. Wild-type Expression Panel: Expression of the target gene was detected in all 13 adult tissue samples tested by RT-PCR, except kidney, skeletal muscle, bone, and adipose.
2. QC Expression: Disruption of the target gene was confirmed by Southern hybridization analysis.

### 46.20.1. PHENOTYPIC ANALYSIS (for disrupted gene: DNA257845 (UNQ503)

### (a) OVERALL PHENOTYPIC SUMMARY:

Mutation of the gene encoding the ortholog ofhuman immunoglobulin superfamily receptor translocation associated 2 (IRTA2) resulted in the male homozygous mutant mice exhibiting an impaired glucose tolerance when compared with that of their gender-matched wild-type littermates and the historical mean. In addition, the (-/-) mice exhibited decreased lean body mass and decreased bone-related measurements. Elevated levels of serum cholesterol and triglycerides were also observed in the mutant (-/-) mice. Disruption of the target gene was confirmed by Southern hybridization analysis.

### (b) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean lean body mass and bone mineral content when compared with that of their gender-matched (+/+) littermates and the historical mean.
micro CT: The male (-/-) mice exhibited decreased mean femoral mid-shaft total area and trabecular thickness when compared with that of their gender-matched (+/+) littermates and the historical mean.

The (-/-) mice analyzed by DEXA and bone micro CT analysis exhibited decreased bone measurements and decreased lean body mass measurements when compared with their (+/+) littermates, suggestive of abnormal bone disorders. Thus, the (-/-) mice exhibited a negative bone phenotype. In addition, the decreased mean lean body mass is indicative of a metabolic disorder related to tissue wasting disorders. The negative bone phenotype indicates that PRO5238 polypeptides or agonists thereof would be useful for maintaining bone homeostasis. In addition, PR05238 polypeptides would be useful in bone healing or for the treatment of arthritis or osteoporosis, whereas antagonists (or inhibitors) of PRO5238polypeptides or its encoding gene would lead to abnormal or pathological bone disorders including inflammatory diseases associated with abnormal bone metabolism including arthritis, osteoporosis and osteopenia.

### (c) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: The (-/-) mice exhibited an increased mean serum alanine amino-transferase level.

### Blood Chemistry/Glucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of 2 wild type and 4 homozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Blood Glucose Levels/Glucose Tolerance Test:

Oral Glucose Tolerance: The male (-/-) mice exhibited an impaired glucose tolerance at 2/3 intervals when compared with that of their gender-matched (+/+) littermates and the historical mean.

Thus, knockout mutant mice exhibited the phenotypic pattern of an impaired glucose homeostasis, and therefor PR05238 polypeptides (or agonists thereof) or its encoding gene would be useful in the treatment of conditions associated with an impaired glucose homeostasis and/or various cardiovascular diseases, including diabetes.

### (d) Phenotypic Analysis: Cardiology

In the area of cardiovascular biology, targets were identified herein for the treatment of hypertension, atherosclerosis, heart failure, stroke, various coronary artery diseases, dyslipidemias such as high cholesterol (hypercholesterolemia)and elevated serum triglycerides (hypertriglyceridemia), diabetes and/or obesity. The phenotypic tests included the measurement of serum cholesterol and triglycerides.

### Blood Lipids

Procedure: A cohort of 4 wild type, 4 heterozygotes and 8 homozygotes were tested in this assay. High cholesterol levels and increased triglyceride blood levels are recognized risk factors in the development of cardiovascular disease and/or diabetes. Measuring blood lipids facilitates the finding of biological switches that regulate blood lipid levels. Inhibition of factors which elevate blood lipid levels may be useful for reducing the risk for cardiovascular disease. In these blood chemistry tests, measurements were recorded using the COBAS Integra 400 (mfr: Roche).

### Results:

Blood Chemistry: The male (-/-) mice exhibited increased mean serum cholesterol and triglyceride levels when compared with that of their gender-matched (+/+) littermates.

As summarized above, the (-/-) mice exhibited increased mean serum cholesterol and triglyceride levels when compared with their gender-matched (+/+) littermates and the historical means. Thus, mutant mice deficient in the PR05238 gene can serve as a model for cardiovascular disease. PR05238 polypeptides or its encoding gene would be useful in regulating blood lipids such as cholesterol and triglycerides. Thus, PR0523 8 polypeptides or agonists thereof would be useful in the treatment of such cardiovascular diseases as hypertension, atherosclerosis, heart failure, stroke, various coronary diseases, hypercholesterolemia, and/or diabetes.

### 46.21. Generation and Analysis of Mice Comprising DNA59211-1450 (UNQ526) Gene Disruptions

In these knockout experiments, the gene encoding PRO1069 polypeptides (designated as DNA59211-1450) (UNQ526) was disrupted. The gene specific information for these studies is as follows: the mutated mouse gene corresponds to nucleotide reference: NM_033648 ACCESSION:NM_033648 NID: gi 16258806 ref NM_033648.1 Mus musculus FXYD domain-containing ion transport regulator 4 (Fxyd4); protein reference: Q9D2W0 ACCESSION:Q9D2WO NID: Mus musculus (Mouse). FXYD domain-containing ion transport regulator 4 precursor (Channel inducing factor) (CHIF); the human gene sequence reference: NM_173160 Homo sapiens FXYD domain containing ion transport regulator 4 (FXYD4); the human protein sequence corresponds to reference: NP_775183 ACCESSION:NP_775183 NID: gi 27764904 ref NP_775183.1 (NM_173160) FXYD domain containing ion transport regulator 4; FXYD domain-containing ion transport regulator 4; channel-inducing factor [Homo sapiens].

The mouse gene of interest is Fxyd4 (FXYD domain-containing ion transport regulator 4), ortholog of human FXYD4. Aliases include Chif (channel inducing factor) and 0610008I02Rik.

FXYD4 is a type I integral plasma membrane protein that likely function as regulator of renal sodium/potassium-ATPase. The protein consists of 89 amino acids, containing a signal peptide, a small extracellular domain, a transmembrane segment, and a small intracellular domain. The transmembrane domain is highly conserved among family members. FXDY4 is expressed primarily in renal collecting duct and is induced by high potassium and aldosterone. FXYD4 likely plays a role in aldosterone-mediated sodium reabsorption in the kidney (Sweadner and Rael, Genomics: 68:41-56 (2000); Beguin et al, EMBO J.: 20:3993-4002 (2001); Garty et al, Am J Physiol Renal Physiol: 283:F607-15 (2002); Aizman et al, Am J Physiol Renal Physiol: 283:F569-77 (2002); Goldschmidt et al, Cell Physiol Biochem: 14:113-20 (2004)).

Aizman and coworkers [Am J Physiol Renal Physiol: 283:F569-77(2002)] investigated the physiological role of FXYD4 using knockout mice. They showed that water intake, glomerular filtration rate, and urine volume under potassium loading was higher in FXYD4 (-/-) mice than in wild-type littermates. Moreover, they showed that potassium loading and inhibition of sodium chloride reabsorption by loop diuretic furosemide caused greater diuresis, hyperkalemia, and lethality in the FXYD4 (-/-) mice than in the wild-type littermates. Aizman and coworkers concluded that FXYD4 likely increases sodium chloride reabsorption and potassium excretion by increasing collecting duct sodium/potassium ATPase activity.

Goldschmidt and coworkers [Cell Physiol Biochem: 14:113-20 (2004)] further investigated the physiological role of FXYD4 using knockout mice. They showed that cAMP-dependent ion transport and amiloride-sensitive sodium transport in colon was lower in FXYD4 (-/-) mice than in wild-type mice. Goldschmidt and coworkers concluded that FXYD4 likely modulates several different ion transport mechanisms indirectly via the sodium/potassium ATPase.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 15 | 42 | 15 | 72 |
| Expected | 18 | 36 | 18 | 72 |

Chi-Sq.= 4.44 Significance= 0.1086091 (hom/n)= 0.22 Avg. Litter Size= 8
Mutation Information
Mutation Type: Homologous Recombination (standard)
Description: Coding exons 1 through 4 were targeted (NCBI accession NM_03364S.1).
1. Wild-type Expression Panel: Expression of the target gene was detected in embryonic stem (ES) cells and in all 13 adult tissue samples tested by RT-PCR, except bone.
2. QC Expression: Disruption of the target gene was confirmed by Southern hybridization analysis.

### 46.21.1. PHENOTYPIC ANALYSIS (for disrupted gene: DNA59211-1450 (UNQ526)

### (a) OVERALL PHENOTYPIC SUMMARY:

Mutation of the gene encoding the ortholog of human FXYD domain-containing ion transport regulator 4 (FXYD4) resulted in a decreased skin fibroblast proliferation rate in female (-/-) mice. Gene disruption was confirmed by Southern blot.

### (b) Adult skin cell proliferation:

Procedure: Skin cells were isolated from 16 week old animals (2 wild type and 4 homozygotes). These were developed into primary fibroblast cultures and the fibroblast proliferation rates were measured in a strictly controlled protocol. The ability of this assay to detect hyper-proliferative and hypo-proliferative phenotypes has been demonstrated with p53 and Ku80. Proliferation was measured using Brdu incorporation.

Specifically, in these studies the skin fibroblast proliferation assay was used. An increase in the number of cells in a standardized culture was used as a measure of relative proliferative capacity. Primary fibroblasts were established from skin biopsies taken from wild type and mutant mice. Duplicate or triplicate cultures of 0.05 million cells were plated and allowed to grow for six days. At the end of the culture period, the number of cells present in the culture was determined using a electronic particle counter.

### Results:

Skin Proliferation: The female (-/-) mice exhibited a decreased mean skin fibroblast proliferation rate when compared with that of their gender-matched (+/+) littermates and the historical mean.

Thus, homozygous mutant mice demonstrated a hypo-proliferative phenotype. As suggested by these observations, antagonists or inhibitors of PRO1069 polypeptides would mimic this hypo-proliferative phenotype and could function as tumor suppressors and would be useful in decreasing abnormal cell proliferation.

### 46.22. Generation and Analysis of Mice Comprising DNA58721-1475 (UNQ554) Gene Disruptions

In these knockout experiments, the gene encoding PRO1111 polypeptides (designated as DNA58721-1475) (UNQ554) was disrupted. The gene specific information for these studies is as follows: the mutated mouse gene corresponds to nucleotide reference: NM_138682 ACCESSION:NM_138682 NID:20373168 Mus musculus Mus musculus LIBG-like protein (MBAG1); protein reference: Q8VI35 ACCESSION:Q8VI35 NID: Mus musculus (Mouse). BRAIN TUMOR-ASSOCIATED PROTEIN MBAG1; the human gene sequence reference: NM_022143 Homo sapiens leucine rich repeat containing 4 (LRRC4); the human protein sequence corresponds to reference: Q9HBW1 ACCESSION:Q9HBW1 NID: Homo sapiens (Human). BRAIN TUMOR ASSOCIATED PROTEIN NAG 14.

The mouse gene of interest is Lrrc4 (leucine rich repeat containing 4), ortholog ofhuman LRRC4. Aliases include MBAG1, Brain tumor associated protein LRRC4, and Nag14.

LRRC4 is a type I integral plasma membrane protein that likely functions as a as a ligand for lipid-anchored axon guidance molecule netrin-G 1. LRRC4 contains a signal peptide, several leucine-rich repeats, an Ig-like domain, a transmembrane segment, and a short C-terminal cytoplasmic domain. LRRC4 likely plays a role in promoting outgrowth and guidance of axons from thalamocortical neurons (Lin et al, Nat Neurosci: 6:1270-6 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 31 | 18 | 68 |
| Expected | 17 | 34 | 17 | 68 |

Chi-Sq.= 0.94 Significance= 0.62500226 (hom/n)= 0.24 Avg. Litter Size= 8
Mutation Information
Mutation Type: Homologous Recombination (standard)
Description: Coding exon 1 was targeted (NCBI accession NM_138682.1).
1. Wild-type Expression Panel: Expression of the target gene was detected in embryonic stem (ES) cells and in all 13 adult tissue samples tested by RT-PCR, except bone.
2. QC Expression: Disruption of the target gene was confirmed by Southern hybridization analysis.

### 46.22.1. PHENOTYPIC ANALYSIS (for disrupted gene: DNA58721-1475 (UNQ554)

### (a) OVERALL PHENOTYPIC SUMMARY:

Mutation of the gene encoding the ortholog of human leucine rich repeat containing 4 (LRRC4) resulted in a decreased skin fibroblast proliferation rate in female (-/-) mice. Most of the (-/-) mutant mice showed impaired hearing during pre-pulse inhibition testing. Gene disruption was confirmed by Southern blot.

### (b) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Prepulse inhibition of the acoustic startle reflex

Prepulse inhibition of the acoustic startle reflex occurs when a loud 120 decibel (dB) startle-inducing tone is preceded by a softer (prepulse) tone. The PPI paradigm consists of six different trial types (70 dB background noise, 120 dB alone, 74dB + 120 dB - pp4, 78 dB + 120 dB - pp8, 82 dB + 120 dB - pp12, and 90 dB+ 120 dB - pp20) each repeated in pseudorandom order six times for a total of 36 trials. The max response to the stimulus (V max) is averaged for each trial type. Animals with a 120 dB average value equal to or below 100 are excluded from analysis. The percent that the prepulse inhibits the animal's response to the startle stimulus is calculated and graphed.

### Results:

PPI: Most of the (-/-) mice failed to exhibit a startle response, suggesting impaired hearing in the mutants. Therefore, prepulse inhibition could not be assessed.

### (c) Adult skin cell proliferation:

Procedure: Skin cells were isolated from 16 week old animals (2 wild type and 4 homozygotes). These were developed into primary fibroblast cultures and the fibroblast proliferation rates were measured in a strictly controlled protocol. The ability of this assay to detect hyper-proliferative and hypo-proliferative phenotypes has been demonstrated with p53 and Ku80. Proliferation was measured using Brdu incorporation.

Specifically, in these studies the skin fibroblast proliferation assay was used. An increase in the number of cells in a standardized culture was used as a measure of relative proliferative capacity. Primary fibroblasts were established from skin biopsies taken from wild type and mutant mice. Duplicate or triplicate cultures of 0.05 million cells were plated and allowed to grow for six days. At the end of the culture period, the number of cells present in the culture was determined using a electronic particle counter.

### Results:

Skin Proliferation: The female (-/-) mice exhibited a decreased mean skin fibroblast proliferation rate when compared with that of their gender-matched (+/+) littermates and the historical mean.

Thus, homozygous mutant mice demonstrated a hypo-proliferative phenotype. As suggested by these observations, antagonists or inhibitors of PRO1111 polypeptides would mimic this hypo-proliferative phenotype and could function as tumor suppressors and would be useful in decreasing abnormal cell proliferation.

### 46.23. Generation and Analysis of Mice Comprising DNA57254-1477 (UNQ556) Gene Disruptions

In these knockout experiments, the gene encoding PRO1113 polypeptides (designated as DNA57254-1477) (UNQ556) was disrupted. The gene specific information for these studies is as follows: the mutated mouse gene corresponds to nucleotide reference: XM_132089 ACCESSION:XM_132089 NID: gi 51710991 ref XM_132089.3 PREDICTED: Mus musculus RIKEN cDNA 3830613022 gene (3830613O22Rik); protein reference: XP_132089 RIKEN cDNA 3830613022 [Mus musculus]; the human gene sequence reference: NM_145290 Homo sapiens G protein-coupled receptor 125 (GPR125); the human protein sequence corresponds to reference: Q8IWK6 ACCESSION:Q8IWK6 NID: Homo sapiens (Human). Probable G-protein coupled receptor 125 precursor (UNQ556/PRO1113).

The mouse gene of interest is Gpr125 (G protein-coupled receptor 125), ortholog of human GPR125. Aliases include 3830613O22Rik, PGR21, TEM5-like, and TEM5L.

GPR125 is an orphan G protein-coupled receptor of the secretin family (Fredriksson et al, Biochem Biophys Res Commun: 301:725-34 (2003)). Secretin family members include receptors for secretin, calcitonin, parathyroid hormone, parathyroid hormone-related peptides, and vasoactive intestinal peptide. All of these receptors activate adenylyl cyclase and phospholipase C signaling pathways (InterPro accession IPR000832). GPR125 is capable of binding with human homologue of Drosophila disc large tumor suppressor gene (DLG1), which functions as a scaffold for receptors and channels. GPR125 expressed in endothelial cells may play a role in tumor angiogenesis (Yamamoto et al, Oncogene: 23:3889-97 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 37 | 17 | 73 |
| Expected | 18.25 | 36.5 | 18.25 | 73 |

Chi-Sq.= 2.89 Significance= 0.23574607 (hom/n)= 0.2 Avg. Litter Size= 9
Mutation Information
Mutation Type: Homologous Recombination (standard)
Description: Coding exons 3 through 5 were targeted (NCBI accession BC052391).
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.23.1. PHENOTYPIC ANALYSIS (for disrupted gene: DNA57254-1477 (UNQ556)

### (a) OVERALL PHENOTYPIC SUMMARY:

Mutation of the gene encoding the ortholog of human G protein-coupled receptor 125 (GPR125) resulted in an increased absolute neutrophil count in the (-/-) mice. The (-/-) mice also exhibited an ocular infection. The homozygous mutant mice exhibited closed eyes that appeared to be crusted over by drainage and an increased median absolute neutrophil count, consistent with an ocular infection in the mutants. In addition, the female homozygous mutant mice exhibited decreased locomotor activity during home-cage activity testing when compared with that of their gender-matched wild-type littermates and the historical mean. There was a trend toward decreased body weight in the female (-/-) mice. Disruption of the target gene was confirmed by Southern hybridization analysis.

### (b) Pathology

Microscopic: Among the 6 (-/-) mice available for analysis, 5 exhibited a purulent exudate in the nasolacrimal ducts, consistent with the crusty eyes and increased tear formation observed clinically.
Gene Expression: LacZ activity was not detected in the panel of tissues by immunohistochemical analysis.
Obvious: The (-/-) mice exhibited closed eyes filled with apparent drainage.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The (-/-) mice exhibited an increased median absolute neutrophil count when compared with that of their (+/+) littermates and the historical mean, which could be contributed to the ocular infection noted in the mutants.

### (d) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The female (-/-) mice exhibited decreased ambulatory counts during the 12-hour habituation period and all light and dark periods when compared with that of their gender-matched (+/+) littermates and the historical mean.

These results are consistent with lethargy or depressive disorders. Antagonists or inhibitors of PRO1113 polypeptides or the PRO1113 encoding gene would be expected to mimic this behavior. Likewise, PRO1113 polypeptides or agonists thereof, would be useful in the treatment of such neurological disorders including depressive disorders or other decreased anxiety-like symptoms such as lethargy, cognitive disorders, hyperalgesia and sensory disorders.

### (e) Bone Metabolism & Body Diagnostics

### Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The female (-/-) mice exhibited decreased mean body weight when compared with that of their gender-matched (+/+) littermates and the historical mean.

Female mutant (-/-) mice deficient in the gene encoding PRO1113 polypeptides show a phenotype consistent with growth retardation, marked by decreased body weight. Thus, antagonists or inhibitors of PRO1113 polypeptides or its encoding gene would mimic these metabolic and growth related effects. On the other hand, PRO1113 polypeptides or agonists thereof would be useful in the prevention and/or treatment of such metabolic disorders as diabetes or other tissue wasting diseases.

### 46.24. Generation and Analysis of Mice Comprising DNA59814-1486 (UNQ567) Gene Disruptions

In these knockout experiments, the gene encoding PRO1130 polypeptides (designated as DNA59814-1486) (UNQ567) was disrupted. The gene specific information for these studies is as follows: the mutated mouse gene corresponds to nucleotide reference: NM_146050 ACCESSION:NM_146050 NID: gi 22164769 ref NM_146050.1 Mus musculus oncoprotein induced transcript 1 (Oitl), XM_214143.1 Rattus norvegicus similar to oncoprotein induced transcript 1 (LOC289949); protein reference: P97805 ACCESSION:P97805 NID: Mus musculus (Mouse). Protein FAM3D precursor (Oncoprotein-induced protein 1) (Protein EF- 7); the human gene sequence reference: NM_138805 Homo sapiens family with sequence similarity 3, member D (FAM3D); the human protein sequence corresponds to reference: Q96BQ1 ACCESSION:Q96BQ1 NID: Homo sapiens (Human). Protein FAM3D precursor.

The mouse gene of interest is Oitl (oncoprotein induced transcript 1), ortholog ofhuman FAM3D (family with sequence similarity 3, member D). Aliases include EF-7, EF7, MGC37550, and 2310076N21Rik.

FAM3D is a putative secreted protein that likely functions as a signal-transducing ligand. The 224-amino acid protein contains a signal peptide and four alpha helices linked together by disulfide bonds similar to a cystine knot, a structure common among secreted growth factors and cytokines. FAM3 D is expressed primarily in placenta (Zhu et al, Genomics: 80:144-50 (2002)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 17 | 37 | 20 | 74 |
| Expected | 18.5 | 37 | 18.5 | 74 |

Chi-Sq.= 2.36 Significance= 0.30727875 (hom/n)= 0.21 Avg. Litter Size= 9
Mutation Information
Mutation Type: Homologous Recombination (standard)
Description: Coding exons 1 and 2 were targeted (NCBI accession NM_146050.1).
1. Wild-type Expression Panel: Expression of the target gene was detected in eye; thymus; spleen; lung; kidney; and stomach, small intestine, and colon among the 13 adult tissue samples tested by RT-PCR.
2. QC Expression: Disruption of the target gene was confirmed by Southern hybridization analysis.

### 46.24.1. PHENOTYPIC ANALYSIS (for disrupted gene: DNA59814-1486 (UNQ567)

### (a) OVERALL PHENOTYPIC SUMMARY:

Mutation of the gene encoding the ortholog of human family with sequence similarity 3, member D (FAM3D) resulted in the male homozygous mutant mice exhibiting a decreased anxiety-like response during open field testing when compared with those of their gender-matched wild-type littermates and the historical means. Disruption of the target gene was confirmed by Southern hybridization analysis.

### (b) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Open field test:

Several targets ofknown drugs have exhibited phenotypes in the open field test. These include knockouts of the seratonin transporter, the dopamine transporter (Giros et al., Nature. 1996 Feb 15;379(6566):606-12), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value. Analyzed wt/ het/ hom: 5/ 4/ 8

### Results:

Openfield2: The male (-/-) mice exhibited an increased median sum time-in-center during open field testing when compared with that of their gender-matched (+/+) littermates and the historical mean, suggesting a decreased anxiety-like response in the mutants.

A notable difference was observed during open field activity testing. The (-/-) mice exhibited an increased median sum time in the center (with hypoactivity) when compared with their gender-matched (+/+) littermates, which is indicative of a decreased anxiety-like response in the mutants. Thus, knockout mice demonstrated a phenotype consistent with depression, generalized anxiety disorders, cognitive disorders, hyperalgesia and sensory disorders and/or bipolar disorders. Thus, PRO1130 polypeptides and agonists thereof would be useful for the treatment or amelioration of the symptoms associated with depressive disorders.

### 46.25. Generation and Analysis of Mice Comprising DNA65412-1523 (UNQ608) Gene Disruptions

In these knockout experiments, the gene encoding PRO1195 polypeptides (designated as DNA65412-1523) (UNQ608) was disrupted. The gene specific information for these studies is as follows: the mutated mouse gene corresponds to nucleotide reference: NM_009475 ACCESSION:NM_009475 NID: gi 6678510 ref NM_009475.1 Mus musculus uterine-specific proline-rich acidic protein (Upa); protein reference: Q60874 ACCESSION:Q60874 NID: Mus musculus (Mouse). Proline-rich acidic protein; the human gene sequence reference: NM_145202 Homo sapiens proline-rich acidic protein 1 (PRAPI); the human protein sequence corresponds to reference: Q96NZ9 ACCESSION:Q96NZ9 NID: Homo sapiens (Human). Proline-rich acidic protein 1.

The mouse gene of interest is Prap1 (proline-rich acidic protein 1), ortholog of human PRAP1. Aliases include Upa and PRO1195.

PRAP1 is a secreted protein that contains a signal peptide and a high content of proline and acidic amino acids. The protein is expressed in epithelial cells from several different tissues, including gastrointestinal tract, pregnant uterus, liver, kidney, and cervix. PRAP1 is down regulated in certain types of cancer and is capable of suppressing growth of cancer cell lines. Thus, PRAP1 may play a role in regulating growth of normal epithelia (Zhang et al, Cancer Res: 63:6658-65 (2003); Kasik and Rice, Am J Obstet Gynecol: 176:452-6 (1997)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 34 | 10 | 65 |
| Expected | 16.25 | 32.5 | 16.25 | 65 |

Chi-Sq.= 2.94 Significance= 0.22992548 (hom/n)= 0.23 Avg. Litter Size= 8
Mutation Information
Mutation Type: Homologous Recombination (standard)
Description: Coding exons 1 through 5 were targeted (NCBI accession NM_009475.1).
1. Wild-type Expression Panel: Expression of the target gene was detected in embryonic stem (ES) cells and in eye; thymus; kidney; stomach, small intestine, and colon; heart; and adipose among the 13 adult tissue samples tested by RT-PCR.
2. QC Expression: Disruption of the target gene was confirmed by Southern hybridization analysis.

### 46.25.1. PHENOTYPIC ANALYSIS (for disrupted gene: DNA65412-1523 (UNQ608)

### (a) OVERALL PHENOTYPIC SUMMARY:

Mutation of the gene encoding the ortholog of human proline-rich acidic protein 1 (PRAP1) resulted in an increased mean serum IgG2a response to ovalbumin challenge and increased serum immunoglobulin levels in (-/-) mice. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Serum Immunoglobulin Isotyping Assay:

The Serum Immunoglobulin Isotyping Assay is performed using a Cytometric Bead Array (CBA) kit. This assay is used to rapidly identify the heavy and light chain isotypes of a mouse monoclonal antibody in a single sample. The values expressed are "relative fluorescence units" and are based on the detection ofkappa light chains. Any value < 6 is not significant.

### Results:

Serum Imm. 2: The (-/-) mice exhibited increased mean serum IgM, IgG1, IgG2a, and IgG2b levels when compared with that of their (+/+) littermates and the historical mean.

Mutant (-/-) mice exhibited elevation of IgG1, IgG2a, IgG2b serum immunoglobulins as well as IgM immunoglobulins compared to their gender-matched (+/+) littermates. IgG immunoglobulins have neutralization effects and to a lesser extent are important for activation of the complement system. Mutant (-/-) mice also exhibited elevation of IgM serum immunoglobulins compared to their gender-matched (+/+) littermates. IgM immunoglobulins are the first to be produced in a humoral immune response for neutralization of bacterial toxins and are particularly important in activating the complement system. The observed phenotype suggests that the PRO1195 polypeptide is a negative regulator of serum immunoglobulins. These immunological abnormalities suggest that inhibitors (antagonists) of PRO1195 polypeptides would be important agents which could stimulate the immune system (such as T cell proliferation) and would find utility in the cases wherein this effect would be beneficial to the individual such as in the case of leukemia, and other types of cancer, and in immunocompromised patients, such as AIDS sufferers. Accordingly, PRO1195 polypeptides or agonists thereof would be useful in inhibiting the immune response and would be useful candidates for suppressing harmful immune responses, e.g. in the case of graft rejection or graft-versus-host diseases.

### Ovalbumin Challenge

Procedure: This assay was carried out on 7 wild types and 8 homozygotes. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immunodominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice exhibited an increased mean serum IgG2a response to ovalbumin challenge when compared with that of their (+/+) littermates and the historical mean.

In summary, the ovalbumin challenge studies indicate that knockout homozygous mice deficient in the gene encoding PRO1195 polypeptides exhibit immunological abnormalities when compared with their wild-type littermates. In particular, the mutant (-/-) mice exhibited an increased ability to elicit an immunological response when challenged with the T-cell dependent OVA antigen. Thus, antagonists (inhibitors) of PRO1195 polypeptides would be useful for stimulating the immune system (such as T cell proliferation) and would find utility in the cases wherein this effect would be beneficial to the individual such as in the case of leukemia, and other types of cancer, and in immuno-compromised patients, such as AIDS sufferers. Accordingly, PRO1195 polypeptides or agonists thereof, would be useful for inhibiting the immune response and thus would be useful candidates for suppressing harmful immune responses, e.g. in the case of graft rejection or graft-versus-host diseases.

### 46.26. Generation and Analysis of Mice Comprising DNA66309-1538 (UNQ641) Gene Disruptions

In these knockout experiments, the gene encoding PRO1271 polypeptides (designated as DNA66309-1538) (UNQ641) was disrupted. The gene specific information for these studies is as follows: the mutated mouse gene corresponds to nucleotide reference: NM_133739 ACCESSION:NM_133739 NID: gi 19526937 ref NM_133739.1 Mus musculus RIKEN cDNA 2310075C12 gene (2310075C12Rik); protein reference: Q91Z22 ACCESSION:Q91Z22 NID: Mus musculus (Mouse). Hypothetical 20.2 kDa protein; the human gene sequence reference: NM_052932 ACCESSION:NM_052932 NID: gi 16418408 ref NM_052932.1 Homo sapiens pro-oncosis receptor inducing membrane injury gene (PORIMIN); the human protein sequence corresponds to reference: Q96QV2 ACCESSION:Q96QV2 NID: Homo sapiens (Human). Porimin.

The mouse gene of interest is RIKEN cDNA 2310075C12 gene, ortholog of human PORIMIN (pro-oncosis receptor inducing membrane injury gene). Aliases include KCT3 and MGC102366.

PORIMIN is a type I integral plasma membrane protein that likely functions as a signal-transducing receptor or cell adhesion molecule. The protein contains a signal peptide, an extracellular domain with several potential O-linked and N-linked glycosylation sites, a transmembrane segment, and a short cytoplasmic C-terminus. Incubation of PORIMIN-expressing cells with a monoclonal antibody specifically reactive with PORIMIN induces oncosis-like cell (Zhang et al, Proc Natl Acad Sci U S A: 95:6290-5 (1998); Ma et al, Proc Natl Acad Sci U S A: 98:9778-83 (2001)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 26 | 33 | 17 | 76 |
| Expected | 19 | 38 | 19 | 76 |

Chi-Sq.= 0.72 Significance= 0.6976763 (hom/n)= 0.23 Avg. Litter Size= 9
Mutation Information
Mutation Type: Homologous Recombination (standard)
Description: Coding exons 3 through 5 were targeted (NCBI accession NM_133739.1).
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.26.1. PHENOTYPIC ANALYSIS (for disrupted gene: DNA66309-1538 (UNQ641)

### (a) OVERALL PHENOTYPIC SUMMARY:

Mutation of the gene encoding the ortholog of human pro-oncosis receptor inducing membrane injury gene (PORIMIN) resulted in the mutant (-/-) mice exhibiting hypoactivity during circadian rhythm testing. The mutant (-/-) mice also showed a trend toward increased body weight and length. Gene disruption was confirmed by Southern blot.

### (b) Bone Metabolism & Body Diagnostics

### Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The female (-/-) mice exhibited increased mean body weight when compared with that of their gender-matched (+/+) littermates and the historical mean.
Length: The female (-/-) mice exhibited increased mean body length when compared with that of their gender-matched (+/+) littermates and the historical mean.

These studies suggest that mutant (-/-) non-human transgenic animals exhibit a negative phenotype that would be associated with obesity. Thus, PRO1271 polypeptides or agonists thereof are essential for normal growth and metabolic processes and especially would be important in the prevention and/or treatment of obesity.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The female (-/-) mice exhibited decreased activity during the 10-hour habituation period when compared with that of their gender-matched (+/+) littermates and the historical mean.

These results are consistent with lethargy or depressive disorders. Antagonists or inhibitors of PRO1271 polypeptides or the PRO1271 encoding gene would be expected to mimic this behavior. Likewise, PRO1271 polypeptides or agonists thereof, would be useful in the treatment of such neurological disorders including depressive disorders or other decreased anxiety-like symptoms such as lethargy, cognitive disorders, hyperalgesia and sensory disorders.

### 46.27. Generation and Analysis of Mice Comprising DNA81757-2512 (UNQ856) Gene Disruptions

In these knockout experiments, the gene encoding PRO1865 polypeptides (designated as DNA81757-2512) (UNQ856) was disrupted. The gene specific information for these studies is as follows: the mutated mouse gene corresponds to nucleotide reference: NM_178382 Mus musculus fibronectin leucine rich transmembrane protein 3 (Flrt3); protein reference: Q8BGT1 ACCESSION:Q8BGT1 NID: Mus musculus (Mouse). Fibronectin leucine rich transmembrane protein 3 (Mus musculus 13 days embryo male testis cDNA, RIKEN full-length enriched library, clone:6030436M19 product:FIBRONECTIN LEUCINE RICH TRANSMEMBRANE PROTEIN 3 homolog); the human gene sequence reference: NM_013281 Homo sapiens fibronectin leucine rich transmembrane protein 3 (FLRT3), transcript variant 1; the human protein sequence corresponds to reference: Q9NZU0 ACCESSION:Q9NZUO NID: Homo sapiens (Human). Leucine-rich repeat transmembrane protein FLRT3 precursor (Fibronectin-like domain-containing leucine-rich transmembrane protein 3).

The mouse gene of interest is Flrt3 (fibronectin leucine rich transmembrane protein 3), ortholog ofhuman FLRT3. Aliases include mKIAA1469, 5530600M07Rik, and C430047I10Rik.

FLRTs are a family of putative type 1 TM proteins expressed in numerous tissues throughout embryonic development and in the adult. There are three family members in vertebrates, each containing nine leucine rich repeats, a fibronectin type III domain, a TM domain, and a short cytoplasmic tail.

FLRT3 is a type I integral plasma membrane protein that likely functions as a cell adhesion molecule or signal-transducing receptor. The protein contains a signal peptide, 10 leucine-rich repeats, a fibronectin domain, a transmembrane segment, and a short cytoplasmic C-terminus. FLRT3 is upregulated by nerve transection or similar nerve injury and overexpression studies suggest a role in promoting neurite outgrowth. FLRT3 is capable of interacting with fibroblast growth factor (FGF) receptor and augmenting FGF signaling. FLRT3 can also promote neurite outgrowth. FLRTs interact not only with FGFRs, but also with each other. Leucine rich repeats are required for this interaction and homotypic cell sorting. FLRT3 is expressed in several different tissues, including central and peripheral nervous systems, kidney, brain, pancreas, skeletal muscle, lung, liver, placenta, and heart. FLRT3 likely plays a role in nervous system development and peripheral nerve regeneration after injury (Lacy et al, Genomics: 62:417-26 (1999); Tsuji et al, Biochem Biophys Res Commun: 313:1086-91 (2004); Robinson et al, Mol Cell Neurosci: 27:202-14 (2004); Bottcher et al, Nat Cell Biol: 6:38-44 2004).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 36 | 2 | 56 |
| Expected | 14 | 28 | 14 | 56 |

Chi-Sq.= 28.64 Significance= 6.03814E-7 (hom/n)= 0.04 Avg. Litter Size= 7
Mutation Information
Mutation Type: Homologous Recombination (standard)
Description: Coding exon 1 was targeted (NCBI accession NM_178382.2).
1. Wild-type Expression Panel: Expression of the target gene was detected in embryonic stem (ES) cells and in all 13 adult tissue samples tested by RT-PCR, except skeletal muscle, bone, and adipose.
2. QC Expression: Disruption of the target gene was confirmed by Southern hybridization analysis.

### 46.27.1. PHENOTYPIC ANALYSIS (for disrupted gene: DNA81757-2512 (UNQ856)

### (a) OVERALL PHENOTYPIC SUMMARY:

Mutation of the gene encoding the ortholog of human fibronectin leucine rich transmembrane protein 3 (FLRT3) resulted in reduced viability of (-/-) mice. Of the 2 homozygous mutant mice born, 1 died during Level I testing. The homozygous mutant mice exhibited signs of growth retardation, including notably decreased mean body weight and length, total tissue mass, lean body mass, and decreased bone-related measurements. The homozygous mutants analyzed also exhibited numerous metabolic, immunological, and blood chemistry abnormalities. In addition, the female mutant exhibited a decreased skin fibroblast proliferation rate. Disruption of the target gene was confirmed by Southern hybridization analysis.

### (b) Pathology

Microscopic: Embryonic lethal. At 12.5 days, there were 44 embryos observed: 2 (-/-) embryos, 16 (+/-) embryos, 12 (+/+) embryos, and 14 resorption moles. However, no structural developmental abnormalities were detected in these 12.5 day embryos by histological examination.
Gene Expression: LacZ activity was detected in the testes among the panel of tissues analyzed by immunohistochemistry.
Obvious: Only 2 (-/-) mice were available for Level 1 analysis, and 1 died during testing.
Embryonic wholemount analysis of UNQ856: Our data suggests that there are numerous sites of overlapping expression of FLRT3 with FLIRT 1 and FLRT2 (midbrain isthmus and zona limitans, somites, muscle, eye, nipples and hair follicles. This broad "vascular" expression is due to lacZ expression in blood cells. Lethality at 8.5-9.5 dpc is likely due to mesoderm insufficiency or migration defects. UNQ856 mutants fail to undergo ventral closure and body rotation. This phenotype is highly penetrant.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Serum Immunoglobulin Isotyping Assay:

The Serum Immunoglobulin Isotyping Assay is performed using a Cytometric Bead Array (CBA) kit. This assay is used to rapidly identify the heavy and light chain isotypes of a mouse monoclonal antibody in a single sample. The values expressed are "relative fluorescence units" and are based on the detection ofkappa light chains. Any value < 6 is not significant.

### Results:

Serum Imm. 2: The single (-/-) mouse exhibited an increased mean serum IgG2b level when compared with that of its (+/+) littermates, the (+/+) mice within the project run, and the historical mean.

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.
Hematology: The (-/-) mouse exhibited an increased mean red cell distribution width and platelet count when compared with those of its (+/+) littermates and the historical means.

Thus, mutant mice deficient in the DNAB1757-2512 gene resulted in a phenotype related to coagulation disorders.

### Flourescence-activated cell-sorting (FA CS) Analysis

### Procedure:

FACS analysis of immune cell composition from peripheral blood was performed including CD4, CD8 and T cell receptor to evaluate T lymphocytes, CD19 for B lymphocytes, CD45 as a leukocyte marker and pan NK for natural killer cells. The FACS analysis was carried out on 2 wild type mice and 1 homozygous mouse and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACSCalibur flow cytometer with CellQuest software.

### Results:

FACS3: The single (-/-) mouse analyzed exhibited an altered distribution of leukocyte subsets in the peripheral blood, characterized by an increased mean percentage of B cells when compared with that of its (+/+) littermates and the historical mean. Thus, PRO1865 polypeptides or agonists thereof function as a negative regulator of B cell production.

### (d) Bone Metabolism & Body Diagnostics

### (1) Tissue Mass & Lean Body Mass Measurements - Dexa

### Dexa Analysis - Test Description:

Procedure: A cohort of wild type, heterozygotes and two homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The (-/-) mice exhibited decreased mean body weight when compared with that of their gender-matched (+/+) littermates and the historical mean.
Length: The (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygotes and one homozygote were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of wild type and one homozygous mouse. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mouse exhibited notably decreased mean total tissue mass, lean body mass, and bone mineral content and density measurements when compared with those of its gender-matched (+/+) littermates and the historical means.
micro CT: The male (-/-) mouse exhibited notably decreased mean femoral mid-shaft cortical thickness and cross-sectional area when compared with those of its gender-matched (+/+) littermates and the historical means. Blood Pressure: The (-/-) mouse exhibited decreased systolic blood pressure when compared with that of their gender-matched (+/+) littermates and the historical mean.

The mutant (-/-) mouse deficient in the gene encoding PRO1865 polypeptides show a phenotype consistent with growth retardation, marked by decreased body weight and length. Thus, antagonists or inhibitors of PRO1865 polypeptides or its encoding gene would mimic these metabolic and growth related effects. On the other hand, PRO1865 polypeptides or agonists thereof would be useful in the prevention and/or treatment of such metabolic disorders as diabetes or other tissue wasting diseases.

In addition, the (-/-) mouse analyzed by DEXA and micro CT exhibited decreased bone measurements and decreased body mass measurements when compared with their (+/+) littermates, suggestive of abnormal bone disorders. Thus, the (-/-) mouse exhibited a negative bone phenotype. In addition, the decreased mean total tissue mass and lean body mass is indicative of a metabolic disorder related to growth retardation and tissue wasting disorders. The negative bone phenotype indicates that PRO1865 polypeptides or agonists thereof would be useful for maintaining bone homeostasis in addition to normal growth development. In addition, PRO1865 polypeptides would be useful in bone healing or for the treatment of arthritis or osteoporosis, whereas antagonists (or inhibitors) of PRO1865 polypeptides or its encoding gene would lead to abnormal or pathological bone disorders including inflammatory diseases associated with abnormal bone metabolism including arthritis, osteoporosis and osteopenia.

### (e) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: The single (-/-) mouse analyzed exhibited increased mean serum albumin, alanine amino transferase, and phosphorus levels when compared with those of its (+/+) littermates and the historical means. Albumin levels were very high indicative of severe dehydration.
Oral Glucose: The single male (-/-) mouse available for analysis exhibited a decreased mean fasting serum glucose level when compared with that of its gender-matched (+/+) littermates and the historical mean.

### (f) Adult skin cell proliferation:

Procedure: Skin cells were isolated from 16 week old animals (2 wild type and 1 homozygous mouse). These were developed into primary fibroblast cultures and the fibroblast proliferation rates were measured in a strictly controlled protocol. The ability ofthis assay to detect hyper-proliferative and hypo-proliferative phenotypes has been demonstrated with p53 and Ku80. Proliferation was measured using Brdu incorporation.

Specifically, in these studies the skin fibroblast proliferation assay was used. An increase in the number of cells in a standardized culture was used as a measure of relative proliferative capacity. Primary fibroblasts were established from skin biopsies taken from wild type and mutant mice. Duplicate or triplicate cultures of 0.05 million cells were plated and allowed to grow for six days. At the end of the culture period, the number of cells present in the culture was determined using a electronic particle counter.

### Results:

Skin Proliferation: The female (-/-) mouse exhibited a decreased skin fibroblast proliferation rate when compared with that of its (+/+) littermates and the historical mean.

Thus, homozygous mutant mouse demonstrated a hypo-proliferative phenotype. As suggested by these observations, antagonists or inhibitors of PRO1865 polypeptides would mimic this hypo-proliferative phenotype.

### (g) Cardiovascular Phenotypic Analysis:

In the area of cardiovascular biology, phenotypic testing was performed to identify potential targets for the treatment of cardiovascular, endothelial or angiogenic disorders. One such phenotypic test included optic fundus photography and angiography to determine the retinal arteriovenous ratio (A/V ratio) in order to flag various eye abnormalities. An abnormal A/V ratio signals such systemic diseases or disorders that may be related to the vascular disease of hypertension (and any disease that causes hypertension, e.g. atherosclerosis), diabetes or other ocular diseases corresponding to ophthalmological disorders. Such eye abnormalities may include but are not limited to the following: retinal abnormality is retinal dysplasia, various retinopathies, restenosis, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

Procedure: A cohort of 4 wild type, 4 heterozygotes and one homozygote were tested in this assay. Optic fundus photography was performed on conscious animals using a Kowa Genesis small animal fundus camera modified according to Hawes and coauthors (Hawes et al., 1999 Molecular Vision 1999; 5:22). Intra-peritoneal injection of fluorescein permitted the acquisition of direct light fundus images and fluorescent angiograms for each examination. In addition to direct ophthalmological changes, this test can detect retinal changes associated with systemic diseases such as diabetes and atherosclerosis or other retinal abnormalities. Pictures were provided of the optic fundus under normal light. The angiographic pictures allowed examination of the arteries and veins of the eye. In addition an artery to vein (A/V) ratio was determined for the eye.

Ophthalmology analysis was performed on generated F2 wild type, heterozygous, and homozygous mutant progeny using the protocol described above. Specifically, the A/V ratio was measured and calculated according to the fundus images with Kowa COMIT+ software. This test takes color photographs through a dilated pupil: the images help in detecting and classifying many diseases. The artery to vein ratio (A/V) is the ratio of the artery diameter to the vein diameter (measured before the bifurcation of the vessels). Many diseases will influence the ratio, i.e., diabetes, cardiovascular disorders, papilledema, optic atrophy or other eye abnormalities such as retinal degeneration (known as retinitis pigmentosa) or retinal dysplasia, vision problems or blindness. Thus, phenotypic observations which result in an increased artery-to-vein ratio in homozygous (-/-) and heterozygous (+/-) mutant progeny compared to wild-type (+/+) littermates would be indicative of such pathological conditions.

### Results:

Fundus: The (-/-) mouse analyzed exhibited a bilateral optic disc lesion.

### 46.28. Generation and Analysis of Mice Comprising DNA54009-2517 (UNQ863) Gene Disruptions

In these knockout experiments, the gene encoding PR01879 polypeptides (designated as DNA54009-2517) (UNQ863) was disrupted. The gene specific information for these studies is as follows: the mutated mouse gene corresponds to nucleotide reference: BC033452 Mus musculus solute carrier family 30 (zinc transporter), member 5, mRNA (cDNA clone MGC:27783 IMAGE:3156622); protein reference: Q8R4H9 ACCESSION:Q8R4H9 NID: Mus musculus (Mouse). Zinc transporter 5 (Similar to zinc transporter ZTL1); the human gene sequence reference: NM_022902 ACCESSION:NM_022902 NID: gi 20070322 refNM_022902.2 Homo sapiens solute carrier family 30 (zinc transporter), member 5 (SLC30A5); the human protein sequence corresponds to reference: Q8TAD4 ACCESSION:Q8TAD4 NID: Homo sapiens (Human). Zinc transporter 5 (Zinc transporter ZnT-5).

The mouse gene of interest is S1c30a5 (solute carrier family 30 [zinc transporter], member 5), ortholog of human SLC30A5. Aliases include ZNT5, ZTL1, ZnT-5, Zntl1, 1810010K08Rik, MGC5499, FLJ12496, and FLJ12756.

SLC30A5 is an integral membrane protein that functions as a zinc transporter. Expression of SLC30A5 is apparently ubiquitous but is most high on pancreatic beta cell secretory granules, which contain a high concentration of zinc. SLC30A5 is also expressed on the apical surface of enterocytes, where it likely plays a role in absorption of dietary zinc (Kambe et al, J Biol Chem: 277:19049-55 (2002); Cragg et al, J Biol Chem: 277:22789-97 (2002)). SLC30A5 is also located on the trans-Golgi network and is upregulated by low levels of zinc (Devergnas et al, Biochem Pharmacol: 68:699-709 (2004)).

Inoue and colleagues [Hum Mol Genet: 11:1775-84 (2002)] investigated the physiological role of SLC30A5 using knockout mice. SLC30A5 homozygous null mice displayed poor growth and impaired osteoblast maturation, resulting in decreased bone density. Moreover, 60% of the male SLC30A5 homozygous null mice died suddenly because of bradyarrhythmia. Inoue and coworkers concluded that SLC30A5 probably plays an important role in maturation of osteoblasts and maintenance of cells of the cardiac conduction system.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 44 | 20 | 85 |
| Expected | 21.25 | 42.5 | 21.25 | 85 |

Chi-Sq.= 1.21 Significance= 0.5460744 (hom/n)= 0.27 Avg. Litter Size= 9
Mutation Information
Mutation Type: Homologous Recombination (standard)
Description: Coding exons 9 through 13 were targeted (NCBI accession NM_022885.1).
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.28.1. PHENOTYPIC ANALYSIS (for disrupted gene: DNA54009-2517 (UNQ863)

### (a) OVERALL PHENOTYPIC SUMMARY:

Mutation of the gene encoding the ortholog of human solute carrier family 30 (zinc transporter), member 5 (SLC30A5) resulted in small (-/-) mice. The homozygous mutant mice were smaller than their gender-matched wild-type littermates, the difference being more notable in the males. In addition, the homozygous mice exhibited decreased total tissue mass, lean body mass, and decreased bone-related measurements. The mutants also exhibited impaired motor strength/coordination during inverted screen testing. Male infertility was noted in the mutant homozygotes. In addition, the (-/-) mice exhibited an increased total white blood cell (WBC) and absolute lymphocyte count. Disruption of the target gene was confirmed by Southern hybridization analysis.

### (b) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder nototherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Inverted Screen Testing:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Inverted Screen Test Data:

The Inverted Screen is used to measure motor strength/coordination. Untrained mice were placed individually on top of a square (7.5 cm x 7.5 cm) wire screen which was mounted horizontally on a metal rod. The rod was then rotated 180 degrees so that the mice were on the bottom of the screens. The following behavioral responses were recorded over a 1 min testing session: fell off, did not climb, and climbed up.

### Results:

| Genotype | Ratio Fell Down % | | Ratio Climbed up % | |
|---|---|---|---|---|
| +/+ (n=8) | 1/8 | 13 | 7/8 | 87.5 |
| -/- (n=8) | 8/8 | 100 | 0/8 | 0 |

A motor strength deficit is apparent when there is a 50% point difference between (-/-) or (+/-) mice and (+/+) mice for the fell down response. 0/8 or 1/8 (-/-) or (+/-) mice not climbing indicates impaired motor coordination. 7/8 or 8/8(-/-) or (+/-) mice climbing up indicates enhanced motor coordination.

The Inverted Screen Test is designed to measure basic sensory & motor observations:
Inverted Screen: All 8 (-/-) mice fell off the inverted screen whereas only 1/8 of the (+/+) mice fell off, suggesting impaired motor strength/coordination in the mutants.

### (c) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The (-/-) mice exhibited decreased mean body weight when compared with that of their gender-matched (+/+) littermates and the historical mean.
Length: The (-/-) mice exhibited decreased mean body length when compared with that of their gender-matched (+/+) littermates and the historical mean.
Fertility: The male (-/-) mouse analyzed produced no pups after 40 days of breeding.
Blood Pressure: The female (-/-) mice exhibited decreased mean systolic blood pressure when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean total tissue mass and lean body mass when compared with those of their gender-matched (+/+) littermates and the historical means. Both male and female (-/-)mice exhibited decreased mean bone mineral content and density measurements. The female (-/-) mice showed a decreased BMC/LBM ratio.
micro CT: The male (-/-) mice exhibited notably decreased mean vertebral trabecular bone measurements and notably decreased mean femoral mid-shaft cortical thickness and cross-sectional area when compared with those of their gender-matched (+/+) littermates and the historical means.

Mutant (-/-) mice deficient in the gene encoding PRO1879 polypeptides show a phenotype consistent with growth retardation, marked by decreased body weight and length. Thus, antagonists or inhibitors of PRO1879 polypeptides or its encoding gene would mimic these metabolic and growth related effects. On the other hand, PRO1879 polypeptides or agonists thereof would be useful in the prevention and/or treatment of such metabolic disorders as diabetes or other tissue wasting diseases.

In addition, the (-/-) mice analyzed by DEXA and micro CT exhibited decreased bone measurements and decreased body mass measurements when compared with their (+/+) littermates, suggestive of abnormal bone disorders. Thus, the (-/-) mice exhibited a negative bone phenotype. In addition, the decreased mean total tissue mass and lean body mass is indicative of a metabolic disorder related to growth retardation and tissue wasting disorders. The negative bone phenotype indicates that PRO1879 polypeptides or agonists thereof would be useful for maintaining bone homeostasis in addition to normal growth development. In addition, PRO1879 polypeptides would be useful in bone healing or for the treatment of arthritis or osteoporosis, whereas antagonists (or inhibitors) of PRO1879 polypeptides or its encoding gene would lead to abnormal or pathological bone disorders including inflammatory diseases associated with abnormal bone metabolism including arthritis, osteoporosis and osteopenia.

### (d) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Hematology Analysis:

Test Description: Blood tests are carried out by Abbott's Cell-Dyn 3500R, an automated hematology analyzer. Some of its features include a five-part WBC differential. 'Patient'reports can cover over 22 parameters in all.
Hematology: The (-/-) mice exhibited an increased mean total white blood cell and absolute lymphocyte counts when compared with those of their (+/+) littermates and the historical means.

### 46.29. Generation and Analysis of Mice Comprising DNA92219-2541 (UNQ1833) Gene Disruptions

In these knockout experiments, the gene encoding PR03446 polypeptides (designated as DNA92219-2541) (UNQ 1833) was disrupted. The gene specific information for these studies is as follows: the mutated mouse gene corresponds to nucleotide reference: AK012157 Mus musculus 10 days embryo whole body cDNA, RIKEN full-length enriched library, clone:2610528A11 product: unknown EST, full insert sequence; protein reference: CAD20705 ACCESSION:CAD20705 NID: Mus musculus unnamed protein product genpept; the human gene sequence reference: NM_207373 Homo sapiens chromosome 10 open reading frame 99 (C10orf99); the human protein sequence corresponds to reference: NP_997256 chromosome 10 open reading frame 99 [Homo sapiens].

The mouse gene of interest is RIKEN cDNA 2610528A11 gene, ortholog of human C10orf99 (chromosome 10 open reading frame 99). Aliases include UNQ1833 and FLJ21763.

C10orf99 is a putative secreted protein (Clark et al, Genome Res: 13:2265-70 (2003)). The protein consists of 81 amino acids and contains a signal peptide.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 36 | 23 | 77 |
| Expected | 19.25 | 38.5 | 19.25 | 77 |

Chi-Sq.= 0.73 Significance= 0.69419664 (hom/n)= 0.27 Avg. Litter Size= 9
Mutation Information
Mutation Type: Homologous Recombination (standard)
Description: Coding exons 1 and 2 were targeted (NCBI accession AK012157).
1. Wild-type Expression Panel: Expression of the target gene was detected in embryonic stem (ES) cells and in all 13 adult tissue samples tested by RT-PCR, except liver, skeletal muscle, bone, and heart.
2. QC Expression: Disruption of the target gene was confirmed by Southern hybridization analysis.

### 46.29.1. PHENOTYPIC ANALYSIS (for disrupted gene: DNA92219-2541 (UNQ1833)

### (a) OVERALL PHENOTYPIC SUMMARY:

Mutation of the gene encoding the ortholog of human chromosome 10 open reading frame 99 (C10orf99) resulted in the (-/-) mice showing decreased body lengths. Gene disruption was confirmed by Southern blot.

### (b) Bone Metabolism & Body Diagnostics

### Tissue Mass & Lean Body Mass Measurements - Dexa

### Dexa Analysis - Test Description:

Procedure: A cohort of wild type, heterozygotes and two homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

The mutant (-/-) mice exhibited decreased body length measurements when compared with their wild-type (+/+) littermate controls and the historical means. Thus, the mutant (-/-) mice showed growth retardation.

### 46.30. Generation and Analysis of Mice Comprising DNA86571-2551 (UNQ1835) Gene Disruptions

In these knockout experiments, the gene encoding PR03543 polypeptides (designated as DNA86571-2551) (UNQ 1835) was disrupted. The gene specific information for these studies is as follows: the mutated mouse gene corresponds to nucleotide reference: NM_009979 ACCESSION:NM_009979 NID: gi 6753545 ref NM_009979.1 Mus musculus cystatin 9 (Cst9); protein reference: Q9Z0H6 ACCESSION:Q9Z0H6 NID: Mus musculus (Mouse). Cystatin 9 precursor (Testatin); the human gene sequence reference: NM_080610 ACCESSION:NM_080610 NID: gi 18104939 refNM_080610.1 Homo sapiens cystatin 9-like (mouse) (CST9L); the human protein sequence corresponds to reference: Q9H4G1 ACCESSION:Q9H4G1 NID: Homo sapiens (Human). Cystatin 9-like precursor.

The mouse gene of interest is Cst9 (cystatin 9), ortholog of human CST9L (cystatin 9-like [mouse]). Aliases include testatin, M12, and bA218C14.1.

CST9L is a secreted protein expressed primarily in fetal gonads and in adult testis (Tohonen et al, 1998; Eriksson et al, 2002). The protein belongs to the cystatin family of protease inhibitors, containing a signal peptide and a cystatin domain (PFAM accession PF00031) that appears to lack amino acids critical for activity. In adult animals, CST9L is expressed in Sertoli cells and pro-spermatogonia (Kanno et al, 1999). CST9L likely plays a role in reproduction (Cornwall and Hsia, 2003).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 13 | 40 | 18 | 71 |
| Expected | 17.75 | 35.5 | 17.75 | 71 |

Chi-Sq.= 6.91 Significance= 0.031587306 (hom/n)=0.29 Avg. Litter Size= 8
Mutation Information
Mutation Type: Homologous Recombination (standard)
Description: Coding exons 1 and 2 were targeted (NCBI accession NM_009979.1).
1. Wild-type Expression Panel: Expression of the target gene was detected in eye, thymus, lung, heart, and adipose among the 13 adult tissue samples tested by RT-PCR.
   Expression of the target gene was detected in embryonic stem (ES) cells and in all 13 adult tissue samples tested by RT-PCR.
2. QC Expression: Disruption of the target gene was confirmed by Southern hybridization analysis.

### 46.30.1. PHENOTYPIC ANALYSIS (for disrupted gene: DNA86571-2551 (UNQ1835)

### (a) OVERALL PHENOTYPIC SUMMARY:

Mutation of the gene encoding the ortholog of human cystatin 9-like (mouse) (CST9L) resulted in the mutant (-/-) mice exhibiting increased bone mineral density measurements. Gene disruption was confirmed by Southern blot.

### (b) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

micro CT: The male (-/-) mice exhibited an increased mean femoral mid-shaft cortical thickness when compared with that of their gender-matched (+/+) littermates and the historical mean.

In summary, the (-/-) mice exhibited increased femoral bone measurements when compared with their gender-matched (+/+) littermates. These results indicate that the knockout mutant phenotype can be associated with such bone abnormalities as osteopetrosis. Osteopetrosis is a condition characterized by abnormal thickening and hardening of bone and abnormal fragility of the bones. As such, PR03543 polypeptides or agonists thereof would be beneficial for the treatment ofosteopetrosis or other osteo-related diseases. On the other hand, inhibitors or antagonists of PR03543 polypeptides would be useful in bone healing.

### 46.31. Generation and Analysis of Mice Comprising DNA77629-2573 (UNQ1885) Gene Disruptions

In these knockout experiments, the gene encoding PR04329 polypeptides (designated as DNA77629-2573) (UNQ1885) was disrupted. The gene specific information for these studies is as follows: the mutated mouse gene corresponds to nucleotide reference: NM_177193 Mus musculus RIKEN cDNA B930052A04 gene (B930052A04Rik); protein reference: Q5RKR3 ACCESSION:Q5RKR3 NID: Mus musculus (Mouse). RIKEN cDNA B930052A04 gene; the human gene sequence reference: NM_020851 Homo sapiens KIAA1465 protein (KIAA1465); the human protein sequence corresponds to reference: Q6UXK2 ACCESSION:Q6UXK2 NID: Homo sapiens (Human). FPLR1885.

The mouse gene of interest is RIKEN cDNA B930052A04 gene, ortholog of human KIAA 1465 protein. Aliases include mKIAA1465 and KIAA1465.

KIAA1465 protein (Okazaki et al, DNA Res: 10: 167-80 (2003)) is a putative plasma membrane protein (Clark et al, Genome Res: 13:2265-70 (2003)), containing a signal peptide, several leucine-rich repeats, an immunoglobulin (Ig)-like domain, a transmembrane segment, and a cytoplasmic C-terminal domain. The extracellular leucine-rich repeats and Ig-like domain are often involved in protein-protein interactions. This domain organization and predicted membrane topology suggests that KIAA1465 protein functions as a cell adhesion molecule or signal-transducing receptor.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 5 | 17 | 0 | 22 |
| Expected | 5.5 | 11 | 5.5 | 22 |

Chi-Sq.= 5.47 Significance= 0.06489401 (hom/n)= 0.14 Avg. Litter Size= 7
Mutation Information
Mutation Type: Homologous Recombination (standard)
Description: The gene consists of 2 exons, with the start codon located in exon 2 (NCBI accession NM_177193.4). Exon 2 was targeted.
1. Wild-type Expression Panel: Expression of the target gene was detected in embryonic stem (ES) cells and in brain, spinal cord, eye, and spleen among 13 adult tissue samples tested by RT-PCR.
2. QC Expression: Disruption of the target gene was confirmed by Southern hybridization analysis.

### 46.31.1. PHENOTYPIC ANALYSIS (for disrupted gene: DNA77629-2573 (UNQ1885)

### (a) OVERALL PHENOTYPIC SUMMARY:

Mutation of the gene encoding the ortholog of a human hypothetical protein (KIAA1465) resulted in lethality of (-/-) mice. The heterozygous mutant mice exhibited signs of growth retardation, including decreased mean body weight and length, total tissue mass, lean body mass, bone mineral content, total body bone mineral density and vertebrae bone mineral density. In addition, the heterozygous mice exhibited an increased serum alanine amino transferase level, decreased tear production, and neurological abnormalities when compared with that of their wild-type littermates and the historical means. The heterozygous mice were also poor breeders, requiring foster mothers and exhibiting fight wounds and reduced litter sizes. Disruption of the target gene was confirmed by Southern hybridization analysis.

### Discussion related to embryonic developmental abnormality of lethality:

Embryonic lethality in knockout mice usually results from various serious developmental problems including but not limited to neuro-degenerative diseases, angiogenic disorders, inflammatory diseases, or where the gene/protein has an important role in basic cell signaling processes in many cell types. In addition, embryonic lethals are useful as potential cancer models. Likewise, the corresponding heterozygous (+/-) mutant animals are particularly useful when they exhibit a phenotype and/or a pathology report which reveals highly informative clues as to the function of the knocked-out gene. For instance, EPO knockout animals were embryonic lethals, but the pathology reports on the embryos showed a profound lack ofRBCs.

### (b) Pathology

Microscopic: Due to embryonic lethality, microscopic analysis was not performed. At 12.5 days there were 23 embryos observed: 4 (-/-) embryos, 9 (+/-) embryos, 6 (+/+) embryos, and 4 resorption moles. The histology images suggested a possible hydrocephalus.
Gene Expression: LacZ activity was not detected in the panel of tissues by immunohistochemical analysis.

### (c) Bone Metabolism & Body Diagnostics

### (1) Tissue Mass & Lean Body Mass Measurements - Dexa

### Dexa Analysis - Test Description:

Procedure: A cohort of wild type and heterozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PDXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Obvious: The (+/-) mice exhibited apparent breeding problems and reduced litter sizes. The mothers cannibalized their pups and were lactation-deficient, requiring foster mothers for the neonates. Several male and female (+/-) mice were euthanized due to fight wounds inflicted during breeding, and many female (+/-) mice never became pregnant.
Weight: The (+/-) mice exhibited decreased mean body weight when compared with that of their gender-matched (+/+) littermates and the historical mean.
Length: The (+/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type and heterozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of wild type and heterozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: Both the male and female (+/-) mice exhibited decreased mean total tissue mass, lean body mass, and bone mineral content, total body vBMD, total body bone mineral density and vertebrae bone mineral density measurements when compared with those of their gender-matched (+/+) littermates and the historical means.
micro CT: The male (+/-) mice exhibited decreased mean femoral mid-shaft cortical thickness and cross-sectional area when compared with those of their gender-matched (+/+) littermates and the historical means.

Mutant (+/-) mice deficient in the gene encoding PR04329 polypeptides show a phenotype consistent with growth retardation, marked by decreased body weight and length. Thus, antagonists or inhibitors of PR04329 polypeptides or its encoding gene would mimic these metabolic and growth related effects. On the other hand, PR04329 polypeptides or agonists thereof would be useful in the prevention and/or treatment of such metabolic disorders as diabetes or other tissue wasting diseases.

In addition, the (+/-) mice analyzed by DEXA and micro CT exhibited decreased bone measurements and decreased body mass measurements when compared with their (+/+) littermates, suggestive of abnormal bone disorders. Thus, the (+/-) mice exhibited a negative bone phenotype. In addition, the decreased mean total tissue mass and lean body mass is indicative of a metabolic disorder related to growth retardation and tissue wasting disorders. The negative bone phenotype indicates that PR04329 polypeptides or agonists thereof would be useful for maintaining bone homeostasis in addition to normal growth development. In addition, PR04329 polypeptides would be useful in bone healing or for the treatment of arthritis or osteoporosis, whereas antagonists (or inhibitors) of PR04329 polypeptides or its encoding gene would lead to abnormal or pathological bone disorders including inflammatory diseases associated with abnormal bone metabolism including arthritis, osteoporosis and osteopenia.

### (d) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type and heterozygous mutant mice. All behavioral tests were done between 12 and 16 weeks of age unless reduced viability necessitates earlier testing. These tests included open field to measure anxiety, activity levels and exploration.

### Open field test:

Several targets ofknown drugs have exhibited phenotypes in the open field test. These include knockouts of the seratonin transporter, the dopamine transporter (Giros et al., Nature. 1996 Feb 15;379(6566):606-12), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value.

### Results:

Openfield2: The (+/-) mice exhibited increased sum total distance and decreased sum center-to-total distance when compared with those of their (+/+) littermates and the historical mean, suggesting increased exploratory behavior and an abnormal exploratory response in the mutants. The (+/-) mice also exhibited an increased median normalized slope, suggesting an abnormal habituation response. Thus, the (+/-) mice exhibited a notably high anxiety response marked by excessive hyperactivity.

In summary, open field testing revealed a phenotype associated with increased anxiety in the heterozygotes which could be associated with mild to moderate anxiety, anxiety due to a general medical condition, and/or bipolar disorders; hyperactivity; sensory disorders; obsessive-compulsive disorders, schizophrenia or a paranoid personality.

### (e) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: The (+/-) mice exhibited an increased median serum alanine amino transferase level when compared with that of their (+/+) littermates and the historical mean.

### 46.32. Generation and Analysis of Mice Comprising DNA87976-2593 (UNQ1906) Gene Disruptions

In these knockout experiments, the gene encoding PR04352 polypeptides (designated as DNA87976-2593) (UNQ1906) was disrupted. The gene specific information for these studies is as follows: the mutated mouse gene corresponds to nucleotide reference: NM_053143 ACCESSION:NM_053143 NID: gi 18087796 ref NM_053143.1 Musmusculusprotocadherinbeta 18 (Pcdhb18); protein reference:Q91Y02 ACCESSION:Q91Y02 NID: Mus musculus (Mouse). Protocadherin beta 18; the human gene sequence reference: NM_018930 ACCESSION:NM_018930 NID: gi 52486036 ref NM_018930.3 Homo sapiens protocadherin beta 10 (PCDHB 10); the human protein sequence corresponds to reference:Q9UN67 ACCESSION:Q9UN67 NID: Homo sapiens (Human). Protocadherin beta 10 precursor (PCDH-beta10).

The mouse gene of interest is Pcdhb18 (protocadherin beta 18), ortholog of human PCDHB10 (protocadherin beta 10). Aliases include Pcdhb9, PcdhbR, and PCDH-BETA10.

PCDHB10 is a type I integral plasma membrane protein of the cadherin superfamily that likely functions as a cell adhesion molecule. The protein contains a signal peptide, six cadherin domains, a transmembrane segment, and a short cytoplasmic tail. PCDHB10 is likely to bind with other PCDHB10 molecules expressed on other cells and to play a role in morphogenesis and synaptic circuit formation (Frank and Kemler, Curr Opin Cell Biol: 14:557-62 (2002); Nollet et al, J Mol Biol: 299:551-72 (2000); Wu et al, Genome Res: 11:389-404 (2001); Yagi and Takeichi, Genes Dev: 14:1169-80 (2000)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 24 | 19 | 62 |
| Expected | 15.5 | 31 | 15.5 | 62 |

Chi-Sq.= 1.11 Significance= 0.57407224 (hom/n)= 0.28 Avg. Litter Size= 9
Mutation Information
Mutation Type: Homologous Recombination (standard)
Description: Coding exon 1 was targeted (NCBI accession NM_053143.1).
1. Wild-type Expression Panel: Expression of the target gene was detected in all 13 adult tissue samples tested by RT-PCR, except skeletal muscle; bone; stomach, small intestine, and colon; and adipose.
2. QC Expression: Disruption of the target gene was confirmed by Southern hybridization analysis.

### 46.32.1. PHENOTYPIC ANALYSIS (for disrupted gene: DNA87976-2593 (UNQ1906)

### (a) OVERALL PHENOTYPIC SUMMARY:

Mutation of the gene encoding the ortholog of human protocadherin beta 10 (PCDHB10) resulted in a decreased skin fibroblast proliferation rate in female (-/-) mice. Gene disruption was confirmed by Southern blot.

### (b) Adult skin cell proliferation:

Procedure: Skin cells were isolated from 16 week old animals (2 wild type and 4 homozygotes). These were developed into primary fibroblast cultures and the fibroblast proliferation rates were measured in a strictly controlled protocol. The ability of this assay to detect hyper-proliferative and hypo-proliferative phenotypes has been demonstrated with p53 and Ku80. Proliferation was measured using Brdu incorporation.

Specifically, in these studies the skin fibroblast proliferation assay was used. An increase in the number of cells in a standardized culture was used as a measure of relative proliferative capacity. Primary fibroblasts were established from skin biopsies taken from wild type and mutant mice. Duplicate or triplicate cultures of 0.05 million cells were plated and allowed to grow for six days. At the end of the culture period, the number of cells present in the culture was determined using a electronic particle counter.

### Results:

Skin Proliferation: The female (-/-) mice exhibited a decreased mean skin fibroblast proliferation rate when compared with that of their gender-matched (+/+) littermates and the historical mean.

Thus, homozygous mutant mice demonstrated a hypo-proliferative phenotype. As suggested by these observations, antagonists or inhibitors of PRO4352 polypeptides would mimic this hypo-proliferative phenotype and could function as tumor suppressors and would be useful in decreasing abnormal cell proliferation.

### 46.33. Generation and Analysis of Mice Comprising DNA82343 (UNQ2453) Gene Disruptions

In these knockout experiments, the gene encoding PR05733 polypeptides (designated as DNA82343) (UNQ2453) was disrupted. The gene specific information for these studies is as follows: the mutated mouse gene corresponds to nucleotide reference: NM_026328 ACCESSION:NM_026328 NID: gi 13385823 refNM_026328.1 Mus musculus RIKEN cDNA 2010002L15 gene (2010002L15Rik); protein reference: Q9D8G5 ACCESSION:Q9D8G5 NID: Mus musculus (Mouse). 2010002L15RIK PROTEIN (RIKEN CDNA 2010002L15 GENE); the human gene sequence reference: NM_032044 ACCESSION:NM_032044 NID: gi 14042979 ref NM_032044.1 Homo sapiens regenerating gene type IV (REG-IV); the human protein sequence corresponds to reference: Q9BYZ8 ACCESSION:Q9BYZ8 NID: Homo sapiens (Human). REGENERATING GENE TYPE IV (REGENERATING GENE TYPE IV PRECURSOR) (GASTROINTESTINAL SECRETORY PROTEIN GISP).

The mouse gene of interest is Reg4 (regenerating islet-derived family, member 4), ortholog of human REG4. Aliases include GISP, RELP, REG-IV, and 2010002L15Rik.

REG4 is a secreted protein expressed primarily in neuroendocrine cells of the small intestine and in parietal cells of the gastric mucosa (Kamarainen et al, Am J Pathol: 163:11-20 (2003)). The protein contains a signal peptide and a C-type lectin domain (Hartupee et al, Biochim Biophys Acta: 1518:287-93 (2001)), which is capable ofbinding with carbohydrate residues in a calcium-dependent manner (Pfam accession PF00059). REG4 is often upregulated in inflammatory mucosal epithelium of individuals with ulcerative colitis or Crohn's disease, suggesting that REG4 may be involved in inflammatory responses in gastrointestinal epithelium (Hartupee et al, Biochim Biophys Acta: 1518:287-93 (2001)); Kamarainen et al, Am J Pathol: 163:11-20 (2003)). REG4 is frequently upregulated in colorectal adenomas and carcinomas, suggesting that REG4 plays a role in colon cancer (Zhang et al, Cancer Lett: 200:69-76 (2003); Violette et al, Int J Cancer: 103:185-93 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 16 | 23 | 6 | 45 |
| Expected | 11.25 | 22.5 | 11.25 | 45 |

Chi-Sq.= 0.61 Significance= 0.73712337 (hom/n)= 0.24 Avg. Litter Size= 7
Mutation Information
Mutation Type: Homologous Recombination (standard)
Description: The gene consists of 6 exons, with the start codon located in exon 2 (NCBI accession NM_026328.1). Exons 4 and 5 were targeted.
1. Wild-type Expression Panel: Expression of the target gene was detected in all 13 adult tissue samples tested by RT-PCR, except spleen, lung, skeletal muscle, bone, and adipose.
2. QC Expression: Disruption of the target gene was confirmed by Southern hybridization analysis.

### 46.33.1. PHENOTYPIC ANALYSIS (for disrupted gene: DNA82343 (UNQ2453)

### (a) OVERALL PHENOTYPIC SUMMARY:

Mutation of the gene encoding the ortholog of human regenerating islet-derived family, member 4 (REG4) resulted in signs of growth retardation in (-/-) mice. The homozygous mutant mice exhibited signs of growth retardation, including decreased mean body weight and length, total fat, total tissue mass, lean body mass and decreased bone-related measurements. The (-/-) mice also exhibited immunological and neurological abnormalities and decreased skin fibroblast proliferation rate. The male (-/-) mice exhibited an impaired glucose tolerance. Disruption of the target gene was confirmed by Southern hybridization analysis.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The (-/-) mice exhibited an increased median total white blood cell count and a decreased median platelet count when compared with that of their (+/+) littermates and the historical means.

Thus, mutant mice deficient in the DNA82343 gene resulted in a phenotype related to coagulation disorders. In this regard, PR05733 polypeptides or agonists thereof would be useful in treating disorders related to abnormal blood coagulation such as hemophilia.

### (c) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The (-/-) mice exhibited decreased mean body weight when compared with that of their gender-matched (+/+) littermates and the historical mean.
Length: The (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean total tissue mass, lean body mass, total fat mass, and percent total body fat when compared with that of their gender-matched (+/+) littermates and the historical means. The female (-/-) mice exhibited decreased total tissue mass, lean body mass, total body bone mineral content, total body bone mineral density, femurs bone mineral density and vertebral bone mineral density.
micro CT: The male (-/-) mice exhibited notably decreased mean femoral mid-shaft cross-sectional area when compared with that of their gender-matched (+/+) littermates and the historical mean.

Mutant (-/-) mice deficient in the gene encoding PR0573 3 polypeptides show a phenotype consistent with growth retardation, marked by decreased body weight and length. Thus, antagonists or inhibitors of PRO5733 polypeptides or its encoding gene would mimic these metabolic and growth related effects. On the other hand, PRO5733 polypeptides or agonists thereof would be useful in the prevention and/or treatment of such metabolic disorders as diabetes or other tissue wasting diseases.

In addition, the (-/-) mice analyzed by DEXA and micro CT exhibited decreased bone measurements and decreased body mass measurements when compared with their (+/+) littermates, suggestive of abnormal bone disorders. Thus, the (-/-) mice exhibited a negative bone phenotype. In addition, the decreased mean total tissue mass, lean body mass and decreased total body fat and fat mass is indicative of a metabolic disorder related to growth retardation and tissue wasting disorders. The negative bone phenotype indicates that PR05733 polypeptides or agonists thereof would be useful for maintaining bone homeostasis in addition to normal growth development. In addition, PR05733 polypeptides would be useful in bone healing or for the treatment of arthritis or osteoporosis, whereas antagonists (or inhibitors) of PRO5733 polypeptides or its encoding gene would lead to abnormal or pathological bone disorders including inflammatory diseases associated with abnormal bone metabolism including arthritis, osteoporosis and osteopenia.

### (d) Blood Chemistry/Glucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of 2 wild type and 4 homozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Results:

### Blood Glucose Levels/Glucose Tolerance Test:

Oral Glucose Tolerance: The male (-/-) mice exhibited an impaired glucose tolerance at the 60-minute interval when compared with that of their gender-matched (+/+) littermates and the historical means. The (-/-) mice also exhibited an increased mean fasting serum glucose level.

These studies indicated that (-/-) mice exhibit a decreased or impaired glucose tolerance in the presence of normal fasting glucose at the intervals tested when compared with their gender-matched (+/+) littermates and the historical means. Thus, knockout mutant mice exhibited the phenotypic pattern of an impaired glucose homeostasis, and therefor PR05733 polypeptides (or agonists thereof) or its encoding gene would be useful in the treatment of conditions associated with an impaired glucose homeostasis and/or various cardiovascular diseases, including diabetes.

### (e) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of 4 wild type, 4 heterozygous and 8 homozygous mutant mice. All behavioral tests were done between 12 and 16 weeks of age unless reduced viability necessitates earlier testing.

### Hot Plate Testing

Test Description: The hot plate test for nociception is carried out by placing each mouse on a small enclosed 55° C hot plate. Latency to a hindlimb response (lick, shake, or jump) is recorded, with a maximum time on the hot plate of 30 sec. Each animal is tested once.

### Results:

Hot Plate: The (-/-) mice exhibited an increased latency to respond during hot plate testing when compared with their (+/+) littermates and the historical mean, suggesting decreased sensitivity to acute pain in the mutants.

### Prepulse inhibition of the acoustic startle reflex

Prepulse inhibition ofthe acoustic startle reflex occurs when a loud 120 decibel (dB) startle-inducing tone is preceded by a softer (prepulse) tone. The PPI paradigm consists of six different trial types (70 dB background noise, 120 dB alone, 74dB + 120 dB - pp4, 78 dB + 120 dB - pp8, 82 dB + 120 dB - pp12, and 90 dB+ 120 dB - pp20) each repeated in pseudo random order six times for a total of 36 trials. The max response to the stimulus (V max) is averaged for each trial type. Animals with a 120 dB average value equal to or below 100 are excluded from analysis. The percent that the prepulse inhibits the animal's response to the startle stimulus is calculated and graphed.

### Results:

PPI: The (-/-) mice exhibited increased median prepulse inhibition during pp4, pp8, and pp12 when compared with the levels for their (+/+) littermates and the historical means, which is an indication of an enhanced sensorimotor gating/attention in the mutants.

### 46.34. Generation and Analysis of Mice Comprising DNA125170-2780 (UNQ3043) Gene Disruptions

In these knockout experiments, the gene encoding PR09859 polypeptides (designated as DNA125170-2780) (UNQ3043) was disrupted. The gene specific information for these studies is as follows: the mutated mouse gene corresponds to nucleotide reference: NM_029875 ACCESSION:NM_029875 NID: gi 37497123 ref NM_029875.2 Mus musculus solute carrier family 35, member E3 (Slc35e3); protein reference: Q6PGC7 ACCESSION:Q6PGC7 NID: Mus musculus (Mouse). Solute carrier family 35, member E3; the human gene sequence reference: NM_018656 Homo sapiens solute carrier family 35, member E3 (SLC35E3); the human protein sequence corresponds to reference: Q7Z769 ACCESSION:Q7Z769 NID: Homo sapiens (Human). Hypothetical protein.

The mouse gene of interest is Slc35e3 (solute carrier family 35, member E3), ortholog of human SLC35E3. Aliases include 9330166G04Rik; BLOV1; solute carrier family 35, member E2; and bladder cancer overexpressed protein.

SLC35E3 is a putative integral plasma membrane protein (Clark et al, Genome Res: 13:2265-70 (2003)), containing nine transmembrane segments and overlapping conserved domain of unknown function DU250 (Pfam accession PF03151). SLC35E3 is structurally related to nucleotide-sugar transporter proteins, such as VRG4 of yeast Saccharomyces cerevisiae. VRG4 is a membrane protein expressed in the Golgi apparatus that transports GDP-mannose into the lumen of the Golgi (Dean et al, J Biol Chem: 272:31908-14 (1997)). Thus, SLC35E3 may function as a transporter.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 14 | 38 | 13 | 65 |
| Expected | 16.25 | 32.5 | 16.25 | 65 |

Chi-Sq.= 0.83 Significance= 0.6603403 (hom/n)= 0.26 Avg. Litter Size= 9
Mutation Information
Mutation Type: Homologous Recombination (standard)
Description: The gene consists of 5 exons, with the start codon located in exon 1 (NCBI accession NM_029875.2). Exons 1 and 2 were targeted.
1. Wild-type Expression Panel: Expression of the target gene was detected in embryonic stem (ES) cells and in all 13 adult tissue samples tested by RT-PCR, except bone.
2. QC Expression: Disruption of the target gene was confirmed by Southern hybridization analysis.

### 46.34.1. PHENOTYPIC ANALYSIS (for disrupted gene: DNA125170-2780 (UNQ3043)

### (a) OVERALL PHENOTYPIC SUMMARY:

Mutation of the gene encoding the ortholog of human solute carrier family 35, member E3 (SLC35E3) resulted in small (-/-) mice, exhibiting immunological abnormalities. The homozygous mutant mice were smaller than their gender-matched wild-type littermates, exhibiting decreased mean body weight and length, total tissue mass, lean body mass and decreased bone mineral content and total body bone mineral density. The mutants also exhibited an increased mean serum IgG1 response to ovalbumin challenge when compared with that of their wild-type littermates and the historical means. Disruption of the target gene was confirmed by Southern hybridization analysis.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Ovalbumin Challenge

Procedure: This assay was carried out on 7 wild types and 8 homozygotes. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immunodominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG 1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice exhibited an increased mean serum IgG1 response to ovalbumin challenge when compared with that of their (+/+) littermates and the historical mean.

In summary, the ovalbumin challenge studies indicate that knockout homozygous mice deficient in the gene encoding PR09859 polypeptides exhibit immunological abnormalities when compared with their wild-type littermates. In particular, the mutant (-/-) mice exhibited an increased ability to elicit an immunological response when challenged with the T-cell dependent OVA antigen. Thus, antagonists (inhibitors) of PR09859 polypeptides would be useful for stimulating the immune system (such as T cell proliferation) and would find utility in the cases wherein this effect would be beneficial to the individual such as in the case of leukemia, and other types of cancer, and in immuno-compromised patients, such as AIDS sufferers. Accordingly, PR09859 polypeptides or agonists thereof, would be useful for inhibiting the immune response and thus would be useful candidates for suppressing harmful immune responses, e.g. in the case of graft rejection or graft-versus-host diseases.

### (c) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The (-/-) mice exhibited decreased mean body weight when compared with that of their gender-matched (+/+) littermates and the historical mean.
Length: The (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

DEXA: Both the male and female (-/-) mice exhibited decreased mean total tissue mass, lean body mass, bone mineral content and total body bone mineral density when compared with those of their gender-matched (+/+) littermates and the historical means.

Mutant (-/-) mice deficient in the gene encoding PR09859 polypeptides show a phenotype consistent with growth retardation, marked by decreased body weight and length and decreased total tissue mass and lean body mass. Thus, antagonists or inhibitors of PR09859 polypeptides or its encoding gene would mimic these metabolic and growth related effects. On the other hand, PR09859 polypeptides or agonists thereof would be useful in the prevention and/or treatment of such metabolic disorders as diabetes or other tissue wasting diseases.

In addition, the (-/-) mice analyzed by DEXA exhibited decreased bone measurements and decreased body mass measurements when compared with their (+/+) littermates, suggestive of abnormal bone disorders. Thus, the (-/-) mice exhibited a negative bone phenotype. In addition, the decreased mean total tissue mass and lean body mass is indicative of a metabolic disorder related to growth retardation and tissue wasting disorders. The negative bone phenotype indicates that PR09859 polypeptides or agonists thereof would be useful for maintaining bone homeostasis in addition to normal growth development. In addition, PRO9859 polypeptides would be useful in bone healing or for the treatment of arthritis or osteoporosis, whereas antagonists (or inhibitors) of PR09859 polypeptides or its encoding gene would lead to abnormal or pathological bone disorders including inflammatory diseases associated with abnormal bone metabolism including arthritis, osteoporosis and osteopenia.

### 46.35. Generation and Analysis of Mice Comprising DNA125151-2784 (UNQ3048) Gene Disruptions

In these knockout experiments, the gene encoding PR09864 polypeptides (designated as DNA125151-2784) (UNQ3048) was disrupted. The gene specific information for these studies is as follows: the mutated mouse gene corresponds to nucleotide reference: XM_131826 Mus musculus RIKEN cDNA 2310043108 gene (2310043I08Rik); protein reference: Q9D702 ACCESSION:Q9D702 NID: Mus musculus (Mouse). 2310043I08Rik protein; the human gene sequence reference: NM_178545 Homo sapiens hypothetical protein LOC339456 (LOC339456); the human protein sequence corresponds to reference: Q8NDY8 ACCESSION:Q8NDY8 NID: Homo sapiens (Human). Muscle protein.

The mouse gene of interest is RIKEN cDNA 2310043I08 gene, ortholog of human hypothetical protein LOC339456.

Hypothetical protein LOC339456 is a putative extracellular protein, containing a signal peptide, a transmembrane segment, and a C-terminus of about 130 amino acids.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 11 | 20 | 11 | 42 |
| Expected | 10.5 | 21 | 10.5 | 42 |

Chi-Sq.= 0.28 Significance= 0.86935824 (hom/n)= 0.24 Avg. Litter Size= 7
Mutation Information
Mutation Type: Homologous Recombination (standard)
Description: Coding exons 1 through 5 were targeted (NCBI accession AK009779).
1. Wild-type Expression Panel: Expression of the target gene was detected in embryonic stem (ES) cells and in all 13 adult tissue samples tested by RT-PCR, except liver.
2. QC Expression: Disruption of the target gene was confirmed by Southern hybridization analysis.

### 46.35.1. PHENOTYPIC ANALYSIS (for disrupted gene: DNA125151-2784 (UNQ3048)

### (a) OVERALL PHENOTYPIC SUMMARY:

Mutation of the gene encoding the ortholog of a hypothetical human protein (LOC339456) resulted in increased locomotor activity in (-/-) mice during both open field and home-cage activity testing when compared with that of their wild-type littermates and the historical means. In addition, the homozygous mutants exhibited an altered distribution of leukocyte subsets in peripheral blood. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination ofthe affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACSCalibur flow cytometer with CellQuest software.

### Results:

FACS3: The (-/-) mice exhibited an altered distribution of leukocyte subsets in the peripheral blood, characterized by decreased mean percentages of CD4 and CD8 cells and an increased mean percentage of B cells when compared with those of their (+/+) littermates and the historical means.

Thus, knocking out the gene which encodes PR09864 polypeptides causes a decrease in the T cell population as well as causing an increase in the B cell population. From these observations, PR09864 polypeptides or the gene encoding PR09864 appears to act as a negative regulator of B cell proliferation. Thus, antagonists or inhibitors of PR09864 polypeptides would be beneficial in enhancing B cell proliferation and depressing T cell proliferation.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The female (-/-) mice exhibited increased ambulatory counts during the 1- and 12-hour habituation periods and during both dark periods when compared with that of their gender-matched (+/+) littermates and the historical mean. These results are consistent with other neurological observations (increased anxiety in open field testing).

### Open field test:

Several targets ofknown drugs have exhibited phenotypes in the open field test. These include knockouts of the seratonin transporter, the dopamine transporter (Giros et al., Nature. 1996 Feb 15;379(6566):606-12), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value.

### Results:

Openfield2: The (-/-) mice exhibited increased median sum total distance when compared with that of their (+/+) littermates and the historical mean, suggesting an increased anxiety-like response in the mutants.

The (-/-) mice demonstrated a decrease median sum time-in-center and an increase in distance travelled when compared to the (+/+) mice, suggesting an increased anxiety-like response in the (-/-) mice. In summary, the open field testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PR09864 polypeptides or agonists thereof would be useful in the treatment of such neurological disorders.

### 46.36. Generation and Analysis of Mice Comprising DNA129549-2798 (UNQ3072) Gene Disruptions

In these knockout experiments, the gene encoding PR09904 polypeptides (designated as DNA129549-2798) (UNQ3072) was disrupted. The gene specific information for these studies is as follows: the mutated mouse gene corresponds to nucleotide reference: NM_053087 ACCESSION:NM_053087 NID: gi 16716372 ref NM_053087.1 Mus musculus epithelial mitogen (Epgn); protein reference: Q924X1 ACCESSION:Q924X1NID: Mus musculus (Mouse). Epigen protein precursor; the human gene sequence reference: XM_171078 PREDICTED: Homo sapiens similar to Epigen protein (LOC255324); the human protein sequence corresponds to reference: XP_171078 PREDICTED: similar to Epigen protein [Homo sapiens].

The mouse gene of interest is Epgn (epithelial mitogen), ortholog of human "similar to Epigen protein." Aliases include epigen, 2310069M11Rik, and LOC255324.

Epgn is a plasma membrane protein that likely functions as a growth factor. The protein contains a signal peptide, an epidermal growth factor (EGF)-like domain, and a transmembrane segment. Epgn is released into the extracellular space by proteolytic cleavage. Epgn activates receptor tyrosine kinase c-erbB-1 and stimulates proliferation of HaCaT cells. Epgn is expressed in testis, heart, and liver and is likely to play a role in growth of epithelial cells and in wound healing (Strachan et al, J Biol Chem: 276:18265-71 (2001); Kochupurakkal et al, J Biol Chem: 280:8503-12 (2005)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 27 | 29 | 24 | 80 |
| Expected | 20 | 40 | 20 | 80 |

Chi-Sq.= 3.86 Significance= 0.1451482 (hom/n)= 0.29 Avg. Litter Size= 9
Mutation Information
Mutation Type: Homologous Recombination (standard)
Description: Coding exons 1 and 2 were targeted (NCBI accession NM_053087.1).
1. Wild-type Expression Panel: Expression of the target gene was detected in embryonic stem (ES) cells and in brain, spinal cord, eye, thymus, spleen, kidney, and heart among 13 adult tissue samples tested by RT-PCR.
2. QC Expression: Disruption of the target gene was confirmed by Southern hybridization analysis.

### 46.36.1. PHENOTYPIC ANALYSIS (for disrupted gene: DNA129549-2798 (UNQ3072)

### (a) OVERALL PHENOTYPIC SUMMARY:

Mutation of the gene encoding the ortholog of human "similar to Epigen protein" resulted in an increased platelet count in the (-/-) mice. In addition, the mutant (-/-) mice also showed an increase in lean body mass and total tissue mass and elevated mean serum cholesterol levels.
Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The (-/-) mice exhibited an increased mean platelet count when compared with that of their (+/+) littermates and the historical mean.

Thus, mutant mice deficient in the DNA129549-2798 gene resulted in a phenotype related to coagulation disorders. In this regard, inhibitors or antagonists of PRO9904 polypeptides would be useful in treating disorders related to abnormal blood coagulation such as hemophilia.

### (c) Phenotypic Analysis: Cardiology

In the area of cardiovascular biology, targets were identified herein for the treatment of hypertension, atherosclerosis, heart failure, stroke, various coronary artery diseases, dyslipidemias such as high cholesterol (hypercholesterolemia)and elevated serum triglycerides (hypertriglyceridemia), diabetes and/or obesity. The phenotypic tests included the measurement of serum cholesterol and triglycerides.

### Blood Lipids

Procedure: A cohort of 4 wild type, 4 heterozygotes and 8 homozygotes were tested in this assay. High cholesterol levels and increased triglyceride blood levels are recognized risk factors in the development of cardiovascular disease and/or diabetes. Measuring blood lipids facilitates the finding of biological switches that regulate blood lipid levels. Inhibition of factors which elevate blood lipid levels may be useful for reducing the risk for cardiovascular disease. In these blood chemistry tests, measurements were recorded using the COBAS Integra 400 (mfr: Roche).

### Results:

Blood Chemistry: The female (-/-) mice exhibited an increased mean serum cholesterol level when compared with that of their gender-matched (+/+) littermates and the historical mean.

As summarized above, the (-/-) mice exhibited increased mean serum cholesterol levels when compared with their gender-matched (+/+) littermates and the historical means. Thus, mutant mice deficient in the PR09904 gene can serve as a model for cardiovascular disease. PR09904 polypeptides or its encoding gene would be useful in regulating blood lipids such as cholesterol. Thus, PR09904 polypeptides or agonists thereof would be useful in the treatment of such cardiovascular diseases as hypertension, atherosclerosis, heart failure, stroke, various coronary diseases, hypercholesterolemia, diabetes and/or obesity.

### (d) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

DEXA: The female (-/-) mice exhibited increased mean lean body mass and total tissue mass when compared with that of their gender-matched (+/+) littermates and the historical mean.

These studies suggest that mutant (-/-) non-human transgenic animals exhibit a negative phenotype that would be associated with obesity. Thus, PR09904 polypeptides or agonists thereof are essential for normal growth and metabolic processes and especially would be important in the prevention and/or treatment of obesity.

### 46.37. Generation and Analysis of Mice Comprising DNA142392-2800 (UNQ3075) Gene Disruptions

In these knockout experiments, the gene encoding PR09907 polypeptides (designated as DNA142392-2800) (UNQ3075) was disrupted. The gene specific information for these studies is as follows: the mutated mouse gene corresponds to nucleotide reference:AY182031 ACCESSION:AY 182031 NID: gi 29542646 gb AY182031.1 Mus musculus leucine-rich repeat transmembrane neuronal 4 protein; protein reference: Q80XG9 ACCESSION:Q80XG9 NID: Mus musculus (Mouse). Leucine-rich repeat transmembrane neuronal 4 protein; the human gene sequence reference: NM_024993 Homo sapiens leucine rich repeat transmembrane neuronal 4 (LRRTM4); the human protein sequence corresponds to reference: Q6ZT31 ACCESSION:Q6ZT31 NID: Homo sapiens (Human). Hypothetical protein FLJ45014.

The mouse gene of interest is Lrrtm4 (leucine rich repeat transmembrane neuronal 4), ortholog of human LRRTM4. Aliases include FLJ12568, A230052N11, and 7530419J18Rik.

LRRTM4 is a putative type I integral plasma membrane protein expressed primarily in the nervous system. The 518-amino acid protein contains a signal peptide, at least 9 leucine-rich repeats flanked by cysteine-rich domains, a transmembrane segment, and a C-terminal segment of about 50 amino acids. Proteins with leucine-rich repeats are likely to be involved in molecular recognition processes, such as signal transduction or cell adhesion (InterPro accession IPR000372). LRRTM4 may play a role in development and maintenance of the vertebrate nervous system (Lauren et al, Genomics: 81:411-21 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 11 | 39 | 19 | 69 |
| Expected | 17.25 | 34.5 | 17.25 | 69 |

Chi-Sq.= 0.1 Significance= 0.95122945 (hom/n)= 0.26 Avg. Litter Size= 9
Mutation Information
Mutation Type: Homologous Recombination (standard)
Description: Coding exon 2 was targeted (NCBI accession NM_178731.2).
1. Wild-type Expression Panel: Expression of the target gene was detected only in brain, spinal cord, and eye among the 13 adult tissue samples tested by RT-PCR.
2. QC Expression: Disruption of the target gene was confirmed by Southern hybridization analysis.

### 46.37.1. PHENOTYPIC ANALYSIS (for disrupted gene: DNA142392-2800 (UNQ3075)

### (a) OVERALL PHENOTYPIC SUMMARY:

Mutation of the gene encoding the ortholog of human leucine rich repeat transmembrane neuronal 4 (LRRTM4) resulted in the male homozygous mutant mice exhibiting an increased anxiety-like response during open field testing when compared with that of their gender-matched wild-type littermates and the historical mean. In addition, the male (-/-)mice exhibited decreased mean vertebral trabecular bone measurements. The (-/-) mice also exhibited decreased mean percentages of CD8 cells. Disruption of target gene was confirmed by Southern hybridization analysis.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample oflysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACSCalibur flow cytometer with CellQuest software.

### Results:

FACS3: The (-/-) mice exhibited an altered distribution of leukocyte subsets in the peripheral blood, characterized by a decreased mean percentage of CD8 cells when compared with that of their (+/+) littermates and the historical mean. Thus, the mutant (-/-) mice show an immunological abnormality marked by a deficiency in cytotoxic T cells which function as the co-receptors for MHC class I molecules. PR09907 polypeptides or agonists thereof, would therefore be beneficial in stimulating CD8 T cell production.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Open field test:

Several targets ofknown drugs have exhibited phenotypes in the open field test. These include knockouts of the seratonin transporter, the dopamine transporter (Giros et al., Nature. 1996 Feb 15;379(6566):606-12), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value.

### Results:

Openfield2: The male (-/-) mice exhibited a decreased sum time-in-center when compared with that of their gender-matched (+/+) littermates and the historical mean, suggesting an increased anxiety-like response in the mutants.

In summary, the open field testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PR09907 polypeptides or agonists thereof would be useful in the treatment of such neurological disorders.

### (d) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

micro CT: The male (-/-) mice exhibited decreased mean vertebral trabecular bone volume, number, thickness, and connectivity density when compared with that of their gender-matched (+/+) littermates and the historical means.

The (-/-) mice analyzed by bone micro CT analysis exhibited decreased bone measurements when compared with their (+/+) littermates, suggestive of abnormal bone disorders. The negative bone phenotype indicates that PR09907 polypeptides or agonists thereof would be useful for maintaining bone homeostasis. In addition, PR09907 polypeptides would be useful in bone healing or for the treatment of arthritis or osteoporosis, whereas antagonists (or inhibitors) of PRO9907polypeptides or its encoding gene would lead to abnormal or pathological bone disorders including inflammatory diseases associated with abnormal bone metabolism including arthritis, osteoporosis and osteopenia.

### 46.38. Generation and Analysis of Mice Comprising DNA125181-2804 (UNQ3082) Gene Disruptions

In these knockout experiments, the gene encoding PRO10013 polypeptides (designated as DNA125181-2804) (UNQ3082) was disrupted. The gene specific information for these studies is as follows: the mutated mouse gene corresponds to nucleotide reference: XM_133861 ACCESSION:XM_133861 NID: gi 51761932 ref XM_133861.4 PREDICTED: Mus musculus RIKEN cDNA 5630400E24 gene (5630400E24Rik); protein reference: XP_133861 similar to Conserved oligomeric Golgi complex component 7 [Mus musculus]; the human gene sequence reference: NM_153603 ACCESSION:NM_153603 NID: gi 23957689 ref NM_153603.1 Homo sapiens component of oligomeric golgi complex 7 (COG7); the human protein sequence corresponds to reference: P83436 ACCESSION:P83436 6 NID: Homo sapiens (Human). Conserved oligomeric Golgi complex component 7.

The mouse gene of interest is Cog7 (component of oligomeric Golgi complex 7), ortholog of human COG7. Aliases include Gm167, 5630400E24Rik, and CDG2E.

COG7 is one of eight subunits of conserved oligomeric Golgi (COG) complex, which functions as a structural component of the Golgi apparatus. COG complex is required for normal Golgi function and is involved in several Golgi processes, such as glycosylation, protein transport, and membrane trafficking. Loss-of-function mutations in COG7 can cause "congenital disorder of glycosylation type IIe," which can result in mental retardation, seizures, hypotonia, liver malfunctions, coagulopathy dysmorphia, and perinatal mortality (Ungar et al, J Cell Biol: 157:405-15 (2002); Wu et al, Nat Med 10:518-23 (2004); Loh and Hong, J Biol Chem 279:24640-8 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 22 | 40 | 0 | 62 |
| Expected | 15.5 | 31 | 15.5 | 62 |

Chi-Sq.= 41.34 Significance= 1.0547099E-9 (hom/n)= 0.0 Avg. Litter Size= 7
Mutation Information
Mutation Type: Homologous Recombination (standard)
Description: Coding exon 1 was targeted (NCBI accession AK030709).
1. Wild-type Expression Panel: Expression of the target gene was detected in embryonic stem (ES) cells and in all 13 adult tissue samples tested by RT-PCR, except lung, skeletal muscle, bone, and adipose.
2. QC Expression: Disruption of the target gene was confirmed by Southern hybridization analysis.

### 46.38.1. PHENOTYPIC ANALYSIS (for disrupted gene: DNA125181-2804 (UNQ3082)

### (a) OVERALL PHENOTYPIC SUMMARY:

Mutation ofthe gene encoding the ortholog of human component of oligomeric Golgi complex 7 (COG7) resulted in lethality of (-/-) mutants. Genetic data indicate that this mutation resulted in lethality of the homozygous mutants. No notable phenotype was observed for the heterozygous mice. Disruption of the target gene was confirmed by Southern hybridization analysis.

### Discussion related to embryonic developmental abnormality of lethality:

Embryonic lethality in knockout mice usually results from various serious developmental problems including but not limited to neuro-degenerative diseases, angiogenic disorders, inflammatory diseases, or where the gene/protein has an important role in basic cell signaling processes in many cell types. In addition, embryonic lethals are useful as potential cancer models. Likewise, the corresponding heterozygous (+/-) mutant animals are particularly useful when they exhibit a phenotype and/or a pathology report which reveals highly informative clues as to the function of the knocked-out gene. For instance, EPO knockout animals were embryonic lethals, but the pathology reports on the embryos showed a profound lack of RBCs.

### (b) Pathology

Microscopic: Due to embryonic lethality, microscopic analysis was not performed. At 12.5 days there were 46 embryos observed: 22 (+/-) embryos, 10 (+/+) embryos, and 14 resorption moles.
Gene Expression: LacZ activity was not detected in the panel of tissues by immunohistochemical analysis.

### 46.39. Generation and Analysis of Mice Comprising DNA336882 (UNQ5043) Gene Disruptions

In these knockout experiments, the gene encoding PR090948 polypeptides (designated as DNA336882) (UNQ5043) was disrupted. The gene specific information for these studies is as follows: the mutated mouse gene corresponds to nucleotide reference: NM_170758 Mus musculus CD300A antigen (Cd300a); protein reference: Q7TN56 ACCESSION:Q7TN56 NID: Mus musculus (Mouse). Mast cell-derived paired immunoglobulin-like receptor1; the human gene sequence reference: NM_007261 Homo sapiens CD300A antigen (CD300A); the human protein sequence corresponds to reference:Q9UGN4 ACCESSION:Q9UGN4 NID: Homo sapiens (Human). CMRF35-H antigen precursor (CMRF35-H9) (CMRF-35-H9) (Inhibitory receptor protein 60) (IRp60) (IRC1/IRC2) (NK inhibitory receptor).

The mouse gene of interest is Cd300a (CD300A antigen), ortholog of human CD300A. Aliases include CMRF35H, B230315M08Rik, Clm8, LMIR1, MAIR-Ia, MMAC8, Pigr4, mcpir1, mast cell-derived paired immunoglobulin-like receptor 1, polymeric immunoglobulin receptor 4, CMRF-35-H9, CMRF-35H, CMRF35H9, IGSF12, IRC1, IRC2, IRp60, CMRF35H leukocyte immunoglobulin-like receptor, and leukocyte membrane antigen.

CD300A is a type I integral plasma membrane protein expressed on leukocytes and is likely to function as a signal-transducing receptor. The protein contains a signal peptide, an immunoglobulin-like domain, a transmembrane segment, and a cytoplasmic domain with several putative immunoreceptor tyrosine-based inhibitory motifs (ITIMs). CD300A may play a role in immune cell function, negatively regulating different types of leukocytes. The CMRF35H gene may be associated with psoriasis (Green et al, Int Immunol: 10:891-9 (1998); Cantoni et al, Eur J Immunol: 29:3148-59 (1999); Clark et al, Tissue Antigens: 55:101-9 2000; Speckman et al, Hum Genet: 112:34-41 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 16 | 28 | 15 | 59 |
| Expected | 14.75 | 29.5 | 14.75 | 59 |

Chi-Sq.= 4.6 Significance= 0.10025885 (hom/n)= 0.29 Avg. Litter Size= 8
Mutation Information
Mutation Type: Homologous Recombination (standard)
Description: Coding exons 2 and 3 were targeted (NCBI accession NM_170758.2).
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.39.1. PHENOTYPIC ANALYSIS (for disrupted gene: DNA336882 (UNQ5043)

### (a) OVERALL PHENOTYPIC SUMMARY:

Mutation of the gene encoding the ortholog of human CD300A antigen (CD300A) resulted in decreased tissue mass, lean body mass and bone mineral content and density measurements in (-/-) mice. Both (+/-) and (-/-) mice exhibited decreased mean body weight and length. Gene disruption was confirmed by Southern blot.

### (b) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: Both the (-/-) and (+/-) mice exhibited decreased mean body weight when compared with that of their gender-matched (+/+) littermates and the historical mean.
Length: Both the (-/-) and (+/-) mice exhibited decreased mean body length when compared with that of their gender-matched (+/+) littermates and the historical mean.
(Male homozygotes and heterozygotes showed 1-2 SD below mean weight and length).

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited notably decreased mean total tissue mass and lean body mass when compared with those of their gender-matched (+/+) littermates and the historical means. In addition, both the male and female (-/-) mice exhibited decreased mean bone mineral content and density measurements.
micro CT: The male (-/-) mice exhibited decreased mean femoral mid-shaft cortical thickness and cross-sectional area when compared with those of their gender-matched (+/+) littermates and the historical means.

The (-/-) mice analyzed by DEXA and bone micro CT analysis exhibited decreased bone measurements and decreased body mass measurements when compared with their (+/+) littermates, suggestive of abnormal bone disorders. Thus, the (-/-) mice exhibited a negative bone phenotype. In addition, the decreased mean total tissue mass and lean body mass as well as decreased mean body weight and length is indicative of a metabolic disorder related to tissue wasting disorders. The negative bone phenotype indicates that PR090948 polypeptides or agonists thereof would be useful for maintaining bone homeostasis. In addition, PR090948 polypeptides would be useful in bone healing or for the treatment of arthritis or osteoporosis, whereas antagonists (or inhibitors) of PRO90948polypeptides or its encoding gene would lead to abnormal or pathological bone disorders including inflammatory diseases associated with abnormal bone metabolism including arthritis, osteoporosis and osteopenia.

### 46.40. Generation and Analysis of Mice Comprising DNA184073 (UNQ5384) Gene Disruptions

In these knockout experiments, the gene encoding PR028694 polypeptides (designated as DNA184073) (UNQ5384) was disrupted. The gene specific information for these studies is as follows: the mutated mouse gene corresponds to nucleotide reference:BC021530 Mus musculus solute carrier family 39 (zinc transporter), member 14; protein reference: Q8VDLO ACCESSION:Q8VDL0 NID: Mus musculus (Mouse). Solute carrier family 39 (Zinc transporter), member 14; the human gene sequence reference: NM_015359 Homo sapiens solute carrier family 39 (zinc transporter), member 14 (SLC39A14); the human protein sequence corresponds to reference: Q6ZME8 ACCESSION:Q6ZME8 NID: Homo sapiens (Human). Hypothetical protein FLJ23971.

The mouse gene of interest is Slc39a14 (solute carrier family 39 [zinc transporter], member 14), ortholog ofhuman SLC39A14. Aliases include fad123, MGC38539, G630015O18Rik, LZT-Hs4, and KIAA0062.

SLC39A14 is a plasma membrane protein that functions as a zinc transporter. Expression of SLC39A14 is ubiquitous but is highest in liver. Zinc is a cofactor for a wide variety of enzymes (Taylor et al, FEBS Lett: 579:427-32 (2005); Taylor and Nicholson, Biochim Biophys Acta 1611:16-30 (2003); Nomura et al, DNA Res: 1:223-9 (1994)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 37 | 20 | 78 |
| Expected | 19.5 | 39 | 19.5 | 78 |

Chi-Sq.= 0.0 Significance= 1.0 (hom/n)= 0.25 Avg. Litter Size= 9
Mutation Information
Mutation Type: Homologous Recombination (standard)
Description: Coding exons 1 through 3 were targeted (NCBI accession NM_144808.3).
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.40.1. PHENOTYPIC ANALYSIS (for disrupted gene: DNA184073 (UNQ5384)

### (a) OVERALL PHENOTYPIC SUMMARY:

Mutation of the gene encoding the ortholog of human solute carrier family 39 (zinc transporter), member 14 (SLC39A14) resulted in small (-/-) mice. The homozygous mutant mice were smaller than their gender-matched wild-type littermates, exhibiting decreased mean body weight and length, total tissue mass, lean body mass, and decreased bone-related measurements. The homozygous mutant mice exhibited neurological abnormalities. Disruption of the target gene was confirmed by Southern hybridization analysis.

### (b) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The male (-/-) mice exhibit decreased mean body weight when compared with that of their gender-matched (+/+) littermates and the historical mean (1-2 SD below mean).
Length: The male (-/-) mice exhibit decreased mean body length when compared with that of their gender-matched (+/+) littermates (1-2 SD below the mean).
Obvious: The (-/-) mice exhibited a head tilt and retropulsion.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

DEXA: The male (-/-) mice exhibited notably decreased mean total tissue mass, lean body mass (LBM), bone mineral content, and femur and vertebrae bone mineral density measurements when compared with those of their (+/+) littermates and the historical means. The female (-/-) mice showed decreased bone mineral content (BMC), BMC/LBM, total body bone mineral density and vertebrae bone mineral density when compared with those of their (+/+) littermates and the historical means.

The (-/-) mice analyzed by DEXA analysis exhibited decreased bone measurements and decreased body mass measurements when compared with their (+/+) littermates, suggestive of abnormal bone disorders. Thus, the (-/-) mice exhibited a negative bone phenotype. In addition, the decreased mean total tissue mass is indicative of a metabolic disorder related to tissue wasting disorders. The negative bone phenotype indicates that PR028694 polypeptides or agonists thereof would be useful for maintaining bone homeostasis. In addition, PR028694 polypeptides would be useful in bone healing or for the treatment of arthritis or osteoporosis, whereas antagonists (or inhibitors) of PR028694 polypeptides or its encoding gene would lead to abnormal or pathological bone disorders including inflammatory diseases associated with abnormal bone metabolism including arthritis, osteoporosis and osteopenia.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Functional Observational Battery (FOB) Test:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Observations:

FOB2: Of the 8 (-/-) mice analyzed, 4 did not rear during the 1-minute observation period. In addition, the (-/-) mice exhibited head tilt and retropulsion. Also, exophthalmus was present in 3 of 8 (-/-) mice; defecation was absent in 3 of 8 (-/-) mice.

### Inverted Screen Testing:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Inverted Screen Test Data:

The Inverted Screen is used to measure motor strength/coordination. Untrained mice were placed individually on top of a square (7.5 cm x 7.5 cm) wire screen which was mounted horizontally on a metal rod. The rod was then rotated 180 degrees so that the mice were on the bottom of the screens. The following behavioral responses were recorded over a 1 min testing session: fell off, did not climb, and climbed up.

### Results:

| Genotype | Ratio Fell Down % | | Ratio Climbed up % | |
|---|---|---|---|---|
| +/+ (n=8) | 1/8 | 13 | 7/8 | 87.5 |
| -/- (n=8) | 8/8 | 100 | 0/8 | 0 |

A motor strength deficit is apparent when there is a 50% point difference between (-/-) or (+/-) mice and (+/+) mice for the fell down response. 0/8 or 1/8 (-/-) or (+/-) mice not climbing indicates impaired motor coordination. 7/8 or 8/8(-/-) or (+/-) mice climbing up indicates enhanced motor coordination.

The Inverted Screen Test is designed to measure basic sensory & motor observations:
Inverted Screen: None of the (-/-) mice climbed up the inverted screen whereas 7/8 (+/+) mice climbed up. These results indicate an impaired motor strength in the mutants.

### Open field test:

Several targets ofknown drugs have exhibited phenotypes in the open field test. These include knockouts of the seratonin transporter, the dopamine transporter (Girosetal., Nature. 1996 Feb 15;379(6566):606-12), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value. Analyzed wt/ het/ hom: 5/ 4/ 8

### Results:

Openfield2: The male (-/-) mice exhibited an increased median sum time-in-center and decreased sum total distance traveled with no rearing during open field testing when compared with that of their gender-matched (+/+) littermates and the historical mean, suggesting a decreased anxiety-like response in the mutants.

A notable difference was observed during open field activity testing. The (-/-) mice exhibited an increased median sum time in the center (with hypoactivity) when compared with their gender-matched (+/+) littermates, which is indicative of a decreased anxiety-like response in the mutants. Thus, knockout mice demonstrated a phenotype consistent with depression, generalized anxiety disorders, cognitive disorders, hyperalgesia and sensory disorders and/or bipolar disorders. Thus, PR028694 polypeptides and agonists thereof would be useful for the treatment or amelioration of the symptoms associated with depressive disorders.

### 46.41. Generation and Analysis of Mice Comprising DNA150163-2842 (UNQ5782) Gene Disruptions

In these knockout experiments, the gene encoding PRO16089 polypeptides (designated as DNA150163-2842) (UNQ5782) was disrupted. The gene specific information for these studies is as follows: the mutated mouse gene corresponds to nucleotide reference: NM_134159 Mus musculus interleukin 17 receptor C (Il17rc); protein reference: Q8K4C2 ACCESSION:Q8K4C2 NID: Mus musculus (Mouse). IL-17RC; the human gene sequence reference: NM_153460 ACCESSION:NM_153460 NID: gi 24430194 refNM_153460.1 Homo sapiens interleukin 17 receptor C (IL-17RC), transcript variant 2; the human protein sequence corresponds to reference: Q8NFS1 ACCESSION:Q8NFS1 NID: Homo sapiens (Human). IL-17RC.

The mouse gene of interest is Il17rc (interleukin 17 receptor C), ortholog of human IL17RC. Aliases include Il17rl, IL-17RC, IL17-RC, IL17-RL, 1110025H02Rik, and MGC10763.

IL17RC is a type I integral plasma membrane protein that likely functions as a signal transducing receptor. The protein contains a signal peptide, a large extracellular segment, a transmembrane segment, and a cytoplasmic domain. Although IL17RC contains no conserved domains, the primary structure of the protein is similar to that of interleukin 17 receptor. Alternative splicing likely gives rise to a number of variant IL17RC proteins, of which some may be soluble extracellular proteins that function as decoy receptors. IL17RC is expressed at high levels in prostate, cartilage, kidney, liver, heart, and muscle. [Haudenschild et al., J Biol Chem: 277:4309-16(2002)]

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 31 | 20 | 69 |
| Expected | 17.25 | 34.5 | 17.25 | 69 |

Chi-Sq.= 0.04 Significance= 0.9801987 (hom/n)= 0.25 Avg. Litter Size= 8
Mutation Information
Mutation Type: Homologous Recombination (standard)
Description: The gene consists of 7 exons, with the start codon located in exon 1 (NCBI accession AK016908). Exons 1 and 2 were targeted.
1. Wild-type Expression Panel: Expression of the target gene was detected in embryonic stem (ES) cells and in all 13 adult tissue samples tested by RT-PCR, except skeletal muscle and bone, stomach, small intestine, and colon. 2. QC Expression: Disruption of the target gene was confirmed by Southern hybridization analysis.

### 46.41.1. PHENOTYPIC ANALYSIS (for disrupted gene: DNA150163-2842 (UNQ5782)

### (a) OVERALL PHENOTYPIC SUMMARY:

Mutation of the gene encoding the ortholog of human interleukin 17 receptor C (IL17RC) resulted in immunological abnormalities in the (-/-) mice. The homozygous mutant mice exhibited increased mean percentages of peritoneal B cells when compared with those of their wild-type littermates and the historical means. The (-/-) mice also showed decreased weight and body lengths. The male knockouts exhibited increased vBMD, total body bone mineral density (BMD) and bone mineral content (BMC); female knockouts showed decreased femur bone mineral density and vertebrae bone mineral density. Disruption of the target gene was confirmed by Southern hybridization analysis.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample oflysed peripheral blood from each mouse with a panel of six lineage-specific antibodies: CD45 PerCP, anti-TCRb APC, CD4 PE, CD8 FITC, pan-NK PE, and CD 19 FITC. The two FITC and PE labeled antibodies stain mutually exclusive cell types. The samples were analyzed using a Becton Dickinson FACSCalibur flow cytometer with Cell Quest software.

### Results:

FACS3: The (-/-) mice exhibited an altered distribution of leukocyte subsets in the peripheral blood, characterized by an increased mean percentage of peritoneal B cells when compared with those of their (+/+) littermates and the historical means.

Thus, knocking out the gene which encodes PRO16089 polypeptides causes an increase in the B cell population. From these observations, PR016089 polypeptides or the gene encoding PR016089 appears to function as a negative regulator of B cell proliferation. Thus, antagonists or inhibitors of PR016089 polypeptides would be beneficial in enhancing B cell proliferation.

### (c) Bone Metabolism & Body Diagnostics

### (1) Tissue Mass & Lean Body Mass Measurements - Dexa

### Dexa Analysis - Test Description:

Procedure: A cohort of wild type, heterozygotes and homozygotes were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The female (-/-) mice exhibited decreased mean body weight when compared with that of their gender-matched (+/+) littermates and the historical mean.
Length: The female (-/-) mice exhibited decreased mean body length when compared with that of their gender-matched (+/+) littermates and the historical mean.

Mutant (-/-) mice deficient in the gene encoding PR016089 polypeptides show a phenotype consistent with growth retardation, marked by decreased body weight and length. Thus, antagonists or inhibitors of PRO16089 polypeptides or its encoding gene would mimic these metabolic and growth related effects. On the other hand, PRO16089 polypeptides or agonists thereof would be useful in the prevention and/or treatment of such metabolic disorders as diabetes or other tissue wasting diseases.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygotes and homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

DEXA: Male knockouts exhibited increased total body vBMD, total body bone mineral density and bone mineral content; however, female knockouts exhibited decreased femur bone mineral density and vertebrae bone mineral density.

### 46.42. Generation and Analysis of Mice Comprising DNA96861-2844 (UNQ5785) Gene Disruptions

In these knockout experiments, the gene encoding PRO19563 polypeptides (designated as DNA96861-2844) (UNQ5785) was disrupted. The gene specific information for these studies is as follows: the mutated mouse gene corresponds to nucleotide reference: XM_126005 PREDICTED: Mus musculus polycystic kidney disease 1 like 1 (Pkd111); protein reference: XP_126005 polycystin-1L1 [Mus musculus]; the human gene sequence reference: NM_138295 Homo sapiens polycystic kidney disease 1 like 1 (PKD1L1); the human protein sequence corresponds to reference: Q8TDX9 Polycystic kidney disease 1-like 1 protein (Polycystin IL1) (UNQ5785/PRO19563) gi|19923084|ref|NP_612152.1| polycystin-1L1 [Homo sapiens] gi|19110438|dbj|BAB85807.1| polycystin-1L1 [Homo sapiens].

The mouse gene of interest is PKD1L1 (polycystic kidney disease 1 like 1), ortholog of human PKD1L1. Aliases include Polycystin 1L1, UNQ5785, and PRO19563.

PKD1L1 is a very large integral plasma membrane protein of about 2,500 amino acids expressed primarily in Leydig cells of testis and in heart. The protein contains two immunoglobulin-like PKD (polycystic kidney disease) domains, an REJ (receptor for egg jelly) domain, and a PLAT (Polycystin-1, Lipoxygenase, Alpha-Toxin) domain within a region containing 11 transmembrane segments. The PKD and REJ domains are predicted to be extracellular, whereas the PLAT domain is predicted to be cytoplasmic. PKD and REJ domains are likely to interact with other proteins or carbohydrates, serving an adhesive function (Pfam accessions PF00801 and PF02010). PLAT domains are found in a variety of membrane or lipid-associated proteins, such as lipoxygenases and pancreatic lipase, and are likely involved in mediating the association of proteins with membranes (Pfam accession PF01477). Thus, PKD1L1 may function in cell adhesion or signal transduction. PKD1L1 may play a role in heart function or development and in the male reproductive system (Yuasa et al, Genomics: 79:376-86 (2002); Lakkis and Zhou, Nephron Exp Nephrol: 93:e3-8 2003).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 36 | 4 | 59 |
| Expected | 14.75 | 29.5 | 14.75 | 59 |

Chi-Sq.= 28.06 Significance= 8.0695355E-7 (hom/n)= 0.1 Avg. Litter Size= 8
Mutation Information
Mutation Type: Homologous Recombination (standard)
Description: Coding exons 10 through 12 were targeted (NCBI accession NM_138295.2 [human]).
1. Wild-type Expression Panel: Expression of the target gene was detected in all 13 adult tissue samples tested by RT-PCR, except skeletal muscle, bone, and adipose.
2. QC Expression: Disruption of the target gene was confirmed by Southern hybridization analysis.

### 46.42.1. PHENOTYPIC ANALYSIS (for disrupted gene: DNA96861-2844 (UNQ5785)

### (a) OVERALL PHENOTYPIC SUMMARY:

Mutation of the gene encoding the ortholog of human polycystic kidney disease 1 like 1 (PKD1L1) resulted in reduced viability of the (-/-) mice. Both necropsy and CAT-Scan revealed situs inversus in some of the homozygous mutant mice analyzed. The (-/-) mice also showed immunological abnormalities and an impaired glucose tolerance. The (-/-) mice exhibited increased body mass and bone mineral density measurements. Disruption of the target gene was confirmed by Southern hybridization analysis.

### (b) Pathology

Microscopic: Of the 6 (-/-) mice examined during necropsy, situs inversus was noted in 2 of the mutants and in several other mutant mice evaluated clinically. Situs inversus is a congenital abnormality characterized by lateral transposition of the viscera (such as the heart or liver). The less than expected number of homozygous (-/-) mice suggests reduced viability of affected embryos. No other significant lesions were noted in the mutants.
CATScan: Of the 5 (-/-) mice analyzed, 3 exhibited complete situs inversus.
Embryonic Expression of UNQ5785: UNQ5785 is expressed in the node and its derivatives as shown by lacZ staining (7.5 dpc). Laterality is established at the level of the NODE by the process of NODAL flow (motile cilia line the pit of the node and spin in one direction. Fluorescent beads placed within the mouse node highlight a circular flow of extra-embryonic fluid. This flow is essential for a shift in Ca++ concentration on the left side of the node. L/R patterning is randomized in UNQ5785 mutants (tail flipping and cardiac looping are randomized in UNQ5785 mutants. In UNQ5785 heterozygous mice (9.5 dpc), UNQ5785 expression is maintained in the midline along the length of the floorplate. Thus, UNQ5785 mutants potentially have cilia defects and can be involved in signal transduction controlling the left-right axis pattern. Cilia have been shown to be essential for signal transduction of the morphogen Sonic Hedge Hog (Sff). Mutations in Shh are strongly correlated with basal cell carcinoma and other cancers.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Acute Phase Response:

Test Description: Bacterial lipopolysaccharide (LPS) is an endotoxin, and as such is a potent inducer of an acute phase response and systemic inflammation. The Level I LPS mice were injected intraperitoneally (i.p.) with a sublethal dose of LPS in 200 µL sterile saline using a 26 gauge needle. The doses were based on the average weight of the mice tested at 1 µg/g body weight 3 hours after injection; a 100ul blood sample was then taken and analyzed for the presence of TNFa, MCP-1, and IL-6 on the FACS Calibur instrument.

### Results:

The (-/-) mice exhibited an increased mean serum IL-6, MCP 1 and TNF-alpha response to LPS challenge when compared with their (+/+) littermates and the historical mean.

In summary, the LPS endotoxin challenge demonstrated that knockout mice deficient in the gene encoding PRO19563 polypeptides exhibit immunological abnormalities when compared with their wild-type littermates. In particular, the mutant mice exhibited an increased ability to elicit an immunological response (TNF-alpha, MCP1 and IL-6 production) when challenged with the LPS endotoxin indicating a pronounced proinflammatory response. These inflammatory cytokines play a critical role in inducing the acute phase response and systemic inflammation. This suggests that antagonists (inhibitors) of PRO19563 polypeptides would stimulate the immune system and would find utility in the cases wherein this effect would be beneficial to the individual such as in the case of leukemia, and other types of cancer, and in immunocompromised patients, such as AIDS sufferers. Accordingly, PRO19563 polypeptides or agonists thereof, would be useful in inhibiting the immune response and would be useful candidates for suppressing harmful immune responses, e.g. in the case of graft rejection or graft-versus-host diseases.

### Flourescence-activated cell-sorting (FACS) Analysis

### Procedure:

FACS analysis of immune cell composition from peripheral blood was performed including CD4, CD8 and T cell receptor to evaluate T lymphocytes, CD19 for B lymphocytes, CD45 as a leukocyte marker and pan NK for natural killer cells. The FACS analysis was carried out on wild type and homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel of six lineage-specific antibodies: CD45 PerCP, anti-TCRb APC, CD4 PE, CD8 FITC, pan-NK PE, and CD 19 FITC. The two FITC and PE labeled antibodies stain mutually exclusive cell types. The samples were analyzed using a Becton Dickinson FACSCalibur flow cytometer with CellQuest software.

### Results:

FACS3: The (-/-) mice exhibited an altered distribution of leukocyte subsets in the peripheral blood, characterized by a decreased mean percentage of CD4 cells and an increased mean percentage of B cells when compared with those of their (+/+) littermates and the historical means.

Thus, knocking out the gene which encodes PRO19563 polypeptides causes a decrease in the T cell population as well as causing an increase in the B cell population. From these observations, PRO19563 polypeptides or the gene encoding PRO19563 appears to function as a negative regulator of B cell proliferation. Thus, antagonists or inhibitors of PR019563 polypeptides would be beneficial in enhancing B cell proliferation and depressing T cell proliferation.

### Hematology Analysis:

Test Description: Blood tests are carried out by Abbott's Cell-Dyn 3500R, an automated hematology analyzer. Some of its features include a five-part WBC differential. 'Patient'reports can cover over 22 parameters in all.

### Results:

Hematology: The (-/-) mice exhibited decreased median white blood cell and absolute neutrophil counts when compared with those of their (+/+) littermates and the historical medians for each.

### (d) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes.

### Results:

Blood Chemistry: The male (-/-) mice exhibited an increased mean serum glucose level when compared with that o f their gender-matched (+/+) littermates and the historical mean. These results are consistent with the observation of an impaired Glucose Tolerance in the mutant (-/-) mice (shown below).

Thus, the mutant (-/-) mice exhibited hyperglycemia which could be associated with an altered glucose metabolism or diabetes.

### (e) Blood ChemistrylGlucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of wild type and male homozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Results:

### Blood Glucose Levels/Glucose Tolerance Test:

Oral Glucose Tolerance: The 2 male (-/-) mice both exhibited impaired glucose tolerance at the T-30 interval when compared with that of their gender-matched (+/+) littermates and the historical mean.

The (-/-) mice exhibited impaired glucose tolerance when compared with their gender-matched (+/+) littermates and the historical means. The (-/-) mice also exhibited an increased mean fasting serum glucose level.

These studies indicated that (-/-) mice exhibit a decreased or impaired glucose tolerance in the presence of normal fasting glucose at the intervals tested when compared with their gender-matched (+/+) littermates and the historical means. Thus, knockout mutant mice exhibited the phenotypic pattern of an impaired glucose homeostasis, and therefor PRO19563 polypeptides (or agonists thereof) or its encoding gene would be useful in the treatment of conditions associated with an impaired glucose homeostasis and/or various cardiovascular diseases, including diabetes.

### 46.43. Generation and Analysis of Mice Comprising DNA131658-2875 (UNQ5835) Gene Disruptions

In these knockout experiments, the gene encoding PRO19675 polypeptides (designated as DNA131658-2875) (UNQ5835) was disrupted. The gene specific information for these studies is as follows: the mutated mouse gene corresponds to nucleotide reference: NM_026035 ACCESSION:NM_026035 NID: gi 21313399 ref NM_026035.1 Mus musculus mitochondrial ribosomal protein L55 (Mrpl55); protein reference: Q9CZ83 ACCESSION:Q9CZ83 NID: Mus musculus (Mouse). 2810038N09Rik protein; the human gene sequence reference: NM_181465 Homo sapiens mitochondrial ribosomal protein L55 (MRPL55), nuclear gene encoding mitochondrial protein, transcript variant 7; the human protein sequence corresponds to reference: Q7Z7F7 ACCESSION:Q7Z7F7 NID: Homo sapiens (Human). MRPL55 protein (Mitochondrial ribosomal protein L55).

The mouse gene of interest is Mrpl55 (mitochondrial ribosomal protein L55), ortholog of human MRPL55. Aliases include 2810038N09Rik, AAVG5835, and PRO19675.

MRPL55 is a subunit of mitochondrial ribosomes (Koc et al, J Biol Chem: 276:43958-69 (2001); O'Brien, Gene: 286:73-9 (2002); Zhang and Gerstein, Genomics: 81:468-80 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 16 | 35 | 0 | 51 |
| Expected | 12.75 | 25.5 | 12.75 | 51 |

Chi-Sq.= 39.82 Significance= 2.2552615E-9 (hom/n)= 0.0 Avg. Litter Size= 7
Mutation Information
Mutation Type: Homologous Recombination (standard)
Description: Coding exons 1 through 3 were targeted (NCBI accession NM_026035.1).
1. Wild-type Expression Panel: Expression of the target gene was detected in embryonic stem (ES) cells and in all 13 adult tissue samples tested by RT-PCR, except bone.
2. QC Expression: Disruption of the target gene was confirmed by Southern hybridization analysis.

### 46.43.1 PHENOTYPIC ANALYSIS (for disrupted gene: DNA131658-2875 (UNQ5835)

### (a) OVERALL PHENOTYPIC SUMMARY:

Mutation of the gene encoding the ortholog of human mitochondrial ribosomal protein L55 (MRPL55) resulted in lethality of (-/-) mutants. Gene disruption was confirmed by Southern blot.

### Discussion related to embryonic developmental abnormality of lethality:

Embryonic lethality in knockout mice usually results from various serious developmental problems including but not limited to neuro-degenerative diseases, angiogenic disorders, inflammatory diseases, or where the gene/protein has an important role in basic cell signaling processes in many cell types. In addition, embryonic lethals are useful as potential cancer models. Likewise, the corresponding heterozygous (+/-) mutant animals are particularly useful when they exhibit a phenotype and/or a pathology report which reveals highly informative clues as to the function of the knocked-out gene. For instance, EPO knockout animals were embryonic lethals, but the pathology reports on the embryos showed a profound lack of RBCs.

### (b) Pathology

Microscopic: Due to embryonic lethality, microscopic analysis was not performed. At 12.5 days, there were 40 embryos observed: 20 (+/-) embryos, 4 (+/+) embryos, and 16 resorption moles.
Gene Expression: LacZ activity was not detected in the panel of tissues by immunohistochemical analysis.

### 46.44. Generation and Analysis of Mice Comprising DNA168061-2897 (UNQ6124) Gene Disruptions

In these knockout experiments, the gene encoding PR020084 polypeptides (designated as DNA168061-2897) (UNQ6124) was disrupted. The gene specific information for these studies is as follows: the mutated mouse gene corresponds to nucleotide reference: NM_199157 Mus musculus interferon kappa precursor (Ifnk); protein reference: Q7TSL0 ACCESSION:Q7TSL0 NID: Mus musculus (Mouse). Interferon kappa; the human gene sequence reference: NM_020124 Homo sapiens interferon kappa precursor (IFNK); the human protein sequence corresponds to reference: Q9P0W0 ACCESSION:Q9POWO NID: Homo sapiens (Human). Interferon-like protein precursor (Interferon kappa precursor).

The mouse gene of interest is Ifnk (interferon kappa precursor), ortholog of human IFNK.

IFNK is a secreted protein and member of the type I interferon family, functioning as a signal-transducing ligand. The protein binds with and activates the same receptor as other type I interferons, activating the JAK/STAT signaling cascade. IFNK is expressed primarily in keratinocytes, where it is upregulated in response to viral infection, double-stranded RNA, interferon-gamma, or interferon-beta. Thus, IFNK likely plays a role in innate immunity, providing cellular protection against viral infection (LaFleur et al, J Biol Chem: 276:39765-71 (2001)). IFNK is also expressed in peritoneal macrophages and is upregulated by double-stranded RNA and interferon-gamma (Vassileva et al, J Immunol: 170:5748-55 (2003)). Moreover, IFNK stimulates the release of cytokines from monocytes and dendritic cells, suggesting that IFNK also plays a role in regulating immune cell functions (Nardelli et al, J Immunol: 169:4822-30 (2002)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 37 | 22 | 78 |
| Expected | 19.5 | 39 | 19.5 | 78 |

Chi-Sq.= 1.11 Significance= 0.57407224 (hom/n)= 0.25 Avg. Litter Size= 9
Mutation Information
Mutation Type: Homologous Recombination (standard)
Description: The gene consists of 2 exons, with the start codon located in exon 1 (NCBI accession NM_199157.1). Exons 1 and 2 were targeted.
1. Wild-type Expression Panel: Expression of the target gene was detected in embryonic stem (ES) cells and in all 13 adult tissue samples tested by RT-PCR, except adipose.
2. QC Expression: Disruption of the target gene was confirmed by Southern hybridization analysis.

### 46.44.1. PHENOTYPIC ANALYSIS (for disrupted gene: DNA168061-2897 (UNQ6124)

### (a) OVERALL PHENOTYPIC SUMMARY:

Mutation of the gene encoding the ortholog of human interferon kappa precursor (IFNK) resulted in the mutant (-/-) mice exhibiting an abnormal habituation response to a novel environment, and decreased CD11bB220-/CD117- cells in the peritoneal cavity. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination ofthe affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample oflysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACSCalibur flow cytometer with CellQuest software.

### Results:

Tissue Specific FACS: The (-/-) mice exhibited a decreased mean percentage of CD11b Hi/B220-/CD117- cells in peritoneal lavage when compared with that of their (+/+) littermates.

Thus, knocking out the gene which encodes PR020084 polypeptides causes an decrease in the T cell subset population. From these observations, PR020084 polypeptides or the gene encoding PR020084 appears to function as a regulator of T cell proliferation.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mutant mice. All behavioral tests were done between 12 and 16 weeks of age unless reduced viability necessitates earlier testing. These tests included open field to measure anxiety, activity levels and exploration.

### Open field test:

Several targets ofknown drugs have exhibited phenotypes in the open field test. These include knockouts of the seratonin transporter, the dopamine transporter (Giros et al., Nature. 1996 Feb 15;379(6566):606-12), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value. Analyzed wt/ het/ hom: 5/ 4/ 8

### Results:

Openfield2: The (-/-) mice exhibited an increased median normalized slope when compared with that of their (+/+) littermates, suggesting an abnormal habituation (decreased or hypoactivity) response in a novel environment.

A notable difference was observed during open field activity testing. The (-/-) mice exhibited hypoactivity when compared with their gender-matched (+/+) littermates, which is indicative of a decreased anxiety-like response in the mutants. Thus, knockout mice demonstrated a phenotype consistent with depression, generalized anxiety disorders, cognitive disorders, hyperalgesia and sensory disorders and/or bipolar disorders. Thus, PRO20084 polypeptides and agonists thereof would be useful for the treatment or amelioration of the symptoms associated with depressive disorders.

### 46.45. Generation and Analysis of Mice Comprising DNA147253-2983 (UNQ6509) Gene Disruptions

In these knockout experiments, the gene encoding PR021434 polypeptides (designated as DNA147253-2983) (UNQ6509) was disrupted. The gene specific information for these studies is as follows: the mutated mouse gene corresponds to nucleotide reference: NM_001001183 Mus musculus cDNA sequence BC054438 (BC054438); protein reference: Q7TQI0 ACCESSION:Q7TQI0 NID: Mus musculus (Mouse). CDNA sequence BC054438; the human gene sequence reference: BC004932 ACCESSION:BC004932 NID:13436268 Homo sapiens Homo sapiens hypothetical protein FLJ20898; the human protein sequence corresponds to reference: Q9BSN7 ACCESSION:Q9BSN7 NID: Homo sapiens (Human).

The mouse gene of interest is cDNA sequence BC054438, ortholog of human C160rf30 (chromosome 16 open reading frame 30). Aliases include CLP24 and FLJ20898.

C16orf30 is an integral plasma membrane protein that likely functions as a modulator of cell adhesion. The protein contains four transmembrane segments and localizes to cell-cell junctions but not tight junctions. C16orf30 is expressed in lung, heart, kidney, and placenta, and C16orf30 expression is increased in response to hypoxia. Overexpression of C16orf30 in vitro decreases cell adhesion and modulates junctional barrier function. C16orf30 may play a role in angiogenesis (Kearsey et al, Eur J Biochem: 271:2584-92 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 43 | 20 | 84 |
| Expected | 21 | 42 | 21 | 84 |

Chi-Sq.= 2.83 Significance= 0.24292563 (hom/n)= 0.22 Avg. Litter Size= 10
Mutation Information
Mutation Type: Homologous Recombination (standard)
Description: Coding exon 1 was targeted (NCBI accession NM_001001183.1).
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 46.45.1. PHENOTYPIC ANALYSIS (for disrupted gene: DNA147253-2983 (UNQ6509)

### (a) OVERALL PHENOTYPIC SUMMARY:

Mutation of the gene encoding the ortholog of human chromosome 16 open reading frame 30 (C16orf30) resulted in immunological abnormalities in (-/-) mice. The mutant (-/-) mice also exhibited decreased total tissue mass and leans body mass and decreased bone-related measurements. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Flourescence-activated cell-sorting (FA CS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACSCalibur flow cytometer with CellQuest software.

### Results:

Tissue Specific FACS: The (-/-) mice exhibited a decreased mean CD4-to-CD8 ratio in spleen when compared with that of their (+/+) littermates. The (-/-) mice also exhibited a decreased mean percentage of B220Hi/CD23+ cells and increased mean percentages of B220Med/CD23- cells and B220+/CD11b Low/CD23- cells in peritoneal lavage.

These observations indicate that there is a change of B cell subtypes in the peritoneal lavage. Also, a decrease in the CD4/CD8 ration in the spleen was observed. Thus, it appears that PR021434 polypeptides act as a regulator for B cell production.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of 4 wild type, 4 heterozygotes and 8 homozygotes were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean total tissue mass and lean body mass when compared with the historical means.
micro CT: The male (-/-) mice exhibited a decreased mean femoral mid-shaft cross-sectional area when compared with that of their gender-matched (+/+) littermates and the historical mean.

The (-/-) mice analyzed by DEXA and bone micro CT analysis exhibited decreased bone measurements and decreased body mass measurements when compared with their (+/+) littermates, suggestive of abnormal bone disorders. Thus, the (-/-) mice exhibited a negative bone phenotype. In addition, the decreased mean total tissue mass and lean body mass is indicative of a metabolic disorder related to tissue wasting disorders. The negative bone phenotype indicates that PR021434 polypeptides or agonists thereof would be useful for maintaining bone homeostasis. In addition, PR021434 polypeptides would be useful in bone healing or for the treatment of arthritis or osteoporosis, whereas antagonists (or inhibitors) of PR021434polypeptides or its encoding gene would lead to abnormal or pathological bone disorders including inflammatory diseases associated with abnormal bone metabolism including arthritis, osteoporosis and osteopenia.

### 46.46. Generation and Analysis of Mice Comprising DNA255255 (UNQ11645) Gene Disruptions

In these knockout experiments, the gene encoding PR050332 polypeptides (designated as DNA255255) (UNQ 11645) was disrupted. The gene specific information for these studies is as follows: the mutated mouse gene corresponds to nucleotide reference: NM_026228 ACCESSION:NM_026228 NID: gi 31541989 refNM_026228.2 Mus musculus RIKEN cDNA 4933419D20 gene (4933419D20Rik); protein reference: Q8BTQ3 ACCESSION:Q8BTQ3 NID: Mus musculus (Mouse). BCG induced integral membrane protein BIGMO-103; the human gene sequence reference: NM_022154 Homo sapiens solute carrier family 39 (zinc transporter), member 8 (SLC39A8); the human protein sequence corresponds to reference: Q9C0K1 ACCESSION:Q9C0K1 NID: Homo sapiens (Human). BCG induced integral membrane protein BIGMo-103 (Up-regulated by BCG- CWS) (Hypothetical protein).

The mouse gene of interest is Slc39a8 (solute carrier family 39 [metal ion transporter], member 8), ortholog of human SLC39A8 (solute carrier family 39 [zinc transporter], member 8). Aliases include BIGM103, LZT-Hs6, and 4933419D20Rik.

SLC39A8 is an integral plasma membrane protein that likely functions as a zinc transporter. The protein contains at least seven transmembrane segments and an overlapping ZIP zinc transporter domain. SLC39A8 is expressed in monocytes in response to treatment with bacteria, bacterial cell wall, or inflammatory cytokines, suggesting that the protein may play a role in innate immunity. Upon differentiation of monocytes to dendritic cells and then macrophages, expression of SLC39A8 is readily detected without stimulation (Begum et al, Genomics: 80:630-45 (2002)). SLC39A8 is expressed in hormonally controlled tissues, such as mammary gland, and is concentrated on vascular endothelial cells of testis. SLC39A8 transports not only zinc but also cadmium, which causes testicular necrosis at toxic cadmium levels. Because zinc is a cofactor for a wide variety of enzymes, SLC39A8 likely plays a role in a number of physiological and disease processes, such as cancer (Taylor et al, Biochem J: 375:51-9 (2003); Dalton et al, Proc Natl Acad Sci U S A: 102:3401-6 (2005); Taylor and Nicholson; Biochim Biophys Acta: 1611:16-30 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 35 | 77 | 1 | 113 |
| Expected | 28.25 | 56.5 | 28.25 | 113 |

Chi-Sq.= 35.34 Significance= 2.1184414E-8 (hom/n)= 0.01 Avg. Litter Size= 7
Mutation Information
Mutation Type: Homologous Recombination (standard)
Description: The gene consists of 8 exons, with the start codon located in exon 1 (NCBI accession NM_026228.2). Exons 3 through 5 were targeted.
1. Wild-type Expression Panel: Panel: Expression of the target gene was detected in embryonic stem (ES) cells and in all 13 adult tissue samples tested by RT-PCR, except skeletal muscle, bone, and adipose.
2. QC Expression: Disruption of the target gene was confirmed by Southern hybridization analysis.

### 46.46.1. PHENOTYPIC ANALYSIS (for disrupted gene: DNA255255 (UNQ11645)

### (a) OVERALL PHENOTYPIC SUMMARY:

Mutation of the gene encoding the ortholog of human solute carrier family 39 (zinc transporter), member 8 (SLC39A8) resulted in lethality of (-/-) mutants. Gene disruption was confirmed by Southern blot.

### Discussion related to embryonic developmental abnormality of lethality:

Embryonic lethality in knockout mice usually results from various serious developmental problems including but not limited to neuro-degenerative diseases, angiogenic disorders, inflammatory diseases, or where the gene/protein has an important role in basic cell signaling processes in many cell types. In addition, embryonic lethals are useful as potential cancer models. Likewise, the corresponding heterozygous (+/-) mutant animals are particularly useful when they exhibit a phenotype and/or a pathology report which reveals highly informative clues as to the function of the knocked-out gene. For instance, EPO knockout animals were embryonic lethals, but the pathology reports on the embryos showed a profound lack of RBCs.

### (b) Pathology

Homozygous lethal. The single (-/-) pup was dead at the time of genotyping.
Microscopic: Due to embryonic lethality, microscopic analysis was not performed. At 12.5 days there were 43 embryos observed: 22 (+/-) embryos, 9 (+/+) embryos, and 12 resorption moles.
Gene Expression: LacZ activity was not detected in the panel of tissues by immunohistochemical analysis.

### 46.47. Generation and Analysis of Mice Comprising DNA228002 (UNQ15965) Gene Disruptions

In these knockout experiments, the gene encoding PR038465 polypeptides (designated as DNA228002) (UNQ15965) was disrupted. The gene specific information for these studies is as follows: the mutated mouse gene corresponds to nucleotide reference: NM_026835 ACCESSION:NM_026835 NID:gi 13386171 refNM_026835.1 Mus musculus membrane-spanning 4-domains, subfamily A, member 6D (Ms4a6d); protein reference: Q99N07 ACCESSION:Q99N07 NID: Mus musculus (Mouse). Membrane-spanning 4-domains subfamily A member 6D (CD20 antigen-like 8); the human gene sequence reference: NM_152852 Homo sapiens membrane-spanning 4-domains, subfamily A, member 6A (MS4A6A), transcript variant 1; the human protein sequence corresponds to reference: Q9H2W1 ACCESSION:Q9H2W1 NID: Homo sapiens (Human). Membrane-spanning 4-domains subfamily A member 6A (Four-span transmembrane protein 3) (CD20 antigen-like 3) (CDA01).

The mouse gene of interest is Ms4a6d (membrane-spanning 4-domains, subfamily A, member 6D), putative ortholog of human MS4A6A (membrane-spanning 4-domains, subfamily A, member 6A). Aliases include 1110058E16Rik, CDA01, MS4A6, 4SPAN3, CD20L3, MST090, MSTP090, 4SPAN3.2, and MGC22650.

MS4A6A is an integral plasma membrane protein that may function as a component of an oligomeric signal-transducing receptor. The protein consists of a cytoplasmic N-terminus, four transmembrane segments highly conserved among MS4A family members, and a cytoplasmic C-terminus. MS4A6A is expressed in B-cell, myelomonocytic, and erythroleukemia cell lines (Liang and Teddar, Genomics: 72:119-27 (2001)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 20 | 38 | 15 | 73 |
| Expected | 18.25 | 36.5 | 18.25 | 73 |

Chi-Sq.= 0.53 Significance= 0.76720595 (hom/n)= 0.23 Avg. Litter Size= 8
Mutation Information
Mutation Type: Homologous Recombination (standard)
Description: Coding exons 1 and 2 and the exon preceding coding exon 1 were targeted (NCBI accession NM_026835.1).
1. Wild-type Expression Panel: Expression of the target gene was detected in all 13 adult tissue samples tested by RT-PCR, except skeletal muscle and stomach, small intestine, and colon.
2. QC Expression: Disruption of the target gene was confirmed by Southern hybridization analysis.

### 46.47.1. PHENOTYPIC ANALYSIS (for disrupted gene: DNA228002 (UNQ15965)

### (a) OVERALL PHENOTYPIC SUMMARY:

Mutation of the gene encoding the ortholog of human membrane-spanning 4-domains, subfamily A, member 6A (MS4A6A) resulted in an increased serum IgG1 response to ovalbumin challenge in (-/-) mice. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Ovalbumin Challenge

Procedure: This assay was carried out on 7 wild types and 8 homozygotes. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immunodominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG 1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice exhibited an increased mean serum IgG1 response to ovalbumin challenge when compared with that of their (+/+) littermates and the historical means.

In summary, the ovalbumin challenge studies indicate that knockout homozygous mice deficient in the gene encoding PR038465 polypeptides exhibit immunological abnormalities when compared with their wild-type littermates. In particular, the mutant (-/-) mice exhibited an increased ability to elicit an immunological response when challenged with the T-cell dependent OVA antigen. Thus, antagonists (inhibitors) of PR038465 polypeptides would be useful for stimulating the immune system (such as T cell proliferation) and would find utility in the cases wherein this effect would be beneficial to the individual such as in the case of leukemia, and other types of cancer, and in immuno-compromised patients, such as AIDS sufferers. Accordingly, PR038465 polypeptides or agonists thereof, would be useful for inhibiting the immune response and thus would be useful candidates for suppressing harmful immune responses, e.g. in the case of graft rejection or graft-versus-host diseases.

### 46.48. Generation and Analysis of Mice Comprising DNA44167-1243 (UNQ305) Gene Disruptions

In these knockout experiments, the gene encoding PR0346 polypeptides (designated as DNA44167-1243) (UNQ305) was disrupted. The gene specific information for these studies is as follows: the mutated mouse gene corresponds to nucleotide reference: NM_178899 Mus musculus expressed sequence AI987662 (AI987662); protein reference: NP_849230 expressed sequence AI987662 [Mus musculus] gi|26341264|dbj|BAC34294.1| unnamed protein product [Mus musculus] gi|26329423|dbj|BAC28450.1| unnamed protein product [Mus musculus]; the human gene sequence reference: NM_198151 Homo sapiens hypothetical protein LOC253012 (LOC253012); the human protein sequence corresponds to reference: Q6UXI0 ACCESSION:Q6UXI0 NID: Homo sapiens (Human). WLKV305.

The mouse gene of interest is "expressed sequence AI987662," ortholog of human "hypothetical protein LOC253012."

Hypothetical protein LOC253012 is a putative integral plasma membrane protein (Clark et al, Genome Res.: 13:2265-70 (2003)) that likely functions as a cell adhesion molecule orreceptor. The protein contains a signal peptide, three immunoglobulin (Ig)-like domains, a transmembrane segment, and a 90-amino acid cytoplasmic domain.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 29 | 35 | 16 | 80 |
| Expected | 20 | 40 | 20 | 80 |

Chi-Sq.= 2.1 Significance= 0.34993777 (hom/n)= 0.24 Avg. Litter Size= 9
Mutation Information
Mutation Type: Homologous Recombination (standard)
Description: Coding exon 2 was targeted (NCBI accession NM_178899.3).
WT Panel: Expression of the target gene was detected in embryonic stem (ES) cells and in all 13 adult tissue samples tested by RT-PCR, except skeletal muscle and bone.
QC Images: Disruption of the target gene was confirmed by Southern hybridization analysis.

### 46.48.1. PHENOTYPIC ANALYSIS (for disrupted gene: DNA44167-1243 (UNQ305)

### (a) OVERALL PHENOTYPIC SUMMARY:

Mutation of the gene encoding the ortholog of a hypothetical human protein (LOC253012) resulted in immunological abnormalities in the mutant (-/-) mice. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Ovalbumin Challenge

Procedure: This assay was carried out on 7 wild types and 8 homozygotes. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immunodominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

The (-/-) mice exhibited an undetectable mean serum IgG2a response to albumin (impaired IgG2a response) when compared with their (+/+) littermates and the historical mean. However, mean serum IgGllevels were increased in response to the ovalbumin challenge.

In summary, the ovalbumin challenge studies indicate that knockout mice deficient in the gene encoding PR0346 polypeptides exhibit immunological abnormalities when compared with their wild-type littermates. In particular, the mutant mice exhibited a decreased ability to elicit an IgG2a immunological response when challenged with the T-cell dependent OVA antigen (thus, an impaired IgG2a response to the antigen was noted). However, the mutant (-/-) mice exhibited an increased ability to elicit an immunological response with respect to IgG 1 when challenged with the T cell dependent Ova antigen.

### Flourescence-activated cell-sorting (FACS) Analysis

### Procedure:

FACS analysis of immune cell composition from peripheral blood was performed including CD4, CD8 and T cell receptor to evaluate T lymphocytes, CD19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACSCalibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACSCalibur flow cytometer with CellQuest software.

### Results:

The mutant (-/-) mice exhibited an increase in percentages of B cells in Peyer's patches with decreased germinal center, isotype-switched B cells (CD38low; IgM negative).

### Acute Phase Response:

Test Description: Bacterial lipopolysaccharide (LPS) is an endotoxin, and as such is a potent inducer of an acute phase response and systemic inflammation. The Level I LPS mice were injected intraperitoneally (i.p.) with a sublethal dose of LPS in 200 µL sterile saline using a 26 gauge needle. The doses were based on the average weight of the mice tested at 1 µg/g body weight 3 hours after injection; a 100u1 blood sample was then taken and analyzed for the presence of TNFa, MCP-1, and IL-6 on the FACS Calibur instrument.

### Results:

The (-/-) mice exhibited an increased Acute Phase response to LPS indicating a proinflammatory response compared with that of their gender-matched (+/+) littermates.

### EXAMPLE 47: Use of PRO218, PRO228, PRO271, PRO273, PRO295, PRO302, PRO305, PRO326, PRO386, PRO655, PRO162, PRO788, PRO792, PRO940, PRO941. PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 as a hybridization probe

The following method describes use of a nucleotide sequence encoding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide as a hybridization probe.

DNA comprising the coding sequence of full-length or mature PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides as disclosed herein is employed as a probe to screen for homologous DNAs (such as those encoding naturally-occurring variants of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides) in human tissue cDNA libraries or human tissue genomic libraries.

Hybridization and washing of filters containing either library DNAs is performed under the following high stringency conditions. Hybridization of radiolabeled PR0218-, PR0228-, PR0271-, PR0273-, PR0295-, PR0302-, PR0305-, PR0326-, PR0386-, PR0655-, PRO162-, PR0788-, PR0792-, PR0940-, PR0941-, PRO1004-, PRO1012-, PRO1016-, PR0474-, PR05238-, PRO1069-, PRO1111-, PRO1113-, PR01130-, PRO1195-, PRO1271-, PRO1865-, PRO1879-, PRO3446-, PR03543-, PR04329-, PR04352-, PR05733-, PR09859-, PR09864-, PR09904-, PR09907-, PRO10013-, PR090948-, PR028694-, PRO16089-, PRO19563-, PRO19675-, PR020084-, PR021434-, PR050332-, PR038465- or PR0346-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1 x SSC and 0.1% SDS at 42°C.

DNAs having a desired sequence identity with the DNA encoding full-length native sequence PR0218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 polypeptides can then be identified using standard techniques known in the art.

### EXAMPLE 48: Expression of PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069. PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 in E. coli

This example illustrates preparation of an unglycosylated form of PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PR01069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides by recombinant expression in E. *coli.*

The DNA sequence encoding a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide is initially amplified using selected PCR primers. The primers should contain restriction enzyme sites which correspond to the restriction enzyme sites on the selected expression vector. A variety of expression vectors may be employed. An example of a suitable vector is pBR322 (derived from E. *coli*; see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PRO5238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 coding region, lambda transcriptional terminator, and an argU gene.

The ligation mixture is then used to transform a selected E. *coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PR0218, PR0228, PR0271, PR0273, PR0295, PR0302, PR0305, PR0326, PR0386, PR0655, PRO162, PR0788, PRO792, PR0940, PR0941, PRO1004, PRO1012, PRO1016, PRO474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 protein can then be purified using a metal chelating column under conditions that allow tight binding of the protein.

PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865. PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 may be expressed in *E. coli* in a poly-His tagged form, using the following procedure. The DNA encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an E. *coli* host based on strain 52 (W3110 fuhA(tonA) Ion galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g (NH₄)₂SO₄, 0.71 g sodium citrate•2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1 M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PR0218, PR0228, PR0271, PR0273, PR0295, PR0302, PR0305, PR0326, PR0386, PR0655, PRO162, PR0788, PRO792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

### EXAMPLE 49: Expression of PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PR020084, PRO21434, PRO50332, PRO38465 or PRO346 in mammalian cells

This example illustrates preparation of a potentially glycosylated form of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR03 8465 or PR0346 polypeptide by recombinant expression in mammalian cells.

The vector, pRK5 (see EP 307,247, published March 15, 1989), is employed as the expression vector. Optionally, the PR0218, PR0228, PR0271, PR0273, PR0295, PR0302, PR0305, PR0326, PR0386, PR0655, PRO162, PR0788, PR0792, PR0940, PRO941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PRO9859, PR09864, PR09904, PR09907, PRO10013, PRO90948, PR028694, PRO16089, PRO19563, PRO19675, PR020084, PR021434, PR050332, PR038465 or PR0346 DNA is ligated into pRK5 with selected restriction enzymes to allow insertion of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 DNA using ligation methods such as described in Sambrook et al., supra. The resulting vector is called pRK5-PRO218, pRK5-PRO228, pRK5-PRO271, pRK5-PRO273, pRK5-PRO295, pRK5-PR0302, pRK5-PRO305, pRK5-PRO326, pRK5-PRO386, pRK5-PRO655, pRK5-PRO162, pRK5-PRO788, pRK5-PRO792, pRK5-PRO940, pRK5-PRO941, pRK5-PRO1004, pRK5-PRO1012, pRK5-PRO1016, pRK5-PR0474, pRK5-PRO5238, pRK5-PRO1069, pRK5-PRO1111, pRK5-PRO1113, pRK5-PRO1130, pRK5-PRO1195, pRK5-PRO1271, pRK5-PRO1865, pRK5-PRO1879, pRK5-PRO3446, pRK5-PRO3543, pRK5-PR04329, pRK5-PRO4352, pRK5-PRO5733, pRK5-PRO9859, pRK5-PRO9864, pRK5-PRO9904, pRK5-PR09907, pRK5-PRO10013,pRK5-PRO90948, pRK5-PRO28694, pRK5-PRO16089, pRK5-PRO19563, pRK5-PR019675, pRK5-PRO20084, pRK5-PRO21434, pRK5-PRO50332, PR038465 or pRK5-PRO346.

The selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO218, pRK5-PRO228, pRK5-PRO271, pRK5-PRO273, pRK5-PRO295, pRK5-PRO302, pRK5-PRO305, pRK5-PRO326, pRK5-PRO386, pRK5-PRO655, pRK5-PRO162, pRK5-PRO788, pRK5-PRO792, pRK5-PRO940, pRK5-PRO941, pRK5-PRO1004, pRK5-PRO1012, pRK5-PRO1016, pRK5-PRO474,pRK5-PRO5238, pRK5-PRO1069, pRK5-PRO1111,pRK5-PRO1113,pRK5-PRO1130, pRK5-PRO1195, pRK5-PRO1271, pRK5-PRO1865, pRK5-PRO1879, pRK5-PRO3446, pRK5-PR03543, pRK5-PRO4329, pRK5-PRO4352, pRK5-PRO5733, pRK5-PRO9859, pRK5-PRO9864, pRK5-PR09904, pRK5-PRO9907, pRK5-PRO10013, pRK5-PRO90948, pRK5-PRO28694, pRK5-PRO16089, pRK5-PR019563, pRK5-PRO19675, pRK5-PRO20084, pRK5-PRO21434, pRK5-PRO50332, PR038465 or pRK5-PR0346 DNA is mixed with about 1 µg DNA encoding the VA RNA gene [Thimmappaya et al., Cell, 31:543 (1982)] and dissolved in 500 µl of 1 mM Tris-HCI, 0.1 mM EDTA, 0.227 M CaC1₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PRO5238, PRO1069, PRO1111, PRO1113, PRO1130, PRO1195, PRO1271, PRO1865, PRO1-879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides. The cultures containing transfected cells may undergo further incubation (in serum free medium) and the medium is tested in selected bioassays.

In an alternative technique, PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PR01069, PRO1111, PRO1113, PRO1130, PRO1195, PR01271, PR01865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PR0218, pRK5-PRO228, pRK5-PRO271, pRK5-PRO273, pRK5-PRO295, pRK5-PRO302, pRK5-PRO305, pRK5-PRO326, pRK5-PRO386, pRK5-PRO655, pRK5-PRO162, pRK5-PRO788, pRK5-PRO792, pRK5-PRO940, pRK5-PRO941, pRK5-PRO1004, pRK5-PRO1012, pRK5-PRO1016, pRK5-PRO474, pRK5-PRO5238, pRK5-PRO1069, pRK5-PRO1111, pRK5-PRO1113, pRK5-PRO1130, pRK5-PRO1195, pRK5-PRO1271, pRK5-PR01865, pRK5-PRO1879, pRK5-PRO3446, pRK5-PRO3543, pRK5-PRO4329, pRK5-PRO4352, pRK5-PR05733, pRK5-PRO9859, pRK5-PRO9864, pRK5-PRO9904, pRK5-PRO9907, pRK5-PRO10013, pRK5-PR090948, pRK5-PRO28694, pRK5-PRO16089, pRK5-PRO19563, pRK5-PRO19675, pRK5-PR020084, pRK5-PR021434, pRK5-PRO50332, PRO38465 or pRK5-PRO346 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16099, PRO19563, PRO19675, PR020084, PR021434, PR050332, PR038465 or PR0346 can then be concentrated and purified by any selected method, such as dialysis and/or column chromatography.

PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PRO474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 can be expressed in CHO cells. The pRK5-PRO218, pRK5-PRO228, pRK5-PRO271, pRK5-PRO273, pRK5-PRO295, pRK5-PRO302, pRK5-PRO305, pRK5-PRO326, pRK5-PRO386, pRK5-PRO655, pRK5-PRO162, pRK5-PRO788, pRK5-PR0792, pRK5-PRO940, pRK5-PRO941, pRK5-PRO1004, pRK5-PRO1012, pRK5-PRO1016, pRK5-PRO474, pRK5-PRO5238,pRK5-PRO1069,pRK5-PRO1111,pRK5-PRO1113,pRK5-PRO1130,pRK5-PRO1195,pRK5-PR01271, pRK5-PRO1865, pRK5-PRO1879, pRK5-PRO3446, pRK5-PRO3543, pRK5-PRO4329, pRK5-PR04352, pRK5-PRO5733, pRK5-PRO9859, pRK5-PRO9864, pRK5-PRO9904, pRK5-PRO9907, pRK5-PRO10013, pRK5-PRO90948, pRK5-PRO28694, pRK5-PRO16089, pRK5-PRO19563, pRK5-PRO19675, pRK5-PR020084, pRK5-PRO21434, pRK5-PRO50332, PR03 8465 or pRK5-PRO346 can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PR01113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the culture medium may be replaced with serum free medium. Preferably, the cultures are incubated for about 6 days, and then the conditioned medium is harvested. The medium containing the expressed PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 can then be concentrated and purified by any selected method.

Epitope-tagged PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 may also be expressed in host CHO cells. The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PR01113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 may be subcloned out of the pRK5 vector. The subclone insert can undergo PCR to fuse in frame with a selected epitope tag such as a poly-his tag into a Baculovirus expression vector. The poly-his tagged PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 can then be concentrated and purified by any selected method, such as by Ni²⁺-chelate affinity chromatography.

PR0218, PR0228, PR0271, PR0273, PR0295, PR0302, PR0305, PR0326, PR0386, PR0655, PR0162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG 1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect® (Qiagen), Dosper® or Fugene® (Boehringer Mannheim). The cells are grown as described in Lucas et al., supra. Approximately 3 x 10⁷ cells are frozen in an ampule for further growth and production as described below.

The ampules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 µm filtered PS20 with 5% 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x 10⁵ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/mL. On day 0, the cell number pH ie determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 µL of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

### EXAMPLE 50: Expression of PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 in Yeast

The following method describes recombinant expression of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 in yeast.

First, yeast expression vectors are constructed for intracellular production or secretion of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PR01879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 from the ADH2/GAPDH promoter. DNA encoding PR0218, PR0228, PR0271, PR0273, PR0295, PR0302, PR0305, PR0326, PR0386, PR0655, PRO162, PR0788, PR0792, PR0940, PR0941, PRO1004, PR01012, PRO1016, PR0474, PR05238, PRO1069, PRO1111, PRO1113, PRO1130, PRO1195, PRO1271, PRO1865, PRO1879, PR03446, PR03543, PRO4329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 and the promoter is inserted into suitable restriction enzyme sites in the selected plasmid to direct intracellular expression of PR0218, PR0228, PR0271, PR0273, PR0295, PR0302, PR0305, PR0326, PR0386, PR0655, PRO162, PR0788, PR0792, PR0940, PRO941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346. For secretion, DNA encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR0523 8, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 can be cloned into the selected plasmid, together with DNA encoding the ADH2/GAPDH promoter, a native PR0218, PR0228, PR0271, PRO273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 signal peptide or other mammalian signal peptide, or, for example, a yeast alpha-factor or invertase secretory signal/leader sequence, and linker sequences (if needed) for expression of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PRO9859, PRO9864, PR09904, PR09907, PRO10013, PR090948, PR028694, PRO16089, PRO19563, PRO19675, PR020084, PR021434, PR050332, PR03 8465 or PR0346 can subsequently be isolated and purified by removing the yeast cells from the fermentation medium by centrifugation and then concentrating the medium using selected cartridge filters. The concentrate containing PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PR01865, PRO1879, PR03446, PR03543, PRO4329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 may further be purified using selected column chromatography resins.

### EXAMPLE 51: Expression of PRO218, PRO228, PRO271, PR0273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PRO38465 or PRO346 in Baculovirus-Infected Insect Cells

The following method describes recombinant expression of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346in Baculovirus-infected insect cells.

The sequence coding for PR0218, PR0228, PR0271, PR0273, PR0295, PR0302, PR0305, PR0326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PR09859, PR09864, PR09904, PR09907, PRO10013, PR090948, PR028694, PRO16089, PRO19563, PRO19675, PR020084, PR021434, PR050332, PR038465 or PR0346 is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 or the desired portion of the coding sequence of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PRO9859, PRO9864, PRO9904, PRO9907, PRO10013, PR090948, PR028694, PRO16089, PRO19563, PRO19675, PR020084, PR021434, PR050332, PR038465 or PR0346 can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or Western blot with Ni²⁺-NTA-conjugated to alkaline phosphatase (Qiagen). Fractions containing the eluted His₁₀-tagged PR0218, PR0228, PR0271, PR0273, PR0295, PR0302, PR0305, PR0326, PR0386, PR0655, PRO162, PR0788, PR0792, PR0940, PR0941, PRO1004, PR01012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 are pooled and dialyzed against loading buffer.

Alternatively, purification of the IgG tagged (or Fc tagged) PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PR01069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 can be performed using known chromatography techniques, including for instance, Protein A or protein G column chromatography.

### EXAMPLE 52: Tissue Expression Profiling Using GeneExpress®

A proprietary database containing gene expression information (GeneExpress®, Gene Logic Inc., Gaithersburg, MD) was analyzed in an attempt to identify polypeptides (and their encoding nucleic acids) whose expression is significantly upregulated in a particular tumor tissue(s) of interest as compared to other tumor(s) and/or normal tissues. Specifically, analysis of the GeneExpress® database was conducted using either software available through Gene Logic Inc., Gaithersburg, MD, for use with the GeneExpress® database or with proprietary software written and developed at Genentech, Inc. for use with the GeneExpress® database. The rating of positive hits in the analysis is based upon several criteria including, for example, tissue specificity, tumor specificity and expression level in normal essential and/or normal proliferating tissues. The following is a list of molecules whose tissue expression profile as determined from an analysis of the GeneExpress® database evidences high tissue expression and significant upregulation of expression in a specific tumor or tumors as compared to other tumor(s) and/or normal tissues and optionally relatively low expression in normal essential and/or normal proliferating tissues. Tissue expression profiling was performed on several UNQ genes the results of which are disclosed in Example 46.

### EXAMPLE 53: Microarray Analysis to Detect Upregulation of UNQ Genes in Cancerous Tumors

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (disease tissue) sample is greater than hybridization signal of a probe from a control (normal tissue) sample, the gene or genes overexpressed in the disease tissue are identified. The implication of this result is that an overexpressed protein in a diseased tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30,2001 and which is herein incorporated by reference.

In the present example, cancerous tumors derived from various human tissues were studied for upregulated gene expression relative to cancerous tumors from different tissue types and/or non-cancerous human tissues in an attempt to identify those polypeptides which are overexpressed in a particular cancerous tumor(s). In certain experiments, cancerous human tumor tissue and non-cancerous human tumor tissue of the same tissue type (often from the same patient) were obtained and analyzed for UNQ polypeptide expression. Additionally, cancerous human tumor tissue from any of a variety of different human tumors was obtained and compared to a "universal" epithelial control sample which was prepared by pooling non-cancerous human tissues of epithelial origin, including liver, kidney, and lung. mRNA isolated from the pooled tissues represents a mixture of expressed gene products from these different tissues. Microarray hybridization experiments using the pooled control samples generated a linear plot in a 2-color analysis. The slope of the line generated in a 2-color analysis was then used to normalize the ratios of (test:control detection) within each experiment. The normalized ratios from various experiments were then compared and used to identify clustering of gene expression. Thus, the pooled "universal control" sample not only allowed effective relative gene expression determinations in a simple 2-sample comparison, it also allowed multi-sample comparisons across several experiments.

In the present experiments, nucleic acid probes derived from the herein described UNQ polypeptide-encoding nucleic acid sequences were used in the creation of the microarray and RNA from various tumor tissues were used for the hybridization thereto. Below is shown the results of these experiments, demonstrating that various UNQ polypeptides ofthe present invention are significantly overexpressed in various human tumor tissues as compared to their normal counterpart tissue(s). Moreover, all of the molecules shown below are significantly overexpressed in their specific tumor tissue(s) as compared to in the "universal" epithelial control. As described above, these data demonstrate that the UNQ polypeptides of the present invention are useful not only as diagnostic markers for the presence of one or more cancerous tumors, but also serve as therapeutic targets for the treatment of those tumors. Microarray analysis was performed on several UNQ genes the results of which are disclosed in Example 46.

### EXAMPLE 54: Quantitative Analysis of UNQ mRNA Expression

In this assay, a 5' nuclease assay (for example, TaqMan®) and real-time quantitative PCR (for example, ABI Prizm 7700 Sequence Detection System® (Perkin Elmer, Applied Biosystems Division, Foster City, CA)), were used to find genes that are significantly overexpressed in a cancerous tumor or tumors as compared to other cancerous tumors or normal non-cancerous tissue. The 5' nuclease assay reaction is a fluorescent PCR-based technique which makes use of the 5' exonuclease activity of Taq DNA polymerase enzyme to monitor gene expression in real time. Two oligonucleotide primers (whose sequences are based upon the gene or EST sequence of interest) are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the PCR amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

The 5' nuclease procedure is run on a real-time quantitative PCR device such as the ABI Prism 7700TM Sequence Detection. The system consists of a thermocycler, laser, charge-coupled device (CCD) camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

The starting material for the screen was mRNA isolated from a variety of different cancerous tissues. The mRNA is quantitated precisely, e.g., fluorometrically. As a negative control, RNA was isolated from various normal tissues of the same tissue type as the cancerous tissues being tested.

5' nuclease assay data are initially expressed as Ct, or the threshold cycle. This is defined as the cycle at which the reporter signal accumulates above the background level of fluorescence. The ΔCt values are used as quantitative measurement of the relative number of starting copies of a particular target sequence in a nucleic acid sample when comparing cancer mRNA results to normal human mRNA results. As one Ct unit corresponds to 1 PCR cycle or approximately a 2-fold relative increase relative to normal, two units corresponds to a 4-fold relative increase, 3 units corresponds to an 8-fold relative increase and so on, one can quantitatively measure the relative fold increase in mRNA expression between two or more different tissues. Using this technique, the molecules have been identified as being significantly overexpressed in a particular tumor(s) as compared to their normal non-cancerous counterpart tissue(s) (from both the same and different tissue donors) and thus, represent excellent polypeptide targets for the diagnosis and therapy of cancer in mammals. Specific results for a UNQ gene are disclosed in Example 46.

### EXAMPLE 55: In situ Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis and aid in chromosome mapping.

*In situ* hybridization was performed following an optimized version ofthe protocol by Lu and Gillett, Cell Vision 1:169-176 (1994), using PCR-generated ³³P-labeled riboprobes. Briefly, formalin-fixed, paraffin-embedded human tissues were sectioned, deparaffinized, deproteinated in proteinase K (20 g/ml) for 15 minutes at 37°C, and further processed for *in situ* hybridization as described by Lu and Gillett, *supra.* A [³³-P] UTP-labeled antisense riboprobe was generated from a PCR product and hybridized at 55°C overnight. The slides were dipped in Kodak NTB2 nuclear track emulsion and exposed for 4 weeks.

### ³³P-Riboprobe synthesis

6.0 µl (125 mCi) of ³³P-UTP (Amersham BF 1002, SA<2000 Ci/mmol) were speed vac dried. To each tube containing dried ³³P-UTP, the following ingredients were added:
2.0 µl 5x transcription buffer
1.0 µl DTT (100 mM)
2.0 µl NTP mix (2.5 mM : 10 µ; each of 10 mM GTP, CTP & ATP + 10 µl H₂O)
1.0 µl UTP (50 µM)
1.0 µl Rnasin
1.0 µl DNA template (1µg)
1.0 µl H₂O
1.0 µl RNA polymerase (for PCR products T3 = AS, T7 = S, usually)

The tubes were incubated at 37°C for one hour. 1.0 µl RQ1 DNase were added, followed by incubation at 37°C for 15 minutes. 90 µl TE (10 mM Tris pH 7.6/1mM EDTA pH 8.0) were added, and the mixture was pipetted onto DE81 paper. The remaining solution was loaded in a Microcon-50 ultrafiltration unit, and spun using program 10 (6 minutes). The filtration unit was inverted over a second tube and spun using program 2 (3 minutes). After the final recovery spin, 100 µl TE were added. 1 µl of the final product was pipetted on DE81 paper and counted in 6 ml of Biofluor II.

The probe was run on a TBE/urea gel. 1-3 µl of the probe or 5 µl of RNA Mrk III were added to 3 µl of loading buffer. After heating on a 95°C heat block for three minutes, the probe was immediately placed on ice. The wells of gel were flushed, the sample loaded, and run at 180-250 volts for 45 minutes. The gel was wrapped in saran wrap and exposed to XAR film with an intensifying screen in -70°C freezer one hour to overnight.

### ³³P-Hybridization

### A. Pretreatment of frozen sections

The slides were removed from the freezer, placed on aluminium trays and thawed at room temperature for 5 minutes. The trays were placed in 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ H₂O). After deproteination in 0.5 µg/ml proteinase K for 10 minutes at 37°C (12.5 µl of 10 mg/ml stock in 250 ml prewarmed RNase-free RNAse buffer), the sections were washed in 0.5 x SSC for 10 minutes at room temperature. The sections were dehydrated in 70%, 95%, 100% ethanol, 2 minutes each.

### B. Pretreatment of paraffin-embedded sections

The slides were deparaffinized, placed in SQ H₂O, and rinsed twice in 2 x SSC at room temperature, for 5 minutes each time. The sections were deproteinated in 20 µg/ml proteinase K (500 µl of 10 mg/ml in 250 ml RNase-free RNase buffer; 37°C, 15 minutes) - human embryo, or 8 x proteinase K (100 µl in 250 ml Rnase buffer, 37°C, 30 minutes) - formalin tissues. Subsequent rinsing in 0.5 x SSC and dehydration were performed as described above.

### C. Prehybridization

The slides were laid out in a plastic box lined with Box buffer (4 x SSC, 50% formamide) - saturated filter paper.

### D. Hybridization

1.0 x 10⁶ cpm probe and 1.0 µl tRNA (50 mg/ml stock) per slide were heated at 95°C for 3 minutes. The slides were cooled on ice, and 48 µl hybridization buffer were added per slide. After vortexing, 50 µl ³³P mix were added to 50 µl prehybridization on slide. The slides were incubated overnight at 55°C.

### E. Washes

Washing was done 2 x 10 minutes with 2xSSC, EDTA at room temperature (400 ml 20 x SSC + 16 ml 0.25M EDTA, V_{f}=4L), followed by RNaseA treatment at 37°C for 30 minutes (500 µl of 10 mg/ml in 250 ml Rnase buffer = 20 µg/ml), The slides were washed 2 x 10 minutes with 2 x SSC, EDTA at room temperature. The stringency wash conditions were as follows: 2 hours at 55°C, 0.1 x SSC, EDTA (20 ml 20 x SSC + 16 ml EDTA, V_{f}=4L).

### F. Oligonucleotides

*In situ* analysis was performed on a variety of DNA sequences disclosed herein. The oligonucleotides employed for these analyses were obtained so as to be complementary to the nucleic acids (or the complements thereof) as shown in the accompanying figures.

### G. Results

*In situ* analysis was performed on a variety of DNA sequences disclosed herein the results of which are disclosed in Example 46.

### EXAMPLE 56: Preparation of Antibodies that Bind PRO218, PRO228, PRO271, PRO273, PRO295, PRO302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PR038465 or PR0346

This example illustrates preparation of monoclonal antibodies which can specifically bind PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PRO9859, PR09864, PR09904, PR09907, PRO10013, PR090948, PR028694, PRO16089, PRO19563, PRO19675, PR020084, PR021434, PR050332, PR038465 or PR0346.

Techniques for producing the monoclonal antibodies are known in the art and are described, for instance, in Goding, supra. Immunogens that may be employed include purified PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides, fusion proteins containing PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides, and cells expressing recombinant PR0218, PR0228, PR0271, PR0273, PR0295, PR0302, PR0305, PR0326, PR0386, PR0655, PRO162, PRO788, PRO792, PR0940, PR0941, PRO1004, PR01012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PR010013, PR090948, PR028694, PR016089, PRO19563, PRO19675, PR020084, PR021434, PR050332, PR038465 or PR0346 polypeptides on the cell surface. Selection of the immunogen can be made by the skilled artisan without undue experimentation.

Mice, such as Balb/c, are immunized with the PR0218, PR0228, PR0271, PR0273, PR0295, PR0302, PR0305, PR0326, PR0386, PR0655, PRO162, PR0788, PR0792, PRO940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PR01012, anti-PRO1016, anti-PR0474, anti-PR05238, anti-PR01069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PR019563, anti-PR019675, anti-PR020084, anti-PR021434, anti-PR050332, anti-PR038465 or anti-PR0346 antibodies.

After a suitable antibody titer has been detected, the animals "positive" for antibodies can be injected with a final intravenous injection of PR0218, PR0228, PR0271, PRO273, PRO295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PR01113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PR0218, PR0228, PR0271, PR0273, PR0295, PR0302, PR0305, PR0326, PR0386, PR0655, PRO162, PR0788, PR0792, PRO940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346. Determination of "positive" hybridoma cells secreting the desired monoclonal antibodies against PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 is within the skill in the art.

The positive hybridoma cells can be injected intraperitoneally into syngeneic Balb/c mice to produce ascites containing the anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PR019563, anti-PRO19675, anti-PR020084, anti-PR021434, anti-PR050332, anti-PR038465 or anti-PR0346 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 57: Purification of PRO218, PRO228, PRO271, PRO273, PRO295, PRO302, PRO305, PRO326, PRO386 PRO655, PRO162, PRO788, PRO792, PRO940, PRO941, PRO1004, PRO1012, PRO1016, PR0474, PRO5238 PRO1069, PRO1111, PRO1113, PRO1130, PRO1195, PRO1271, PRO1865, PRO1879, PR03446, PRO3543, PRO4329, PRO4352, PRO5733, PRO9859, PRO9864, PRO9904, PRO9907, PRO10013, PRO90948, PRO28694 PRO16089 PRO19563, PRO19675, PRO20084, PRO21434, PRO50332, PRO38465 or PR0346 Polypeptides Using Specific Antibodies

Native or recombinant PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PRO3543, PRO4329, PR04352, PR05733, PR09859, PR09864, PR09904, PR09907, PRO10013, PR090948, PR028694, PRO16089, PRO19563, PRO19675, PRO20084, PR021434, PR050332, PR038465 or PR0346 polypeptides may be purified by a variety of standard techniques in the art of protein purification. For example, pro-PRO218, pro-PRO228, pro-PRO271, pro-PRO273, pro-PRO295, pro-PRO302, pro-PRO305, pro-PRO326, pro-PRO386, pro-PRO655, pro-PRO162, pro-PRO788, pro-PRO792, pro-PRO940, pro-PRO941, pro-PRO1004, pro-PRO1012, pro-PRO1016, pro-PRO474, pro-PRO5238, pro-PRO1069, pro-PRO1111, pro-PRO1113, pro-PRO1130, pro-PRO1195, pro-PRO1271, pro-PRO1865, pro-PRO1879, pro-PRO3446, pro-PRO3543, pro-PR04329, pro-PRO4352, pro-PRO5733, pro-PRO9859, pro-PRO9864, pro-PRO9904, pro-PRO9907, pro-PRO10013, pro-PRO90948, pro-PRO28694, pro-PRO16089, pro-PRO19563, pro-PRO19675, pro-PRO20084, pro-PRO21434, pro-PRO50332, pro-PRO38465 or pro-PRO346 polypeptide, mature PR0218,mature PR0228, mature PR0271, mature PR0273, mature PR0295, mature PR0302, mature PR0305, mature PR0326, mature PR03 86, mature PR0655, mature PRO162, mature PR0788, mature PR0792, mature PR0940, mature PR0941, mature PRO1004, mature PRO1012, mature PRO1016, mature PR0474, mature PR05238, mature PRO1069, mature PRO1111, mature PRO1113, mature PR01130, mature PRO1195, mature PR01271,mature PRO1865, mature PRO1879, mature PR03446, mature PR03543, mature PR04329, mature PR04352, mature PR05733, mature PR09859, mature PR09864, mature PR09904, mature PR09907, mature PRO10013, mature PR090948, mature PR028694, mature PRO16089, mature PRO19563, mature PRO19675, mature PR020084, mature PR021434, mature PR050332, mature PR038465 or mature PR0346 polypeptide, or pre-PRO218, pre-PR0228, pre-PR0271, pre-PR0273, pre-PR0295, pre-PR0302, pre-PR0305, pre-PR0326, pre-PR0386, pre-PR0655, pre-PRO162, pre-PR0788, pre-PR0792, pre-PR0940, pre-PRO941, pre-PRO1004, pre-PRO1012, pre-PRO1016, pre-PRO474,pre-PRO5238,pre-PRO1069,pre-PRO1111pre-PRO1113,pre-PRO1130,pre-PRO1195,pre-PRO1271, pre-PRO1865, pre-PRO1879, pre-PR03446, pre-PR03543, pre-PR04329, pre-PR04352, pre-PR05733, pre-PR09859, pre-PR09864, pre-PR09904, pre-PR09907, pre-PRO10013, pre-PR090948, pre-PR028694, pre-PR016089, pre-PRO19563, pre-PRO19675, pre-PR020084, pre-PR021434, pre-PR050332, pre-PR038465 or pre-PR0346 polypeptide is purified by immunoaffinity chromatography using antibodies specific for the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PR01865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide of interest. In general, an immunoaffinity column is constructed by covalently coupling the anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{™} (Pharmacia LKB Biotechnology). The antibody is coupled to the resin, the resin is blocked, and the derivative resin is washed according to the manufacturer's instructions.

Such an immunoaffinity column is utilized in the purification of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide by preparing a fraction from cells containing PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide in a soluble form. This preparation is derived by solubilization of the whole cell or of a subcellular fraction obtained via differential centrifugation by the addition of detergent or by other methods well known in the art. Alternatively, soluble polypeptide containing a signal sequence may be secreted in useful quantity into the medium in which the cells are grown.

A soluble PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PR01113, PRO1130, PR01195, PRO1271, PRO1865, PRO1879, PR03446, PR03543, PR04329, PR04352, PR05733, PR09859, PRO9864, PRO9904, PR09907, PRO10013, PR090948, PR028694, PRO16089, PRO19563, PRO19675, PR020084, PR021434, PR050332, PR038465 or PR0346 polypeptide-containing preparation is passed over the immunoaffinity column, and the column is washed under conditions that allow the preferential absorbance of PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide (*e.g.*, high ionic strength buffers in the presence of detergent). Then, the column is eluted under conditions that disrupt antibody/PRO218, antibody/PRO228, antibody/PRO271, antibody/PRO273, antibody/PRO295, antibody/PRO302, antibody/PRO305, antibody/PRO326, antibody/PRO386, antibody/PRO655, antibody/PRO162, antibody/PRO788, antibody/PRO792, antibody/PRO940, antibody/PRO941, antibody/PRO1004, antibody/PRO1012, antibody/PRO1016, antibody/PRO474, antibody/PRO5238, antibody/PRO1069, antibody/PRO1111, antibody/PRO1113, antibody/PRO1130, antibody/PRO1195, antibody/PRO1271, antibody/PRO1865, antibody/PRO1879, antibody/PRO3446, antibody/PRO3543, antibody/PRO4329, antibody/PRO4352, antibody/PRO5733, antibody/PRO9859, antibody/PRO9864, antibody/PRO9904, antibody/PRO9907, antibody/PRO10013, antibody/PRO90948, antibody/PRO28694, antibody/PRO16089, antibody/PRO19563, antibody/PRO19675, antibody/PRO20084, antibody/PRO21434, antibody/PRO50332,antibody/PRO38465 or antibody/PRO346 polypeptide binding (*e.g.*, a low pH buffer such as approximately pH 2-3, or a high concentration of a chaotrope such as urea or thiocyanate ion), and PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PR01865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide is collected.

### EXAMPLE 58: Drug Screening

This invention is particularly useful for screening compounds by using PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptides or binding fragment thereof in any of a variety of drug screening techniques. The PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or fragment. Drugs are screened against such transformed cells in competitive binding assays. Such cells, either in viable or fixed form, can be used for standard binding assays. One may measure, for example, the formation of complexes between PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or a fragment and the agent being tested. Alternatively, one can examine the diminution in complex formation between the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide and its target cell or target receptors caused by the agent being tested.

Thus, the present invention provides methods of screening for drugs or any other agents which can affect a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide-associated disease or disorder. These methods comprise contacting such an agent with an PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or fragment thereof and assaying (I) for the presence of a complex between the agent and the PR0218, PR0228, PR0271, PR0273, PR0295, PR0302, PRO305, PRO326, PRO386, PRO655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PRO3446, PRO3543, PRO4329, PRO4352, PRO5733, PRO9859, PRO9864, PRO9904, PRO9907, PRO10013, PRO90948, PRO28694, PRO16089, PRO19563, PRO19675, PRO20084, PRO21434, PRO50332, PR038465 or PR0346 polypeptide or fragment, or (ii) for the presence of a complex between the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or fragment and the cell, by methods well known in the art. In such competitive binding assays, the PR0218, PR0228, PR0271, PR0273, PRO295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or fragment is typically labeled. After suitable incubation, free PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PRO9859, PRO9864, PR09904, PR09907, PRO10013, PR090948, PR028694, PRO16089, PRO19563, PRO19675, PR020084, PR021434, PR050332, PR038465 or PR0346 polypeptide or fragment is separated from that present in bound form, and the amount of free or uncomplexed label is a measure ofthe ability ofthe particular agent to bind to PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or to interfere with the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide, the peptide test compounds are reacted with PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide and washed. Bound PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide is detected by methods well known in the art. Purified PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PRO4329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide specifically compete with a test compound for binding to PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or fragments thereof. In this manner, the antibodies can be used to detect the presence of any peptide which shares one or more antigenic determinants with PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.

### EXAMPLE 59: Rational Drug Design

The goal of rational drug design is to produce structural analogs of biologically active polypeptide of interest (*i.e.*, a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide) or of small molecules with which they interact, *e.g.,* agonists, antagonists, or inhibitors. Any of these examples can be used to fashion drugs which are more active or stable forms of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide or which enhance or interfere with the function of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide *in vivo* (*c.f.,* Hodgson, Bio/Technology, 9: 19-21 (1991)).

In one approach, the three-dimensional structure of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, or of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide-inhibitor complex, is determined by x-ray crystallography, by computer modeling or, most typically, by a combination of the two approaches. Both the shape and charges of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide must be ascertained to elucidate the structure and to determine active site(s) of the molecule. Less often, useful information regarding the structure of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide may be gained by modeling based on the structure of homologous proteins. In both cases, relevant structural information is used to design analogous PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists ofnative peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide may be made available to perform such analytical studies as X-ray crystallography. In addition, knowledge of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide amino acid sequence provided herein will provide guidance to those employing computer modeling techniques in place of or in addition to x-ray crystallography.
The following lettered paragraphs preceding the claims define further aspects and embodiments of the invention.
A. A method of identifying a phenotype associated with a disruption of a gene which encodes for a PRO218, PR0228, PR0271, PR0273, PR0295, PR0302, PR0305, PR0326, PR0386, PR0655, PRO162, PRO788, PRO792, PRO940, PRO941, PRO1004, PRO1012, PRO1016, PRO474, PRO5238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of a gene which is an ortholog of a human gene that encodes for a PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) measuring a physiological characteristic of the non-human transgenic animal; and
   (c) comparing the measured physiological characteristic with that of a gender matched wild-type animal, wherein the physiological characteristic of the non-human transgenic animal that differs from the physiological characteristic of the wild-type animal is identified as a phenotype resulting from the gene disruption in the non-human transgenic animal.
B. The method of A, wherein the non-human transgenic animal is heterozygous for the disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
C. The method of A, wherein the phenotype exhibited by the non-human transgenic animal as compared with gender matched wild-type littermates is at least one of the following: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
D. The method of C, wherein the neurological disorder is an increased anxiety-like response during open field activity testing.
E. The method of C, wherein the neurological disorder is a decreased anxiety-like response during open field activity testing.
F. The method of C, wherein the neurological disorder is an abnormal circadian rhythm during home-cage activity testing.
G. The method of C, wherein the neurological disorder is an enhanced motor coordination during inverted screen testing.
H. The method of C, wherein the neurological disorder is an impaired motor coordination during inverted screen testing.
I The method of C, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
J. The method of C. wherein the eye abnormality is a retinal abnormality.
K. The method of C, wherein the eye abnormality is consistent with vision problems or blindness.
L. The method of K, wherein the retinal abnormality is consistent with retinitis pigmentosa.
M. The method of K, wherein the retinal abnormality is characterized by retinal degeneration or retinal dysplasia.
N. The method of K, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
O. The method of C, wherein the eye abnormality is a cataract.
P. The method of O, wherein the cataract is consistent with systemic diseases such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
Q The method of C, wherein the developmental abnormality comprises embryonic lethality or reduced viability.
R. The method of C, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
S. The method of C, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonias, idiopathic pulmonary fibrosis and hypersensitivity pneumonitis; or transplantation associated diseases including graft rejection and graft -versus-host disease.
T. The method of C, wherein the bone metabolic abnormality or disorder is arthritis, osteoporosis or osteopetrosis.
U. The method of A, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits ofoptic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages of CD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages of CD4 cells and increased percentages of B cells in tissues; increase in percentages of B cells in Peyer's patches;; decreased germinal center, isotype-switched B cells in Peyer's patches (CD381ow;IgM negative); decreased CD23 intensity in spleen; increased mean percentages ofB220 Med/CD23- cells and B220+/CD 11b-Low/CD23- cells in peritoneal lavage; increased mean percentages of B cells in peripheral blood; decreased CD4 and CD8 T cells and increased B cells; increase in peritoneal B cells; reduction in CD11b-Hi cells in peritoneal cavity; decreased mean CD4 to CD8 ratio in spleen; decreased CD8 cells; decreased mean percentages of B220+/CD23+ cells and B220+/CD 11bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG1; increased mean serum IgG2a; increased mean serum IgG2b; decreased mean serum IgG1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.
V. An isolated cell derived from a non-human transgenic animal whose genome comprises a disruption of a gene which is an ortholog of a human gene that encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
W. The isolated cell of V which is a murine cell.
X. The isolated cell of W, wherein the murine cell is an embryonic stem cell.
Y. The isolated cell of V, wherein the non-human transgenic animal exhibits at least one of the following phenotypes compared with gender matched wild-type littermates: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
Z. A method of identifying an agent that modulates a phenotype associated with a disruption of a gene which encodes for a PR0218, PR0228, PR0271, PR0273, PR0295, PR0302, PR0305, PR0326, PR0386, PR0655, PRO162, PR0788, PR0792, PRO940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of a gene which is an ortholog of a human gene that encodes for the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PRO4352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) measuring a physiological characteristic of the non-human transgenic animal of (a);
   (c) comparing the measured physiological characteristic of (b) with that of a gender matched wild-type animal, wherein the physiological characteristic of the non-human transgenic animal that differs from the physiological characteristic of the wild-type animal is identified as a phenotype resulting from the gene disruption in the non-human transgenic animal;
   (d) administering a test agent to the non-human transgenic animal of (a); and
   (e) determining whether the test agent modulates the identified phenotype associated with gene disruption in the non-human transgenic animal.
AA. The method of Z, wherein the phenotype associated with the gene disruption comprises a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
AB. The method of AA, wherein the neurological disorder is an increased anxiety-like response during open field activity testing.
AC. The method of AA, wherein the neurological disorder is a decreased anxiety-like response during open field activity testing.
AD. The method of AA, wherein the neurological disorder is an abnormal circadian rhythm during home-cage activity testing.
AE. The method of AA, wherein the neurological disorder is an enhanced motor coordination during inverted screen testing.
AF. The method of AA, wherein the neurological disorder is an impaired motor coordination during inverted screen testing.
AG. The method of AA, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
AH. The method of AA, wherein the eye abnormality is a retinal abnormality.
AI. The method of AA, wherein the eye abnormality is consistent with vision problems or blindness.
AJ. The method of AH, wherein the retinal abnormality is consistent with retinitis pigmentosa.
AK. The method of AH, wherein the retinal abnormality is characterized by retinal degeneration or retinal dysplasia.
AL. The method of AH, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
AM. The method of AA, wherein the eye abnormality is a cataract.
AN. The method of AM, wherein the cataract is consistent with systemic diseases such as human Down's . syndrome, Hallerman-Streiffsyndrome, Lowe syndrome, galactosemia, Marfan syndrome, Trismoy 13-15, Alport syndrome, myotonic dystrophy, Fabry disease, hypoparathroidism or Conradi syndrome.
AO. The method of AA, wherein the developmental abnormality comprises embryonic lethality or reduced viability.
AP. The method of AA, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.
AQ. The method of AA, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation-associated diseases including graft rejection and graft -versus-host disease.
AR. The method of AA, wherein said bone metabolic abnormality or disorder is arthritis, osteoporosis or osteopetrosis.
AS. The method of Z, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits ofoptic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages of CD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages of CD4 cells and increased percentages ofB cells in tissues; increase in percentages of B cells in Peyer's patches;; decreased germinal center, isotype-switched B cells in Peyer's patches (CD3 8low;IgM negative); decreased CD23 intensity in spleen; increased mean percentages of B220 Med/CD23- cells and B220+/CD11b-Low/CD23- cells in peritoneal lavage; increased mean percentages of B cells in peripheral blood; decreased CD4 and CD8 T cells and increased B cells; increase in peritoneal B cells; reduction in CD11b-Hi cells in peritoneal cavity; decreased mean CD4 to CD8 ratio in spleen; decreased CD8 cells; decreased mean percentages of B220+/CD23+ cells and B220+/CD11 bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG1; increased mean serum IgG2a; increased mean serum IgG2b; decreased mean serum IgG 1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.
AT. An agent identified by the method of Z.
AU. The agent of AT, which is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PR01069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
AV. The agent of AU, wherein the agonist is an anti-PR0218, anti-PR0228, anti-PR0271, anti-PR0273, anti-PR0295, anti-PRO302, anti-PR0305, anti-PR0326, anti-PR0386, anti-PR0655, anti-PR0162, anti-PR0788, anti-PR0792, anti-PR0940, anti-PRO941, anti-PRO1004, anti-PR01012, anti-PRO1016, anti-PR0474, anti-PR05238, anti-PRO1069, anti-PRO1111, anti-PRO1113, anti-PR01130, anti-PR01195, anti-PRO1271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibody.
AW. The agent of AU, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibody.
A method of identifying an agent that modulates a physiological characteristic associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of a gene which is an ortholog of a human gene that encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) measuring a physiological characteristic exhibited by the non-human transgenic animal of (a);
   (c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
   (d) administering a test agent to the non-human transgenic animal of (a); and
   (e) determining whether the physiological characteristic associated with gene disruption is modulated.
AY. The method of AX, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle) ;abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits ofoptic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages of CD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages of CD4 cells and increased percentages of B cells in tissues; increase in percentages of B cells in Peyer's patches;; decreased germinal center, isotype-switched B cells in Peyer's patches (CD381ow;IgM negative); decreased CD23 intensity in spleen; increased mean percentages of B220 Med/CD23- cells and B220+/CD11b-Low/CD23- cells in peritoneal lavage; increased mean percentages ofB cells in peripheral blood; decreased CD4 and CD8 T cells and increased B cells; increase in peritoneal B cells; reduction in CD11b-Hi cells in peritoneal cavity; decreased mean CD4 to CD8 ratio in spleen; decreased CD8 cells; decreased mean percentages of B220+/CD23+cells and B220+/CD11 bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG1; increased mean serum IgG2a; increased mean serum IgG2b; decreased mean serum IgG 1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.
AZ. An agent identified by the method of AX.
BA. The agent of AZ which is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
BB. The agent of BA, wherein the agonist is an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PRO474, anti-PR05238, anti-PR01069, anti-PRO1111, anti-PRO1113, anti-PRO1130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibody.
BC. The agent of BA, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PRO474, anti-PR05238, anti-PRO1069, anti-PRO1111, anti-PRO1113, anti-PRO1130, anti-PRO1195, anti-PRO1271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PR019563, anti-PRO19675, anti-PR020084, anti-PR021434, anti-PR050332, anti-PR038465 or anti-PR0346 antibody.
BD. A method of identifying an agent which modulates a behavior associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PR01012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PRO346 polypeptide, the method comprising:
   (a) providing a non-human transgenic animal whose genome comprises a disruption of a gene which is an ortholog of a human gene that encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) observing the behavior exhibited by the non-human transgenic animal of (a);
   (c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
   (d) administering a test agent to the non-human transgenic animal of (a); and
   (e) determining whether the agent modulates the behavior associated with gene disruption.
BE. The method of BD, wherein the behavior is an increased anxiety-like response during open field activity testing.
BF. The method of BD, wherein the behavior is a decreased anxiety-like response during open field activity testing.
BG. The method of BD, wherein the behavior is an abnormal circadian rhythm during home-cage activity testing.
BH. The method of BD, wherein the behavior is an enhanced motor coordination during inverted screen testing.
BI. The method of BD, wherein the behavior is an impaired motor coordination during inverted screen testing.
BJ. The method of BD, wherein the behavior is depression, generalized anxiety disorders, attention deficit disorder, sleep disorder, hyperactivity disorder, obsessive compulsive disorder, schizophrenia, cognitive disorders, hyperalgesia or sensory disorders.
BK. An agent identified by the method of BD.
BL. The agent of BK which is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
BM. The agent of BL, wherein the agonist is an anti-PR0218, anti-PR0228, anti-PR0271, anti-PR0273,
   anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PR01004, anti-PRO1012, anti-PRO1016, anti-PRO474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PRO19675, anti-PR020084, anti-PR021434, anti-PR050332, anti-PR038465 or anti-PR0346 antibody.
BN. The agent of BL wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PR01879, anti-PR03446, anti-PR03543, anti-PR04329, anti-PR04352, anti-PR05733, anti-PR09859, anti-PR09864, anti-PRO9904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
BO. A method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in a gene which encodes for a PR0218, PR0228, PR0271, PR0273, PR0295, PR0302, PR0305, PR0326, PR0386, PR0655, PRO162, PR0788, PR0792, PR0940, PRO941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PRO4352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of a gene which is an ortholog of a human gene that encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PR016089, PR019563, PR019675, PR020084, PR021434, PR050332, PR038465 or PR0346 polypeptide;
   (b) administering a test agent to said non-human transgenic animal; and
   (c) determining whether said test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in the non-human transgenic animal.
BP. The method of BO, wherein the neurological disorder is an increased anxiety-like response during open field activity testing.
BQ. The method of BO, wherein the neurological disorder is a decreased anxiety-like response during open field activity testing.
BR. The method of BO, wherein the neurological disorder is an abnormal circadian rhythm during home-cage activity testing.
BS. The method of BO, wherein the neurological disorder is an enhanced motor coordination during inverted screen testing.
BT. The method of BO, wherein the neurological disorder is an impaired motor coordination during inverted screen testing.
BU. The method of BO, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
BV. The method of BO, wherein the eye abnormality is a retinal abnormality.
BW. The method of BO, wherein the eye abnormality is consistent with vision problems or blindness.
BX. The method of BV, wherein the retinal abnormality is consistent with retinitis pigmentosa.
BY, The method of BV, wherein the retinal abnormality is characterized by retinal degeneration or retinal dysplasia.
BZ. The method of BV, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
CA. The method of BO, wherein the eye abnormality is a cataract.
CB. The method of CA, wherein the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
CC. The method of BO, wherein the developmental abnormality comprises embryonic lethality or reduced viability.
CD. The method of BO, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
CE. The method of BO, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft-versus-host disease.
CF. The method of BO, wherein said bone metabolic abnormality or disorder is arthritis, osteoporosis or osteopetrosis.
CG. The method of BO, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; abnormal circadian rhythm during home-cage activity testing (low activity during the light phase; altered sleep/wake cycle);abnormal circadian rhythm during home-cage activity testing including decreased ambulatory counts; hypoactivity with no circadian rhythm; abnormal circadian rhythm during home-cage activity testing including increased ambulatory counts; decreased rearing; increased sensitivity to stress induced hyperthermia (increased anxiety); impaired motor coordination during inverted screen testing; head tilt and retropulsion; increased prepulse inhibition response indicating enhanced sensorimotor gating/attention; decreased startle response during prepulse inhibition testing; no startle response indicating deafness or impaired hearing; decreased prepulse inhibition with impaired sensorimotor gating/attention; increased latency to respond in hot plate testing; decreased latency to respond in hot plate testing; opthamological abnormalities; impaired vision; white deposits of optic disc region; ocular infection and neutrophilia; bilateral optic disc lesion; decreased tear production; decreased heart rate; increased mean systolic blood pressure; decreased mean systolic blood pressure; increased mean fasting serum glucose levels; decreased mean serum glucose levels; increased mean serum cholesterol levels; decreased mean serum cholesterol levels; increased mean serum triglyceride levels; decreased mean serum triglyceride levels; impaired glucose tolerance; increased mean serum albumin, alanine amino transferase and phosphorus levels; increased mean serum alkaline phosphatase levels; urinary nitrites present; increased total white blood cell (WBC) count; decreased total white blood cell (WBC) count and absolute neutrophil count; increased mean absolute neutrophil count; increased mean absolute lymphocyte count; increased mean platelet count; increased mean red cell distribution width; decreased mean platelet count; reduced percentage of CD4 spleen thymocytes; decreased percentages of CD4 cells in the periphery resulting in increased percentages of B cells in lymph organs; CD4 cells exhibit a more activated/memory phenotype (CD62Llow, CD44hi); developmental defect in CD4+ cells; decreased percentages of CD4 cells and increased percentages of B cells in blood; decreased percentages ofCD4 cells and increased percentages ofB cells in tissues; increase in percentages of B cells in Peyer's patches;; decreased germinal center, isotype-switched B cells in Peyer's patches (CD381ow;IgM negative); decreased CD23 intensity in spleen; increased mean percentages of B220 Med/CD23- cells and B220+/CD11b-Low/CD23- cells in peritoneal lavage; increased mean percentages of B cells in peripheral blood; decreased CD4 and CD8 T cells and increased B cells; increase in peritoneal B cells; reduction in CD11b-Hi cells in peritoneal cavity; decreased mean CD4 to CD8 ratio in spleen; decreased CD8 cells; decreased mean percentages of B220+/CD23+ cells and B220+/CD 11bLow/CD23- cells in peritoneal lavage; increased mean serum IgG 1 response to ovalbumin challenge; increased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increased mean serum IgA; increase mean serum IgG1; increased mean serum IgG2a; increased mean serum IgG2b; decreased mean serum IgG 1 response to ovalbumin challenge; decreased mean serum IgG2a response to ovalbumin challenge; failure in ovalbumin response; decreased mean serum IgA level; decreased mean serum IgG2a level; decreased skin fibroblast proliferation rate; increased mean percent of total body fat and total fat mass; increased mean body weight; increased mean body length; increased total tissue mass (TTM); increased bone mineral density (BMD); increase in bone mineral content (BMC); increased mean femoral midshaft cortical thickness; decreased mean percent of total body fat and total fat mass; decreased mean body weight; decreased mean body length; decreased mean body weight and length in heterozygotes; decreased total tissue mass (TTM); decreased lean body mass (LBM); decreased femoral bone mineral density (BMD); decreased vertebral bone mineral density (BMD); decreased bone mineral density (BMD) in total body; decreased bone mineral content (BMC); decreased bone mineral density index; decreased volumetric bone mineral density (vBMD); decreased mean femoral midshaft cortical thickness; decreased mean femoral midshaft cross-sectional area; decreased mean vertebral trabecular bone volume, number and connectivity density; osteopetrosis; osteoporosis; moderate kidney hydronephrosis; hydrocephalus; enlarged liver; induced in activated T cells; induced in activated NK cells and dendritic cells; myeloid B cell expression; hyperplasia of sebaceous glands and multifocal hyperplasia of the epidermis (acanthosis and hyperkeratosis); moderate dermatitis; increased extramedullary hematopoeisis in liver and spleen; myeloid hyperplasia of the bone marrow; encephalitis due to Group B streptococcus; meningitis due to *E. Coli* infection; lymphocytic infiltrates in salivary glands, pancreas and lungs; poor breeders requiring foster mothers; decreased litter size; homozygous mice were small and dehydrated; vacuolar degeneration of testes resulting in decreased sperm production and infertility; defective spermatogenesis in the testes; hypospermia and defective spermatozoa in the epididymus; male infertility; decreased testes weight; growth retardation; small mice and failure to thrive; reduced viability; reduced viability with situs invertus; and homozygous embryonic lethality.
CH. An agent identified by the method of BO.
CI. The agent of CH which is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
CJ. The agent of CI, wherein the agonist is an anti-PR0218, anti-PR0228, anti-PR0271, anti-PR0273,
   anti-PR0295, anti-PR0302, anti-PR0305, anti-PR0326, anti-PR0386, anti-PR0655, anti-PRO162, anti-PRO788, anti-PR0792, anti-PR0940, anti-PR0941, anti-PRO1004, anti-PRO1012, anti-PR01016, anti-PR0474, anti-PR05238, anti-PRO1069, anti-PRO1111, anti-PRO1113, anti-PRO1130, anti-PRO1195, anti-PRO1271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PR019563, anti-PRO19675, anti-PR020084, anti-PR021434, anti-PR050332, anti-PR038465 or anti-PR0346 antibody.
CK. The agent of CI, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PR01865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
CL. A therapeutic agent identified by the method of BO.
CM. A method of identifying an agent that modulates the expression of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) contacting a test agent with a host cell expressing a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide; and
   (b) determining whether the test agent modulates the expression of the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide by the host cell.
CN. An agent identified by the method of CM.
CO. The agent of CN which is an agonist or antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
CP. The agent of CO, wherein the agonist is an anti-PR0218, anti-PR0228, anti-PR0271, anti-PR0273,
   anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PR01069, anti-PRO1111, anti-PRO1113, anti-PRO1130, anti-PRO1195, anti-PR01271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PR016089, anti-PRO19563, anti-PR019675, anti-PR020084, anti-PR021434, anti-PR050332, anti-PR038465 or anti-PR0346 antibody.
CQ. The agent of cO, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PR01004, anti-PRO1012, anti-PR01016, anti-PRO474, anti-PR05238, anti-PRO1069, anti-PRO1111, anti-PRO1113, anti-PRO1130, anti-PRO1195, anti-PRO1271, anti-PRO1865, anti-PRO1879, anti-PR03446, anti-PR03543, anti-PR04329, anti-PR04352, anti-PR05733, anti-PR09859, anti-PR09864, anti-PR09904, anti-PRO9907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
CR. A method of evaluating a therapeutic agent capable of affecting a condition associated with a disruption of a gene which encodes for a PR0218, PR0228, PR0271, PR0273, PR0295, PR0302, PR0305, PR0326, PR0386, PR0655, PRO162, PR0788, PR0792, PR0940, PRO941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of a gene which is an ortholog of a human gene that encodes for the PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide;
   (b) measuring a physiological characteristic of the non-human transgenic animal of (a);
   (c) comparing the measured physiological characteristic of (b) with that of a gender matched wild-type animal, wherein the physiological characteristic of the non-human transgenic animal that differs from the physiological characteristic of the wild-type animal is identified as a condition resulting from the gene disruption in the non-human transgenic animal;
   (d) administering a test agent to the non-human transgenic animal of (a); and
   (e) evaluating the effects of the test agent on the identified condition associated with gene disruption in the non-human transgenic animal.
CS. The method of CR, wherein the condition is a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
CT. A therapeutic agent identified by the method of CR.
CU. The therapeutic agent of CT which is an agonist or antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PR01271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide.
CV. The therapeutic agent of CU, wherein the agonist is an anti-PR0218, anti-PR0228, anti-PRO271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
CW. The therapeutic agent of CH, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PRO1271, anti-PRO1865, anti-PR01879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PRO20084, anti-PR021434, anti-PR050332, anti-PR038465 or anti-PR0346 antibody.
CX. A pharmaceutical composition comprising the therapeutic agent of CT.
CY. A method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject in need of such treatment whom may already have the disorder, or may be prone to have the disorder or may be in whom the disorder is to be prevented, a therapeutically effective amount of the therapeutic agent of CL, or agonists or antagonists thereof, thereby effectively treating or preventing or ameliorating said disorder.
CZ. The method of CY, wherein the neurological disorder is an increased anxiety-like response during open field activity testing.
DA. The method of CY, wherein the neurological disorder is a decreased anxiety-like response during open field activity testing.
DB. The method of CY, wherein the neurological disorder is an abnormal circadian rhythm during home-cage activity testing.
DC. The method of CY, wherein the neurological disorder is an enhanced motor coordination during inverted screen testing.
DD. The method of CY, wherein the neurological disorder is an impaired motor coordination during inverted screen testing.
DE. The method of CY, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
DF. The method of CY, wherein the eye abnormality is a retinal abnormality.
DG. The method of CY, wherein the eye abnormality is consistent with vision problems or blindness.
DH. The method of DF, wherein the retinal abnormality is consistent with retinitis pigmentosa.
DI. The method of DF, wherein the retinal abnormality is characterized by retinal degeneration or retinal dysplasia.
DJ. The method of DF, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
DK. The method of CY, wherein the eye abnormality is a cataract.
DL. The method of DK, wherein the cataract is a systemic disease such as human Down's syndrome, Hallerman-Streiffsyndrome, Lowe syndrome, galactosemia, Marfan syndrome, Trismoy 13-15, Alport syndrome, myotonic dystrophy, Fabry disease, hypoparathroidism or Conradi syndrome.
DM. The method of CY, wherein the developmental abnormality comprises embryonic lethality or reduced viability.
DN. The method of CY, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
DO. The method of CY, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
DP. The method of CY, wherein said bone metabolic abnormality or disorder is arthritis, osteoporosis or osteopetrosis.
DQ. A method of modulating a phenotype associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PR01130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject whom may already have the phenotype, or may be prone to have the phenotype or may be in whom the phenotype is to be prevented, an effective amount of the agent of AT, or agonists or antagonists thereof, thereby effectively modulating the phenotype.
DR. A method of modulating a physiological characteristic associated with a disruption of a gene which encodes for a PR0218, PR0228, PR0271, PR0273, PR0295, PR0302, PR0305, PR0326, PR0386, PR0655, PRO162, PRO788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PRO3543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide, the method comprising administering to a subject whom may already exhibit the physiological characteristic, or may be prone to exhibit the physiological characteristic or may be in whom the physiological characteristic is to be prevented, an effective amount of the agent of Claim 52, or agonists or antagonists thereof, thereby effectively modulating the physiological characteristic.
DS. A method of modulating a behavior associated with a disruption of a gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject whom may already exhibit the behavior, or may be prone to exhibit the behavior or may be in whom the exhibited behavior is to be prevented, an effective amount of the agent of Claim BK or agonists or antagonists thereof, thereby effectively modulating the behavior.
DT. A method of modulating the expression of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PR01271, PR01865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a host cell expressing said PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PRO4352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, an effective amount of the agent of CN, or agonists or antagonists thereof, thereby effectively modulating the expression of said polypeptide.
DU. A method of modulating a condition associated with a disruption of a gene which encodes for a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject whom may have the condition, or may be prone to have the condition or may be in whom the condition is to be prevented, a therapeutically effective amount of the therapeutic agent of Claim CT, or agonists or antagonists thereof, thereby effectively modulating the condition.
DV. A method of identifying an agent that mimics a condition or phenotype associated with a disruption in a gene which encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PRO9864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of a gene which is an ortholog of a human gene that encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) measuring a physiological characteristic of the non-human transgenic animal of (a);
   (c) comparing the measured physiological characteristic of (b) with that of a gender matched wild-type animal, wherein the physiological characteristic of the non-human transgenic animal that differs from the physiological characteristic of the gender matched wild-type animal is identified as a condition or phenotype resulting from the gene disruption in the non-human transgenic animal;
   (d) administering a test agent to said gender matched wild-type animal; and
   (e) determining whether said test agent mimics the condition or phenotype initially observed in the non-human transgenic animal.
DW. The method of DV, wherein the condition or phenotype associated with the disruption of the gene which is an ortholog of a human gene that encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide is enhanced glucose tolerance.
DX The method of DV, wherein the condition or phenotype associated with the disruption of the gene which is an ortholog of a human gene that encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide is increased insulin sensitivity.
DY. An agent identified by the method of DV.
DZ. The agent of DY which is an antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
EA. The agent of DZ, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PR0162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PR01865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
EB. A method of mimicking a condition or phenotype associated with a disruption of a gene which encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PRO474, PR05238, PRO1069, PRO1111, PR01113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject in whom the condition or phenotype is to be mimicked, an effective amount of the agent of Claim 129 or an antagonist of a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PR01069, PRO1111, PRO1113, PRO1130, PRO1195, PR01271, PR01865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, thereby effectively mimicking the condition or phenotype.
EC. The method of EB, wherein the condition or phenotype associated with the disruption of the gene which is an ortholog of a human gene that encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PRO90948, PR028694, PRO16089, PRO19563, PRO19675, PR020084, PR021434, PR050332, PR038465 or PR0346 polypeptide is enhanced glucose tolerance.
ED. The method of Claim EB, wherein the condition or phenotype associated with the disruption of the gene which is an ortholog of a human gene that encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide is increased insulin sensitivity.
EE. A method of evaluating a therapeutic agent capable of mimicking a condition or phenotype associated with a disruption of a gene which encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising:
   (a) providing a non-human transgenic animal whose genome comprises a disruption of a gene which is an ortholog of a human gene that encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide;
   (b) measuring a physiological characteristic of the non-human transgenic animal of (a);
   (c) comparing the measured physiological characteristic of (b) with that of a gender matched wild-type animal, wherein the physiological characteristic of the non-human transgenic animal that differs from the physiological characteristic of the gender matched wild-type animal is identified as a condition or phenotype resulting from the gene disruption in the non-human transgenic animal;
   (d) administering a test agent to said gender matched wild-type animal of (c); and
   (e) evaluating the ability of the test agent to mimic the condition or phenotype associated with gene disruption in the non-human transgenic animal.
EF. A therapeutic agent identified by the method of EE.
EG. The therapeutic agent of EF, which is an antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PR01069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide.
EH. The therapeutic agent of EG, wherein the antagonist is an anti-PR0218, anti-PR0228, anti-PR0271, anti-PR0273, anti-PR0295, anti-PR0302, anti-PR0305, anti-PR0326, anti-PR0386, anti-PR0655, anti-PRO162, anti-PR0788, anti-PR0792, anti-PR0940, anti-PR0941, anti-PRO1004, anti-PRO1012, anti-PRO1016, anti-PR0474, anti-PR05238, anti-PRO1069, anti-PRO1111, anti-PRO1113, anti-PRO1130, anti-PRO1195, anti-PR01271, anti-PRO1865, anti-PRO1879, anti-PR03446, anti-PR03543, anti-PR04329, anti-PR04352, anti-PR05733, anti-PR09859, anti-PR09864, anti-PR09904, anti-PR09907, anti-PRO10013, anti-PR090948, anti-PR028694, anti-PRO16089, anti-PRO19563, anti-PRO19675, anti-PR020084, anti-PR021434, anti-PR050332, anti-PR038465 or anti-PR0346 antibody.
EI. A pharmaceutical composition comprising the therapeutic agent of EF.
EJ. A method of mimicking a condition or phenotype associated with a disruption of a gene which encodes a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject in whom the condition or phenotype disorder is to be mimicked, a therapeutically effective amount of the therapeutic agent of Claim 136, or an antagonist of a PRO218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PRO1195, PRO1271, PRO1865, PRO1879, PR03446, PR03543, PRO4329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, thereby effectively mimicking the condition or phenotype.
EK. A method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PR0218, PR0228, PR0271, PR0273, PR0295, PR0302, PR0305, PR0326, PR0386, PR0655, PRO162, PR0788, PR0792, PR0940, PR0941, PRO1004, PRO1012, PRO1016, PR0474, PR05238, PRO1069, PRO1111, PRO1113, PRO1130, PR01195, PRO1271, PRO1865, PRO1879, PR03446, PR03543, PR04329, PR04352, PR05733, PR09859, PR09864, PR09904, PR09907, PRO10013, PR090948, PR028694, PRO16089, PRO19563, PRO19675, PR020084, PR021434, PR050332, PR038465 or PR0346 polypeptide, the method comprising administering to a subject whom may have the neurological disorder; cardiovascular, endothelial or angiogenic disorder; eye abnormality; immunological disorder; oncological disorder; bone metabolic abnormality or disorder; lipid metabolic disorder; or developmental abnormality, a therapeutic agent of CL, or agonists or antagonists thereof, thereby ameliorating or modulating the disorder.

## Claims

1. A method of identifying a phenotype associated with a disruption of a gene which encodes for a PR0940 polypeptide, the method comprising:
(a) providing a non-human transgenic animal whose genome comprises a disruption of a gene which is an ortholog of a human gene that encodes for a PR0940 polypeptide;
(b) measuring a physiological characteristic of the non-human transgenic animal; and
(c) comparing the measured physiological characteristic with that of a gender matched wild-type animal, wherein the physiological characteristic of the non-human transgenic animal that differs from the physiological characteristic of the wild-type animal is identified as a phenotype resulting from the gene disruption in the non-human transgenic animal.

2. The method of Claim 1, wherein the non-human transgenic animal is heterozygous for the disruption of a gene which encodes for a PR0940 polypeptide.

3. A method of identifying an agent that modulates a phenotype associated with a disruption of a gene which encodes for a PR0940 polypeptide, the method comprising:
(a) providing a non-human transgenic animal whose genome comprises a disruption of a gene which is an ortholog of a human gene that encodes for the PR0940 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a phenotype resulting from the gene disruption in the non-human transgenic animal;
(d) administering a test agent to the non-human transgenic animal of (a); and
(e) determining whether the test agent modulates the identified phenotype associated with gene disruption in the non-human transgenic animal.

4. A method of identifying an agent that modulates a physiological characteristic associated with a disruption of a gene which encodes for a PR0940 polypeptide, the method comprising:
(a) providing a non-human transgenic animal whose genome comprises a disruption of a gene which is an ortholog of a human gene that encodes for a PR0940 polypeptide;
(b) measuring a physiological characteristic exhibited by the non-human transgenic animal of (a);
(c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
(d) administering a test agent to the non-human transgenic animal of (a); and
(e) determining whether the physiological characteristic associated with gene disruption is modulated.

5. The method of Claim 1 or Claim 3, wherein the phenotype exhibited by the non-human transgenic animal as compared with gender matched wild-type littermates is at least one of the following: a neurological disorder; or a bone metabolic abnormality or disorder.

6. A method of identifying an agent that ameliorates or modulates a neurological disorder; or a bone metabolic abnormality or disorder associated with a disruption in a gene which encodes for a PR0940 polypeptide, the method comprising:
(a) providing a non-human transgenic animal whose genome comprises a disruption of a gene which is an ortholog of a human gene that encodes for a PR0940 polypeptide;
(b) administering a test agent to said non-human transgenic animal; and
(c) determining whether said test agent ameliorates or modulates the neurological disorder; or bone metabolic abnormality or disorder in the non-human transgenic animal.

7. The method of Claim 5 or Claim 6, wherein the neurological disorder is an abnormal circadian rhythm during home-cage activity testing, or wherein the bone metabolic abnormality or disorder is osteopetrosis.

8. The method of any one of Claims 1, 3, 4 and 6, wherein the non-human transgenic animal exhibits at least one of the following physiological characteristics compared with gender matched wild-type littermates: abnormal circadian rhythm during home-cage activity testing (low activity during the light phase; altered sleep/wake cycle); increased sensitivity to stress induced hyperthermia (increased anxiety); increased bone mineral density (BMD); and increase in bone mineral content (BMC).

9. A method of identifying an agent which modulates a behavior associated with a disruption of a gene which encodes for a PR0940 polypeptide, the method comprising:
(a) providing a non-human transgenic animal whose genome comprises a disruption of a gene which is an ortholog of a human gene that encodes for a PR0940 polypeptide;
(b) observing the behavior exhibited by the non-human transgenic animal of (a);
(c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
(d) administering a test agent to the non-human transgenic animal of (a); and
(e) determining whether the agent modulates the behavior associated with gene disruption.

10. The method of Claim 9, wherein the behavior is an abnormal circadian rhythm during home-cage activity testing.

11. A method of identifying an agent that modulates the expression of a PR0940 polypeptide, the method comprising:
(a) contacting a test agent with a host cell expressing a PR0940 polypeptide; and
(b) determining whether the test agent modulates the expression of the PR0940 polypeptide by the host cell.

12. A method of evaluating a therapeutic agent capable of affecting a condition associated with a disruption of a gene which encodes for a PR0940 polypeptide, the method comprising:
(a) providing a non-human transgenic animal whose genome comprises a disruption of a gene which is an ortholog of a human gene that encodes for the PR0940 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a condition resulting from the gene disruption in the non-human transgenic animal;
(d) administering a test agent to the non-human transgenic animal of (a); and
(e) evaluating the effects of the test agent on the identified condition associated with gene disruption in the non-human transgenic animal.

13. The method of Claim 12, wherein the condition is a neurological disorder; or a bone metabolic abnormality or disorder.

14. A method of identifying an agent that mimics a condition or phenotype associated with a disruption in a gene which encodes a PR0940 polypeptide, the method comprising:
(a) providing a non-human transgenic animal whose genome comprises a disruption of a gene which is an ortholog of a human gene that encodes a PR0940 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the gender matched wild-type animal is identified as a condition or phenotype resulting from the gene disruption in the non-human transgenic animal;
(d) administering a test agent to said gender matched wild-type animal; and
(e) determining whether said test agent mimics the condition or phenotype initially observed in the non-human transgenic animal.

15. A method of evaluating a therapeutic agent capable of mimicking a condition or phenotype associated with a disruption of a gene which encodes a PR0940 polypeptide, the method comprising:
(a) providing a non-human transgenic animal whose genome comprises a disruption of a gene which is an ortholog of a human gene that encodes a PR0940 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the gender matched wild-type animal is identified as a condition or phenotype resulting from the gene disruption in the non-human transgenic animal;
(d) administering a test agent to said gender matched wild-type animal of (c); and
(e) evaluating the ability of the test agent to mimic the condition or phenotype associated with gene disruption in the non-human transgenic animal.

16. An isolated cell derived from a non-human transgenic animal whose genome comprises a disruption of a gene which is an ortholog of a human gene that encodes for a PR0940 polypeptide, preferably a murine cell, more preferably a murine cell embryonic stem cell.

17. The isolated cell of Claim 16, wherein the non-human transgenic animal exhibits at least one of the following phenotypes compared with gender matched wild-type littermates: a neurological disorder; or a bone metabolic abnormality or disorder.

18. An agent identified by the method of any one of Claims 3, 4, 6, 9, 11 and 14 or a therapeutic agent identified by the method of Claim 12 or Claim 15.

19. The agent or therapeutic agent of Claim 18 which is an agonist or antagonist of a PR0940 polypeptide.

20. The agent or therapeutic agent of Claim 19, wherein the agonist or antagonist is an anti-PR0940 antibody.

21. A therapeutic agent identified by the method of Claim 6.

22. A pharmaceutical composition comprising the therapeutic agent of Claim 18.

23. A method of
(i) treating or preventing or ameliorating a neurological disorder; or bone metabolic abnormality or disorder, the method comprising administering to a subject in need of such treatment whom may already have the disorder, or may be prone to have the disorder or may be in whom the disorder is to be prevented, a therapeutically effective amount of the therapeutic agent of Claim 21, or agonists or antagonists thereof, thereby effectively treating or preventing or ameliorating said disorder;
(ii) modulating a phenotype associated with a disruption of a gene which encodes for a PR0940 polypeptide, the method comprising administering to a subject whom may already have the phenotype, or may be prone to have the phenotype or may be in whom the phenotype is to be prevented, an effective amount of the agent of Claim 18 as dependent from Claim 3, or agonists or antagonists thereof, thereby effectively modulating the phenotype;
(iii) modulating a physiological characteristic associated with a disruption of a gene which encodes for a PR0940 polypeptide, the method comprising administering to a subject whom may already exhibit the physiological characteristic, or may be prone to exhibit the physiological characteristic or may be in whom the physiological characteristic is to be prevented, an effective amount of the agent of Claim 18 as dependent from Claim 4, or agonists or antagonists thereof, thereby effectively modulating the physiological characteristic;
(iv) modulating a behavior associated with a disruption of a gene which encodes for a PR0940 polypeptide, the method comprising administering to a subject whom may already exhibit the behavior, or may be prone to exhibit the behavior or may be in whom the exhibited behavior is to be prevented, an effective amount of the agent of Claim 18 as dependent from Claim 9, or agonists or antagonists thereof, thereby effectively modulating the behavior;
(v) modulating the expression of a PR0940 polypeptide, the method comprising administering to a host cell expressing said PR0940 polypeptide, an effective amount of the agent of Claim 18 as dependent from Claim 11, or agonists or antagonists thereof, thereby effectively modulating the expression of said polypeptide;
(vi) modulating a condition associated with a disruption of a gene which encodes for a PR0940 polypeptide, the method comprising administering to a subject whom may have the condition, or may be prone to have the condition or may be in whom the condition is to be prevented, a therapeutically effective amount of the therapeutic agent of Claim 18 as dependent from Claim 12, or agonists or antagonists thereof, thereby effectively modulating the condition;
(vii) mimicking a condition or phenotype associated with a disruption of a gene which encodes a PR0940 polypeptide, the method comprising administering to a subject in whom the condition or phenotype is to be mimicked, an effective amount of the agent of Claim 18 as dependent from Claim 14 or an antagonist of a PR0940 polypeptide, thereby effectively mimicking the condition or phenotype;
(viii) mimicking a condition or phenotype associated with a disruption of a gene which encodes a PR0940 polypeptide, the method comprising administering to a subject in whom the condition or phenotype disorder is to be mimicked, a therapeutically effective amount of the therapeutic agent of Claim 18 as dependent from Claim 15, or an antagonist of a PR0940 polypeptide, thereby effectively mimicking the condition or phenotype.

24. A method of identifying an agent that ameliorates or modulates a neurological disorder; or a bone metabolic abnormality or disorder associated with a disruption in the gene which encodes for a PR0940 polypeptide, the method comprising administering to a subject whom may have the neurological disorder; or bone metabolic abnormality or disorder, a therapeutic agent of Claim 21, or agonists or antagonists thereof, thereby ameliorating or modulating the disorder.
